(19) Europäisches Patentamt
European Patent Office
Office européen des brevets

(11) **EP 4 534 100 A1**

(12) **EUROPEAN PATENT APPLICATION**
published in accordance with Art. 153(4) EPC

(43) Date of publication:
**09.04.2025 Bulletin 2025/15**

(21) Application number: **23811904.4**

(22) Date of filing: **26.05.2023**

(51) International Patent Classification (IPC):
***A61K 45/00*** *(2006.01)* ***A61K 9/127*** *(2025.01)*
***A61K 31/497*** *(2006.01)* ***A61K 31/506*** *(2006.01)*
***A61K 31/5377*** *(2006.01)* ***A61K 39/395*** *(2006.01)*
***A61K 47/02*** *(2006.01)* ***A61K 47/24*** *(2006.01)*
***A61K 47/26*** *(2006.01)* ***A61K 47/28*** *(2006.01)*
***A61K 47/32*** *(2006.01)* ***A61K 47/34*** *(2017.01)*
***A61P 35/00*** *(2006.01)* ***A61P 43/00*** *(2006.01)*

(52) Cooperative Patent Classification (CPC):
**A61K 9/127; A61K 31/497; A61K 31/506; A61K 31/5377; A61K 39/395; A61K 45/00; A61K 47/02; A61K 47/24; A61K 47/26; A61K 47/28; A61K 47/32; A61K 47/34; A61P 35/00; A61P 43/00**

(86) International application number:
**PCT/JP2023/019755**

(87) International publication number:
**WO 2023/229032 (30.11.2023 Gazette 2023/48)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **27.05.2022 JP 2022087166**

(71) Applicant: **Sumitomo Pharma Co., Ltd.**
**Osaka-shi, Osaka 541-0045 (JP)**

(72) Inventors:
- **KUMAGAI, Yuka**
  **Osaka-shi, Osaka 554-0022 (JP)**
- **UMEHARA, Hiroki**
  **Osaka-shi, Osaka 554-0022 (JP)**
- **OISHI, Jun**
  **Osaka-shi, Osaka 541-0045 (JP)**
- **KAWAKAMI, Ryuhei**
  **Osaka-shi, Osaka 554-0022 (JP)**
- **INAGAKI, Ryosaku**
  **Osaka-shi, Osaka 554-0022 (JP)**
- **OE, Seina**
  **Osaka-shi, Osaka 554-0022 (JP)**
- **SHIMIZU, Kana**
  **Osaka-shi, Osaka 554-0022 (JP)**
- **OTA, Yosuke**
  **Osaka-shi, Osaka 554-0022 (JP)**

(74) Representative: **Vossius & Partner**
**Patentanwälte Rechtsanwälte mbB**
**Siebertstraße 3**
**81675 München (DE)**

(54) **THERAPEUTIC FOR CANCER REFRACTORY TO IMMUNE CHECKPOINT INHIBITOR**

(57) Provided is a compound that exerts an anticancer action based on CHK1 inhibition. The present disclosure was completed by finding that a compound represented by formula (1) or a pharmaceutically acceptable salt thereof exhibits an excellent antitumor action by having a potent inhibitory action against CHK1:



Processed by Luminess, 75001 PARIS (FR)

(Cont. next page)

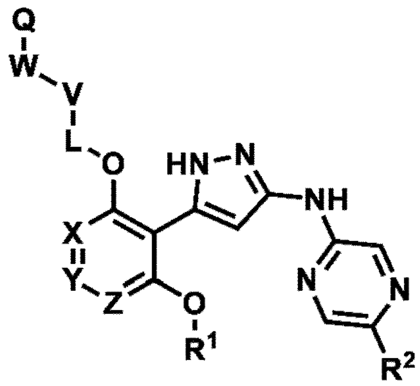

(1)

wherein $R^1$, $R^2$, L, V, W, and Q are as defined herein, X, Y, and Z each independently represent $CR^8$ or a nitrogen atom, wherein X, Y, and Z are not simultaneously $CR^8$, and $R^8$ is as defined herein.

Fig.11

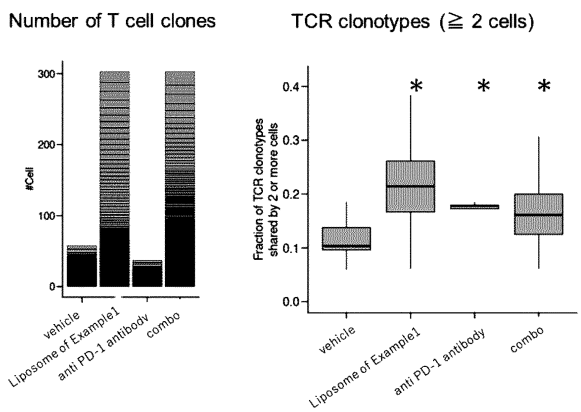

## Description

[Technical Field]

The present disclosure relates to a CHK1 inhibitor, a liposome encapsulating a CHK1 inhibitor, or combined use of the same and an immune checkpoint inhibitor, as therapeutic drugs for cancer exhibiting therapeutic resistance to an immune checkpoint inhibitor.

[Background Art]

CHK1 is a serine/threonine protein kinase downstream of ATR in a checkpoint signaling pathway of DNA damage during a cell cycle. In mammals, CHK1 is ATR dependently phosphorylated in response to a DNA damage induced by ionizing radiation or the like, or DNA replication stress due to excessive cell growth or genome instability in cancer cells or the like (Non Patent Literatures 1 to 5). Through such phosphorylation that activates CHK1, CHK1 phosphorylates CDC25A, and dephosphorylation of cyclin E/CDK2 by CDC25A is inhibited, and progression from the S phase is discontinued. CHK1 also phosphorylates CDC25C, and dephosphorylation of cyclin B/CDK1 by CDC25C is inhibited, and progression from the G2M phase is discontinued. In either case, regulation of CDK activity induces discontinuation of the cell cycle and obstruction of cell division in the presence of a DNA damage to promote repair of the DNA damage. In a cancer cell, inhibition of CHK1 suppresses a checkpoint function of a DNA damage in a cell cycle induces DNA repair deficiency, uncontrollable DNA synthesis, etc. in the presence of a DNA damage, resulting in induction of a DNA fragmentation, replication catastrophe, and cell death (Patent Literatures 1 to 2).

Since their launch, immune checkpoint inhibitors have established themselves as the fourth therapy in cancer therapy after surgery, radiation therapy, and chemotherapy. These immune checkpoint inhibitors are currently approved in a wide range of cancer types including melanoma. It has been reported that a characteristic of its efficacy is that it brings about a durable clinical response; however, this efficacy is seen only in some patients, and there is a need to improve the response rate, for example, the overall response rate of nivolumab in head and neck squamous cell carcinoma of 13.3% (Non Patent Literature 7).

Various CHK1 inhibitors (Patent Literatures 1 to 4) have been reported.

[Citation List]

[Patent Literature]

[PTL 1]
WO 2010/077758
[PTL 2]
WO 2012/064548
[PTL 3]
WO 2017/132928

[Non Patent Literature]

[NPL 1]
Dai Y and Grant S, Clin Cancer Res, 16(2):376-383 (2010)
[NPL 2]
Benada J and Macurek L, Biomolecules, 5:1912-1937 (2015)
[NPL 3]
King C, Mol Cancer Ther, 14(9):2004-2013 (2015)
[NPL 4]
Dent P, Expert Opinion on Investigational drugs, 28(12):1095-1100 (2019)
[NPL 5]
Evangelisti G. et al, Expert Opinion on Investigational Drugs, 29(8):779-792 (2020)
[NPL 6]
David H. et al. J Clin Oncol, 34:1764-1771 (2016)

[NPL 7]
Raffaele A.et al. Expert Opinion on Biological Therapy,19:3, 169-171 (2019)

[Summary of the Invention]

[Solution to Problem]

The present disclosure provides a drug for treating cancer exhibiting therapeutic resistance to an immune checkpoint inhibitor or for preventing recurrence of cancer after remission.

As a result of intensive studies, the present inventors have found that by continuously exposing a drug to cancer tissue by a CHK1 inhibitor or a liposome encapsulating a CHK1 inhibitor, a cytocidal effect and antigen presentation are enhanced, and M2 macrophages decrease and the intratumoral environment changes to one that attracts CD8 positive T cells (inflammatory microenvironment), and have found that the present drug exhibits an excellent antitumor effect on cancer exhibiting therapeutic resistance to an immune checkpoint inhibitor. Furthermore, the present inventors have found that when an immune checkpoint inhibitor is used concomitantly with the inhibitor or liposome, a more excellent antitumor effect is exhibited. In addition, the present inventors have found that when the inhibitor or liposome is used concomitantly with an immune checkpoint inhibitor, CD8 positive T cells having a memory function increase, and tumor is rejected or tumor growth is suppressed at the time of secondary transplantation, and thus, a prophylactic effect on the recurrence of cancer after remission is exhibited, thereby completing the present disclosure.

Accordingly, the present disclosure is as follows.

[Item 1]

A therapeutic agent and/or prophylactic agent for a cancer patient exhibiting therapeutic resistance to an immune checkpoint inhibitor, comprising, as an active ingredient, a CHK1 inhibitor, or a liposome encapsulating a CHK1 inhibitor.

[Item 2]

The therapeutic agent and/or prophylactic agent of item **1,** wherein the CHK1 inhibitor is used concomitantly with an immune checkpoint inhibitor.

[Item 3]

The therapeutic agent and/or prophylactic agent of item **2,** wherein the CHK1 inhibitor and the immune checkpoint inhibitor are administered simultaneously.

[Item 4]

The therapeutic agent and/or prophylactic agent of any one of item 2 or **3,** wherein the immune checkpoint inhibitor is administered after the CHK1 inhibitor is administered.

[Item 5]

The therapeutic agent and/or prophylactic agent of any one of items 2 to **4,** wherein the anti-PD-1 antibody is further administered after the CHK1 inhibitor and the immune checkpoint inhibitor are administered simultaneously.

[Item 6]

The therapeutic agent and/or prophylactic agent of any one of items 2 to **4,** wherein the anti-PD-1 antibody is further administered three days or later after the CHK1 inhibitor and the immune checkpoint inhibitor are administered simultaneously.

[Item 7]

The therapeutic agent and/or prophylactic agent of any one of items 2 to **6,** wherein the immune checkpoint inhibitor is an anti-PD-1 antibody or an anti-PD-L1 antibody.

[Item 8]

The therapeutic agent and/or prophylactic agent of any one of items 2 to **6,** wherein the immune checkpoint inhibitor is an anti-PD-1 antibody.

[Item 9]

The therapeutic agent and/or prophylactic agent of any one of items 2 to **6,** wherein the anti-PD-1 antibody is pembrolizumab, nivolumab, spartalizumab, or cemiplimab.

[Item 10]

The therapeutic agent and/or prophylactic agent of any one of items 1 to **9,** wherein the number of CD3 positive cells in the tumor tissue of the cancer patient is lower than that in tumor tissue of an anti-PD-1 antibody-responsive patient.

[Item 11]

The therapeutic agent and/or prophylactic agent of any one of items 1 to 10, wherein the number of CD8 positive cells in the tumor tissue of the cancer patient is lower than that in tumor tissue of an anti-PD-1 antibody-responsive patient.

[Item 12]

The therapeutic agent and/or prophylactic agent of any one of items 1 to 11, wherein the number of CD8 positive cells in the tumor tissue of the cancer patient is 0.135 or less.

[Item 13]

The therapeutic agent and/or prophylactic agent of any one of items 1 to 12, wherein an M1-M2 ratio of macrophages in the tumor tissue of the cancer patient is 21 or less.

[Item 14]

The therapeutic agent and/or prophylactic agent of any one of items 1 to 13, wherein the number of CD206 positive cells in the tumor tissue of the cancer patient is higher than that in tumor tissue of an anti-PD-1 antibody-responsive patient.

[Item 15]

The therapeutic agent and/or prophylactic agent of any one of items 1 to 14, wherein the number of IAd positive cells in the tumor tissue of the cancer patient is lower than that in tumor tissue of an anti-PD-1 antibody-responsive patient.

[Item 16]

The therapeutic agent and/or prophylactic agent of any one of items 1 to 15, wherein the number of CD62L negative CD44 positive cells in the lymph nodes of the cancer patient is decreased compared to lymph nodes in an anti-PD-1 antibody-responsive patient.

[Item 17]

The therapeutic agent and/or prophylactic agent of any one of items 1 to 16, wherein PD-L1 expression in the tumor tissue of the cancer patient is lower than that in tumor tissue of an anti-PD-1 antibody-responsive patient.

[Item 18]

The therapeutic agent and/or prophylactic agent of any one of items 1 to 17, wherein VEGFa expression in the tumor tissue of the cancer patient is higher than that in tumor tissue of an anti-PD-1 antibody-responsive patient.

[Item 19]

The therapeutic agent and/or prophylactic agent of any one of items 1 to 17, wherein Granzyme B expression in the tumor tissue of the cancer patient is lower than that in tumor tissue of an anti-PD-1 antibody-responsive patient.

[Item 20]

The therapeutic agent and/or prophylactic agent of any one of items 1 to 17, wherein Interferon γ expression in the tumor tissue of the cancer patient is lower than that in tumor tissue of an anti-PD-1 antibody-responsive patient.

[Item 21]

The therapeutic agent and/or prophylactic agent of any one of items 1 to 17, wherein H2ab1 expression in the tumor tissue of the cancer patient is lower than that in tumor tissue of an anti-PD-1 antibody-responsive patient.

[Item 22]

The therapeutic agent and/or prophylactic agent of any one of items 1 to 17, wherein CD80 expression in the tumor tissue of the cancer patient is lower than that in tumor tissue of an anti-PD-1 antibody-responsive patient.

[Item 23]

The therapeutic agent and/or prophylactic agent of any one of items 1 to 17, wherein Mrc1 expression in the tumor tissue of the cancer patient is higher than that in tumor tissue of an anti-PD-1 antibody-responsive patient.

[Item 24]

The therapeutic agent and/or prophylactic agent of any one of items 1 to 17, wherein Arginase1 expression in the tumor tissue of the cancer patient is higher than that in tumor tissue of an anti-PD-1 antibody-responsive patient.

[Item 25]

The therapeutic agent and/or prophylactic agent of any one of items 1 to 17, wherein DNA replication stress in the tumor tissue of the cancer patient is lower than that in tumor tissue of an anti-PD-1 antibody-responsive patient.

[Item 26]

A therapeutic agent and/or prophylactic agent for the prophylaxis of recurrence of cancer after remission, comprising, as an active ingredient, a CHK1 inhibitor, or a liposome encapsulating a CHK1 inhibitor.

[Item 27]

The therapeutic agent and/or prophylactic agent of item 26, wherein the CHK1 inhibitor is used concomitantly with an immune checkpoint inhibitor.

[Item 28]

The therapeutic agent and/or prophylactic agent of any one of item 26 or 27, wherein the immune checkpoint inhibitor is an anti-PD-1 antibody or an anti-PD-L1 antibody.

[Item 29]

The therapeutic agent and/or prophylactic agent of any one of item 26 or 27, wherein the immune checkpoint inhibitor is an anti-PD-1 antibody.

[Item 30]

The therapeutic agent and/or prophylactic agent of any one of item 26 or 27, wherein the anti-PD-1 antibody is

pembrolizumab, nivolumab, spartalizumab, or cemiplimab.

[Item 31]

The therapeutic agent and/or prophylactic agent of any one of items 26 to 30, wherein the cancer is at least one type of cancer selected from the group consisting of squamous cell carcinoma, melanoma, acute leukemia, chronic lymphocytic leukemia, chronic myelogenous leukemia, myelodysplastic syndrome, polycythemia vera, malignant lymphoma, plasma cell tumor, multiple myeloma, brain tumor, head and neck cancer, head and neck squamous cell carcinoma, esophageal cancer, thyroid cancer, lung cancer, small cell lung cancer, non small cell lung cancer, thymoma/thymic carcinoma, breast cancer, triple negative breast cancer, gastric cancer, gallbladder/bile duct cancer, liver cancer, hepatocellular carcinoma, pancreatic cancer, colon cancer, rectal cancer, MSI-high colon cancer, anal cancer, gastrointestinal stromal tumor, choriocarcinoma, endometrial cancer, cervical cancer, ovarian cancer, platinum-resistant ovarian cancer, PARP inhibitor-resistant ovarian cancer, bladder cancer, urothelial cancer, renal cancer, renal cell cancer, prostate cancer, testicular tumor, testicular germ cell tumor, ovarian germ cell tumor, Wilms tumor, malignant melanoma, neuroblastoma, osteosarcoma, Ewing sarcoma, chondrosarcoma, soft tissue sarcoma, or skin cancer.

[Item 32]

The therapeutic agent and/or prophylactic agent of any one of items 26 to 30, wherein the cancer is at least one type of cancer selected from the group consisting of melanoma, squamous cell carcinoma, non small cell lung cancer, small cell lung cancer, head and neck squamous cell carcinoma, urothelial cancer, non-muscle invasive bladder cancer, kidney cancer, hepatocellular carcinoma, MSI-high colon cancer, esophageal cancer, colon cancer, rectal cancer, breast cancer, or endometrial cancer.

[Item 33]

The therapeutic agent and/or prophylactic agent of any one of items 26 to 30, wherein the cancer is at least one type of cancer selected from the group consisting of head and neck cancer, squamous cell carcinoma, non small cell lung cancer, anal cancer, breast cancer, triple negative breast cancer, ovarian cancer, platinum-resistant ovarian cancer, or endometrial cancer.

[Item 34]

The therapeutic agent and/or prophylactic agent of any one of items 26 to 30, wherein the cancer is a Tumor Mutation Burden-High (TMB-High) solid tumor.

[Item 35]

The therapeutic agent and/or prophylactic agent of any one of items 1 to 34, wherein the liposome further comprises a phospholipid.

[Item 36]

The therapeutic agent and/or prophylactic agent of item 35, wherein the phospholipid is one selected from the group consisting of phosphatidylcholine, phosphatidylglycerol, phosphatidic acid, phosphatidylethanolamine, phosphatidylserine, phosphatidylinositol, sphingomyelin, soybean lecithin, egg yolk lecithin, hydrogenated egg yolk lecithin, and hydrogenated soybean lecithin or a combination of two or more thereof.

[Item 37]

The therapeutic agent and/or prophylactic agent of any one of items 1 to 36, wherein the liposome further comprises sterol.

[Item 38]

The therapeutic agent and/or prophylactic agent of any one of items 1 to 37, wherein the sterol is cholesterol.

[Item 39]

The therapeutic agent and/or prophylactic agent of any one of items 1 to 38, wherein the liposome further comprises a polymer-modified lipid.

[Item 40]

The therapeutic agent and/or prophylactic agent of item 39, wherein a polymer moiety of the polymer-modified lipid is polyethylene glycol, polypropylene glycol, polyvinyl alcohol, polyvinylpyrrolidone, methoxypolyethylene glycol, methoxypolypropylene glycol, methoxypolyvinyl alcohol, methoxypolyvinylpyrrolidone, ethoxypolyethylene glycol, ethoxypolypropylene glycol, ethoxypolyvinyl alcohol, ethoxypolyvinylpyrrolidone, propoxypolyethylene glycol, propoxypolypropylene glycol, propoxypolyvinyl alcohol, or propoxypolyvinylpyrrolidone.

[Item 41]

The therapeutic agent and/or prophylactic agent of item 39 or 40, wherein a lipid moiety of the polymer-modified lipid is phosphatidylethanolamine or diacylglycerol.

[Item 42]

The therapeutic agent and/or prophylactic agent of any one of items 1 to 41, wherein the liposome encapsulating a CHK1 inhibitor comprises

(1) 40 to 70 mol% of phospholipid,
(2) 30 to 50 mol% of cholesterol, and
(3) 1 to 10 mol% of polymer-modified lipid.

[Item 43]

The therapeutic agent and/or prophylactic agent of any one of items 1 to 42, wherein the liposome further comprises an additive selected from the group consisting of inorganic acids, inorganic acid salts, organic acids, organic acid salts, saccharides, buffer, antioxidants, and polymers.

[Item 1A]

Use of a CHK1 inhibitor, or a liposome encapsulating a CHK1 inhibitor, for the manufacture of a therapeutic agent and/or prophylactic agent for a cancer patient exhibiting therapeutic resistance to an immune checkpoint inhibitor.

[Item 2A]

The use of item 1A, wherein the CHK1 inhibitor is used concomitantly with an immune checkpoint inhibitor.

[Item 3A]

The use of item 2A, wherein the CHK1 inhibitor and the immune checkpoint inhibitor are administered simultaneously.

[Item 4A]

The use of any one of item 2A or 3A, wherein the immune checkpoint inhibitor is administered after the CHK1 inhibitor is administered.

[Item 5A]

The use of any one of items 2A to 4A, wherein the anti-PD-1 antibody is further administered after the CHK1 inhibitor and the immune checkpoint inhibitor are administered simultaneously.

[Item 6A]

The use of any one of items 2A to 4A, wherein the anti-PD-1 antibody is further administered three days or later after the CHK1 inhibitor and the immune checkpoint inhibitor are administered simultaneously.

[Item 7A]

The use of any one of items 2A to 6A, wherein the immune checkpoint inhibitor is an anti-PD-1 antibody or an anti-PD-L1 antibody.

[Item 8A]

The use of any one of items 2A to 6A, wherein the immune checkpoint inhibitor is an anti-PD-1 antibody.

[Item 9A]

The use of any one of items 2A to 6A, wherein the anti-PD-1 antibody is pembrolizumab, nivolumab, spartalizumab, or cemiplimab.

[Item 10A]

The use of any one of items 1A to 9A, wherein the number of CD3 positive cells in the tumor tissue of the cancer patient is lower than that in tumor tissue of an anti-PD-1 antibody-responsive patient.

[Item 11A]

The use of any one of items 1A to 10A, wherein the number of CD8 positive cells in the tumor tissue of the cancer patient is lower than that in tumor tissue of an anti-PD-1 antibody-responsive patient.

[Item 12A]

The use of any one of items 1A to 11A, wherein the number of CD8 positive cells in the tumor tissue of the cancer patient is 0.135 or less.

[Item 13A]

The use of any one of items 1A to 12A, wherein an M1-M2 ratio of macrophages in the tumor tissue of the cancer patient is 21 or less.

[Item 14A]

The use of any one of items 1A to 13A, wherein the number of CD206 positive cells in the tumor tissue of the cancer patient is higher than that in tumor tissue of an anti-PD-1 antibody-responsive patient.

[Item 15A]

The use of any one of items 1A to 14A, wherein the number of IAd positive cells in the tumor tissue of the cancer patient is lower than that in tumor tissue of an anti-PD-1 antibody-responsive patient.

[Item 16A]

The use of any one of items 1A to 15A, wherein the number of CD62L negative CD44 positive cells in the lymph nodes of the cancer patient is decreased compared to lymph nodes in an anti-PD-1 antibody-responsive patient.

[Item 17A]

The use of any one of items 1A to 16A, wherein PD-L1 expression in the tumor tissue of the cancer patient is lower than that in tumor tissue of an anti-PD-1 antibody-responsive patient.

[Item 18A]

The use of any one of items 1A to 17A, wherein VEGFa expression in the tumor tissue of the cancer patient is higher than that in tumor tissue of an anti-PD-1 antibody-responsive patient.

[Item 19A]

The use of any one of items 1A to 17A, wherein Granzyme B expression in the tumor tissue of the cancer patient is lower than that in tumor tissue of an anti-PD-1 antibody-responsive patient.

[Item 20A]

The use of any one of items 1A to 17A, wherein Interferon $\gamma$ expression in the tumor tissue of the cancer patient is lower than that in tumor tissue of an anti-PD-1 antibody-responsive patient.

[Item 21A]

The use of any one of items 1A to 17A, wherein H2ab1 expression in the tumor tissue of the cancer patient is lower than that in tumor tissue of an anti-PD-1 antibody-responsive patient.

[Item 22A]

The use of any one of items 1A to 17A, wherein CD80 expression in the tumor tissue of the cancer patient is lower than that in tumor tissue of an anti-PD-1 antibody-responsive patient.

[Item 23A]

The use of any one of items 1A to 17A, wherein Mrc1 expression in the tumor tissue of the cancer patient is higher than that in tumor tissue of an anti-PD-1 antibody-responsive patient.

[Item 24A]

The use of any one of items 1A to 17A, wherein Arginase1 expression in the tumor tissue of the cancer patient is higher than that in tumor tissue of an anti-PD-1 antibody-responsive patient.

[Item 25A]

The use of any one of items 1A to 17A, wherein DNA replication stress in the tumor tissue of the cancer patient is lower than that in tumor tissue of an anti-PD-1 antibody-responsive patient.

[Item 26A]

Use of a CHK1 inhibitor, or a liposome encapsulating a CHK1 inhibitor, for the manufacture of a therapeutic agent and/or prophylactic agent for the prophylaxis of recurrence of cancer after remission.

[Item 27A]

The use of item 26A, wherein the CHK1 inhibitor is used concomitantly with an immune checkpoint inhibitor.

[Item 28A]

The use of any one of item 26A or 27A, wherein the immune checkpoint inhibitor is an anti-PD-1 antibody or an anti-PD-L1 antibody.

[Item 29A]

The use of any one of item 26A or 27A, wherein the immune checkpoint inhibitor is an anti-PD-1 antibody.

[Item 30A]

The use of any one of item 26A or 27A, wherein the anti-PD-1 antibody is pembrolizumab, nivolumab, spartalizumab, or cemiplimab.

[Item 31A]

The use of any one of items 26A to 30A, wherein the cancer is at least one type of cancer selected from the group consisting of squamous cell carcinoma, melanoma, acute leukemia, chronic lymphocytic leukemia, chronic myelogenous leukemia, myelodysplastic syndrome, polycythemia vera, malignant lymphoma, plasma cell tumor, multiple myeloma, brain tumor, head and neck cancer, head and neck squamous cell carcinoma, esophageal cancer, thyroid cancer, lung cancer, small cell lung cancer, non small cell lung cancer, thymoma/thymic carcinoma, breast cancer, triple negative breast cancer, gastric cancer, gallbladder/bile duct cancer, liver cancer, hepatocellular carcinoma, pancreatic cancer, colon cancer, rectal cancer, MSI-high colon cancer, anal cancer, gastrointestinal stromal tumor, choriocarcinoma, endometrial cancer, cervical cancer, ovarian cancer, platinum-resistant ovarian cancer, PARP inhibitor-resistant ovarian cancer, bladder cancer, urothelial cancer, renal cancer, renal cell cancer, prostate cancer, testicular tumor, testicular germ cell tumor, ovarian germ cell tumor, Wilms tumor, malignant melanoma, neuroblastoma, osteosarcoma, Ewing sarcoma, chondrosarcoma, soft tissue sarcoma, or skin cancer.

[Item 32A]

The use of any one of items 26A to 30A, wherein the cancer is melanoma, non small cell lung cancer, small cell lung cancer, head and neck squamous cell carcinoma, urothelial cancer, non-muscle invasive bladder cancer, kidney cancer, hepatocellular carcinoma, MSI-high colon cancer, esophageal cancer, colon and rectal cancer, breast cancer, or endometrial cancer.

[Item 33A]

The use of any one of items 26A to 30A, wherein the cancer is at least one type of cancer selected from the group consisting of head and neck cancer, squamous cell carcinoma, non small cell lung cancer, anal cancer, breast cancer, triple negative breast cancer, ovarian cancer, platinum-resistant ovarian cancer, or endometrial cancer.

[Item 34A]

The use of any one of items 26A to 30A, wherein the cancer is a Tumor Mutation Burden-High (TMB-High) solid tumor.

[Item 35A]

The use of any one of items 1A to 34A, wherein the liposome further comprises a phospholipid.

[Item 36A]

The use of item 35A, wherein the phospholipid is one selected from the group consisting of phosphatidylcholine, phosphatidylglycerol, phosphatidic acid, phosphatidylethanolamine, phosphatidylserine, phosphatidylinositol, sphingomyelin, soybean lecithin, egg yolk lecithin, hydrogenated egg yolk lecithin, and hydrogenated soybean lecithin or a combination of two or more thereof.

[Item 37A]

The use of any one of items 1A to 36A, wherein the liposome further comprises sterol.

[Item 38A]

The use of any one of items 1A to 37A, wherein the sterol is cholesterol.

[Item 39A]

The use of any one of items 1A to 38A, wherein the liposome further comprises a polymer-modified lipid.

[Item 40A]

The use of item 39A, wherein a polymer moiety of the polymer-modified lipid is polyethylene glycol, polypropylene glycol, polyvinyl alcohol, polyvinylpyrrolidone, methoxypolyethylene glycol, methoxypolypropylene glycol, methoxypolyvinyl alcohol, methoxypolyvinylpyrrolidone, ethoxypolyethylene glycol, ethoxypolypropylene glycol, ethoxypolyvinyl alcohol, ethoxypolyvinylpyrrolidone, propoxypolyethylene glycol, propoxypolypropylene glycol, propoxypolyvinyl alcohol, or propoxypolyvinylpyrrolidone.

[Item 41A]

The use of item 39A or 40A, wherein a lipid moiety of the polymer-modified lipid is phosphatidylethanolamine or diacylglycerol.

[Item 42A]

The use of any one of items 1A to 41A, wherein the liposome encapsulating a CHK1 inhibitor comprises

(1) 40 to 70 mol% of phospholipid,
(2) 30 to 50 mol% of cholesterol, and
(3) 1 to 10 mol% of polymer-modified lipid.

[Item 43A]

The use of any one of items 1A to 42A, wherein the liposome further comprises an additive selected from the group consisting of inorganic acids, inorganic acid salts, organic acids, organic acid salts, saccharides, buffer, antioxidants, and polymers.

[Item 1B]

A therapeutic agent and/or prophylactic agent for use in the treatment and/or prophylaxis of a cancer patient exhibiting therapeutic resistance to an immune checkpoint inhibitor, comprising, as an active ingredient, a CHK1 inhibitor, or a liposome encapsulating a CHK1 inhibitor.

[Item 2B]

The therapeutic agent and/or prophylactic agent of item 1B, wherein the CHK1 inhibitor is used concomitantly with an immune checkpoint inhibitor.

[Item 3B]

The therapeutic agent and/or prophylactic agent of item 2B, wherein the CHK1 inhibitor and the immune checkpoint inhibitor are administered simultaneously.

[Item 4B]

The therapeutic agent and/or prophylactic agent of any one of item 2B or 3B, wherein the immune checkpoint inhibitor is administered after the CHK1 inhibitor is administered.

[Item 5B]

The therapeutic agent and/or prophylactic agent of any one of items 2B to 4B, wherein the anti-PD-1 antibody is further administered after the CHK1 inhibitor and the immune checkpoint inhibitor are administered simultaneously.

[Item 6B]

The therapeutic agent and/or prophylactic agent of any one of items 2B to 4B, wherein the anti-PD-1 antibody is further administered three days or later after the CHK1 inhibitor and the immune checkpoint inhibitor are administered simultaneously.

[Item 7B]

The therapeutic agent and/or prophylactic agent of any one of items 2B to 6B, wherein the immune checkpoint inhibitor is an anti-PD-1 antibody or an anti-PD-L1 antibody.

[Item 8B]

The therapeutic agent and/or prophylactic agent of any one of items 2B to 6B, wherein the immune checkpoint inhibitor is an anti-PD-1 antibody.

[Item 9B]

The therapeutic agent and/or prophylactic agent of any one of items 2B to 6B, wherein the anti-PD-1 antibody is pembrolizumab, nivolumab, spartalizumab, or cemiplimab.

[Item 10B]

The therapeutic agent and/or prophylactic agent of any one of items 1B to 9B, wherein the number of CD3 positive cells in the tumor tissue of the cancer patient is lower than that in tumor tissue of an anti-PD-1 antibody-responsive patient.

[Item 11B]

The therapeutic agent and/or prophylactic agent of any one of items 1B to 10B, wherein the number of CD8 positive cells in the tumor tissue of the cancer patient is lower than that in tumor tissue of an anti-PD-1 antibody-responsive patient.

[Item 12B]

The therapeutic agent and/or prophylactic agent of any one of items 1B to 11B, wherein the number of CD8 positive cells in the tumor tissue of the cancer patient is 0.135 or less.

[Item 13B]

The therapeutic agent and/or prophylactic agent of any one of items 1B to 12B, wherein an M1-M2 ratio of macrophages in the tumor tissue of the cancer patient is 21 or less.

[Item 14B]

The therapeutic agent and/or prophylactic agent of any one of items 1B to 13B, wherein the number of CD206 positive cells in the tumor tissue of the cancer patient is higher than that in tumor tissue of an anti-PD-1 antibody-responsive patient.

[Item 15B]

The therapeutic agent and/or prophylactic agent of any one of items 1B to 14B, wherein the number of IAd positive cells in the tumor tissue of the cancer patient is lower than that in tumor tissue of an anti-PD-1 antibody-responsive patient.

[Item 16B]

The therapeutic agent and/or prophylactic agent of any one of items 1B to 15B, wherein the number of CD62L negative CD44 positive cells in the lymph nodes of the cancer patient is decreased compared to lymph nodes in an anti-PD-1 antibody-responsive patient.

[Item 17B]

The therapeutic agent and/or prophylactic agent of any one of items 1B to 16B, wherein PD-L1 expression in the tumor tissue of the cancer patient is lower than that in tumor tissue of an anti-PD-1 antibody-responsive patient.

[Item 18B]

The therapeutic agent and/or prophylactic agent of any one of items 1B to 17B, wherein VEGFa expression in the tumor tissue of the cancer patient is higher than that in tumor tissue of an anti-PD-1 antibody-responsive patient.

[Item 19B]

The therapeutic agent and/or prophylactic agent of any one of items 1B to 17B, wherein Granzyme B expression in the tumor tissue of the cancer patient is lower than that in tumor tissue of an anti-PD-1 antibody-responsive patient.

[Item 20B]

The therapeutic agent and/or prophylactic agent of any one of items 1B to 17B, wherein Interferon γ expression in the tumor tissue of the cancer patient is lower than that in tumor tissue of an anti-PD-1 antibody-responsive patient.

[Item 21B]

The therapeutic agent and/or prophylactic agent of any one of items 1B to 17B, wherein H2ab1 expression in the tumor tissue of the cancer patient is lower than that in tumor tissue of an anti-PD-1 antibody-responsive patient.

[Item 22B]

The therapeutic agent and/or prophylactic agent of any one of items 1B to 17B, wherein CD80 expression in the tumor tissue of the cancer patient is lower than that in tumor tissue of an anti-PD-1 antibody-responsive patient.

[Item 23B]

The therapeutic agent and/or prophylactic agent of any one of items 1B to 17B, wherein Mrc1 expression in the tumor tissue of the cancer patient is higher than that in tumor tissue of an anti-PD-1 antibody-responsive patient.

[Item 24B]

The therapeutic agent and/or prophylactic agent of any one of items 1B to 17B, wherein Arginase1 expression in the tumor tissue of the cancer patient is higher than that in tumor tissue of an anti-PD-1 antibody-responsive patient.

[Item 25B]

The therapeutic agent and/or prophylactic agent of any one of items 1B to 17B, wherein DNA replication stress in the tumor tissue of the cancer patient is lower than that in tumor tissue of an anti-PD-1 antibody-responsive patient.

[Item 26B]

A therapeutic agent and/or prophylactic agent for use in in the prophylaxis of recurrence of cancer after remission, comprising, as an active ingredient, a CHK1 inhibitor, or a liposome encapsulating a CHK1 inhibitor.

[Item 27B]

The therapeutic agent and/or prophylactic agent of item 26B, wherein the CHK1 inhibitor is used concomitantly with an immune checkpoint inhibitor.

[Item 28B]

The therapeutic agent and/or prophylactic agent of any one of item 26B or 27B, wherein the immune checkpoint

inhibitor is an anti-PD-1 antibody or an anti-PD-L1 antibody.

[Item 29B]

The therapeutic agent and/or prophylactic agent of any one of item 26B or 27B, wherein the immune checkpoint inhibitor is an anti-PD-1 antibody.

[Item 30B]

The therapeutic agent and/or prophylactic agent of any one of item 26B or 27B, wherein the anti-PD-1 antibody is pembrolizumab, nivolumab, spartalizumab, or cemiplimab.

[Item 31B]

The therapeutic agent and/or prophylactic agent of any one of items 26B to 30B, wherein the cancer is at least one type of cancer selected from the group consisting of squamous cell carcinoma, melanoma, acute leukemia, chronic lymphocytic leukemia, chronic myelogenous leukemia, myelodysplastic syndrome, polycythemia vera, malignant lymphoma, plasma cell tumor, multiple myeloma, brain tumor, head and neck cancer, head and neck squamous cell carcinoma, esophageal cancer, thyroid cancer, lung cancer, small cell lung cancer, non small cell lung cancer, thymoma/thymic carcinoma, breast cancer, triple negative breast cancer, gastric cancer, gallbladder/bile duct cancer, liver cancer, hepatocellular carcinoma, pancreatic cancer, colon cancer, rectal cancer, MSI-high colon cancer, anal cancer, gastrointestinal stromal tumor, choriocarcinoma, endometrial cancer, cervical cancer, ovarian cancer, platinum-resistant ovarian cancer, PARP inhibitor-resistant ovarian cancer, bladder cancer, urothelial cancer, renal cancer, renal cell cancer, prostate cancer, testicular tumor, testicular germ cell tumor, ovarian germ cell tumor, Wilms tumor, malignant melanoma, neuroblastoma, osteosarcoma, Ewing sarcoma, chondrosarcoma, soft tissue sarcoma, or skin cancer.

[Item 32B]

The therapeutic agent and/or prophylactic agent of any one of items 26B to 30B, wherein the cancer is melanoma, non small cell lung cancer, small cell lung cancer, head and neck squamous cell carcinoma, urothelial cancer, non-muscle invasive bladder cancer, kidney cancer, hepatocellular carcinoma, MSI-high colon cancer, esophageal cancer, colon and rectal cancer, breast cancer, or endometrial cancer.

[Item 33B]

The therapeutic agent and/or prophylactic agent of any one of items 26B to 30B, wherein the cancer is at least one type of cancer selected from the group consisting of head and neck cancer, squamous cell carcinoma, non small cell lung cancer, anal cancer, breast cancer, triple negative breast cancer, ovarian cancer, platinum-resistant ovarian cancer, or endometrial cancer.

[Item 34B]

The therapeutic agent and/or prophylactic agent of any one of items 26B to 30B, wherein the cancer is a Tumor Mutation Burden-High (TMB-High) solid tumor.

[Item 35B]

The therapeutic agent and/or prophylactic agent of any one of items 1B to 34B, wherein the liposome further comprises a phospholipid.

[Item 36B]

The therapeutic agent and/or prophylactic agent of item 35B, wherein the phospholipid is one selected from the group consisting of phosphatidylcholine, phosphatidylglycerol, phosphatidic acid, phosphatidylethanolamine, phosphatidylserine, phosphatidylinositol, sphingomyelin, soybean lecithin, egg yolk lecithin, hydrogenated egg yolk lecithin, and hydrogenated soybean lecithin or a combination of two or more thereof.

[Item 37B]

The therapeutic agent and/or prophylactic agent of any one of items 1B to 36B, wherein the liposome further comprises sterol.

[Item 38B]

The therapeutic agent and/or prophylactic agent of any one of items 1B to 37B, wherein the sterol is cholesterol.

[Item 39B]

The therapeutic agent and/or prophylactic agent of any one of items 1B to 38B, wherein the liposome further comprises a polymer-modified lipid.

[Item 40B]

The therapeutic agent and/or prophylactic agent of item 39B, wherein a polymer moiety of the polymer-modified lipid is polyethylene glycol, polypropylene glycol, polyvinyl alcohol, polyvinylpyrrolidone, methoxypolyethylene glycol, methoxypolypropylene glycol, methoxypolyvinyl alcohol, methoxypolyvinylpyrrolidone, ethoxypolyethylene glycol, ethoxypolypropylene glycol, ethoxypolyvinyl alcohol, ethoxypolyvinylpyrrolidone, propoxypolyethylene glycol, propoxypolypropylene glycol, propoxypolyvinyl alcohol, or propoxypolyvinylpyrrolidone.

[Item 41B]

The therapeutic agent and/or prophylactic agent of item 39B or 40B, wherein a lipid moiety of the polymer-modified lipid is phosphatidylethanolamine or diacylglycerol.

[Item 42B]

The therapeutic agent and/or prophylactic agent of any one of items 1B to 41B, wherein the liposome encapsulating a CHK1 inhibitor comprises

(1) 40 to 70 mol% of phospholipid,
(2) 30 to 50 mol% of cholesterol, and
(3) 1 to 10 mol% of polymer-modified lipid.

[Item 43B]

The therapeutic agent and/or prophylactic agent of any one of items 1B to 42B, wherein the liposome further comprises an additive selected from the group consisting of inorganic acids, inorganic acid salts, organic acids, organic acid salts, saccharides, buffer, antioxidants, and polymers.

[Item 1C]

A method of treating and/or preventing a cancer patient exhibiting therapeutic resistance to an immune checkpoint inhibitor, comprising administering a CHK1 inhibitor, or a liposome encapsulating a CHK1 inhibitor, to the patient.

[Item 2C]

The method of item 1C, wherein the CHK1 inhibitor is used concomitantly with an immune checkpoint inhibitor.

[Item 3C]

The method of item 2C, wherein the CHK1 inhibitor and the immune checkpoint inhibitor are administered simultaneously.

[Item 4C]

The method of any one of item 2C or 3C, wherein the immune checkpoint inhibitor is administered after the CHK1 inhibitor is administered.

[Item 5C]

The method of any one of items 2C to 4C, wherein the anti-PD-1 antibody is further administered after the CHK1 inhibitor and the immune checkpoint inhibitor are administered simultaneously.

[Item 6C]

The method of any one of items 2C to 4C, wherein the anti-PD-1 antibody is further administered three days or later after the CHK1 inhibitor and the immune checkpoint inhibitor are administered simultaneously.

[Item 7C]

The method of any one of items 2C to 6C, wherein the immune checkpoint inhibitor is an anti-PD-1 antibody or an anti-PD-L1 antibody.

[Item 8C]

The method of any one of items 2C to 6C, wherein the immune checkpoint inhibitor is an anti-PD-1 antibody.

[Item 9C]

The method of any one of items 2C to 6C, wherein the anti-PD-1 antibody is pembrolizumab, nivolumab, spartalizumab, or cemiplimab.

[Item 10C]

The method of any one of items 1C to 9C, wherein the number of CD3 positive cells in the tumor tissue of the cancer patient is lower than that in tumor tissue of an anti-PD-1 antibody-responsive patient.

[Item 11C]

The method of any one of items 1C to 10C, wherein the number of CD8 positive cells in the tumor tissue of the cancer patient is lower than that in tumor tissue of an anti-PD-1 antibody-responsive patient.

[Item 12C]

The method of any one of items 1C to 11C, wherein the number of CD8 positive cells in the tumor tissue of the cancer patient is 0.135 or less.

[Item 13C]

The method of any one of items 1C to 12C, wherein an M1-M2 ratio of macrophages in the tumor tissue of the cancer patient is 21 or less.

[Item 14C]

The method of any one of items 1C to 13C, wherein the number of CD206 positive cells in the tumor tissue of the cancer patient is higher than that in tumor tissue of an anti-PD-1 antibody-responsive patient.

[Item 15C]

The method of any one of items 1C to 14C, wherein the number of IAd positive cells in the tumor tissue of the cancer patient is lower than that in tumor tissue of an anti-PD-1 antibody-responsive patient.

[Item 16C]

The method of any one of items 1C to 15C, wherein the number of CD62L negative CD44 positive cells in the lymph nodes of the cancer patient is decreased compared to lymph nodes in an anti-PD-1 antibody-responsive patient.

[Item 17C]

The method of any one of items 1C to 16C, wherein PD-L1 expression in the tumor tissue of the cancer patient is lower than that in tumor tissue of an anti-PD-1 antibody-responsive patient.

[Item 18C]

The method of any one of items 1C to 17C, wherein VEGFa expression in the tumor tissue of the cancer patient is higher than that in tumor tissue of an anti-PD-1 antibody-responsive patient.

[Item 19C]

The method of any one of items 1C to 17C, wherein Granzyme B expression in the tumor tissue of the cancer patient is lower than that in tumor tissue of an anti-PD-1 antibody-responsive patient.

[Item 20C]

The method of any one of items 1C to 17C, wherein Interferon $\gamma$ expression in the tumor tissue of the cancer patient is lower than that in tumor tissue of an anti-PD-1 antibody-responsive patient.

[Item 21C]

The method of any one of items 1C to 17C, wherein H2ab1 expression in the tumor tissue of the cancer patient is lower than that in tumor tissue of an anti-PD-1 antibody-responsive patient.

[Item 22C]

The method of any one of items 1C to 17C, wherein CD80 expression in the tumor tissue of the cancer patient is lower than that in tumor tissue of an anti-PD-1 antibody-responsive patient.

[Item 23C]

The method of any one of items 1C to 17C, wherein Mrc1 expression in the tumor tissue of the cancer patient is higher than that in tumor tissue of an anti-PD-1 antibody-responsive patient.

[Item 24C]

The method of any one of items 1C to 17C, wherein Arginase1 expression in the tumor tissue of the cancer patient is higher than that in tumor tissue of an anti-PD-1 antibody-responsive patient.

[Item 25C]

The method of any one of items 1C to 17C, wherein DNA replication stress in the tumor tissue of the cancer patient is lower than that in tumor tissue of an anti-PD-1 antibody-responsive patient.

[Item 26C]

A method of preventing recurrence of cancer after remission, comprising administering a CHK1 inhibitor, or a liposome encapsulating a CHK1 inhibitor, to a patient.

[Item 27C]

The method of item 26C, wherein the CHK1 inhibitor is used concomitantly with an immune checkpoint inhibitor.

[Item 28C]

The method of any one of item 26C or 27C, wherein the immune checkpoint inhibitor is an anti-PD-1 antibody or an anti-PD-L1 antibody.

[Item 29C]

The method of any one of item 26A or 27C, wherein the immune checkpoint inhibitor is an anti-PD-1 antibody.

[Item 30C]

The method of any one of item 26C or 27C, wherein the anti-PD-1 antibody is pembrolizumab, nivolumab, spartalizumab, or cemiplimab.

[Item 31C]

The method of any one of items 26C to 30C, wherein the cancer is at least one type of cancer selected from the group consisting of squamous cell carcinoma, melanoma, acute leukemia, chronic lymphocytic leukemia, chronic myelogenous leukemia, myelodysplastic syndrome, polycythemia vera, malignant lymphoma, plasma cell tumor, multiple myeloma, brain tumor, head and neck cancer, head and neck squamous cell carcinoma, esophageal cancer, thyroid cancer, lung cancer, small cell lung cancer, non small cell lung cancer, thymoma/thymic carcinoma, breast cancer, triple negative breast cancer, gastric cancer, gallbladder/bile duct cancer, liver cancer, hepatocellular carcinoma, pancreatic cancer, colon cancer, rectal cancer, MSI-high colon cancer, anal cancer, gastrointestinal stromal tumor, choriocarcinoma, endometrial cancer, cervical cancer, ovarian cancer, platinum-resistant ovarian cancer, PARP inhibitor-resistant ovarian cancer, bladder cancer, urothelial cancer, renal cancer, renal cell cancer, prostate cancer, testicular tumor, testicular germ cell tumor, ovarian germ cell tumor, Wilms tumor, malignant melanoma, neuroblastoma, osteosarcoma, Ewing sarcoma, chondrosarcoma, soft tissue sarcoma, or skin cancer.

[Item 32C]

The method of any one of items 26C to 30C, wherein the cancer is melanoma, non small cell lung cancer, small cell lung cancer, head and neck squamous cell carcinoma, urothelial cancer, non-muscle invasive bladder cancer, kidney cancer, hepatocellular carcinoma, MSI-high colon cancer, esophageal cancer, colon and rectal cancer, breast cancer, or endometrial cancer.

[Item 33C]

The method of any one of items 26C to 30C, wherein the cancer is at least one type of cancer selected from the group consisting of head and neck cancer, squamous cell carcinoma, non small cell lung cancer, anal cancer, breast cancer, triple negative breast cancer, ovarian cancer, platinum-resistant ovarian cancer, or endometrial cancer.

[Item 34C]

The method of any one of items 26C to 30C, wherein the cancer is a Tumor Mutation Burden-High (TMB-High) solid tumor.

[Item 35C]

The method of any one of items 1C to 34C, wherein the liposome further comprises a phospholipid.

[Item 36C]

The method of item 35C, wherein the phospholipid is one selected from the group consisting of phosphatidylcholine, phosphatidylglycerol, phosphatidic acid, phosphatidylethanolamine, phosphatidylserine, phosphatidylinositol, sphingomyelin, soybean lecithin, egg yolk lecithin, hydrogenated egg yolk lecithin, and hydrogenated soybean lecithin or a combination of two or more thereof.

[Item 37C]

The method of any one of items 1C to 36C, wherein the liposome further comprises sterol.

[Item 38C]

The method of any one of items 1C to 37C, wherein the sterol is cholesterol.

[Item 39C]

The method of any one of items 1C to 36C, wherein the liposome further comprises a polymer-modified lipid.

[Item 40C]

The method of item 39C, wherein a polymer moiety of the polymer-modified lipid is polyethylene glycol, polypropylene glycol, polyvinyl alcohol, polyvinylpyrrolidone, methoxypolyethylene glycol, methoxypolypropylene glycol, methoxypolyvinyl alcohol, methoxypolyvinylpyrrolidone, ethoxypolyethylene glycol, ethoxypolypropylene glycol, ethoxypolyvinyl alcohol, ethoxypolyvinylpyrrolidone, propoxypolyethylene glycol, propoxypolypropylene glycol, propoxypolyvinyl alcohol, or propoxypolyvinylpyrrolidone.

[Item 41C]

The method of item 39C or 40C, wherein a lipid moiety of the polymer-modified lipid is phosphatidylethanolamine or diacylglycerol.

[Item 42C]

The method of any one of items 1C to 41C, wherein the liposome encapsulating a CHK1 inhibitor comprises

(1) 40 to 70 mol% of phospholipid,
(2) 30 to 50 mol% of cholesterol, and
(3) 1 to 10 mol% of polymer-modified lipid.

[Item 43C]

The method of any one of items 1C to 42C, wherein the liposome further comprises an additive selected from the group consisting of inorganic acids, inorganic acid salts, organic acids, organic acid salts, saccharides, buffer, antioxidants, and polymers.

[Item 44]

The therapeutic agent and/or prophylactic agent of any one of items 1 to 43, wherein the CHK1 inhibitor is a compound represented by formula (1):

[Chemical Formula 6]

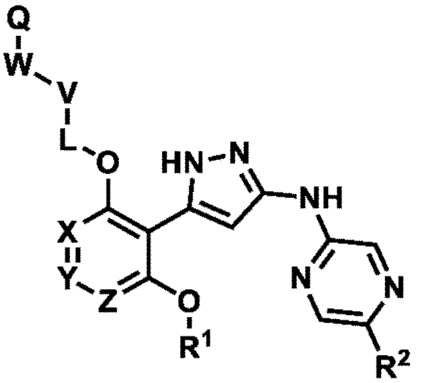

(1)

or a pharmaceutically acceptable salt thereof, wherein

$R^1$ represents a hydrogen atom, optionally substituted $C_{1-6}$ alkyl, optionally substituted $C_{3-10}$ cycloalkyl, an optionally substituted 3- to 10-membered saturated heterocyclic group, optionally substituted $C_{6-10}$ aryl, or optionally substituted 5-to 12-membered heteroaryl,
$R^2$ represents a hydrogen atom, a halogen atom, cyano, nitro, carboxyl, sulfonic acid, phosphoric acid, $-OR^3$, $-SR^3$, $-COR^4$, $-CO_2R^4$, $-CO_NR^5R^6$, $-SO_2R^4$, $-SO_2NR^5R^6$, $-OCOR^4$, $-OCO_2R^4$, $-OCONR^5R^6$, $-NR^5R^6$, $-NR^7COR^4$, $-NR^7CO_2R^4$, $-NR^7CONR^5R^6$, $-NR^7SO_2R^4$, $-NR^7SO_2NR^5R^6$, optionally substituted $C_{1-6}$ alkyl, optionally substituted $C_{2-6}$ alkenyl, optionally substituted $C_{2-6}$ alkynyl, optionally substituted $C_{3-10}$ cycloalkyl, an optionally substituted 3- to 10-membered saturated heterocyclic group, optionally substituted $C_{6-10}$ aryl, or optionally substituted 5-to 12-membered heteroaryl,
$R^3$ represents a hydrogen atom or $C_{1-6}$ alkyl,
$R^4$ represents $C_{1-6}$ alkyl,
$R^5$, $R^6$, and $R^7$ each independently represent a hydrogen atom or $C_{1-6}$ alkyl, wherein $R^5$ and $R^6$ that attach to the same nitrogen atom, when both are $C_{1-6}$ alkyl, together with the nitrogen atom to which each is attached, may form a 3- to 8-membered nitrogen-containing saturated heterocycle,
X, Y, and Z each independently represent $CR^8$ or a nitrogen atom, wherein X, Y, and Z are not simultaneously $CR^8$,
$R^8$, if there are multiple instances, each independently represent a hydrogen atom, a halogen atom, cyano, nitro, carboxyl, sulfonic acid, phosphoric acid, $-OR^9$, $-SR^9$, $-COR^{10}$, $-CO_2R^{10}$, $-CONR^{11}R^{12}$, $-SO_2R^{10}$, $-SO_2NR^{11}R^{12}$, $-OCOR^{10}$, $-OCO_2R^{10}$, $-OCONR^{11}R^{12}$, $-NR^{11}R^{12}$, $-NR^{13}COR^{10}$, $-NR^{13}CO_2R^{10}$, $-NR^{13}CONR^{11}R^{12}$, $-NR^{13}SO_2R^{10}$, $-NR^{13}SO_2NR^{11}R^{12}$, optionally substituted $C_{1-6}$ alkyl, optionally substituted $C_{2-6}$ alkenyl, optionally substituted $C_{2-6}$ alkynyl, optionally substituted $C_{3-10}$ cycloalkyl, an optionally substituted 3- to 10-membered saturated heterocyclic group, optionally substituted $C_{6-10}$ aryl, or optionally substituted 5-to 12-membered heteroaryl,
$R^9$ represents a hydrogen atom or $C_{1-6}$ alkyl,
$R^{10}$ represents $C_{1-6}$ alkyl,
$R^{11}$, $R^{12}$, and $R^{13}$ each independently represent a hydrogen atom or $C_{1-6}$ alkyl, wherein $R^{11}$ and $R^{12}$ that attach to the same nitrogen atom, when both are $C_{1-6}$ alkyl, together with the nitrogen atom to which each is attached, may form a 3- to 8-membered nitrogen-containing saturated heterocycle,
L represents a single bond or optionally substituted $C_{1-6}$ alkylene,
V represents a single bond, optionally substituted $C_{3-10}$ cycloalkylene, or an optionally substituted 3- to 10-membered divalent saturated heterocyclic group,
W represents a single bond or optionally substituted $C_{1-6}$ alkylene,
Q represents a hydrogen atom or $NHR^{14}$, and
$R^{14}$ represents a hydrogen atom, optionally substituted $C_{1-6}$ alkyl, optionally substituted $C_{3-10}$ cycloalkyl, or an optionally substituted 3- to 10-membered saturated heterocyclic group.

[Item 45]

The therapeutic agent and/or prophylactic agent of item 44, wherein

optionally substituted $C_{1-6}$ alkyl, optionally substituted $C_{2-6}$ alkenyl, optionally substituted $C_{2-6}$ alkynyl, optionally substituted $C_{3-10}$ cycloalkyl, an optionally substituted 3- to 10-membered saturated heterocyclic group, optionally

substituted $C_{6-10}$ aryl, optionally substituted 5- to 12-membered heteroaryl, optionally substituted $C_{1-6}$ alkylene, optionally substituted $C_{3-10}$ cycloalkylene, or an optionally substituted 3-to 10-membered divalent saturated heterocyclic group in $R^1$, $R^2$, $R^8$, $R^{14}$, L, V, and W is, each independently, optionally substituted with 1 to 5 of the same or different substituents selected from the group consisting of

(1) a halogen atom,
(2) a hydroxyl group,
(3) $C_{6-10}$ aryl,
(4) 5- to 12-membered heteroaryl,
(5) $C_{1-6}$ alkyl,
(6) $C_{2-6}$ alkenyl,
(7) $C_{2-6}$ alkynyl,
(8) $C_{1-6}$ alkoxy,
(9) $C_{1-6}$ alkylthio
(10) $C_{3-10}$ cycloalkyl,
(11) a 3- to 10-membered saturated heterocyclic group,
(12) carboxyl,
(13) $-COR^{15}$,
(14) $-CO_2R^{15}$,
(15) $-CONR^{16}R^{17}$,
(16) $-NR^{16}R^{17}$,
(17) $-NR^{18}COR^{15}$,
(18) $-NR^{18}CO_2R^{15}$,
(19) $-NR^{18}SO_2R^{15}$,
(20) $-NR^{18}CONR^{16}R^{17}$,
(21) $-NR^{18}SO_2NR^{16}R^{17}$,
(22) $-SO_2R^{15}$,
(23) $-SO_2NR^{16}R^{17}$,
(24) $-OCOR^{15}$,
(25) $-OCO_2R^{15}$,
(26) $-OCONR^{16}R^{17}$,
(27) sulfonic acid,
(28) phosphoric acid,
(29) cyano, and
(30) nitro,

wherein the groups represented by (3) $C_{6-10}$ aryl, (4) 5-to 12-membered heteroaryl, (5) $C_{1-6}$ alkyl, (6) $C_{2-6}$ alkenyl, (7) $C_{2-6}$ alkynyl, (8) $C_{1-6}$ alkoxy, (9) $C_{1-6}$ alkylthio, (9) $C_{3-10}$ cycloalkyl, and (10) a 3- to 10-membered saturated heterocyclic group are optionally substituted with 1 to 5 of the same or different substituents selected from the group consisting of

(a) a halogen atom,
(b) a hydroxyl group,
(c) $C_{6-10}$ aryl,
(d) 5- to 12-membered heteroaryl,
(e) $C_{1-6}$ alkyl,
(f) $C_{2-6}$ alkenyl,
(g) $C_{2-6}$ alkynyl,
(h) $C_{1-6}$ alkoxy,
(i) $C_{3-10}$ cycloalkyl,
(j) a 3- to 10-membered saturated heterocyclic group,
(k) carboxyl,
(1) $-COR^{15}$,
(m) $-CO_2R^{15}$,
(n) $-CONR^{16}R^{17}$,
(o) $-NR^{16}R^{17}$,
(p) $-NR^{18}COR^{15}$,
(q) $-NR^{18}SO_2R^{15}$,

(r) $-SO_2R^{15}$,
(s) $-SO_2NR^{16}R^{17}$,
(t) sulfonic acid,
(u) phosphoric acid,
(v) cyano, and
(w) nitro,

$R^{15}$, if there are multiple instances, are each independently $C_{1-6}$ alkyl,
$R^{16}$ and $R^{17}$ are each independently a hydrogen atom or $C_{1-6}$ alkyl, and if there are multiple instances of $R^{16}$ or $R^{17}$, each of $R^{16}$ or $R^{17}$ may be the same or different, wherein $R^{16}$ and $R^{17}$ that attach to the same nitrogen atom, when both are $C_{1-6}$ alkyl, together with the nitrogen atom to which each is attached, may form a 3- to 8-membered nitrogen-containing saturated heterocycle, and
$R^{18}$ is a hydrogen atom or $C_{1-6}$ alkyl.

[Item 46]

The therapeutic agent and/or prophylactic agent of any one of items 44 to 45, wherein $R^1$ is a hydrogen atom, or $C_{1-6}$ alkyl optionally substituted with 1 to 3 fluorine atoms.

[Item 47]

The therapeutic agent and/or prophylactic agent of any one of items 44 to 46, wherein $R^1$ is a methyl group.

[Item 48]

The therapeutic agent and/or prophylactic agent of any one of items 44 to 47, wherein $R^2$ is a hydrogen atom, a halogen atom, cyano, $-OR^3$, $C_{1-6}$ alkyl, $C_{3-10}$ cycloalkyl, or a 3- to 10-membered saturated heterocyclic group.

[Item 49]

The therapeutic agent and/or prophylactic agent of any one of items 44 to 46, wherein $R^2$ is cyano.

[Item 50]

The therapeutic agent and/or prophylactic agent of any one of items 44 to 49, wherein $R^8$, if there are multiple instances, are each independently a hydrogen atom, a halogen atom, cyano, $-OR^9$, $-CO_2R^{10}$, $-CONR^{11}R^{12}$, $-NR^{11}R^{12}$, $-NR^{13}COR^{10}$, $C_{1-6}$ alkyl (wherein the alkyl is optionally substituted with 1 to 3 of the same or different substituents selected from the group consisting of a fluorine atom, a chlorine atom, a bromine atom, a hydroxyl group, phenyl, 5- to 6-membered heteroaryl, $C_{1-6}$ alkoxy, $C_{3-7}$ cycloalkyl, a 3- to 7-membered saturated heterocyclic group, $-CONR^{16}R^{17}$, $-NR^{16}R^{17}$, and cyano), $C_{3-10}$ cycloalkyl (wherein the cycloalkyl is optionally substituted with 1 to 3 of the same or different substituents selected from the group consisting of a fluorine atom, a chlorine atom, a bromine atom, a hydroxyl group, phenyl, 5- to 6-membered heteroaryl, $C_{1-6}$ alkyl, $C_{1-6}$ alkoxy, $C_{3-7}$ cycloalkyl, a 3- to 7-membered saturated heterocyclic group, $-CONR^{16}R^{17}$, $-NR^{16}R^{17}$, and cyano), a 3- to 10-membered saturated heterocyclic group (wherein the saturated heterocyclic group is optionally substituted with 1 to 3 of the same or different substituents selected from the group consisting of a fluorine atom, a chlorine atom, a bromine atom, a hydroxyl group, phenyl, 5- to 6-membered heteroaryl, $C_{1-6}$ alkyl, $C_{1-6}$ alkoxy, $C_{3-7}$ cycloalkyl, a 3- to 7-membered saturated heterocyclic group, $-CONR^{16}R^{17}$, $- NR^{16}R^{17}$, and cyano), phenyl (wherein the phenyl is optionally substituted with 1 to 3 of the same or different substituents selected from the group consisting of a fluorine atom, a chlorine atom, a bromine atom, a hydroxyl group, phenyl, 5- to 6-membered heteroaryl, $C_{1-6}$ alkyl, $C_{1-6}$ alkoxy, $C_{3-7}$ cycloalkyl, a 3- to 7-membered saturated heterocyclic group, $-CONR^{16}R^{17}$, $- NR^{16}R^{17}$, and cyano), or 5- to 6-membered heteroaryl (wherein the heteroaryl is optionally substituted with 1 to 3 of the same or different substituents selected from the group consisting of a fluorine atom, a chlorine atom, a bromine atom, a hydroxyl group, phenyl, 5- to 6-membered heteroaryl, $C_{1-6}$ alkyl, $C_{1-6}$ alkoxy, $C_{3-7}$ cycloalkyl, a 3- to 7-membered saturated heterocyclic group, $-CONR^{16}R^{17}$, $-NR^{16}R^{17}$, and cyano) .

[Item 51]

The therapeutic agent and/or prophylactic agent of any one of items 44 to 50, wherein L is

a single bond, or
$C_{1-6}$ alkylene (wherein the alkylene is optionally substituted with 1 to 3 of the same or different substituents selected from the group consisting of a fluorine atom, a hydroxyl group, $C_{1-3}$ alkoxy, $-NR^{16}R^{17}$, and cyano) .

[Item 52]

The therapeutic agent and/or prophylactic agent of any one of items 44 to 51, wherein V is

a single bond,
$C_{3-10}$ cycloalkylene (wherein the cycloalkylene is optionally substituted with 1 to 3 of the same or different substituents selected from the group consisting of a fluorine atom, a hydroxyl group, $C_{1-3}$ alkyl, $C_{1-3}$ alkoxy, $-NR^{16}R^{17}$, and cyano), or
a 3- to 10-membered divalent saturated heterocyclic group (wherein the saturated heterocyclic group is optionally substituted with 1 to 3 of the same or different substituents selected from the group consisting of a fluorine atom, a hydroxyl group, $C_{1-3}$ alkyl optionally substituted with 1 to 2 hydroxyl groups or fluorine atoms, $C_{1-3}$ alkoxy, $-NR^{16}R^{17}$, and cyano).

[Item 53]

The therapeutic agent and/or prophylactic agent of any one of items 44 to 52, wherein W is

a single bond, or
$C_{1-6}$ alkylene (wherein the alkylene is optionally substituted with 1 to 3 of the same or different substituents selected from the group consisting of a fluorine atom, a hydroxyl group, $C_{1-3}$ alkoxy, $-NR^{16}R^{17}$, and cyano) .

[Item 54]

The therapeutic agent and/or prophylactic agent of any one of items 44 to 53, wherein $R^{14}$ is

a hydrogen atom,
$C_{1-6}$ alkyl (wherein the alkyl is optionally substituted with 1 to 3 of the same or different substituents selected from the group consisting of a fluorine atom, a hydroxyl group, $C_{1-3}$ alkoxy, $-NR^{16}R^{17}$, and cyano),
$C_{3-10}$ cycloalkyl (wherein the cycloalkyl is optionally substituted with 1 to 3 of the same or different substituents selected from the group consisting of a fluorine atom, a hydroxyl group, $C_{1-3}$ alkyl, $C_{1-3}$ alkoxy, $-NR^{16}R^{17}$, and cyano), or
a 3- to 10-membered saturated heterocyclic group (wherein the saturated heterocyclic group is optionally substituted with 1 to 3 of the same or different substituents selected from the group consisting of a fluorine atom, a hydroxyl group, $C_{1-3}$ alkyl, $C_{1-3}$ alkoxy, $-NR^{16}R^{17}$, and cyano).

[Item 55]

The therapeutic agent and/or prophylactic agent of item 44, wherein formula (1) is represented by formula (2):

[Chemical Formula 7]

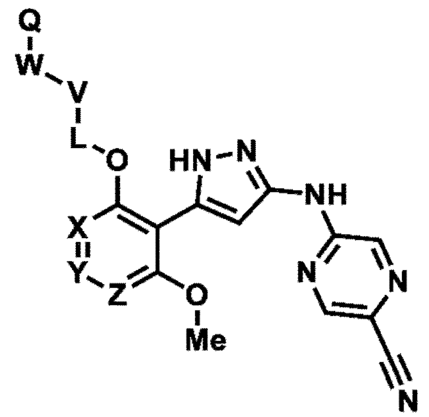

(2)

wherein

X, Y, and Z each independently represent $CR^8$ or a nitrogen atom, wherein X, Y, and Z are not simultaneously $CR^8$, $R^8$, if there are multiple instances, each independently represent

a hydrogen atom,
a fluorine atom,
a chlorine atom,
a bromine atom,
$C_{1-6}$ alkyl (wherein the alkyl is optionally substituted with 1 to 2 of the same or different substituents selected from the group consisting of a fluorine atom, a hydroxyl group, $C_{1-3}$ alkoxy, $-NR^{16}R^{17}$, and cyano), or
5- to 6-membered heteroaryl (wherein the heteroaryl is optionally substituted with 1 to 2 of the same or different substituents selected from the group consisting of a fluorine atom, a hydroxyl group, $C_{1-3}$ alkyl, $C_{1-3}$ alkoxy, $-NR^{16}R^{17}$, and cyano),

L represents

a single bond, or
$C_{1-6}$ alkylene (wherein the alkylene is optionally substituted with 1 to 3 of the same or different substituents selected from the group consisting of a fluorine atom, a hydroxyl group, $C_{1-3}$ alkoxy, $-NR^{16}R^{17}$, and cyano),

V represents

a single bond,
$C_{3-10}$ cycloalkylene (wherein the cycloalkylene is optionally substituted with 1 to 3 of the same or different substituents selected from the group consisting of a fluorine atom, a hydroxyl group, $C_{1-3}$ alkyl, $C_{1-3}$ alkoxy, $-NR^{16}R^{17}$, and cyano), or
a 3- to 10-membered divalent saturated heterocyclic group (wherein the saturated heterocyclic group is optionally substituted with 1 to 3 of the same or different substituents selected from the group consisting of a fluorine atom, a hydroxyl group, $C_{1-3}$ alkyl optionally substituted with 1 to 2 hydroxyl groups or fluorine atoms, $C_{1-3}$ alkoxy, $-NR^{16}R^{17}$, and cyano),

W represents

a single bond, or
$C_{1-6}$ alkylene (wherein the alkylene is optionally substituted with 1 to 3 of the same or different substituents selected from the group consisting of a fluorine atom, a hydroxyl group, $C_{1-3}$ alkoxy, $-NR^{16}R^{17}$, and cyano),

Q represents a hydrogen atom or $NHR^{14}$,
$R^{14}$ represents

a hydrogen atom,
$C_{1-6}$ alkyl (wherein the alkyl is optionally substituted with 1 to 3 of the same or different substituents selected from the group consisting of a fluorine atom, a hydroxyl group, $C_{1-3}$ alkoxy, $-NR^{16}R^{17}$, and cyano),
$C_{3-10}$ cycloalkyl (wherein the cycloalkyl is optionally substituted with 1 to 3 of the same or different substituents selected from the group consisting of a fluorine atom, a hydroxyl group, $C_{1-3}$ alkyl, $C_{1-3}$ alkoxy, $-NR^{16}R^{17}$, and cyano), or
a 3- to 10-membered saturated heterocyclic group (wherein the saturated heterocyclic group is optionally substituted with 1 to 3 of the same or different substituents selected from the group consisting of a fluorine atom, a hydroxyl group, $C_{1-3}$ alkyl, $C_{1-3}$ alkoxy, $-NR^{16}R^{17}$, and cyano), and

$R^{16}$ and $R^{17}$ each independently represent a hydrogen atom or $C_{1-6}$ alkyl, and if there are multiple instances of $R^{16}$ or $R^{17}$, each of $R^{16}$ or $R^{17}$ may be the same or different, wherein $R^{16}$ and $R^{17}$ that attach to the same nitrogen atom, when both are $C_{1-6}$ alkyl, together with the nitrogen atom to which each is attached, may form a 3- to 8-membered nitrogen-containing saturated heterocycle.

[Item 56]

The therapeutic agent and/or prophylactic agent of any one of items 44 to 55, wherein one or two of X, Y, and Z represents a nitrogen atom.

[Item 57]

The therapeutic agent and/or prophylactic agent of any one of items 44 to 56, wherein

X is a nitrogen atom, and
Y and Z are $CR^8$.

[Item 58]

The therapeutic agent and/or prophylactic agent of any one of items 44 to 56, wherein

Y is a nitrogen atom, and
X and Z are $CR^8$.

[Item 59]

The therapeutic agent and/or prophylactic agent of any one of items 44 to 56, wherein

Z is a nitrogen atom, and
X and Y are $CR^8$.

[Item 60]

The therapeutic agent and/or prophylactic agent of any one of items 44 to 59, wherein $R^8$, if there are multiple instances, are each independently

a hydrogen atom,
a fluorine atom,
a chlorine atom,
a bromine atom, or
$C_{1-6}$ alkyl (wherein the alkyl is optionally substituted with 1 to 2 of the same or different substituents selected from the group consisting of a fluorine atom, a hydroxyl group, and $C_{1-3}$ alkoxy) .

[Item 61]

The therapeutic agent and/or prophylactic agent of any one of items 44 to 60, wherein L is

a single bond, or

$C_{1-6}$ alkylene (wherein the alkylene is optionally substituted with 1 to 2 of the same or different substituents selected from the group consisting of a fluorine atom, a hydroxyl group, and cyano).

[Item 62]

The therapeutic agent and/or prophylactic agent of any one of items 44 to 61, wherein V is

a single bond,
$C_{3-7}$ cycloalkylene (wherein the cycloalkylene is optionally substituted with 1 to 2 of the same or different substituents selected from the group consisting of a fluorine atom, a hydroxyl group, $C_{1-3}$ alkyl optionally substituted with 1 to 2 hydroxyl groups, and cyano), or
a 3- to 7-membered divalent saturated heterocyclic group (wherein the saturated heterocyclic group is optionally substituted with 1 to 2 of the same or different substituents selected from the group consisting of a fluorine atom, a hydroxyl group, $C_{1-3}$ alkyl optionally substituted with 1 to 2 hydroxyl groups or fluorine atoms, and cyano).

[Item 63]

The therapeutic agent and/or prophylactic agent of any one of items 44 to 62, wherein W is

a single bond, or
$C_{1-6}$ alkylene (wherein the alkylene is optionally substituted with 1 to 2 of the same or different substituents selected from the group consisting of a fluorine atom, a hydroxyl group, and cyano).

[Item 64]

The therapeutic agent and/or prophylactic agent of any one of items 44 to 63, wherein $R^{14}$ is

a hydrogen atom, or
$C_{1-6}$ alkyl (wherein the alkyl is optionally substituted with 1 to 2 of the same or different substituents selected from the group consisting of a fluorine atom, a hydroxyl group, and cyano) .

[Item 65]

The therapeutic agent and/or prophylactic agent of any one of items 44 to 64, wherein Q is

a hydrogen atom,
$NH_2$, or
NHMe.

[Item 66]

The therapeutic agent and/or prophylactic agent of item 44, wherein formula (1) is represented by formula (3):

[Chemical Formula 8]

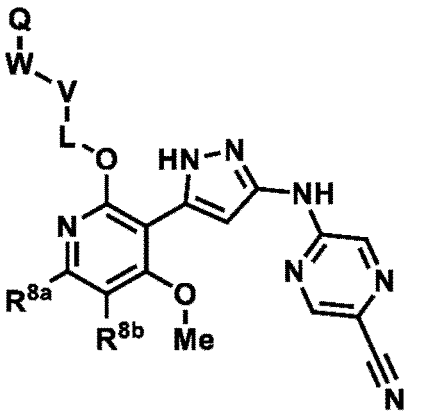

(3)

wherein,

$R^{8a}$ and $R^{8b}$ each independently represent a hydrogen atom, a fluorine atom, a chlorine atom, a bromine atom, or $C_{1-3}$ alkyl,
L represents a single bond or $C_{1-6}$ alkylene optionally substituted with 1 hydroxyl group,
V represents

a single bond,
$C_{3-7}$ cycloalkylene (wherein the cycloalkylene is optionally substituted with 1 substituent selected from the group consisting of a hydroxyl group and $C_{1-3}$ alkyl), or
a 3- to 7-membered divalent saturated heterocyclic group (wherein the saturated heterocyclic group is optionally substituted with 1 substituent selected from the group consisting of a fluorine atom, cyano, a hydroxyl group, and $C_{1-3}$ alkyl),

W represents

a single bond, or
$C_{1-3}$ alkylene optionally substituted with 1 hydroxyl group, and

Q is a hydrogen atom or $NH_2$.

[Item 67]

The therapeutic agent and/or prophylactic agent of item 66, wherein $R^{8a}$ and $R^{8b}$ are each independently a hydrogen atom, a fluorine atom or a chlorine atom.

[Item 68]

The therapeutic agent and/or prophylactic agent of item 66, wherein $R^{8a}$ and $R^{8b}$ are each independently a hydrogen atom, or a chlorine atom.

[Item 69]

The therapeutic agent and/or prophylactic agent of any one of items 66 to 68, wherein L is $C_{1-3}$ alkylene.

[Item 70]

The therapeutic agent and/or prophylactic agent of any one of items 66 to 69, wherein V is a single bond.

[Item 71]

The therapeutic agent and/or prophylactic agent of any one of items 66 to 70, wherein V is $C_{3-7}$ cycloalkylene.

[Item 72]

The therapeutic agent and/or prophylactic agent of any one of items 66 to 71, wherein W is a single bond.

[Item 73]

The therapeutic agent and/or prophylactic agent of any one of items 66 to 71, wherein W is $C_{1-3}$ alkylene.

[Item 74]

The therapeutic agent and/or prophylactic agent of any one of items 66 to 73, wherein Q is $NH_2$.

[Item 75]

The therapeutic agent and/or prophylactic agent of item 44, wherein formula (1) is represented by formula (4):

[Chemical Formula 9]

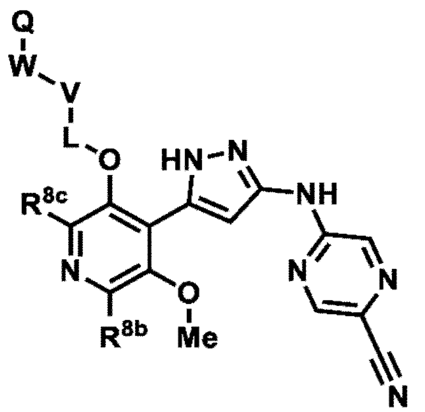

(4)

wherein

$R^{8b}$ and $R^{8c}$ each independently represent a hydrogen atom, a fluorine atom, a chlorine atom, a bromine atom, or $C_{1-3}$ alkyl,
L represents

a single bond, or
$C_{1-6}$ alkylene (wherein the alkylene is optionally substituted with 1 substituent selected from the group consisting of a fluorine atom, a hydroxyl group, and cyano),

V represents

a single bond,
$C_{3-7}$ cycloalkylene (wherein the cycloalkylene is optionally substituted with 1 substituent selected from the group consisting of a hydroxyl group and $C_{1-3}$ alkyl), or
a 3- to 7-membered divalent saturated heterocyclic group (wherein the saturated heterocyclic group is optionally substituted with 1 substituent selected from the group consisting of a fluorine atom, cyano, a hydroxyl group, and $C_{1-3}$ alkyl optionally substituted with 1 to 3 hydroxyl groups and fluorine atoms),

W represents

a single bond, or
$C_{1-3}$ alkylene optionally substituted with 1 hydroxyl group, and

Q is a hydrogen atom, $NH_2$, or NHMe.

[Item 76]

The therapeutic agent and/or prophylactic agent of item 75, wherein $R^{8b}$ and $R^{8c}$ are hydrogen atoms.

[Item 77]

The therapeutic agent and/or prophylactic agent of any one of item 75 or 76, wherein L is

a single bond, or
$C_{1-6}$ alkylene optionally substituted with 1 hydroxyl group or 1 fluorine atom.

[Item 78]

The therapeutic agent and/or prophylactic agent of any one of items 75 to 77, wherein V is

a single bond,
$C_{3-7}$ cycloalkylene, or
a 3- to 7-membered divalent saturated heterocyclic group (wherein the saturated heterocyclic group is optionally substituted with 1 substituent selected from the group consisting of a fluorine atom, a hydroxyl group, and $C_{1-3}$ alkyl).

[Item 79]

The therapeutic agent and/or prophylactic agent of any one of items 75 to 78, wherein W is

a single bond, or
$C_{1-3}$ alkylene.

[Item 80]

The therapeutic agent and/or prophylactic agent of any one of items 75 to 79, wherein Q is a hydrogen atom.

[Item 81]

The therapeutic agent and/or prophylactic agent of any one of items 75 to 79, wherein Q is $NH_2$.

[Item 82]

The therapeutic agent and/or prophylactic agent of any one of items 75 to 79, wherein Q is NHMe.

[Item 83]

The therapeutic agent and/or prophylactic agent of item 44, wherein formula (1) is represented by formula (5):

[Chemical Formula 10]

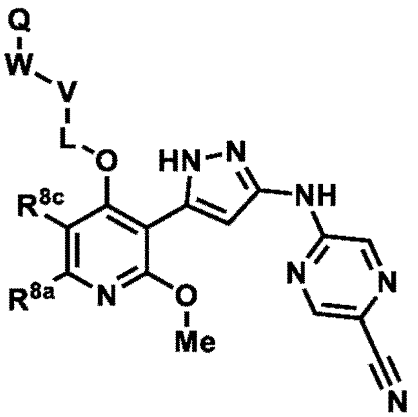

(5)

wherein

$R^{8a}$ and $R^{8c}$ each independently represent a hydrogen atom, a fluorine atom, a chlorine atom, a bromine atom, or $C_{1-3}$ alkyl,
L represents a single bond, or $C_{1-6}$ alkylene optionally substituted with 1 hydroxyl group,
V represents

a single bond,
$C_{3-7}$ cycloalkylene (wherein the cycloalkylene is optionally substituted with 1 substituent selected from the group consisting of a hydroxyl group and $C_{1-3}$ alkyl), or
a 3- to 7-membered divalent saturated heterocyclic group (wherein the saturated heterocyclic group is optionally substituted with 1 substituent selected from the group consisting of a fluorine atom, cyano, a hydroxyl group, and $C_{1-3}$ alkyl optionally substituted with 1 hydroxyl group),

W represents

a single bond, or
$C_{1-3}$ alkylene optionally substituted with 1 hydroxyl group, and

Q is a hydrogen atom or $NH_2$.

[Item 84]

The therapeutic agent and/or prophylactic agent of item 83, wherein $R^{8a}$ and $R^{8c}$ are hydrogen atoms.

[Item 85]

The therapeutic agent and/or prophylactic agent of any one of item 83 or 84, wherein L is $C_{1-6}$ alkylene optionally substituted with 1 hydroxyl group.

[Item 86]

The therapeutic agent and/or prophylactic agent of any one of items 83 to 85, wherein V is

a single bond,
$C_{3-7}$ cycloalkylene, or
a 3- to 7-membered divalent saturated heterocyclic group (wherein the saturated heterocyclic group is optionally substituted with 1 substituent selected from the group consisting of a hydroxyl group and $C_{1-3}$ alkyl optionally substituted with 1 hydroxyl group).

[Item 87]

The therapeutic agent and/or prophylactic agent of any one of items 83 to 86, herein W is

a single bond, or
$C_{1-3}$ alkylene.

[Item 88]

The therapeutic agent and/or prophylactic agent of any one of items 83 to 87, wherein Q is a hydrogen atom.

[Item 89]

The therapeutic agent and/or prophylactic agent of any one of items 83 to 87, wherein Q is $NH_2$.

[Item 90]

The therapeutic agent and/or prophylactic agent of item 44, wherein formula (1) is represented by formula (6):

[Chemical Formula 11]

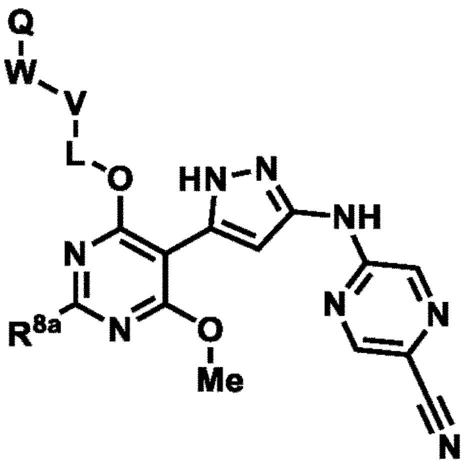

(6)

wherein

$R^{8a}$ represents a hydrogen atom, a fluorine atom, a chlorine atom, a bromine atom, or $C_{1-3}$ alkyl,
L represents

a single bond, or
$C_{1-6}$ alkylene (wherein the alkylene is optionally substituted with 1 substituent selected from the group consisting of a fluorine atom, a hydroxyl group, and cyano),

V represents

a single bond,
$C_{3-7}$ cycloalkylene (wherein the cycloalkylene is optionally substituted with 1 substituent selected from the group consisting of a hydroxyl group and $C_{1-3}$ alkyl), or
a 3- to 7-membered divalent saturated heterocyclic group (wherein the saturated heterocyclic group is optionally substituted with 1 substituent selected from the group consisting of a fluorine atom, cyano, a hydroxyl group, and $C_{1-3}$ alkyl optionally substituted with 1 to 3 fluorine atoms),

W represents

a single bond, or

$C_{1-3}$ alkylene optionally substituted with 1 hydroxyl group, and

Q is a hydrogen atom, $NH_2$, or NHMe.

[Item 91]

The therapeutic agent and/or prophylactic agent of item 90, wherein $R^{8a}$ is a hydrogen atom.

[Item 92]

The therapeutic agent and/or prophylactic agent of any one of item 90 or 91, wherein L is $C_{1-4}$ alkylene.

[Item 93]

The therapeutic agent and/or prophylactic agent of any one of items 90 to 92, wherein V is

a single bond, or
$C_{3-7}$ cycloalkylene.

[Item 94]

The therapeutic agent and/or prophylactic agent of any one of items 90 to 93, wherein W is

a single bond, or
$C_{1-3}$ alkylene.

[Item 95]

The therapeutic agent and/or prophylactic agent of any one of items 90 to 94, wherein Q is $NH_2$.

[Item 96]

The therapeutic agent and/or prophylactic agent of item 44, comprising a compound selected from the following compounds, or a pharmaceutically acceptable salt thereof:

5-({5-[2-(3-aminopropoxy)-4-methoxypyridin-3-yl]-1H-pyrazol-3-yl}amino)pyrazine-2-carbonitrile,
5-({5-[2-(3-aminopropoxy)-6-chloro-4-methoxypyridin-3-yl]-1H-pyrazol-3-yl}amino)pyrazine-2-carbonitrile,
5-({5-[3-(3-aminopropoxy)-5-methoxypyridin-4-yl]-1H-pyrazol-3-yl}amino)pyrazine-2-carbonitrile,
5-({5-[4-(3-aminopropoxy)-2-methoxypyridin-3-yl]-1H-pyrazol-3-yl}amino)pyrazine-2-carbonitrile,
5-[(5-{3-[(3-fluoroazetidin-3-yl)methoxy]-5-methoxypyridin-4-yl}-1H-pyrazol-3-yl)amino]pyrazine-2-carbonitrile,
5-[(5-{2-methoxy-4-[(3-methylazetidin-3-yl)methoxy]pyridin-3-yl}-1H-pyrazol-3-yl)amino]pyrazine-2-carbonitrile,
5-[(5-{4-[(3-hydroxyazetidin-3-yl)methoxy]-2-methoxypyridin-3-yl}-1H-pyrazol-3-yl)amino]pyrazine-2-carbonitrile,
5-{[5-(4-{[3-(hydroxymethyl)azetidin-3-yl]methoxy}-2-methoxypyridin-3-yl)-1H-pyrazol-3-yl]amino}pyrazine-2-carbonitrile,
5-[(5-{3-[(3R)-3-aminobutoxy]-5-methoxypyridin-4-yl}-1H-pyrazol-3-yl)amino]pyrazine-2-carbonitrile,
5-[(5-{3-[(3S)-3-aminobutoxy]-5-methoxypyridin-4-yl}-1H-pyrazol-3-yl)amino]pyrazine-2-carbonitrile,
5-{[5-(3-{[1-(aminomethyl)cyclopropyl]methoxy}-5-methoxypyridin-4-yl)-1H-pyrazol-3-yl]amino}pyrazine-2-carbonitrile,
5-[(5-{3-methoxy-5-[(morpholin-2-yl)methoxy]pyridin-4-yl}-1H-pyrazol-3-yl)amino]pyrazine-2-carbonitrile,
5-[(5-{3-methoxy-5-[(morpholin-2-yl)methoxy]pyridin-4-yl}-1H-pyrazol-3-yl)amino]pyrazine-2-carbonitrile,
5-[(5-{3-[(2S)-3-amino-2-hydroxypropoxy]-5-methoxypyridin-4-yl}-1H-pyrazol-3-yl)amino]pyrazine-2-carbonitrile,
N-{5-[2-(3-aminopropoxy)-4-methoxypyridin-3-yl]-1H-pyrazol-3-yl}-5-chloropyrazin-2-amine,
N-{5-[2-(3-aminopropoxy)-4-methoxypyridin-3-yl]-1H-pyrazol-3-yl}-5-(trifluoromethyl)pyrazin-2-amine,
5-({5-[4-(3-aminopropoxy)-6-methoxypyrimidin-5-yl]-1H-pyrazol-3-yl}amino)pyrazine-2-carbonitrile,
5-({5-[3-(azetidin-3-yl)methoxy-5-methoxypyridin-4-yl]-1H-pyrazol-3-yl}amino)pyrazine-2-carbonitrile,
5-{[5-(3-{[(1R,3S)-3-aminocyclohexyl]oxy}-5-methoxypyridin-4-yl)-1H-pyrazol-3-yl]amino}pyrazine-2-carbonitrile,
(S)-5-[(5-{3-[(3-fluoropyrrolidin-3-yl)methoxy]-5-methoxypyridin-4-yl}-1H-pyrazol-3-yl)amino]pyrazine-2-carbonitrile,

(S)-5-[(5-{3-methoxy-[5-(pyrrolidin-3-yl)methoxy]pyridin-4-yl}-1H-pyrazol-3-yl)amino]pyrazine-2-carbonitrile,
(R)-5-[(5-{3-[(3-fluoropyrrolidin-3-yl)methoxy]-5-methoxypyridin-4-yl}-1H-pyrazol-3-yl)amino]pyrazine-2-carbonitrile,
5-{[5-(4-{[1-(aminomethyl)cyclopropyl]methoxy}-6-methoxypyrimidin-5-yl)-1H-pyrazol-3-yl]amino}pyrazine-2-carbonitrile,
5-({5-[3-(3-aminopropoxy)-5-(fluoromethoxy)pyridin-4-yl]-1H-pyrazol-3-yl}amino)pyrazine-2-carbonitrile,
5-[(5-{3-methoxy-5-[(3-methylazetidin-3-yl)methoxy]pyridin-4-yl}-1H-pyrazol-3-yl)amino]pyrazine-2-carbonitrile,
5-{[5-(3-{[3-(difluoromethyl)azetidin-3-yl]methoxy}-5-methoxypyridin-4-yl)-1H-pyrazol-3-yl]amino}pyrazine-2-carbonitrile,
5-[(5-{3-[(2S)-3-amino-2-methoxypropoxy]-5-methoxypyridin-4-yl}-1H-pyrazol-3-yl)amino]pyrazine-2-carbonitrile,
5-[(5-{3-[(2R)-3-amino-2-methoxypropoxy]-5-methoxypyridin-4-yl}-1H-pyrazol-3-yl)amino]pyrazine-2-carbonitrile,
5-[(5-{3-[(2S)-3-amino-2-fluoropropoxy]-5-methoxypyridin-4-yl}-1H-pyrazol-3-yl)amino]pyrazine-2-carbonitrile,
5-[(5-{3-[(2R)-3-amino-2-fluoropropoxy]-5-methoxypyridin-4-yl}-1H-pyrazol-3-yl)amino]pyrazine-2-carbonitrile,
5-[(5-{3-methoxy-5-[3-(methylamino)propoxy]pyridin-4-yl}-1H-pyrazol-3-yl)amino]pyrazine-2-carbonitrile,
5-{[5-(3-{[(1R,3R)-3-aminocyclopentyl]oxy}-5-methoxypyridin-4-yl)-1H-pyrazol-3-yl]amino}pyrazine-2-carbonitrile,
5-[(5-{3-[(1R)-1-(azetidin-3-yl)ethoxy]-5-methoxypyridin-4-yl}-1H-pyrazol-3-yl)amino]pyrazine-2-carbonitrile,
5-[(5-{3-[(1R)-1-(3-hydroxyazetidin-3-yl)ethoxy]-5-methoxypyridin-4-yl}-1H-pyrazol-3-yl)amino]pyrazine-2-carbonitrile,
5-{[5-(3-methoxy-5-{[(1R,3R)-3-(methylamino)cyclopentyl]oxy}pyridin-4-yl)-1H-pyrazol-3-yl]amino}pyrazine-2-carbonitrile,
5-{[5-(3-{[(1R,2S,4S,5S)-4-aminobicyclo[3.1.0]hexan-2-yl]oxy}-5-methoxypyridin-4-yl)-1H-pyrazol-3-yl]amino}pyrazine-2-carbonitrile,
5-{[5-(2-{[1-(aminomethyl)cyclopropyl]methoxy}-4-methoxypyridin-3-yl)-1H-pyrazol-3-yl]amino}pyrazine-2-carbonitrile, and
5-{[5-(4-{[1-(aminomethyl)cyclopropyl]methoxy}-2-methoxypyridin-3-yl)-1H-pyrazol-3-yl]amino}pyrazine-2-carbonitrile.

[Item 97]

The therapeutic agent and/or prophylactic agent of item 44, comprising a compound selected from the following compounds, or a pharmaceutically acceptable salt thereof:

5-({5-[2-(3-aminopropoxy)-4-methoxypyridin-3-yl]-1H-pyrazol-3-yl}amino)pyrazine-2-carbonitrile,
5-({5-[3-(3-aminopropoxy)-5-methoxypyridin-4-yl]-1H-pyrazol-3-yl}amino)pyrazine-2-carbonitrile, and
5-({5-[4-(3-aminopropoxy)-2-methoxypyridin-3-yl]-1H-pyrazol-3-yl}amino)pyrazine-2-carbonitrile.

[Item 98]

The therapeutic agent and/or prophylactic agent of item 44, comprising a compound selected from the following compound, or a pharmaceutically acceptable salt thereof:
5-({5-[4-(3-aminopropoxy)-2-methoxypyridin-3-yl]-1H-pyrazol-3-yl}amino)pyrazine-2-carbonitrile.

[Item 99]

The therapeutic agent and/or prophylactic agent of item 44, comprising the following compound or a pharmaceutically acceptable salt thereof:
5-({5-[2-(3-aminopropoxy)-4-methoxypyridin-3-yl]-1H-pyrazol-3-yl}amino)pyrazine-2-carbonitrile.

[Item 100]

The therapeutic agent and/or prophylactic agent of item 44, comprising the following compound or a pharmaceutically acceptable salt thereof:
5-({5-[3-(3-aminopropoxy)-5-methoxypyridin-4-yl]-1H-pyrazol-3-yl}amino)pyrazine-2-carbonitrile.

[Effect of the Invention]

According to the present disclosure, there is provided a therapeutic agent and/or prophylactic agent for a cancer patient exhibiting therapeutic resistance to an immune checkpoint inhibitor, comprising, as an active ingredient, a CHK1

inhibitor or a liposome encapsulating a CHK1 inhibitor. Furthermore, in a case where an immune checkpoint inhibitor is used concomitantly with the inhibitor or liposome, an excellent antitumor effect is exhibited as compared with a single agent. In addition, the inhibitor, the liposome, or combined use of them with an immune checkpoint inhibitor also exhibits an effect of preventing recurrence of cancer after remission.

The present disclosure provides a CHK1 inhibitor comprising a 5-heteroaryl-1H-pyrazol-3-amine derivative or a pharmaceutically acceptable salt thereof. The compound of the present disclosure has both an excellent CHK1 inhibitory activity as well as high safety. The compound is encapsulated in a liposome and is controllably released from the liposome to sustainably act on cancer to exhibit a potent antitumor effect. The compound of the present disclosure is useful as a therapeutic drug against CHK1-related diseases. Specifically, the compound can be applied to patients with pancreatic cancer, ovarian cancer, osteosarcoma, Ewing sarcoma, chondrosarcoma, soft tissue sarcoma, or the like.

The present disclosure elucidated that compounds which exhibited a pharmacological action on ovarian cancer patients in Phase 2 trials did not achieve maximization of efficacy from 72 hours of sustained exposure expected from a non-clinical trial relative to a 5-phenyl-1H-pyrazol-3-amine derivative prexasertib, whereas the compound of the present disclosure or a salt thereof can be a further improvement thereof. In comparison to prexasertib that results in a side effect due to a high maximum blood concentration, the compound of the present disclosure and a salt thereof did not have such a side effect in a single administration. While a side effect due to drug exposure for an extended period was observed, such a side effect was not observed with the compound of the present disclosure or a salt thereof in 3-day continuous administration (Non Patent Literature 3, Non Patent Literature 6). In addition, the inventors have newly found that prexasertib has a hepatotoxic risk, while the compound of present application has a reduced hepatotoxic risk. In view of the above, the compound of the present disclosure has both an excellent CHK1 inhibitory activity and high safety.

In particular, a compound having a pyridine ring with a nitrogen atom at a specific position represented by formula (3) has a characteristic of having a high antitumor activity as well as safety while also having excellent pharmacokinetics.

In particular, the compound of Example 1 encompassed by formula (3) has characteristics of exhibiting a higher CHK1 inhibitory activity and having a more potent cell growth suppression effect as well as higher safety, compared to prexasertib. The compound of Example 1 also has characteristics of exhibiting excellent pharmacokinetics as well as efficient encapsulation in a liposome. A liposome formulation of the compound of Example 1 has an excellent antitumor effect as well as high safety, and exerts an even more significant antitumor effect when used concomitantly with an existing anticancer agent.

[Brief Description of Drawings]

[Figure 1] Figure 1 shows the X ray powder diffraction pattern of form I of a compound of Example 39. The horizontal axis indicates diffraction angle 2θ(°), and the vertical axis indicates the count (the same applies to Figures 2 to 6 hereinafter).

[Figure 2] Figure 2 shows the X ray powder diffraction pattern of form II of a compound of Example 40.

[Figure 3] Figure 3 shows the X ray powder diffraction pattern of form III of a compound of Example 41.

[Figure 4] Figure 4 shows the X ray powder diffraction pattern of form IV of a compound of Example 42.

[Figure 5] Figure 5 shows the X ray powder diffraction pattern of form V of a compound of Example 43.

[Figure 6] Figure 6 shows the X ray powder diffraction pattern of form VI of a compound of Example 44.

[Figure 7] Figure 7 is a diagram showing tumor PD responses using ES-2 bearing mice for Test A of Test Example 11, prexasertib solution formulation or liposome formulation. PD responses were evaluated by detecting the expression levels of γH2AX and Tubulin by Western blotting, quantifying each band intensity with ImageJ software, and computing the relative value of the expression level of γH2AX with the expression level of Tubulin. The vertical axis indicates the relative value of γH2AX.

[Figure 8] Figure 8 is a diagram showing antitumor PD responses using ES-2 bearing mice for Test B of Test Example 11, prexasertib solution formulation or liposome formulation of Example 1. PD responses were evaluated by detecting the expression levels of γH2AX and Tubulin by Western blotting, quantifying each band intensity with ImageJ software, and computing the relative value of the expression level of γH2AX with the expression level of Tubulin. The vertical axis indicates the relative value of γH2AX.

[Figure 9] Figure 9 is a diagram showing antitumor PD responses using ES-2 bearing mice for Test C of Test Example 11 or liposome formulation of Example 1. PD responses were evaluated by detecting the expression levels of γH2AX and Tubulin by Western blotting, quantifying each band intensity with ImageJ software, and computing the relative value of the expression level of γH2AX with the expression level of Tubulin. The vertical axis indicates the relative value of γH2AX.

[Figure 10] Figure 10 is a diagram showing an example of an administration regimen of an anti-PD-1 antibody and a liposome formulation of Example 1. The liposome formulation of Example 1 is administered simultaneously with an

anti-PD-1 antibody, and then administered at 1 week/time (A1), 2 weeks/time (A2), 3 weeks/time (A3), 4 weeks/time (A4), or 5 weeks/time (A5). The anti-PD-1 antibody is administered simultaneously with Example 1, and then administered at 2 weeks/time (B1), 3 weeks/time (B2), 4 weeks/time (B3), or 6 weeks/time (B4) according to an individual regimen. That is, Example 1 and the anti-PD-1 antibody are administered according to an administration regimen selected by any combination thereof. Preferably, examples of the combination of the administration regimens include A1 and B1, A1 and B2, A1 and B3, A1 and B4, A1 and B5, A2 and B1, A2 and B2, A2 and B3, A2 and B4, A2 and B5, A3 and B1, A3 and B2, A3 and B3, A3 and B4, A3 and B5, A4 and B1, A4 and B2, A4 and B3, A4 and B4, A4 and B5, A5 and B1, A5 and B2, A5 and B3, A5 and B4, and A5 and B5.

[Figure 11] Figure 11 is a diagram showing the number of cells in each level of clones of CD8 positive T cells and TCR repertoire clonotype in the liposome formulation of Example 1 in the test of Test Example 28.

[Figure 12] Figure 12 is a diagram showing CD8 score of the liposome formulation of Example 1 in the test of Test Example 28.

[Figure 13] Figure 13 is a diagram showing M1-M2 ratio of the liposome formulation of Example 1 in the test of Test Example 28.

[Figure 14] Figure 14 is a diagram showing the expression levels of ssDNA by the treatment of Example 1 in the test of Test Example 34.

[Description of Embodiments]

The present disclosure is described hereinafter in more detail. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. The terms used herein should also be understood as being used in the meaning that is commonly used in the art, unless specifically noted otherwise. Thus, unless defined otherwise, all terminologies and scientific technical terms that are used herein have the same meaning as the general understanding of those skilled in the art to which the present disclosure pertains. In case of a contradiction, the present specification (including the definitions) takes precedence.

The terms that are used herein are described hereinafter.

As used herein, the number of substituents in a group defined as "optionally substituted" is not particularly limited, as long as they are substitutable. The description of each group is also applicable when the group is a substituent or a part of another group, unless specifically noted otherwise.

Examples of a "halogen atom" include a fluorine atom, a chlorine atom, a bromine atom, an iodine atom, and the like. A halogen atom is preferably a fluorine atom or a chlorine atom.

"$C_{1-6}$ alkyl" refers to alkyl with 1 to 6 carbon atoms, and "$C_6$ alkyl" refers to alkyl with 6 carbon atoms. The same applies to other numbers.

"$C_{1-6}$ alkyl" refers to a linear or branched saturated hydrocarbon group with 1 to 6 carbon atoms. $C_{1-6}$ alkyl is preferably "$C_{1-4}$ alkyl", and more preferably "$C_{1-3}$ alkyl". Specific examples of "$C_{1-3}$ alkyl" include methyl, ethyl, propyl, *1*-methylethyl, and the like. Specific examples of "$C_{1-4}$ alkyl" include, in addition to the specific examples for the "$C_{1-3}$ alkyl" described above, butyl, 1,1-dimethylethyl, 1-methylpropyl, 2-methylpropyl, and the like. Specific examples of "$C_{1-6}$ alkyl" include, in addition to the specific examples for the "$C_{1-4}$ alkyl" described above, pentyl, 1,1-dimethylpropyl, 1,2-dimethylpropyl, 1-methylbutyl, 2-methylbutyl, 4-methylpentyl, 3-methylpentyl, 2-methylpentyl, 1-methylpentyl, hexyl, and the like.

"$C_{2-6}$ alkenyl" refers to a linear or branched unsaturated hydrocarbon group with 2 to 6 carbon atoms, comprising 1 to 3 double bonds. Preferred examples of "$C_{2-6}$ alkenyl" include "$C_{2-4}$ alkenyl". Specific examples of "$C_{2-4}$ alkenyl" include vinyl, propenyl, methylpropenyl, butenyl, and the like. Specific examples of "$C_{2-6}$ alkenyl" include, in addition to the specific examples for the "$C_{2-4}$ alkenyl" described above, pentenyl, hexenyl, and the like.

"$C_{2-6}$ alkynyl" refers to a linear or branched unsaturated hydrocarbon group with 2 to 6 carbon atoms, comprising one triple bonds. Preferred examples of "$C_{2-6}$ alkynyl" include "$C_{2-4}$ alkynyl". Specific examples of "$C_{2-4}$ alkynyl" include, in addition to the specific examples for the "$C_{2-4}$ alkynyl" described above, propynyl, methylpropynyl, butynyl, and the like. Specific examples of "$C_{2-6}$ alkynyl" include methylbutynyl, pentynyl, hexynyl, and the like.

"$C_{1-6}$ alkoxy" is "$C_{1-6}$ alkyloxy", and the "$C_{1-6}$ alkyl" moiety is defined the same as the "$C_{1-6}$ alkyl". "$C_{1-6}$ alkoxy" is preferably "$C_{1-4}$ alkoxy", and more preferably "$C_{1-3}$ alkoxy". Specific examples of "$C_{1-3}$ alkoxy" include methoxy, ethoxy, propoxy, 1-methylethoxy, and the like. Specific examples of "$C_{1-4}$ alkoxy" include, in addition to the specific examples for the "$C_{1-3}$ alkoxy" described above, butoxy, 1,1-dimethylethoxy, 1-methylpropoxy, 2-methylpropoxy, and the like. Specific examples of "$C_{1-6}$ alkoxy" include, in addition to the specific examples for the "$C_{1-4}$ alkoxy" described above, pentyloxy, 1,1-dimethylpropoxy, 1,2-dimethylpropoxy, 1-methylbutoxy, 2-methylbutoxy, 4-methylpentyloxy, 3-methylpentyloxy, 2-methylpentyloxy, 1-methylpentyloxy, hexyloxy, and the like.

The "$C_{1-6}$ alkyl" moiety of "$C_{1-6}$ alkylthio" is defined by the same as the "$C_{1-6}$ alkyl". "$C_{1-6}$ alkylthio" is preferably

"$C_{1-4}$ alkylthio", and more preferably "$C_{1-3}$ alkylthio". Specific examples of "$C_{1-3}$ alkylthio" include methylthio, ethylthio, propylthio, 1-methylethylthio, and the like. Specific examples of "$C_{1-4}$ alkylthio" include, in addition to the specific examples for the "$C_{1-3}$ alkylthio" described above, butylthio, 1,1-dimethylethylthio, 1-methylpropylthio, 2-methylpropylthio, and the like. Specific examples of "$C_{1-6}$ alkylthio" include, in addition to the specific examples for the "$C_{1-4}$ alkylthio" described above, pentylthio, 1,1-dimethylpropylthio, 1,2-dimethylpropylthio, 1-methylbutylthio, 2-methylbutylthio, 4-methylpentylthio, 3-methylpentylthio, 2-methylpentylthio, 1-methylpentylthio, hexylthio, and the like.

"$C_{1-6}$ alkylene" refers to a linear or branched divalent saturated hydrocarbon group with 1 to 6 carbon atoms. "$C_{1-6}$ alkylene" is preferably "$C_{1-4}$ alkylene", and more preferably "$C_{1-3}$ alkylene". Specific examples of "$C_{1-3}$ alkylene" include methylene, ethylene, propylene, trimethylene, and the like. Specific examples of "$C_{1-4}$ alkylene" include, in addition to the specific examples for the "$C_{1-3}$ alkylene" described above, butylene, 1,1-dimethylethylene, 1,2-dimethylethylene, 1-methyltrimethylene, 2-methyltrimethylene, and the like. Specific examples of "$C_{1-6}$ alkylene" include, in addition to the specific examples for the "$C_{1-4}$ alkylene" described above, pentylene, 1,1-dimethyltrimethylene, 1,2-dimethyltrimethylene, 1-methylbutylene, 2-methylbutylene, 1-methylpentylene, 2-methylpentylene, 3-methylpentylene, hexylene, and the like.

"$C_{3-10}$ cycloalkyl" refers to a cyclic saturated hydrocarbon group with 3 to 10 carbon atoms, including those with a partially unsaturated bond and those with a crosslinked structure. "$C_{3-10}$ cycloalkyl" is preferably "$C_{3-7}$ cycloalkyl". Specific examples of "$C_{3-7}$ cycloalkyl" include cyclopropyl, cyclobutyl, cyclopentyl, cyclohexyl, cycloheptyl, and the like. Specific examples of "$C_{3-10}$ cycloalkyl" include, in addition to the specific examples for the "$C_{3-7}$ cycloalkyl" described above, cyclooctyl, cyclononyl, cyclodecyl, adamantyl, and the like.

"$C_{3-10}$ cycloalkyl" encompasses those that are bicyclic from fusing the $C_{3-10}$ cycloalkyl described above with an aromatic hydrocarbon ring. Specific examples of such a fused compound include the structure represented below and the like.

[Chemical Formula 12]

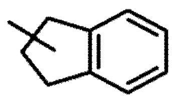

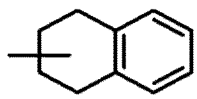

Specific examples of the crosslinked structure described above include the structure represented below and the like.

[Chemical Formula 13]

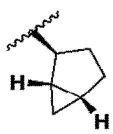

"$C_{3-10}$ cycloalkylene" refers to a cyclic divalent saturated hydrocarbon group with 3 to 10 carbon atoms, including those that have a partially unsaturated bond and those with a crosslinked structure. "$C_{3-10}$ cycloalkylene" is preferably "$C_{3-7}$ cycloalkylene". Specific examples of "$C_{3-7}$ cycloalkylene" include cyclopropylene, cyclobutylene, cyclopentylene, cyclohexylene, cycloheptylene, and the like. Specific examples of "$C_{3-10}$ cycloalkyl" include, in addition to the specific examples for the "$C_{3-7}$ cycloalkyl" described above, cyclooctylene, cyclononylene, cyclodecylene, adamantylene, and the like.

Specific examples of the crosslinked structure described above include the structure represented below and the like.

[Chemical Formula 14]

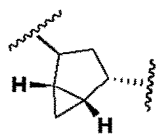

"3- to 10-membered saturated carbocyclic ring" refers to a cyclic saturated hydrocarbon with 3 to 10 carbon atoms. "3-to 10-membered saturated carbocyclic ring" is preferably "4- to 6-membered saturated carbocyclic ring". Specific examples of "4- to 6-membered saturated carbocyclic ring" include a cyclobutane ring, cyclopentane ring, cyclohexane ring, and the like. Specific examples of "3- to 10-membered saturated carbocyclic ring" include, in addition to the specific examples for the "4- to 6-membered saturated carbocyclic ring", a cyclopropane ring, cycloheptane ring, cyclooctane, cyclononane, cyclodecane, and the like.

"3- to 10-membered saturated heterocyclic group" refers to a monovalent saturated heterocyclic group comprised of 1 to 2 atoms independently selected from the group consisting of a nitrogen atom, an oxygen atom, and a sulfur atom, and 2 to 9 carbon atoms, including those having a partially unsaturated bond and those with a crosslinked structure. Atoms constituting a ring may include an atom that is oxidized such as -C(O)-, - S(O)-, or $-SO_2-$. "3- to 10-membered saturated heterocyclic group" is preferably "4- to 7-membered monocyclic saturated heterocyclic group". Specific examples of "4- to 7-membered monocyclic saturated heterocyclic group" include oxetanyl, azetidinyl, tetrahydrofuryl, pyrrolidinyl, imidazolidinyl, piperidinyl, morpholinyl, thiomorpholinyl, dioxothiomorpholinyl, hexamethyleniminyl, oxazolidinyl, thiazolidinyl, oxoimidazolidinyl, dioxoimidazolidinyl, oxooxazolidinyl, dioxooxazolidinyl, dioxothiazolidinyl, tetrahydrofuranyl, tetrahydropyranyl, and the like. Examples of "3- to 10-membered saturated heterocyclic group" include, in addition to the specific examples for the "4- to 7-membered monocyclic saturated heterocyclic group" described above, oxiranyl, aziridinyl, and the like.

"3- to 10-membered saturated heterocyclic group" encompasses those that are bicyclic forming a fused ring of the 3- to 10-membered saturated heterocyclic group and a 6-membered aromatic hydrocarbon ring or 6-membered aromatic heterocycle. Examples of the 6-membered aromatic hydrocarbon ring forming a fused ring include a benzene ring and the like. Examples of the 6-membered aromatic heterocycle forming a fused ring includes pyridine, pyrimidine, pyridazine, and the like. Specific examples of bicyclic "3- to 10-membered saturated heterocyclic group" forming a fused ring include dihydroindolyl, dihydroisoindolyl, dihydropurinyl, dihydrothiazolopyrimidinyl, dihydrobenzodioxanyl, isoindolyl, indazolyl, tetrahydroquinolinyl, tetrahydroisoquinolinyl, tetrahydronaphthyridinyl, and the like.

"3- to 8-membered nitrogen-containing saturated heterocycle" refers to a saturated heterocycle comprised of 1 nitrogen atom and 2 to 7 carbon atoms. "3- to 8-membered nitrogen-containing saturated heterocycle" is preferably "4- to 6-membered nitrogen-containing saturated heterocycle". Specific examples of "4- to 6-membered nitrogen-containing saturated heterocycle" include an azetidine ring, pyrrolidine ring, piperidine ring, and the like. Specific examples of "3- to 8-membered nitrogen-containing saturated heterocycle" include, in addition to the specific examples for the "4- to 6-membered nitrogen-containing saturated heterocycle" described above, an aziridine ring, azepane ring, azocane ring, and the like.

"3- to 10-membered divalent saturated heterocyclic group" refers to a divalent saturated heterocyclic group comprised of 1 to 2 atoms independently selected from the group consisting of a nitrogen atom, an oxygen atom, and a sulfur atom and 2 to 9 carbon atoms, including those having a partially unsaturated bond and those with a crosslinked structure. Atoms constituting a ring may include an atom that is oxidized such as -C(O)-, - S(O)-, or $-SO_2-$. "3- to 10-membered saturated heterocyclic group" is preferably "4- to 7-membered monocyclic saturated heterocyclic group". Specific examples of "4- to 7-membered monocyclic saturated heterocyclic group" include oxetanylene, azetidinylene, tetrahydrofurylene, pyrrolidinylene, imidazolidinylene, piperidinylene, morpholinylene, thiomorpholinylene, dioxothiomorpholinylene, hexamethyleniminylene, oxazolidinylene, thiazolidinylene, oxoimidazolidinylene, dioxooimidazolidinylene, oxooxazolidinylene, dioxooxazolidinylene, dioxothiazolidinylene, tetrahydrofuranylene, tetrahydropyranylene, and the like. Examples of "3- to 10-membered saturated heterocyclic group" include, in addition to the specific examples for the "4- to 7-membered monocyclic saturated heterocyclic group" described above, oxiranylene, aziridinylene, and the like.

"3- to 10-membered saturated heterocyclic group" encompasses those that are bicyclic forming a fused ring of the 3- to 10-membered saturated heterocyclic group and a 6-membered aromatic hydrocarbon ring or 6-membered aromatic heterocycle. Examples of the 6-membered aromatic hydrocarbon ring forming a fused ring include a benzene ring and the like. Examples of the 6-membered aromatic heterocycle forming a fused ring include pyridine, pyrimidine, pyridazine, and the like. Specific examples of bicyclic "3- to 10-membered saturated heterocyclic group" forming a fused ring include dihydroindolylene, dihydroisoindolylene, dihydropurinylene, dihydrothiazolopyrimidinylene, dihydrobenzodioxanylene, isoindolylene, indazolylene, tetrahydroquinolylene, tetrahydroisoquinolinylene, tetrahydronaphthylidinylene, and the like.

"$C_{6-10}$ aryl" refers to an aromatic hydrocarbon ring group with 6 to 10 carbon atoms. Specific examples of "$C_{6-10}$ aryl" include phenyl, 1-naphthyl, 2-naphthyl, and the like. Preferred examples thereof include phenyl.

"$C_{6-10}$ aryl" encompasses those that are bicyclic forming a fused ring of the $C_{6-10}$ aryl and $C_{4-6}$ cycloalkyl or 5- to 6-membered saturated heterocycle. Specific examples of bicyclic "$C_{6-10}$ aryl" forming a fused ring include the groups represented by the following and the like.

[Chemical Formula 15]

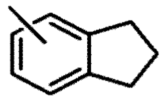 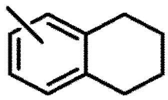 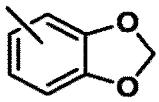 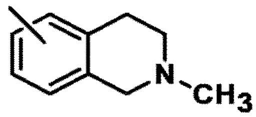

"Aromatic hydrocarbon ring" refers to the cyclic moiety of the "$C_{6-10}$ aryl" described above.

"5- to 12-membered heteroaryl" refers to a cyclic group of a monocyclic 5- to 7-membered aromatic heterocycle or a cyclic group of a bicyclic 8- to 12-membered aromatic heterocycle, comprising 1 to 4 atoms independently selected from the group consisting of a nitrogen atom, an oxygen atom, and a sulfur atom. This is preferably "5- to 7-membered monocyclic heteroaryl", more preferably pyridyl, pyrimidinyl, quinolyl, or isoquinolyl, and more preferably pyridyl. Specific examples of "5- to 7-membered monocyclic heteroaryl" include pyridyl, pyridazinyl, isothiazolyl, pyrrolyl, furyl, thienyl, thiazolyl, imidazolyl, pyrimidinyl, thiadiazolyl, pyrazolyl, oxazolyl, isooxazolyl, pyrazinyl, triazinyl, triazolyl, oxadiazolyl, triazolyl, tetrazolyl, and the like. Specific examples of "5- to 12-membered heteroaryl" include, in addition to the specific examples for the "5- to 7-membered monocyclic heteroaryl" described above, indolyl, indazolyl, chromenyl, quinolyl, isoquinolyl, benzofuranyl, benzothienyl, benzooxazolyl, benzothiazolyl, benzoisooxazolyl, benzoisothiazolyl, benzotriazolyl, benzoimidazolyl, and the like.

"Aromatic heterocycle" refers to a cyclic moiety of the "5- to 12-membered heteroaryl" described above.

"Cancer" and "tumor" are defined the same in the present disclosure, both referring to malignant tumor and encompassing cancer, sarcoma, and hematological malignancy. Specific examples of "cancer" and "tumor" include acute leukemia, chronic lymphocytic leukemia, chronic myelogenous leukemia, myelodysplastic syndrome, polycythemia vera, malignant lymphoma, plasma cell tumor, multiple myeloma, brain tumor, head and neck cancer, esophageal cancer, thyroid cancer, small cell lung cancer, non-small cell lung cancer, thymoma/thymic carcinoma, breast cancer, gastric cancer, gallbladder/bile duct cancer, liver cancer, hepatocellular carcinoma, pancreatic cancer, colon cancer, rectal cancer, anal cancer, gastrointestinal stromal tumor, choriocarcinoma, endometrial cancer, cervical cancer, ovarian cancer, bladder cancer, urothelial cancer, renal cancer, renal cell cancer, prostate cancer, testicular tumor, testicular germ cell tumor, ovarian germ cell tumor, Wilms tumor, malignant melanoma, neuroblastoma, osteosarcoma, Ewing sarcoma, chondrosarcoma, soft tissue sarcoma, skin cancer, and the like.

Prexasertib is a compound with the following structure.

[Chemical Formula 16]

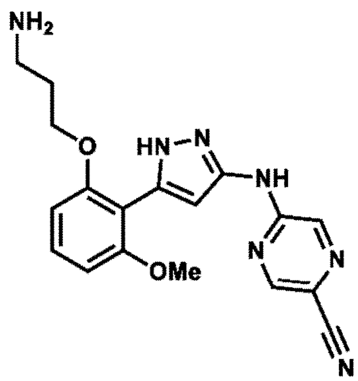

In the compound of the present disclosure represented by formula (1), (2), (3), (4), or (5), $R^1$, $R^2$, $R^3$, $R^4$, $R^5$, $R^6$, $R^7$, $R^8$, $R^{8a}$, $R^{8b}$, $R^{8c}$, $R^9$, $R^{10}$, $R^{11}$, $R^{12}$, $R^{13}$, $R^{14}$, $R^{15}$, $R^{16}$, $R^{17}$, $R^{18}$, X, Y, Z, L, V, W, Q, and Y are preferably the following, but the technical scope of the present disclosure is not limited to the scope of the compounds listed below.

Preferred embodiments of $R^1$ include $C_{1-6}$ alkyl optionally substituted with 1 to 3 fluorine atoms.

More preferred embodiments of $R^1$ include an ethyl group or methyl group optionally substituted with 1 to 3 fluorine atoms.

Still more preferred embodiments of $R^1$ include a methyl group optionally substituted with 1 to 3 fluorine atoms.

Yet still more preferred embodiments of $R^1$ include a methyl group.

Preferred embodiments of $R^2$ include a hydrogen atom, a halogen atom, cyano, $-OR^3$, $C_{1-6}$ alkyl optionally substituted with 1 to 3 halogen atoms, $C_{3-10}$ cycloalkyl, and a 3- to 10-membered saturated heterocyclic group.

More preferred embodiments of $R^2$ include a hydrogen atom, a halogen atom, cyano, and $C_{1-6}$ alkyl optionally

substituted with 1 to 3 halogen atoms.

Still more preferred embodiments of $R^2$ include a halogen atom, cyano, and $C_{1-6}$ alkyl optionally substituted with 1 to 3 halogen atoms.

Yet still more preferred embodiments of $R^2$ include cyano.

Preferred embodiments of $R^3$ include a hydrogen atom and $C_{1-6}$ alkyl.

More preferred embodiments of $R^3$ include $C_{1-6}$ alkyl.

Still more preferred embodiments of $R^3$ include $C_{1-3}$ alkyl.

Yet still more preferred embodiments of $R^3$ include a methyl group.

Preferred embodiments of $R^4$ include $C_{1-3}$ alkyl.

More preferred embodiments of $R^4$ include a methyl group.

Preferred embodiments of $R^5$, $R^6$, and $R^7$ include a hydrogen atom and $C_{1-6}$ alkyl.

More preferred embodiments of $R^5$, $R^6$, and $R^7$ include $C_{1-6}$ alkyl.

Still more preferred embodiments of $R^5$, $R^6$, and $R^7$ include $C_{1-3}$ alkyl.

Yet still more preferred embodiments of $R^5$, $R^6$, and $R^7$ include a methyl group.

Preferred embodiments of $R^8$ include a hydrogen atom, a fluorine atom, a chlorine atom, a bromine atom, $C_{1-6}$ alkyl (wherein the alkyl is optionally substituted with 1 to 2 of the same or different substituents selected from the group consisting of a fluorine atom, a hydroxyl group, $C_{1-3}$ alkoxy, - $NR^{16}R^{17}$, and cyano), and 5- to 6-membered heteroaryl (wherein the heteroaryl is optionally substituted with 1 to 2 of the same or different substituents selected from the group consisting of a fluorine atom, a hydroxyl group, $C_{1-3}$ alkyl, $C_{1-3}$ alkoxy, $-NR^{16}R^{17}$, and cyano).

More preferred embodiments of $R^8$ include a hydrogen atom, a fluorine atom, a chlorine atom, a bromine atom, and $C_{1-6}$ alkyl (wherein the alkyl is optionally substituted with 1 to 2 of the same or different substituents selected from the group consisting of a fluorine atom, a hydroxyl group, $C_{1-3}$ alkoxy, - $NR^{16}R^{17}$, and cyano) .

Still more preferred embodiments of $R^8$ include a hydrogen atom, a fluorine atom, a chlorine atom, a bromine atom, and $C_{1-3}$ alkyl.

Still more preferred embodiments of $R^8$ include a hydrogen atom and a chlorine atom.

Preferred embodiments of $R^{8a}$, $R^{8b}$, and $R^{8c}$ include a hydrogen atom, a fluorine atom, a chlorine atom, and a bromine atom.

More preferred embodiments of $R^{8a}$, $R^{8b}$, and $R^{8c}$ include a hydrogen atom and a chlorine atom.

Still more preferred embodiments of $R^{8a}$, $R^{8b}$, and $R^{8c}$ include a hydrogen atom.

Preferred embodiments of $R^9$ include a hydrogen atom and $C_{1-6}$ alkyl.

More preferred embodiments of $R^9$ include $C_{1-3}$ alkyl.

Still more preferred embodiments of $R^9$ include a methyl group.

Preferred embodiments of $R^{10}$ include $C_{1-3}$ alkyl.

Still more preferred embodiments of $R^{10}$ include a methyl group.

Preferred embodiments of $R^{11}$, $R^{12}$, and $R^{13}$ include a hydrogen atom and $C_{1-6}$ alkyl.

More preferred embodiments of $R^{11}$, $R^{12}$, and $R^{13}$ include $C_{1-3}$ alkyl.

Still more preferred embodiments of $R^{11}$, $R^{12}$, and $R^{13}$ include a methyl group.

Preferred embodiments of $R^{14}$ include a hydrogen atom, $C_{1-6}$ alkyl (wherein the alkyl is optionally substituted with 1 to 3 of the same or different substituents selected from the group consisting of a fluorine atom, a hydroxyl group, $C_{1-3}$ alkoxy, - $NR^{16}R^{17}$, and cyano), $C_{3-10}$ cycloalkyl (wherein the cycloalkylene is optionally substituted with 1 to 3 of the same or different substituents selected from the group consisting of a fluorine atom, a hydroxyl group, $C_{1-3}$ alkyl, $C_{1-3}$ alkoxy, $-NR^{16}R^{17}$, and cyano), and a 3- to 10-membered saturated heterocyclic group (wherein the saturated heterocyclic group is optionally substituted with 1 to 3 of the same or different substituents selected from the group consisting of a fluorine atom, a hydroxyl group, $C_{1-3}$ alkyl, $C_{1-3}$ alkoxy, $-NR^{16}R^{17}$, and cyano).

More preferred embodiments of $R^{14}$ include a hydrogen atom and $C_{1-6}$ alkyl (wherein the alkyl is optionally substituted with 1 to 3 of the same or different substituents selected from the group consisting of a fluorine atom, a hydroxyl group, $C_{1-3}$ alkoxy, $-NR^{16}R^{17}$, and cyano).

More preferred embodiments of $R^{14}$ include a hydrogen atom and $C_{1-3}$ alkyl (wherein the alkyl is optionally substituted with 1 substituent selected from the group consisting of a fluorine atom, a hydroxyl group, and cyano).

Still more preferred embodiments of $R^{14}$ include a hydrogen atom and a methyl group.

Preferred embodiments of $R^{15}$ include $C_{1-6}$ alkyl.

More preferred embodiments of $R^{15}$ include $C_{1-3}$ alkyl.

More preferred embodiments of $R^{15}$ include a methyl group.

Preferred embodiments of $R^{16}$ and $R^{17}$ include a hydrogen atom and $C_{1-6}$ alkyl.

More preferred embodiments of $R^{16}$ and $R^{17}$ include $C_{1-6}$ alkyl.

Still more preferred embodiments of $R^{16}$ and $R^{17}$ include $C_{1-3}$ alkyl.

Still more preferred embodiments of $R^{16}$ and $R^{17}$ include a methyl group.

Preferred embodiments of $R^{18}$ include a hydrogen atom and $C_{1-6}$ alkyl.

More preferred embodiments of $R^{18}$ include $C_{1-6}$ alkyl.

More preferred embodiments of $R^{18}$ include $C_{1-3}$ alkyl.

Still more preferred embodiments of $R^{18}$ include a methyl group.

Preferred embodiments of X, Y, and Z include $CR^8$ and a nitrogen atom. For the compound or pharmaceutically acceptable salt of the present disclosure, X, Y, and Z are not simultaneously $CR^8$ in the present disclosure.

In another preferred embodiment of X, Y, and Z, one or two of X, Y, and Z represents a nitrogen atom.

In another preferred embodiment of X, Y, and Z, X is $CR^8$, and Y and Z are nitrogen atoms.

In still another preferred embodiment of X, Y, and Z, Y is $CR^8$, and X and Z are nitrogen atoms.

In another different preferred embodiment of X, Y, and Z, Z is $CR^8$, and X and Y are nitrogen atoms.

Preferred embodiments of L include a single bond and $C_{1-6}$ alkylene (wherein the alkylene is optionally substituted with 1 to 3 of the same or different substituents selected from the group consisting of a fluorine atom, a hydroxyl group, $C_{1-3}$ alkoxy, $-NR^{16}R^{17}$, and cyano) .

More preferred embodiments of L include a single bond, and $C_{1-6}$ alkylene (wherein the alkylene is optionally substituted with 1 substituent selected from the group consisting of a fluorine atom, a hydroxyl group, and cyano).

More preferred embodiments of L include a single bond, and $C_{1-6}$ alkylene (wherein the alkylene is optionally substituted with 1 substituent selected from the group consisting of a fluorine atom and a hydroxyl group).

Still more preferred embodiments of L include a single bond and $C_{1-6}$ alkylene optionally substituted with 1 hydroxyl group.

Preferred embodiments of V include a single bond, $C_{3-10}$ cycloalkylene (wherein the cycloalkylene is optionally substituted with 1 to 3 of the same or different substituents selected from the group consisting of a fluorine atom, a hydroxyl group, $C_{1-3}$ alkyl, $C_{1-3}$ alkoxy, $-NR^{16}R^{17}$, and cyano), and a 3- to 10-membered divalent saturated heterocyclic group (wherein the saturated heterocyclic group is optionally substituted with 1 to 3 of the same or different substituents selected from the group consisting of a fluorine atom, a hydroxyl group, $C_{1-3}$ alkyl optionally substituted with 1 to 2 hydroxyl groups or fluorine atoms, $C_{1-3}$ alkoxy, $-NR^{16}R^{17}$, and cyano).

More preferred embodiments of V include a single bond, $C_{3-7}$ cycloalkylene (wherein the cycloalkylene is optionally substituted with 1 to 2 of the same or different substituents selected from the group consisting of a fluorine atom, a hydroxyl group, $C_{1-3}$ alkyl optionally substituted with 1 to 2 hydroxyl groups, and cyano), and a 3- to 7-membered divalent saturated heterocyclic group (wherein the saturated heterocyclic group is optionally substituted with 1 to 2 of the same or different substituents selected from the group consisting of a fluorine atom, a hydroxyl group, $C_{1-3}$ alkyl optionally substituted with 1 to 2 hydroxyl groups or fluorine atoms, and cyano).

More preferred embodiments of V include a single bond, $C_{3-7}$ cycloalkylene (wherein the cycloalkylene is optionally substituted with 1 to 2 of the same or different substituents selected from the group consisting of a fluorine atom, a hydroxyl group, and $C_{1-3}$ alkyl optionally substituted with 1 to 2 hydroxyl groups or fluorine atoms), and a 3- to 7-membered divalent saturated heterocyclic group (wherein the saturated heterocyclic group is optionally substituted with 1 to 2 of the same or different substituents selected from the group consisting of a fluorine atom, a hydroxyl group, and $C_{1-3}$ alkyl optionally substituted with 1 to 2 hydroxyl groups).

Still more preferred embodiments of V include a single bond, $C_{3-7}$ cycloalkylene (wherein the cycloalkylene is optionally substituted with 1 substituent selected from the group consisting of a hydroxyl group and $C_{1-3}$ alkyl), and a 3-to 7-membered divalent saturated heterocyclic group (wherein the 3- to 7-membered saturated heterocyclic group is optionally substituted with 1 substituent selected from the group consisting of a fluorine atom, cyano, a hydroxyl group, and $C_{1-3}$ alkyl).

Preferred embodiments of W include a single bond and $C_{1-6}$ alkylene (wherein the alkylene is optionally substituted with 1 to 3 of the same or different substituents selected from the group consisting of a fluorine atom, a hydroxyl group, $C_{1-3}$ alkoxy, $-NR^{16}R^{17}$, and cyano) .

More preferred embodiments of W include a single bond and $C_{1-3}$ alkylene (wherein the alkylene is optionally substituted with 1 substituent selected from the group consisting of a fluorine atom, a hydroxyl group, and cyano).

More preferred embodiments of W include a single bond and $C_{1-3}$ alkylene (wherein the alkylene is optionally substituted with 1 substituent selected from the group consisting of a fluorine atom and a hydroxyl group).

Still more preferred embodiments of W include a single bond and $C_{1-3}$ alkylene optionally substituted with 1 hydroxyl group.

Preferred embodiments of Q include a hydrogen atom and $NHR^{14}$.

More preferred embodiments of Q include a hydrogen atom, $NH_2$, and NHMe.

More preferred embodiments of Q include a hydrogen atom and $NH_2$.

Still more preferred embodiments of Q include a hydrogen atom.

Still another preferred embodiments of Q include $NH_2$.

One embodiment of the compound represented by formula (1) includes the following (A).

(A) A compound or a pharmaceutically acceptable salt thereof, wherein

$R^1$ is an ethyl group or methyl group optionally substituted with 1 to 3 fluorine atoms,
$R^2$ is a hydrogen atom, a halogen atom, cyano, $-OR^3$, $C_{1-6}$ alkyl optionally substituted with 1 to 3 halogen atoms, $C_{3-10}$ cycloalkyl, or a 3- to 10-membered saturated heterocyclic group,
$R^3$ is a hydrogen atom or $C_{1-6}$ alkyl,
X, Y, and Z each independently represent $CR^8$ or a nitrogen atom, wherein X, Y, and Z are not simultaneously $CR^8$,
$R^8$ is a hydrogen atom, a fluorine atom, a chlorine atom, a bromine atom, $C_{1-6}$ alkyl (wherein the alkyl is optionally substituted with 1 to 2 of the same or different substituents selected from the group consisting of a fluorine atom, a hydroxyl group, $C_{1-3}$ alkoxy, $-NR^{16}R^{17}$, and cyano), or 5- to 6-membered heteroaryl (wherein the heteroaryl is optionally substituted with 1 to 2 of the same or different substituents selected from the group consisting of a fluorine atom, a hydroxyl group, $C_{1-3}$ alkyl, $C_{1-3}$ alkoxy, $-NR^{16}R^{17}$, and cyano),
L is a single bond or $C_{1-6}$ alkylene (wherein the alkylene is optionally substituted with 1 to 3 of the same or different substituents selected from the group consisting of a fluorine atom, a hydroxyl group, $C_{1-3}$ alkoxy, $-NR^{16}R^{17}$, and cyano),
V is a single bond, $C_{3-10}$ cycloalkylene (wherein the cycloalkylene is optionally substituted with 1 to 3 of the same or different substituents selected from the group consisting of a fluorine atom, a hydroxyl group, $C_{1-3}$ alkyl, $C_{1-3}$ alkoxy, $- NR^{16}R^{17}$, and cyano), or a 3- to 10-membered divalent saturated heterocyclic group (wherein the saturated heterocyclic group is optionally substituted with 1 to 3 of the same or different substituents selected from the group consisting of a fluorine atom, a hydroxyl group, $C_{1-3}$ alkyl optionally substituted with 1 to 2 hydroxyl groups or fluorine atoms, $C_{1-3}$ alkoxy, $-NR^{16}R^{17}$, and cyano),
W is a single bond or $C_{1-6}$ alkylene (wherein the alkylene is optionally substituted with 1 to 3 of the same or different substituents selected from the group consisting of a fluorine atom, a hydroxyl group, $C_{1-3}$ alkoxy, $-NR^{16}R^{17}$, and cyano),
Q is a hydrogen atom or $NHR^{14}$,
$R^{14}$ is a hydrogen atom, $C_{1-6}$ alkyl (wherein the alkyl is optionally substituted with 1 to 3 of the same or different substituents selected from the group consisting of a fluorine atom, a hydroxyl group, $C_{1-3}$ alkoxy, $-NR^{16}R^{17}$, and cyano), $C_{3-10}$ cycloalkyl (wherein the cycloalkylene is optionally substituted with 1 to 3 of the same or different substituents selected from the group consisting of a fluorine atom, a hydroxyl group, $C_{1-3}$ alkyl, $C_{1-3}$ alkoxy, $-NR^{16}R^{17}$, and cyano), or a 3- to 10-membered saturated heterocyclic group (wherein the saturated heterocyclic group is optionally substituted with 1 to 3 of the same or different substituents selected from the group consisting of a fluorine atom, a hydroxyl group, $C_{1-3}$ alkyl, $C_{1-3}$ alkoxy, $-NR^{16}R^{17}$, and cyano), and
$R^{16}$ and $R^{17}$ are hydrogen atoms or $C_{1-6}$ alkyl.

An embodiment of the compound represented by formula (1) includes the following (B).

(B) A compound or a pharmaceutically acceptable salt thereof, wherein
$R^1$ is a methyl group optionally substituted with 1 to 3 fluorine atoms,
$R^2$ is a halogen atom, cyano, or $C_{1-6}$ alkyl optionally substituted with 1 to 3 halogen atoms,
X, Y, and Z each independently represent $CR^8$ or a nitrogen atom, wherein X, Y, and Z are not simultaneously $CR^8$,
$R^8$ is a hydrogen atom, a fluorine atom, a chlorine atom, a bromine atom, or $C_{1-3}$ alkyl,
L is a single bond or $C_{1-6}$ alkylene (wherein the alkylene is optionally substituted with 1 to 3 of the same or different substituents selected from the group consisting of a fluorine atom, a hydroxyl group, $C_{1-3}$ alkoxy, $-NR^{16}R^{17}$, and cyano),
V is a single bond, $C_{3-10}$ cycloalkylene (wherein the cycloalkylene is optionally substituted with 1 to 3 of the same or different substituents selected from the group consisting of a fluorine atom, a hydroxyl group, $C_{1-3}$ alkyl, $C_{1-3}$ alkoxy, $-NR^{16}R^{17}$, and cyano), or a 3- to 10-membered divalent saturated heterocyclic group (wherein the saturated heterocyclic group is optionally substituted with 1 to 3 of the same or different substituents selected from the group consisting of a fluorine atom, a hydroxyl group, $C_{1-3}$ alkyl optionally substituted with 1 to 2 hydroxyl groups or fluorine atoms, $C_{1-3}$ alkoxy, $-NR^{16}R^{17}$, and cyano),
W is a single bond or $C_{1-6}$ alkylene (wherein the alkylene is optionally substituted with 1 to 3 of the same or different substituents selected from the group consisting of a fluorine atom, a hydroxyl group, $C_{1-3}$ alkoxy, $-NR^{16}R^{17}$, and cyano),
Q is a hydrogen atom or $NHR^{14}$,
$R^{14}$ is a hydrogen atom, $C_{1-6}$ alkyl (wherein the alkyl is optionally substituted with 1 to 3 of the same or different substituents selected from the group consisting of a fluorine atom, a hydroxyl group, $C_{1-3}$ alkoxy, $-NR^{16}R^{17}$, and cyano), $C_{3-10}$ cycloalkyl (wherein the cycloalkylene is optionally substituted with 1 to 3 of the same or different substituents selected from the group consisting of a fluorine atom, a hydroxyl group, $C_{1-3}$ alkyl, $C_{1-3}$ alkoxy, $-NR^{16}R^{17}$, and cyano), or a 3- to 10-membered saturated heterocyclic group (wherein the saturated heterocyclic group is optionally substituted with 1 to 3 of the same or different substituents selected from the group consisting of a fluorine

atom, a hydroxyl group, $C_{1-3}$ alkyl, $C_{1-3}$ alkoxy, $-NR^{16}R^{17}$, and cyano), and
$R^{16}$ and $R^{17}$ are hydrogen atoms or $C_{1-6}$ alkyl.

An embodiment of the compound represented by formula (1) includes the following (C).

(C) A compound or a pharmaceutically acceptable salt thereof, wherein
$R^1$ is a methyl group or a fluoromethyl group,
$R^2$ is a chlorine atom, cyano, or a trifluoromethyl group,
X, Y, and Z each independently represent $CR^8$ or a nitrogen atom, wherein X, Y, and Z are not simultaneously $CR^8$,
$R^8$ is a hydrogen atom, a fluorine atom, a chlorine atom, a bromine atom, or $C_{1-3}$ alkyl,
L is a single bond or $C_{1-6}$ alkylene (wherein the alkylene is optionally substituted with 1 to 3 of the same or different substituents selected from the group consisting of a fluorine atom, a hydroxyl group, $C_{1-3}$ alkoxy, $-NR^{16}R^{17}$, and cyano),
V is a single bond, $C_{3-10}$ cycloalkylene (wherein the cycloalkylene is optionally substituted with 1 to 3 of the same or different substituents selected from the group consisting of a fluorine atom, a hydroxyl group, $C_{1-3}$ alkyl, $C_{1-3}$ alkoxy, $-NR^{16}R^{17}$, and cyano), or a 3- to 10-membered divalent saturated heterocyclic group (wherein the saturated heterocyclic group is optionally substituted with 1 to 3 of the same or different substituents selected from the group consisting of a fluorine atom, a hydroxyl group, $C_{1-3}$ alkyl optionally substituted with 1 to 2 hydroxyl groups or fluorine atoms, $C_{1-3}$ alkoxy, $-NR^{16}R^{17}$, and cyano),
W is a single bond or $C_{1-6}$ alkylene (wherein the alkylene is optionally substituted with 1 to 3 of the same or different substituents selected from the group consisting of a fluorine atom, a hydroxyl group, $C_{1-3}$ alkoxy, $-NR^{16}R^{17}$, and cyano),
Q is a hydrogen atom or $NHR^{14}$,
$R^{14}$ is a hydrogen atom or $C_{1-3}$ alkyl (wherein the alkyl is optionally substituted with 1 substituent selected from the group consisting of a fluorine atom, a hydroxyl group, and cyano), and
$R^{16}$ and $R^{17}$ are hydrogen atoms or $C_{1-6}$ alkyl.

An embodiment of the compound represented by formula (1) includes the following (D).

(D) A compound or a pharmaceutically acceptable salt thereof, wherein
$R^1$ is a methyl group,
$R^2$ is cyano,
X, Y, and Z each independently represent $CR^8$ or a nitrogen atom, wherein X, Y, and Z are not simultaneously $CR^8$,
$R^8$ is a hydrogen atom, a fluorine atom, a chlorine atom, a bromine atom, or $C_{1-3}$ alkyl,
L is a single bond or $C_{1-6}$ alkylene (wherein the alkylene is optionally substituted with 1 substituent selected from the group consisting of a fluorine atom, a hydroxyl group, and cyano),
V is a single bond, $C_{3-7}$ cycloalkylene (wherein the cycloalkylene is optionally substituted with 1 to 2 of the same or different substituents selected from the group consisting of a fluorine atom, a hydroxyl group, $C_{1-3}$ alkyl optionally substituted with 1 to 2 hydroxyl groups, and cyano), or a 3- to 7-membered divalent saturated heterocyclic group (wherein the saturated heterocyclic group is optionally substituted with 1 to 2 of the same or different substituents selected from the group consisting of a fluorine atom, a hydroxyl group, $C_{1-3}$ alkyl optionally substituted with 1 to 2 hydroxyl groups or fluorine atoms, and cyano),
W is a single bond or $C_{1-3}$ alkylene (wherein the alkylene is optionally substituted with 1 substituent selected from the group consisting of a fluorine atom, a hydroxyl group, and cyano), and
Q is a hydrogen atom, $NH_2$, or NHMe.

An embodiment of the compound represented by formula (1) or (2) includes the following (E).

(E) A compound or a pharmaceutically acceptable salt thereof, wherein
X, Y, and Z each independently represent $CR^8$ or a nitrogen atom, wherein X, Y, and Z are not simultaneously $CR^8$,
$R^8$ is a hydrogen atom or a chlorine atom,
L is a single bond or $C_{1-6}$ alkylene optionally substituted with 1 hydroxyl group or fluorine atom,
V is a single bond, $C_{3-7}$ cycloalkylene (wherein the cycloalkylene is optionally substituted with 1 substituent selected from the group consisting of a hydroxyl group and $C_{1-3}$ alkyl), or a 3- to 7-membered divalent saturated heterocyclic group (wherein the saturated heterocyclic group is optionally substituted with 1 substituent selected from the group consisting of a fluorine atom, cyano, a hydroxyl group, and $C_{1-3}$ alkyl optionally substituted with 1 to 2 hydroxyl groups or fluorine atoms),
W is a single bond or $C_{1-3}$ alkylene optionally substituted with 1 hydroxyl group, and

Q is a hydrogen atom, $NH_2$, or NHMe.

An embodiment of the compound represented by formula (3) includes the following (F).

(F) A compound or a pharmaceutically acceptable salt thereof, wherein
$R^{8a}$ and $R^{8b}$ are each independently a hydrogen atom or a chlorine atom,
L is a single bond or $C_{1-3}$ alkylene optionally substituted with 1 hydroxyl group,
V is a single bond, $C_{3-7}$ cycloalkylene (wherein the cycloalkylene is optionally substituted with 1 substituent selected from the group consisting of a hydroxyl group and $C_{1-3}$ alkyl), or a 3- to 7-membered divalent saturated heterocyclic group (wherein the saturated heterocyclic group is optionally substituted with 1 substituent selected from the group consisting of a fluorine atom, cyano, a hydroxyl group, and $C_{1-3}$ alkyl),
W is a single bond or $C_{1-3}$ alkylene optionally substituted with 1 hydroxyl group, and
Q is a hydrogen atom or $NH_2$.

An embodiment of the compound represented by formula (3) includes the following (G).

(G) A compound or a pharmaceutically acceptable salt thereof, wherein
$R^{8a}$ and $R^{8b}$ are each independently a hydrogen atom or a chlorine atom,
L is $C_{1-3}$ alkylene optionally substituted with 1 hydroxyl group,
V is a single bond,
W is a single bond or $C_{1-3}$ alkylene optionally substituted with 1 hydroxyl group, and
Q is $NH_2$.

An embodiment of the compound represented by formula (3) includes the following (H).

(H) A compound or a pharmaceutically acceptable salt thereof, wherein
$R^{8a}$ and $R^{8b}$ are each independently a hydrogen atom,
L is $C_{1-3}$ alkylene optionally substituted with 1 hydroxyl group,
V is $C_{3-7}$ cycloalkylene,
W is $C_{1-3}$ alkylene optionally substituted with 1 hydroxyl group, and
Q is $NH_2$.

An embodiment of the compound represented by formula (4) includes the following (I).

(I) A compound or a pharmaceutically acceptable salt thereof, wherein
$R^{8b}$ and $R^{8c}$ are hydrogen atoms,
L is a single bond or $C_{1-3}$ alkylene optionally substituted with 1 hydroxyl group or fluorine atom,
V is a single bond, $C_{3-7}$ cycloalkylene (wherein the cycloalkylene is optionally substituted with 1 substituent selected from the group consisting of a hydroxyl group and $C_{1-3}$ alkyl), or a 3- to 7-membered divalent saturated heterocyclic group (wherein the saturated heterocyclic group is optionally substituted with 1 substituent selected from the group consisting of a fluorine atom, cyano, a hydroxyl group, and $C_{1-3}$ alkyl optionally substituted with 1 to 3 hydroxyl groups or fluorine atoms),
W is a single bond or $C_{1-3}$ alkylene optionally substituted with 1 hydroxyl group, and
Q is a hydrogen atom, $NH_2$, or NHMe.

An embodiment of the compound represented by formula (4) includes the following (J).

(J) A compound or a pharmaceutically acceptable salt thereof, wherein
$R^{8b}$ and $R^{8c}$ are each independently a hydrogen atom,
L is $C_{1-3}$ alkylene optionally substituted with 1 hydroxyl group or fluorine atom,
V is a 3- to 7-membered divalent saturated heterocyclic group (wherein the saturated heterocyclic group is optionally substituted with 1 substituent selected from the group consisting of a fluorine atom, cyano, a hydroxyl group, and $C_{1-3}$ alkyl optionally substituted with 1 to 3 hydroxyl groups and fluorine atoms),
W is a single bond, and
Q is a hydrogen atom.

An embodiment of the compound represented by formula (4) includes the following (K).

(K) A compound or a pharmaceutically acceptable salt thereof, wherein
$R^{8b}$ and $R^{8c}$ are each independently a hydrogen atom,
L is $C_{1-3}$ alkylene optionally substituted with 1 hydroxyl group or fluorine atom,
V is $C_{3-7}$ cycloalkylene (wherein the cycloalkylene is optionally substituted with 1 substituent selected from the group consisting of a hydroxyl group and $C_{1-3}$ alkyl),
W is $C_{1-3}$ alkylene optionally substituted with 1 hydroxyl group, and
Q is $NH_2$.

(L) A compound or a pharmaceutically acceptable salt thereof, wherein
$R^{8b}$ and $R^{8c}$ are each independently a hydrogen atom,
L is a single bond,
V is $C_{3-7}$ cycloalkylene (wherein the cycloalkylene is optionally substituted with 1 substituent selected from the group consisting of a hydroxyl group and $C_{1-3}$ alkyl),
W is a single bond, and
Q is $NH_2$ or NHMe.

An embodiment of the compound represented by formula (4) includes the following (M).

(M) A compound or a pharmaceutically acceptable salt thereof, wherein
$R^{8b}$ and $R^{8c}$ are each independently a hydrogen atom,
L is $C_{1-3}$ alkylene optionally substituted with 1 hydroxyl group or fluorine atom,
V is a single bond,
W is a single bond or $C_{1-3}$ alkylene optionally substituted with 1 hydroxyl group, and
Q is $NH_2$ or NHMe.

An embodiment of the compound represented by formula (5) includes the following (N).

(N) A compound or a pharmaceutically acceptable salt thereof, wherein
$R^{8a}$ and $R^{8c}$ are each independently a hydrogen atom,
L is a single bond or $C_{1-3}$ alkylene optionally substituted with 1 hydroxyl group,
V is a single bond, $C_{3-7}$ cycloalkylene (wherein the cycloalkylene is optionally substituted with 1 substituent selected from the group consisting of a hydroxyl group and $C_{1-3}$ alkyl), or a 3- to 7-membered divalent saturated heterocyclic group (wherein the saturated heterocyclic group is optionally substituted with 1 substituent selected from the group consisting of a fluorine atom, cyano, a hydroxyl group, and $C_{1-3}$ alkyl optionally substituted with 1 to 2 hydroxyl groups),
W is a single bond or $C_{1-3}$ alkylene optionally substituted with 1 hydroxyl group, and
Q is a hydrogen atom or $NH_2$.

An embodiment of the compound represented by formula (5) includes the following (O).

(O) A compound or a pharmaceutically acceptable salt thereof, wherein
$R^{8a}$ and $R^{8c}$ are each independently a hydrogen atom,
L is $C_{1-3}$ alkylene optionally substituted with 1 hydroxyl group,
V is a 3- to 7-membered divalent saturated heterocyclic group (wherein the saturated heterocyclic group is optionally substituted with 1 substituent selected from the group consisting of a hydroxyl group and $C_{1-3}$ alkyl optionally substituted with 1 hydroxyl group),
W is a single bond, and
Q is a hydrogen atom.

An embodiment of the compound represented by formula (5) includes the following (P).

(P) A compound or a pharmaceutically acceptable salt thereof, wherein
$R^{8a}$ and $R^{8c}$ are each independently a hydrogen atom,
L is $C_{1-3}$ alkylene optionally substituted with 1 hydroxyl group,
V is a single bond,
W is a single bond or $C_{1-3}$ alkylene optionally substituted with 1 hydroxyl group, and
Q is $NH_2$.

An embodiment of the compound represented by formula (5) includes the following (Q).

(Q) A compound or a pharmaceutically acceptable salt thereof, wherein
$R^{8a}$ are each independently a hydrogen atom,
L is $C_{1-3}$ alkylene optionally substituted with 1 hydroxyl group,
V is $C_{3-7}$ cycloalkylene,
W is $C_{1-3}$ alkylene optionally substituted with 1 hydroxyl group, and
Q is $NH_2$.

An embodiment of the compound represented by formula (6) includes the following (R).

(R) A compound or a pharmaceutically acceptable salt thereof, wherein
$R^{8a}$ is a hydrogen atom,
L is $C_{1-3}$ alkylene,
V is a single bond or $C_{3-7}$ cycloalkylene,
W is a single bond or $C_{1-3}$ alkylene, and
Q is $NH_2$.

Another embodiment of the invention includes 5-({5-[2-(3-aminopropoxy)-4-methoxypyridin-3-yl]-1H-pyrazol-3-yl}amino)pyrazine-2-carbonitrile hydrochloride, which is a crystalline form (form I) having an X ray powder diffraction pattern having a characteristic peak at at least 7.2° ± 0.2° in terms of 2θ.

Another embodiment of the invention includes 5-({5-[2-(3-aminopropoxy)-4-methoxypyridin-3-yl]-1H-pyrazol-3-yl}amino)pyrazine-2-carbonitrile hydrochloride, which is a crystalline form (form I) having an X ray powder diffraction pattern having a characteristic peak at at least 8.8° ± 0.2° in terms of 2θ

Another embodiment of the invention includes 5-({5-[2-(3-aminopropoxy)-4-methoxypyridin-3-yl]-1H-pyrazol-3-yl}amino)pyrazine-2-carbonitrile hydrochloride, which is a crystalline form (form I) having an X ray powder diffraction pattern having characteristic peaks at at least 7.2° ± 0.2° and 8.8° ± 0.2° in terms of 2θ.

Another embodiment of the invention includes 5-({5-[2-(3-aminopropoxy)-4-methoxypyridin-3-yl]-1H-pyrazol-3-yl}amino)pyrazine-2-carbonitrile hydrochloride of a crystalline form of form I, having an X ray powder diffraction pattern having characteristic peaks at 7.2° ± 0.2°, 8.8° ± 0.2°, 9.8° ± 0.2°, 10.2° ± 0.2°, 10.7° ± 0.2°, 16.7° ± 0.2°, 18.5° ± 0.2°, 26.2° ± 0.2°, 27.0° ± 0.2°, and 26.4° ± 0.2° in terms of 2θ. The crystal is identified by 4 or 5 selected from these 10 peaks.

Another embodiment of the invention includes 5-({5-[2-(3-aminopropoxy)-4-methoxypyridin-3-yl]-1H-pyrazol-3-yl}amino)pyrazine-2-carbonitrile phosphate, which is a crystalline form (form II) having an X ray powder diffraction pattern having a characteristic peak at at least 6.8° ± 0.2° in terms of 2θ.

Another embodiment of the invention includes 5-({5-[2-(3-aminopropoxy)-4-methoxypyridin-3-yl]-1H-pyrazol-3-yl}amino)pyrazine-2-carbonitrile phosphate, which is a crystalline form (form II) having an X ray powder diffraction pattern having a characteristic peak at at least 13.0° ± 0.2° in terms of 2θ.

Another embodiment of the invention includes 5-({5-[2-(3-aminopropoxy)-4-methoxypyridin-3-yl]-1H-pyrazol-3-yl}amino)pyrazine-2-carbonitrile phosphate, which is a crystalline form (form II) having an X ray powder diffraction pattern having characteristic peaks at at least 6.8° ± 0.2° and 13.0° ± 0.2° in terms of 2θ.

Another embodiment of the invention includes 5-({5-[2-(3-aminopropoxy)-4-methoxypyridin-3-yl]-1H-pyrazol-3-yl}amino)pyrazine-2-carbonitrile phosphate of a crystalline form of form II, having an X ray powder diffraction pattern having characteristic peaks at 6.8° ± 0.2°, 7.5° ± 0.2°, 11.7° ± 0.2°, 11.9° ± 0.2°, 13.0° ± 0.2°, 16.4° ± 0.2°, 19.3° ± 0.2°, 20.4° ± 0.2°, 22.7° ± 0.2°, and 24.3° ± 0.2° in terms of 2θ. The crystal is identified by having 4 or 5 selected from these 10 peaks.

Another embodiment of the invention includes 5-({5-[2-(3-aminopropoxy)-4-methoxypyridin-3-yl]-1H-pyrazol-3-yl}amino)pyrazine-2-carbonitrile tosylate, which is a crystalline form (form III) having an X ray powder diffraction pattern having a characteristic peak at at least 6.0° ± 0.2° in terms of 2θ.

Another embodiment of the invention includes 5-({5-[2-(3-aminopropoxy)-4-methoxypyridin-3-yl]-1H-pyrazol-3-yl}amino)pyrazine-2-carbonitrile tosylate, which is a crystalline form (form III) having an X ray powder diffraction pattern having a characteristic peak at at least 17.0° ± 0.2° in terms of 2θ.

Another embodiment of the invention includes 5-({5-[2-(3-aminopropoxy)-4-methoxypyridin-3-yl]-1H-pyrazol-3-yl}amino)pyrazine-2-carbonitrile tosylate, which is a crystalline form (form III) having an X ray powder diffraction pattern having characteristic peaks at at least 6.0° ± 0.2° and 17.0° ± 0.2° in terms of 2θ.

Another embodiment of the invention includes 5-({5-[2-(3-aminopropoxy)-4-methoxypyridin-3-yl]-1H-pyrazol-3-yl}amino)pyrazine-2-carbonitrile tosylate of a crystalline form of form III, having an X ray powder diffraction pattern having characteristic peaks at 6.0° ± 0.2°, 9.0° ± 0.2°, 12.1° ± 0.2°, 14.4° ± 0.2°, 16.2° ± 0.2°, 17.0° ± 0.2°, 22.8° ± 0.2°, and 26.3° ± 0.2° in terms of 2θ. The crystal is identified by having 4 or 5 selected from these 10 peaks.

Another embodiment of the invention includes 5-({5-[2-(3-aminopropoxy)-4-methoxypyridin-3-yl]-1H-pyrazol-3-yl}amino)pyrazine-2-carbonitrile, which is a crystalline form (form IV) having an X ray powder diffraction pattern having a characteristic peak at at least 9.3° ± 0.2° in terms of 2θ.

Another embodiment of the invention includes 5-({5-[2-(3-aminopropoxy)-4-methoxypyridin-3-yl]-1H-pyrazol-3-yl}amino)pyrazine-2-carbonitrile, which is a crystalline form (form IV) having an X ray powder diffraction pattern having a characteristic peak at at least 10.2° ± 0.2° in terms of 2θ.

Another embodiment of the invention includes 5-({5-[2-(3-aminopropoxy)-4-methoxypyridin-3-yl]-1H-pyrazol-3-yl}amino)pyrazine-2-carbonitrile, which is a crystalline form (form IV) having an X ray powder diffraction pattern having characteristic peaks at at least 9.3° ± 0.2° and 10.2° ± 0.2° in terms of 2θ.

Another embodiment of the invention includes 5-({5-[2-(3-aminopropoxy)-4-methoxypyridin-3-yl]-1H-pyrazol-3-yl}amino)pyrazine-2-carbonitrile of a crystalline form of form IV, having an X ray powder diffraction pattern having characteristic peaks at 9.3° ± 0.2°, 10.2° ± 0.2°, 10.7° ± 0.2°, 13.6° ± 0.2°, 16.7° ± 0.2°, 17.1° ± 0.2°, 17.8° ± 0.2°, 18.6° ± 0.2°, 26.1° ± 0.2°, and 26.4° ± 0.2° in terms of 2θ. The crystal is identified by having 4 or 5 selected from these 10 peaks.

Another embodiment of the invention includes 5-({5-[2-(3-aminopropoxy)-4-methoxypyridin-3-yl]-1H-pyrazol-3-yl}amino)pyrazine-2-carbonitrile, which is a crystalline form (form V) having an X ray powder diffraction pattern having a characteristic peak at at least 7.9° ± 0.2° in terms of 2θ.

Another embodiment of the invention includes 5-({5-[2-(3-aminopropoxy)-4-methoxypyridin-3-yl]-1H-pyrazol-3-yl}amino)pyrazine-2-carbonitrile, which is a crystalline form (form V) having an X ray powder diffraction pattern having a characteristic peak at at least 8.7° ± 0.2° in terms of 2θ.

Another embodiment of the invention includes 5-({5-[2-(3-aminopropoxy)-4-methoxypyridin-3-yl]-1H-pyrazol-3-yl}amino)pyrazine-2-carbonitrile, which is a crystalline form (form V) having an X ray powder diffraction pattern having characteristic peaks at at least 7.9° ± 0.2° and 8.7° ± 0.2° in terms of 2θ.

Another embodiment of the invention includes 5-({5-[2-(3-aminopropoxy)-4-methoxypyridin-3-yl]-1H-pyrazol-3-yl}amino)pyrazine-2-carbonitrile of a crystalline form of form V, having an X ray powder diffraction pattern having characteristic peaks at 7.9° ± 0.2°, 8.7° ± 0.2°, 12.2° ± 0.2°, 13.1° ± 0.2°, 15.9° ± 0.2°, 17.6° ± 0.2°, 19.9° ± 0.2°, 21.9° ± 0.2°, 22.8° ± 0.2°, and 26.6° ± 0.2° in terms of 2θ. The crystal is identified by having 4 or 5 selected from these 10 peaks.

Another embodiment of the invention includes 5-({5-[2-(3-aminopropoxy)-4-methoxypyridin-3-yl]-1H-pyrazol-3-yl}amino)pyrazine-2-carbonitrile, which is a crystalline form (form VI) having an X ray powder diffraction pattern having a characteristic peak at at least 5.3° ± 0.2° in terms of 2θ.

Another embodiment of the invention includes 5-({5-[2-(3-aminopropoxy)-4-methoxypyridin-3-yl]-1H-pyrazol-3-yl}amino)pyrazine-2-carbonitrile, which is a crystalline form (form VI) having an X ray powder diffraction pattern having a characteristic peak at at least 5.7° ± 0.2° in terms of 2θ.

Another embodiment of the invention includes 5-({5-[2-(3-aminopropoxy)-4-methoxypyridin-3-yl]-1H-pyrazol-3-yl}amino)pyrazine-2-carbonitrile, which is a crystalline form (form VI) having an X ray powder diffraction pattern having characteristic peaks at at least 5.3° ± 0.2° and 5.7° ± 0.2° in terms of 2θ.

Another embodiment of the invention includes 5-({5-[2-(3-aminopropoxy)-4-methoxypyridin-3-yl]-1H-pyrazol-3-yl}amino)pyrazine-2-carbonitrile of a crystalline form of form VI, having an X ray powder diffraction pattern having characteristic peaks at 5.3° ± 0.2°, 5.7° ± 0.2°, 7.0° ± 0.2°, 7.3° ± 0.2°, 7.8° ± 0.2°, 8.4° ± 0.2°, 9.3° ± 0.2°, 10.5° ± 0.2°, 11.5° ± 0.2°, and 14.1° ± 0.2° in terms of 2θ. The crystal is identified by having 4 or 5 selected from these 10 peaks.

When the compound of the present disclosure is administered, the amount used varies depending on the symptom, age, dosing method, or the like. For example for intravenous administration, an effect is expected by administering 0.01 mg (preferably 0.1 mg) as a lower limit and 1000 mg (preferably 100 mg) as an upper limit, once or several times daily depending on the symptoms to adults. Examples of dosing schedules include a single dose administration, once daily administration for 3 consecutive days, twice daily administration for 7 consecutive days, and the like. Each dosing method described above can also be repeated with an interval of about 1 day to about 60 days.

The compound of the present disclosure can be administered through parenteral administration or oral administration, preferably through a parenteral method and more preferably through an intravenous injection. The compound of the present disclosure is preferably formulated as a pharmaceutically acceptable carrier such as a liposome and administered.

A liposome encapsulating the compound of the present disclosure comprises at least one type of phospholipid. Examples of phospholipid include phosphatidylcholine, phosphatidylglycerol, phosphatidic acid, phosphatidylethanolamine, phosphatidylserine, phosphatidylinositol, sphingomyelin, and the like.

A fatty acid residue in a phospholipid is not particularly limited. Examples thereof include saturated or unsaturated fatty acid residues with 14 to 18 carbons. Specific examples thereof include myristic acid, palmitic acid, stearic acid, oleic acid, linoleic acid, and other fatty acid derived acyl groups. Egg yolk lecithins, soybean lecithins, and other naturally derived phospholipids, and hydrogenated egg yolk lecithin, hydrogenated soybean lecithin (also known as

hydrogenated soybean phospholipid or hydrogenated soybean phosphatidylcholine), and the like from hydrogenating an unsaturated fatty acid residue thereof can also be used.

Although the amount (molar fraction) of phospholipid added with respect to the entire liposomal membrane components is not particularly limited, the amount is preferably 30 to 80% and more preferably 40 to 70%.

A liposome encapsulating the compound of the present disclosure can comprise sterols. Examples of sterols include cholesterol, β-sitosterol, stigmasterol, campesterol, brassicasterol, ergosterol, fucosterol, and the like. Preferred sterols include cholesterol. Although the amount (molar fraction) of sterols added with respect to the entire liposomal membrane components is not particularly limited, the amount is preferably 0 to 60%, more preferably 10 to 50%, and still more preferably 30 to 50%.

A liposome encapsulating the compound of the invention can comprise a polymer-modified lipid to improve residency *in vivo.* Although the amount (molar fraction) of polymer-modified lipid added with respect to the entire liposomal membrane components is not particularly limited, the amount is preferably 0 to 20%, and more preferably 1 to 10%. The polymer moiety of a polymer-modified lipid is preferably a hydrophilic polymer, and more preferably a hydrophilic polymer having an end that is not bound to a lipid which is alkoxylated. Specific examples of a polymer moiety of a polymer-modified lipid include, but are not particularly limited to, polyethylene glycol, polypropylene glycol, polyvinyl alcohol, polyvinylpyrrolidone, methoxypolyethylene glycol, methoxypolypropylene glycol, methoxypolyvinyl alcohol, methoxypolyvinylpyrrolidone, ethoxypolyethylene glycol, ethoxypolypropylene glycol, ethoxypolyvinyl alcohol, ethoxypolyvinylpyrrolidone, propoxypolyethylene glycol, propoxypolypropylene glycol, propoxypolyvinyl alcohol, and propoxypolyvinylpyrrolidone. Preferred examples of a polymer moiety of a polymer-modified lipid include polyethylene glycol, methoxypolyethylene glycol, methoxypolypropylene glycol, ethoxypolyethylene glycol, ethoxypolypropylene glycol, propoxypolyethylene glycol, and propoxypolypropylene glycol. More preferred examples of a polymer moiety of a polymer-modified lipid include polyethylene glycol, methoxypolyethylene glycol, ethoxypolyethylene glycol, ethoxypolypropylene glycol, and propoxypolyethylene glycol. Still more preferred examples of a polymer moiety of a polymer-modified lipid include polyethylene glycol and methoxypolyethylene glycol. The most preferred examples of a polymer moiety of a polymer-modified lipid include methoxypolyethylene glycol. The molecular weight of the polymer moiety of a polymer-modified lipid is not particularly limited, but is, for example, 100 to 10000 daltons, preferably 1000 to 7000 daltons, more preferably 1500 to 5000 daltons, and most preferably 1500 to 3000 daltons.

The specific lipid moiety of a polymer-modified lipid is not particularly limited. Examples thereof include phosphatidylethanolamine and diacylglycerol. The lipid moiety of a polymer-modified lipid is preferably phosphatidylethanolamine having a saturated or unsaturated fatty acid residue with 14 to 18 carbons or diacylglycerol having a saturated or unsaturated fatty acid residue with 14 to 18 carbons, more preferably phosphatidylethanolamine having a saturated fatty acid residue with 14 to 18 carbons or diacylglycerol having a saturated fatty acid residue with 14 to 18 carbons, still more preferably phosphatidylethanolamine with a palmitoyl group or stearoyl group or diacylglycerol with a palmitoyl group or stearoyl group. The lipid moiety of a polymer-modified lipid is most preferably distearoylphosphatidylethanolamine.

A liposome encapsulating the compound of the present disclosure can comprise a pharmaceutically acceptable additive. Examples of additives include inorganic acids, inorganic acid salts, organic acids, organic acid salts, saccharides, buffer, antioxidants, and polymers.

Examples of the inorganic acids include phosphoric acid, hydrochloric acid, and sulfuric acid.

Examples of the inorganic acid salts include sodium hydrogen phosphate, sodium chloride, ammonium sulfate, and magnesium sulfate.

Examples of the organic acids include citric acid, acetic acid, succinic acid, and tartaric acid.

Examples of the organic acid salts include sodium citrate, sodium acetate, disodium succinate, and sodium tartrate.

Examples of the saccharides include glucose, sucrose, mannitol, sorbitol, and trehalose.

Examples of the buffer include L-arginine, L-histidine, trometamol (trishydroxymethylaminomethane, Tris), and salts thereof.

Examples of the antioxidants include sodium sulfite, L-cysteine, sodium thioglycolate, sodium thiosulfate, ascorbic acid, and tocopherol.

Examples of the polymers include polyvinyl alcohol, polyvinylpyrrolidone, carboxyvinyl polymer, and carboxymethylcellulose sodium.

The manufacturing method of the compound of the present disclosure represented by formula (1) is exemplified hereinafter with examples, but the manufacturing method of the compound of the present disclosure is not limited thereto. A compound used in the following manufacturing method may form a salt, as long as the reaction is not affected.

The compound of the present disclosure can be manufactured using a known compound as the starting material by, for example, Manufacturing Method A, B, C, D, E, F, G, H, I, J, K, L, M, N, O, P, Q, or R described below, a method in accordance therewith, or an appropriate combination of synthetic methods that are well known to those skilled in the art. The compound of the present disclosure other than formula (a2) can also be manufactured by a method in accordance

therewith or an appropriate combination of synthetic methods that are well known to those skilled in the art.

Manufacturing Method A

The compound of the present disclosure represented by formula (a2) can be manufactured by, for example, the following method.

[Chemical Formula 17]

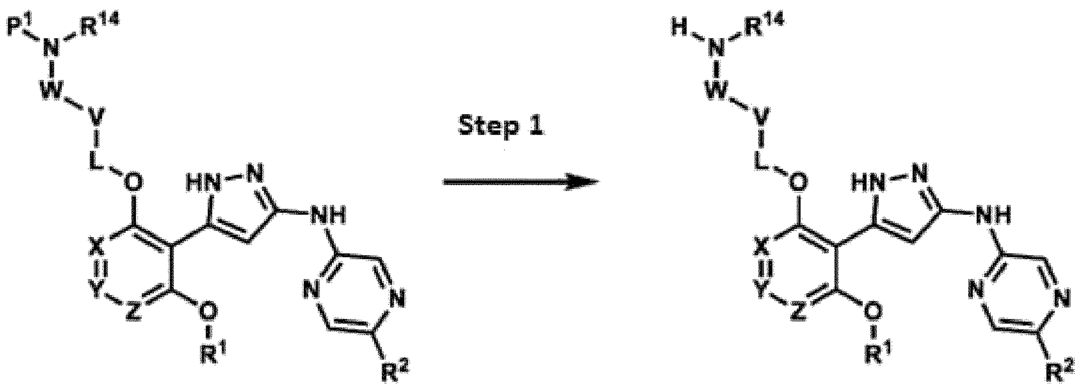


(a1)

(a2)

wherein $R^1$, $R^2$, $R^{14}$, X, Y, Z, L, V, and W are defined the same as the description in item 1, and $P^1$ refers to an amino protecting group. Examples of $P^1$ include the amino protecting groups described in Protective Groups in Organic Synthesis (authors: Theodora W. Greene, Peter G. M. Wuts, publisher: John Wiley & Sons, Inc., 1999) and the like.

[Step 1]

Compound (a2) can be manufactured by removing protecting group $P^1$ of compound (a1) obtained by the following manufacturing method. This step can be performed in accordance with, for example, the method described in Protective Groups in Organic Synthesis (authors: Theodora W. Greene, Peter G. M. Wuts, publisher: John Wiley & Sons, Inc., 1999) or the like.

Manufacturing Method B

The compound of the present disclosure represented by formula (a1) can be manufactured, for example, by the following method.

[Chemical Formula 18]

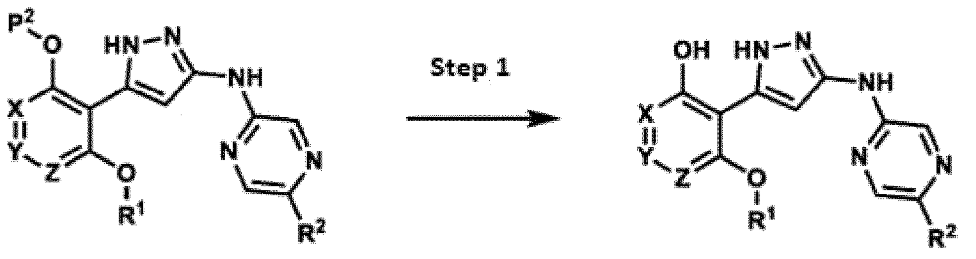


(b1) (b2)

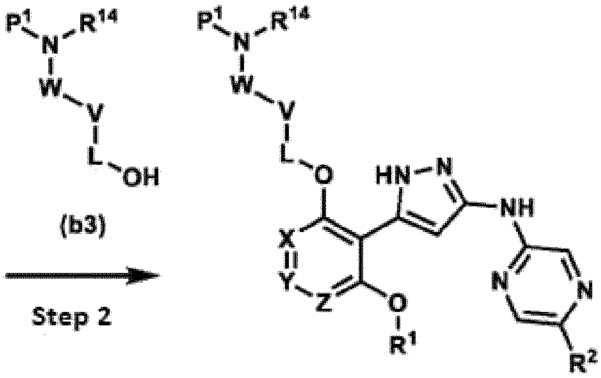


(a1)

wherein $R^1$, $R^2$, $R^{14}$, X, Y, Z, L, V, and W are defined the same as the description in item 1, $P^1$ refers to an amino protecting group, and $P^2$ refers to a phenol protecting group. Examples of $P^1$ include the amino protecting groups described in Protective Groups in Organic Synthesis (authors: Theodora W. Greene, Peter G. M. Wuts, publisher: John Wiley & Sons, Inc., 1999) and the like, and examples of $P^2$ include the phenol protecting groups described in Protective Groups in Organic Synthesis (authors: Theodora W. Greene, Peter G. M. Wuts, publisher: John Wiley & Sons, Inc., 1999) and the like.

Compound (b3) is available as a commercial product.

[Step 1]

Compound (b2) can be manufactured by removing the protecting group $P^2$ of compound (b1) obtained by the following manufacturing method. This step can be performed in accordance with, for example, the method described in Protective Groups in Organic Synthesis (authors: Theodora W. Greene, Peter G. M. Wuts, publisher: John Wiley & Sons, Inc., 1999) or the like.

[Step 2]

Compound (a1) can be manufactured by a Mitsunobu reaction between compound (b2) and compound (b3) in a suitable solvent in the presence of a Mitsunobu reaction.

Examples of Mitsunobu reagents include diethyl azodicarboxylate (DEAD), diisopropyl azodicarboxylate (DIAD), N,N,N',N'-tetraisopropyl azodicarboxamide (TIPA), 1,1'-(azodicarbonyl)dipiperidine (ADDP), N,N,N',N'-tetramethylazodicarboxamide (TMAD), triphenylphosphine, tributylphosphine, and the like. Cyanomethylene trimethylphosphorane (CMMP) and cyanomethylene tributylphophorane (CMBP) can also be used.

A solvent is not particularly limited as long as it is a solvent that does not react under the reaction conditions of this step. Examples thereof include ether solvents such as diethyl ether, diisopropyl ether, tetrahydrofuran, methylcyclopentyl ether, anisole, and 1,4-dioxane; aromatic hydrocarbon solvents such as benzene, toluene, chlorobenzene, and xylene; ester solvents such as ethyl acetate and methyl acetate; aprotic solvents such as N,N-dimethylformamide, N,N-dimethylacetamide, N-methyl-2-pyrrolidinone, 1,3-dimethyl-2-imidazolidinone, and dimethyl sulfoxide; mixtures thereof,

and the like. Preferred examples of solvents include toluene, benzene, THF, 1,4-dioxane, mixtures thereof, and the like.

The reaction time is generally 5 minutes to 72 hours, and preferably 12 hours to 24 hours.

The reaction temperature is generally 0°C to 100°C and preferably 0°C to 50°C.

Manufacturing Method C

The compound of the present disclosure represented by formula (b1) can be manufactured, for example, by the following method.

[Chemical Formula 19]

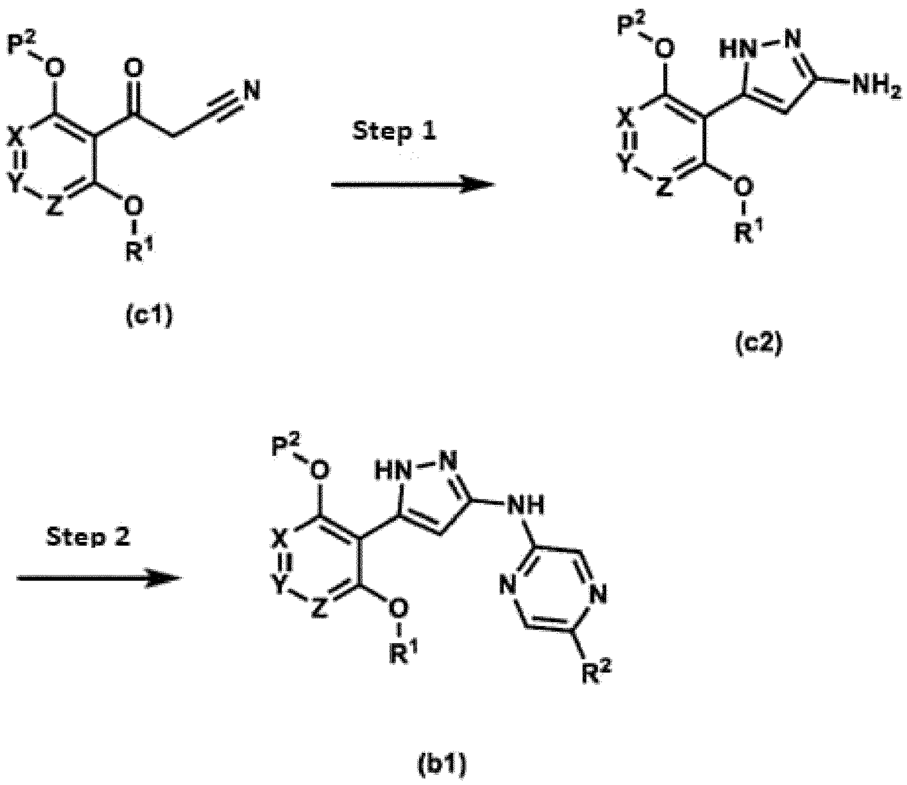


(c1) (c2) (b1)

wherein $R^1$, $R^2$, X, Y, and Z are defined the same as the description in item 1, and $P^2$ refers to a phenol protecting group. Examples of $P^2$ include the phenol protecting groups described in Protective Groups in Organic Synthesis (authors: Theodora W. Greene, Peter G. M. Wuts, publisher: John Wiley & Sons, Inc., 1999) and the like.

[Step 1]

Compound (c2) can be manufactured by reacting compound (c1) with hydrazine monohydrate in the presence or absence of a suitable acid in a suitable solvent.

Examples of acids include acetic acid, propionic acid, p-toluenesulfonic acid, methanesulfonic acid, ethanesulfonic acid, hydrochloric acid, sulfuric acid, camphorsulfonic acid, and the like. An acid is preferably acetic acid, p-toluenesulfonic acid, or the like.

A solvent is not particularly limited as long as it is a solvent that does not react under the reaction conditions of this step. Examples thereof include ether solvents such as diethyl ether, diisopropyl ether, tetrahydrofuran, methylcyclopentyl ether, anisole, and 1,4-dioxane; aromatic hydrocarbon solvents such as benzene, toluene, chlorobenzene, and xylene; ester solvents such as ethyl acetate and methyl acetate; aprotic solvents such as N,N-dimethylformamide, N,N-dimethylacetamide, N-methyl-2-pyrrolidinone, 1,3-dimethyl-2-imidazolidinone, and dimethyl sulfoxide; alcohol solvents such as methanol, ethanol, isopropyl alcohol, and butanol; and mixtures thereof. Preferred solvents include 1,4-dioxane, toluene, ethanol, and the like.

The reaction time is generally 5 minutes to 72 hours, and preferably 12 hours to 24 hours.

The reaction temperature is generally 0°C to 200°C, and preferably 50°C to 100°C.

[Step 2]

Compound (b1) can be manufactured by reacting compound (c2) with a 5-chloropyrazine derivative in the presence of a suitable base in a suitable solvent. Compound (b1) can also be manufactured by reacting compound (c2) with a 5-chloropyrazine derivative in the presence of a suitable palladium catalyst, ligand, and base in a suitable solvent.
Examples of the base include organic bases such as triethylamine, diisopropylethylamine, tributylamine, 1,5-diazabicyclo[4.3.0]non-5-ene (DBN), 1,8-diazabicyclo[5.4.0]undec-7-ene (DBU), pyridine, dimethylaminopyridine, picoline, N-methylmorpholine (NMM), and N-ethylmorpholine, and inorganic bases such as sodium bicarbonate, potassium bicarbonate, sodium carbonate, potassium carbonate, cesium carbonate, sodium hydroxide, and potassium hydroxide. Preferred examples of bases include triethylamine, diisopropylethylamine, N-ethylmorpholine, potassium carbonate, cesium carbonate, and the like.
Examples of palladium catalysts include salts of palladium chloride or palladium acetate, zerovalent palladium complexes such as tris(dibenzylideneacetone)dipalladium(0) chloroform adduct, and the like.
Examples of ligands include 4,5-bis(diphenylphosphino)-9,9-dimethylxanthene (xantphos), 2,2'-bis(diphenylphosphino)-1,1'-binaphthyl (BINAP), 2-dicyclohexylphosphino-2',6'-dimethoxybiphenyl (SPhos), 2-dicyclohexylphosphino-2',4',6'-triisopropylbiphenyl (XPhos), and other phosphine ligands. Preferred examples of ligands include 4,5-bis(diphenylphosphino)-9,9-dimethylxanthene (xantphos).
A solvent is not particularly limited as long as it is a solvent that does not react under the reaction conditions of this step. Examples thereof include ether solvents such as diethyl ether, diisopropyl ether, tetrahydrofuran, methylcyclopentyl ether, anisole, and 1,4-dioxane; aromatic hydrocarbon solvents such as benzene, toluene, chlorobenzene, and xylene; ester solvents such as ethyl acetate and methyl acetate; aprotic solvents such as N,N-dimethylformamide, N,N-dimethylacetamide, N-methyl-2-pyrrolidinone, 1,3-dimethyl-2-imidazolidinone, and dimethyl sulfoxide; and mixtures thereof. Preferred examples of solvents include tetrahydrofuran, 1,4-dioxane, toluene, dimethyl sulfoxide, and the like.
The reaction time is generally 5 minutes to 48 hours, and preferably 1 hour to 6 hours.
The reaction temperature is generally 0°C to 200°C, and preferably 50°C to 100°C.

Manufacturing Method D

The compound of the present disclosure represented by formula (c1) can be manufactured, for example, by the following method.

[Chemical Formula 20]

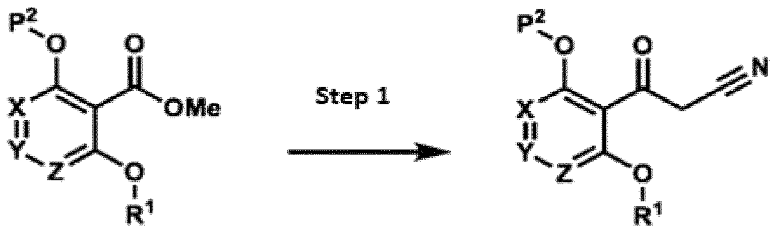


(d1) (c1)

wherein $R^1$, X, Y, and Z are defined the same as the description in item 1, and $P^2$ refers to a phenol protecting group. Examples of $P^2$ include the phenol protecting groups described in Protective Groups in Organic Synthesis (authors: Theodora W. Greene, Peter G. M. Wuts, publisher: John Wiley & Sons, Inc., 1999) and the like.

[Step 1]

Compound (c1) can be manufactured by reacting compound (d1) with acetonitrile in the presence of a suitable base in a suitable solvent.
An inorganic base can be used as the base. Examples of the inorganic base include hydrides such as sodium hydride, halogenated alkali such as potassium fluoride, alkali hydroxides such as sodium hydroxide and potassium hydroxide, alkali carbonates such as sodium carbonate, potassium carbonate, cesium carbonate, and sodium bicarbonate, alkali alkoxides such as sodium ethoxide, sodium tert-butoxide, and potassium tert-butoxide, and alkali metals such as n-butyllithium, methyllithium, and isopropylmagnesium bromide. Preferred examples of bases include sodium ethoxide, sodium tert-butoxide, potassium tert-butoxide, n-butyllithium, and the like.
A solvent is not particularly limited as long as it is a solvent that does not react under the reaction conditions of this

step. Examples thereof include ether solvents such as diethyl ether, diisopropyl ether, tetrahydrofuran, methylcyclopentyl ether, anisole, and 1,4-dioxane; aromatic hydrocarbon solvents such as benzene, toluene, chlorobenzene, and xylene; ester solvents such as ethyl acetate and methyl acetate; aprotic solvents such as N,N-dimethylformamide, N,N-dimethylacetamide, N-methyl-2-pyrrolidinone, 1,3-dimethyl-2-imidazolidinone, and dimethyl sulfoxide; alcohol solvents such as methanol, ethanol, isopropyl alcohol, and butanol; and mixtures thereof. Preferred solvents include tetrahydrofuran, toluene, and the like.

The reaction time is generally 5 minutes to 72 hours, and preferably 12 hours to 24 hours.

The reaction temperature is generally 0°C to 200°C, and preferably 50°C to 100°C.

Manufacturing Method E

The compound of the present disclosure represented by formula (b1) can be manufactured, for example, by the following method.

[Chemical Formula 21]

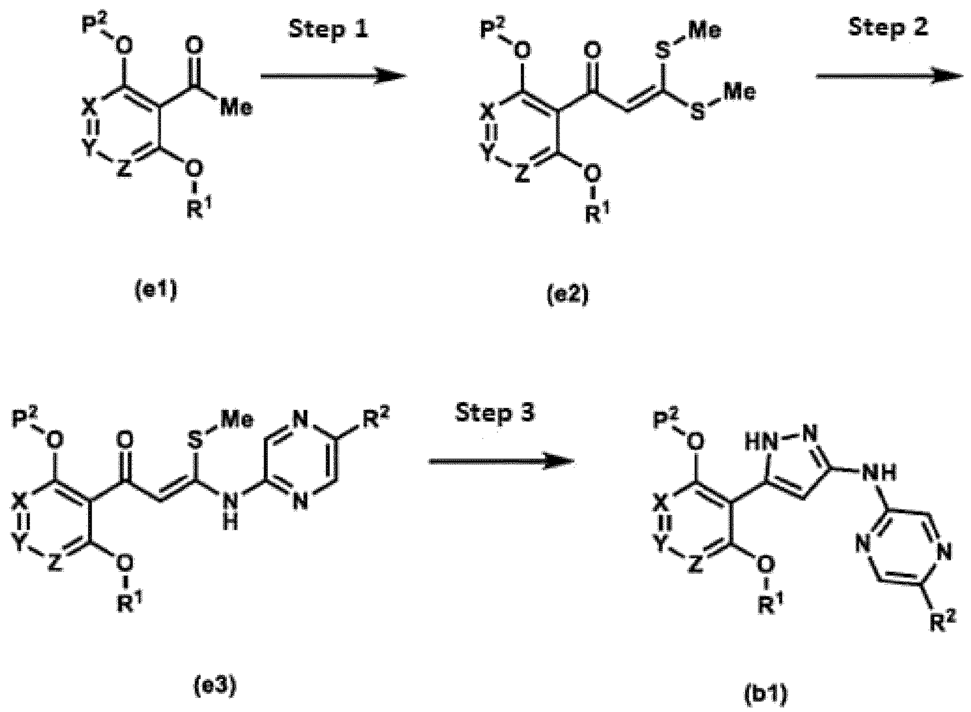


(e1) (e2) (e3) (b1)

wherein $R^1$, $R^2$, X, Y, and Z are defined the same as the description in item 1, and $P^2$ refers to a phenol protecting group. Examples of $P^2$ include the phenol protecting groups described in Protective Groups in Organic Synthesis (authors: Theodora W. Greene, Peter G. M. Wuts, publisher: John Wiley & Sons, Inc., 1999) and the like.

[Step 1]

Compound (e2) can be manufactured by reacting compound (e1) with carbon disulfide and iodomethane in the presence of a suitable base in a suitable solvent.

An inorganic base can be used as the base. Examples of the inorganic base include hydrides such as sodium hydride, alkali halides such as potassium fluoride, alkali hydroxides such as sodium hydroxide and potassium hydroxide, alkali carbonates such as sodium carbonate, potassium carbonate, cesium carbonate, and sodium bicarbonate, alkali alkoxides such as sodium ethoxide, sodium tert-butoxide, and potassium tert-butoxide, and alkali metals such as n-butyllithium, methyllithium, and isopropylmagnesium bromide. Preferred examples of bases include sodium hydride, potassium tert-butoxide, and the like.

A solvent is not particularly limited as long as it is a solvent that does not react under the reaction conditions of this step. Examples thereof include ether solvents such as diethyl ether, diisopropyl ether, tetrahydrofuran, methylcyclopentyl ether, anisole, and 1,4-dioxane; aromatic hydrocarbon solvents such as benzene, toluene, chlorobenzene, and xylene; ester solvents such as ethyl acetate and methyl acetate; aprotic solvents such as N,N-dimethylformamide, N,N-dimethylacetamide, N-methyl-2-pyrrolidinone, 1,3-dimethyl-2-imidazolidinone, and dimethyl sulfoxide; alcohol solvents such as methanol, ethanol, isopropyl alcohol, and butanol; and mixtures thereof. Preferred examples of solvents include tetrahydrofuran, toluene, N,N-dimethylformamide, and the like.

The reaction time is generally 5 minutes to 72 hours, and preferably 30 minutes to 2 hours.

The reaction temperature is generally -78°C to 200°C, and preferably 0°C to 25°C.

[Step 2]

Compound (e3) can be manufactured by reacting compound (e2) with a 5-aminopyrazine derivative in the presence of a suitable base in a suitable solvent.

Examples of the base include organic bases such as triethylamine, diisopropylethylamine, tributylamine, 1,5-diazabicyclo[4.3.0]non-5-ene (DBN), 1,8-diazabicyclo[5.4.0]undec-7-ene (DBU), pyridine, dimethylaminopyridine, picoline, N-methylmorpholine (NMM), and N-ethylmorpholine, and inorganic bases such as sodium bicarbonate, potassium bicarbonate, sodium carbonate, potassium carbonate, sodium hydroxide, and potassium hydroxide. Preferred examples of bases include triethylamine, diisopropylethylamine, and the like.

A solvent is not particularly limited as long as it is a solvent that does not react under the reaction conditions of this step. Examples thereof include ether solvents such as diethyl ether, diisopropyl ether, tetrahydrofuran, methylcyclopentyl ether, anisole, and 1,4-dioxane; aromatic hydrocarbon solvents such as benzene, toluene, chlorobenzene, and xylene; ester solvents such as ethyl acetate and methyl acetate; aprotic solvents such as N,N-dimethylformamide, N,N-dimethylacetamide, N-methyl-2-pyrrolidinone, 1,3-dimethyl-2-imidazolidinone, and dimethyl sulfoxide; and mixtures thereof. Preferred examples of solvents include tetrahydrofuran, toluene, dimethyl sulfoxide, and the like.

The reaction time is generally 5 minutes to 48 hours, and preferably 2 hours to 8 hours.

The reaction temperature is generally 0°C to 200°C, and preferably 50°C to 100°C.

[Step 3]

Compound (b1) can be manufactured by reacting compound (e3) with a hydrazine monohydrate in the presence or absence of a suitable acid in a suitable solvent.

Examples of acids include acetic acid, propionic acid, p-toluenesulfonic acid, methanesulfonic acid, ethanesulfonic acid, hydrochloric acid, sulfuric acid, camphorsulfonic acid, and the like. An acid is preferably acetic acid.

A solvent is not particularly limited as long as it is a solvent that does not react under the reaction conditions of this step. Examples thereof include ether solvents such as diethyl ether, diisopropyl ether, tetrahydrofuran, methylcyclopentyl ether, anisole, and 1,4-dioxane; aromatic hydrocarbon solvents such as benzene, toluene, chlorobenzene, and xylene; ester solvents such as ethyl acetate and methyl acetate; aprotic solvents such as N,N-dimethylformamide, N,N-dimethylacetamide, N-methyl-2-pyrrolidinone, 1,3-dimethyl-2-imidazolidinone, and dimethyl sulfoxide; alcohol solvents such as methanol, ethanol, isopropyl alcohol, and butanol; and mixtures thereof. Preferred examples of solvents include 1,4-dioxane, ethanol, and the like.

The reaction time is generally 5 minutes to 72 hours, and preferably 12 hours to 24 hours.

The reaction temperature is generally 0°C to 200°C, and preferably 50°C to 100°C.

Manufacturing Method F

The compound of the present disclosure represented by formula (c1) can be manufactured, for example, by the following method.

[Chemical Formula 22]

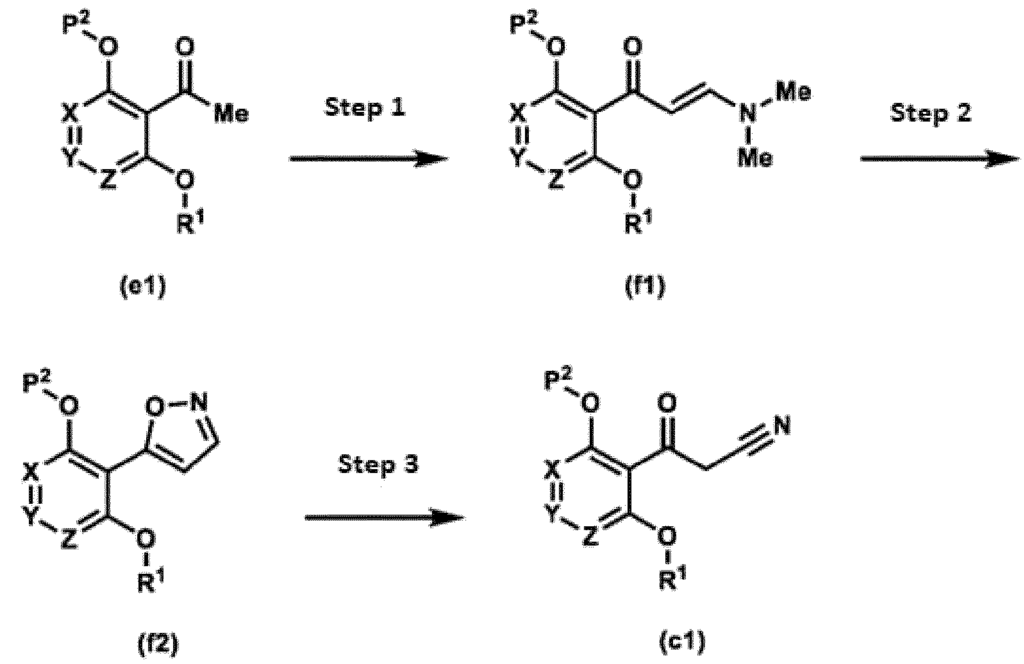


(e1) (f1) (f2) (c1)

wherein $R^1$, X, Y, and Z are defined the same as the description in item 1, and $P^2$ refers to a phenol protecting group. Examples of $P^2$ include the phenol protecting groups described in Protective Groups in Organic Synthesis (authors: Theodora W. Greene, Peter G. M. Wuts, publisher: John Wiley & Sons, Inc., 1999) and the like.

[Step 1]

Compound (f1) can be manufactured by reacting compound (e1) with dimethylformamide dimethyl acetal in a suitable solvent.
A solvent is not particularly limited as long as it is a solvent that does not react under the reaction conditions of this step. Examples thereof include ether solvents such as diethyl ether, diisopropyl ether, tetrahydrofuran, methylcyclopentyl ether, anisole, and 1,4-dioxane; aromatic hydrocarbon solvents such as benzene, toluene, chlorobenzene, and xylene; ester solvents such as ethyl acetate and methyl acetate; aprotic solvents such as N,N-dimethylformamide, N,N-dimethylacetamide, N-methyl-2-pyrrolidinone, 1,3-dimethyl-2-imidazolidinone, and dimethyl sulfoxide; and mixtures thereof. Preferred examples of solvents include N,N-dimethylformamide and the like.
The reaction time is generally 5 minutes to 48 hours, and preferably 24 hours to 48 hours.
The reaction temperature is generally 0°C to 200°C, and preferably 60°C to 120°C.

[Step 2]

Compound (f2) can be manufactured by reacting compound (f1) with hydroxyamine hydrochloride in a suitable solvent.
A solvent is not particularly limited as long as it is a solvent that does not react under the reaction conditions of this step. Examples thereof include ether solvents such as diethyl ether, diisopropyl ether, tetrahydrofuran, methylcyclopentyl ether, anisole, and 1,4-dioxane; aromatic hydrocarbon solvents such as benzene, toluene, chlorobenzene, and xylene; ester solvents such as ethyl acetate and methyl acetate; aprotic solvents such as N,N-dimethylformamide, N,N-dimethylacetamide, N-methyl-2-pyrrolidinone, 1,3-dimethyl-2-imidazolidinone, and dimethyl sulfoxide; alcohol solvents such as methanol, ethanol, isopropyl alcohol, and butanol; and mixtures thereof. Preferred examples of solvents include ethanol, isopropyl alcohol, and the like.
The reaction time is generally 5 minutes to 48 hours, and preferably 6 hours to 12 hours.
The reaction temperature is generally 0°C to 200°C, and preferably 40°C to 80°C.

[Step 3]

Compound (c1) can be manufactured by reacting compound (f2) with a base in a suitable solvent.
Examples of the base include inorganic bases such as sodium bicarbonate, potassium bicarbonate, sodium carbonate, potassium carbonate, sodium hydroxide, and potassium hydroxide. Preferred examples of the base include

sodium hydroxide, potassium hydroxide, and the like.

A solvent is not particularly limited as long as it is a solvent that does not react under the reaction conditions of this step. Examples thereof include ether solvents such as diethyl ether, diisopropyl ether, tetrahydrofuran, methylcyclopentyl ether, anisole, and 1,4-dioxane; aromatic hydrocarbon solvents such as benzene, toluene, chlorobenzene, and xylene; ester solvents such as ethyl acetate and methyl acetate; aprotic solvents such as N,N-dimethylformamide, N,N-dimethylacetamide, N-methyl-2-pyrrolidinone, 1,3-dimethyl-2-imidazolidinone, and dimethyl sulfoxide; protic solvents such as water, methanol, ethanol, isopropyl alcohol, and butanol; and mixtures thereof. Preferred examples of solvents include mixtures of water and ethanol, etc.

The reaction time is generally 5 minutes to 48 hours, and preferably 1 hour to 6 hours.

The reaction temperature is generally 0°C to 200°C, and preferably 20°C to 50°C.

Manufaturing Method G

The compound of the present disclosure represented by formula (a1) can be manufactured, for example, by the following method.

[Chemical Formula 23]

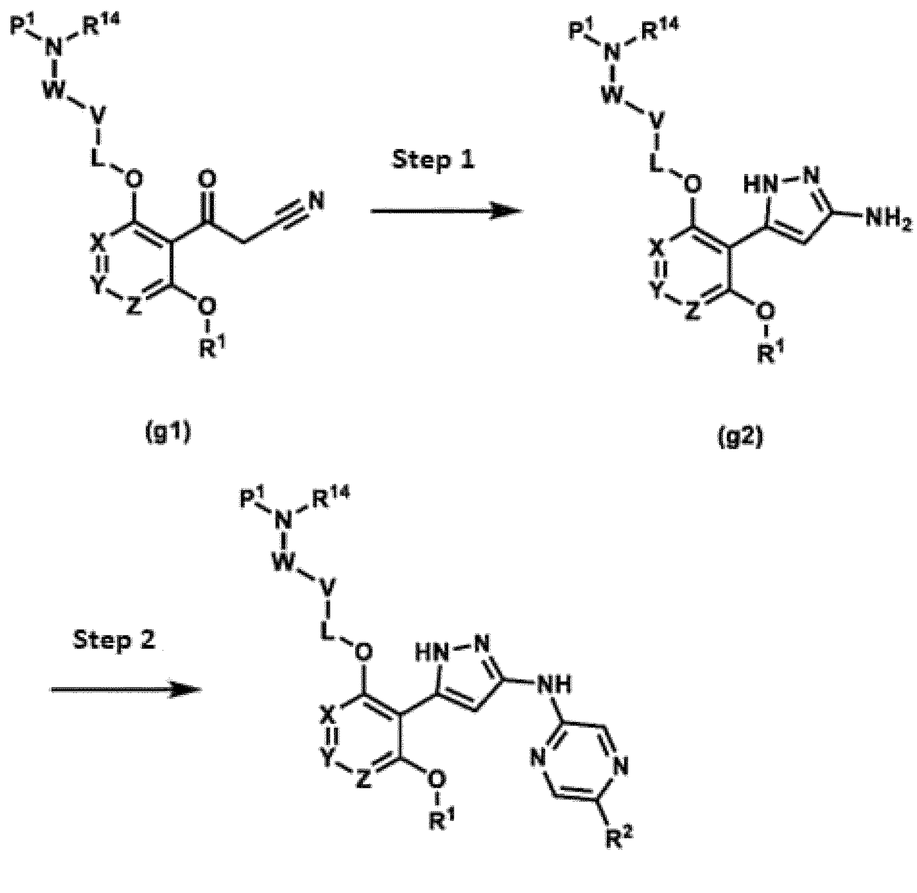


(g1)

(g2)

(a1)

wherein $R^1$, $R^2$, $R^{14}$, X, Y, Z, L, V, and W are defined the same as the description in item 1, and $P^1$ refers to an amino protecting group. Examples of $P^1$ include the amino protecting groups described in Protective Groups in Organic Synthesis (authors: Theodora W. Greene, Peter G. M. Wuts, publisher: John Wiley & Sons, Inc., 1999) and the like.

[Step 1]

Compound (g2) can be manufactured from compound (g1) obtained through a manufacturing method described below, by the method described in step 1 of Manufacturing Method C or a method in accordance therewith.

[Step 2]

Compound (a1) can be manufactured from compound (g2) by the method described in step 2 of Manufacturing Method C or a method in accordance therewith.

Manufacturing Method H

A compound represented by formula (a1) can be manufactured, for example, by the following method.

[Chemical Formula 24]

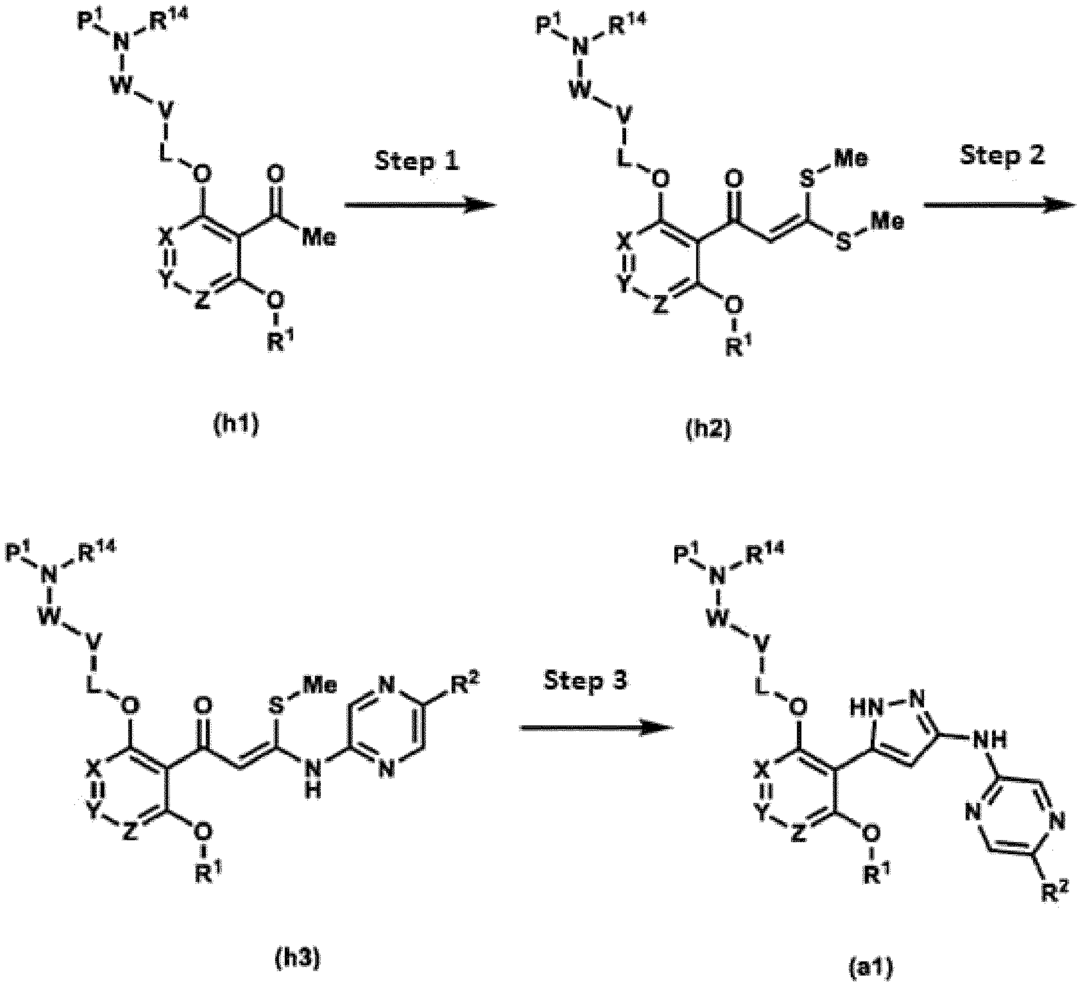


(h1) (h2) (h3) (a1)

wherein $R^1$, $R^2$, $R^{14}$, X, Y, Z, L, V, and W are defined the same as the description in item 1, and $P^1$ refers to an amino protecting group. Examples of $P^1$ include the amino protecting groups described in Protective Groups in Organic Synthesis (authors: Theodora W. Greene, Peter G. M. Wuts, publisher: John Wiley & Sons, Inc., 1999) and the like.

[Step 1]

Compound (h2) can be manufactured from compound (h1) obtained through the following method, by the method described in step 1 of Manufacturing Method E or a method in accordance therewith.

[Step 2]

Compound (h3) can be manufactured from compound (h2) by the method described in step 2 of Manufacturing Method E or a method in accordance therewith.

[Step 3]

Compound (a1) can be manufactured from compound (h3) by the method described in step 3 of Manufacturing Method E or a method in accordance therewith.

Manufacturing Method I

A compound represented by formula (g1) can be manufactured, for example, by the following method.

[Chemical Formula 25]

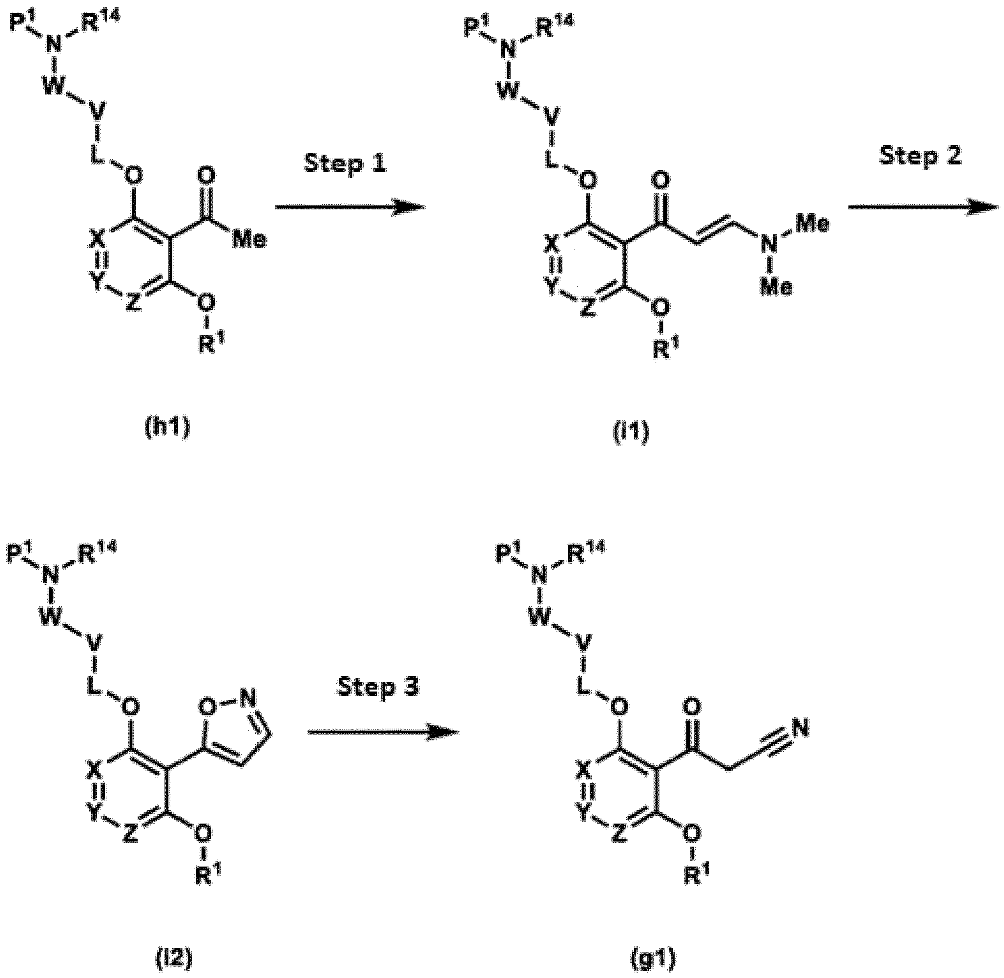


(h1) (i1)

(i2) (g1)

wherein $R^1$, $R^{14}$, X, Y, Z, L, V, and W are defined the same as the description in item 1, and $P^1$ refers to an amino protecting group. Examples of $P^1$ include the amino protecting groups described in Protective Groups in Organic Synthesis (authors: Theodora W. Greene, Peter G. M. Wuts, publisher: John Wiley & Sons, Inc., 1999) and the like.

[Step 1]

Compound (i1) can be manufactured from compound (h1) obtained through the following manufacturing method, by the method described in step 1 of Manufacturing Method F or a method in accordance therewith.

[Step 2]

Compound (i2) can be manufactured from compound (i1) by the method described in step 2 of Manufacturing Method F or a method in accordance therewith.

[Step 3]

Compound (g1) can be manufactured from compound (i2) by the method described in step 3 of Manufacturing Method F or a method in accordance therewith.

Manufacturing Method J

A compound represented by formula (g1) can also be manufactured by, for example, the following method.

[Chemical Formula 26]

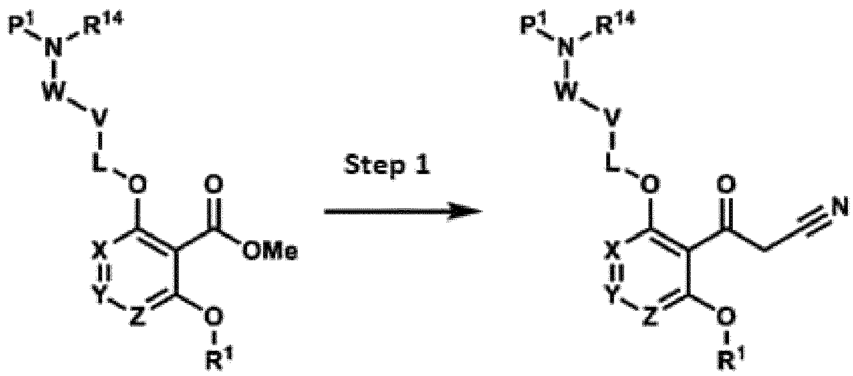


(j1) (g1)

wherein $R^1$, $R^{14}$, X, Y, Z, L, V, and W are defined the same as the description in item 1, and $P^1$ refers to an amino protecting group. Examples of $P^1$ include the amino protecting groups described in Protective Groups in Organic Synthesis (authors: Theodora W. Greene, Peter G. M. Wuts, publisher: John Wiley & Sons, Inc., 1999) and the like.

[Step 1]

Compound (g1) can be manufactured from compound (j1) obtained through a manufacturing method described below, by the method described in step 1 of Manufacturing Method D or a method in accordance therewith.

Manufacturing Method K

The compound of the present disclosure represented by formula (d1) can be manufactured, for example, by the following method.

[Chemical Formula 27]

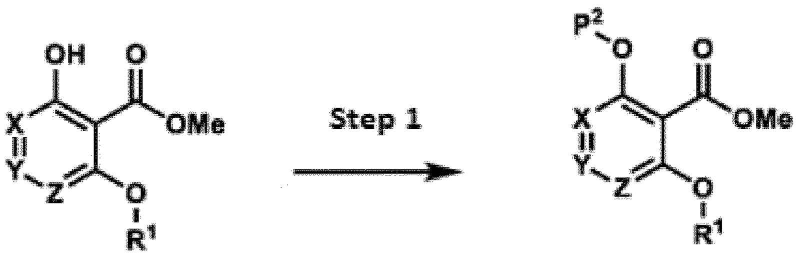


(k1) (d1)

wherein $R^1$, X, Y, and Z are defined the same as the description in item 1, and $P^2$ refers to a phenol protecting group. Examples of $P^2$ include the phenol protecting groups described in Protective Groups in Organic Synthesis (authors: Theodora W. Greene, Peter G. M. Wuts, publisher: John Wiley & Sons, Inc., 1999) and the like.

Compound (k1) is available as a commercial product.

[Step 1]

Compound (d1) can be manufactured from compound (k1) by, for example, the method described in Protective Groups in Organic Synthesis (authors: Theodora W. Greene, Peter G. M. Wuts, publisher: John Wiley & Sons, Inc., 1999).

Manufacturing Method L

The compound of the present disclosure represented by formula (e1) can be manufactured, for example, by the following method.

[Chemical Formula 28]

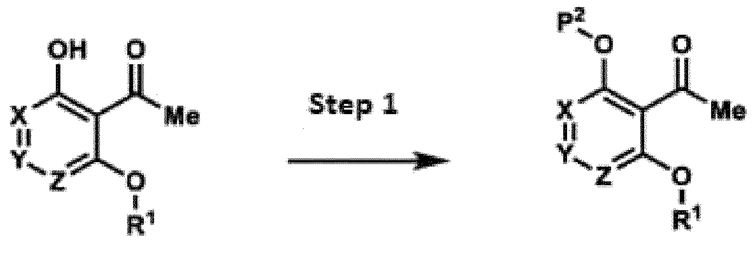


(l1)

(e1)

wherein $R^1$, X, Y, and Z are defined the same as the description in item 1, and $P^2$ refers to a phenol protecting group. Examples of $P^2$ include the phenol protecting groups described in Protective Groups in Organic Synthesis (authors: Theodora W. Greene, Peter G. M. Wuts, publisher: John Wiley & Sons, Inc., 1999) and the like.

Compound (l1) is available as a commercial product.

[Step 1]

Compound (e1) can be manufactured from compound (l1) by the method described in step 1 of Manufacturing Method K or a method in accordance therewith.

Manufacturing Method M

The compound of the present disclosure represented by formula (j1) can be manufactured, for example, by the following method.

[Chemical Formula 29]

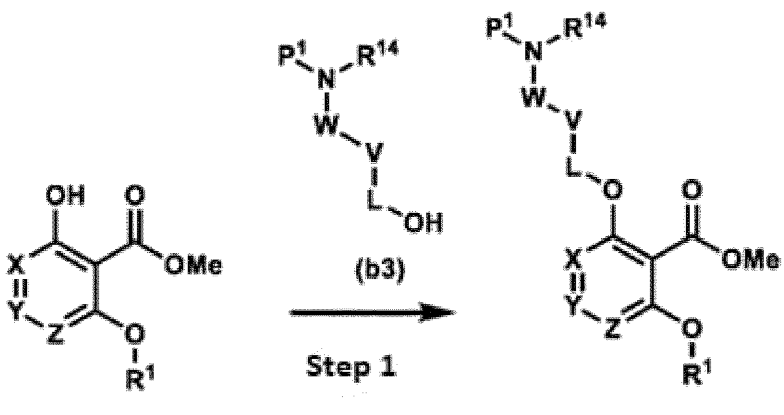


(k1)

(j1)

wherein $R^1$, $R^{14}$, X, Y, Z, L, V, and W are defined the same as the description in item 1, and $P^1$ refers to an amino protecting group. Examples of $P^1$ include the amino protecting groups described in Protective Groups in Organic Synthesis (authors: Theodora W. Greene, Peter G. M. Wuts, publisher: John Wiley & Sons, Inc., 1999) and the like.

Compound (k1) and compound (b3) are available as commercial products.

[Step 1]

Compound (j1) can be manufactured from compound (k1) and compound (b3) by the method described in step 2 of Manufacturing Method B or a method in accordance therewith.

Manufacturing Method N

The compound of the present disclosure represented by formula (j1) can be manufactured, for example, by the following method.

[Chemical Formula 30]

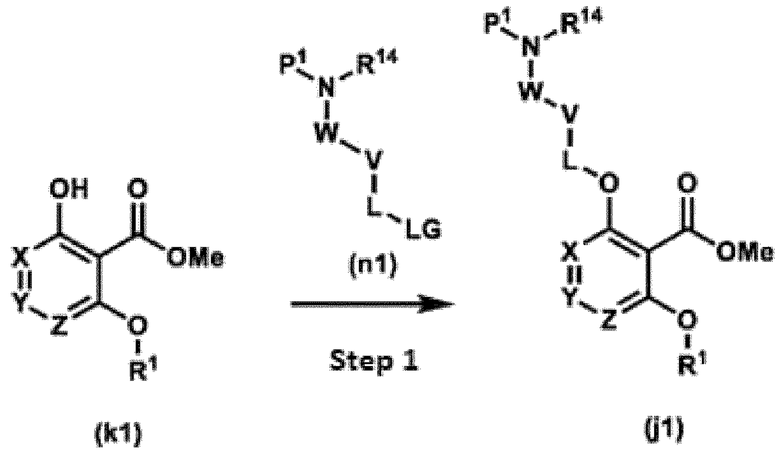


wherein $R^1$, $R^{14}$, X, Y, Z, L, V, and W are defined the same as the description in item 1, $P^1$ refers to an amino protecting group, and LG refers to a leaving group. Examples of $P^1$ include the amino protecting groups described in Protective Groups in Organic Synthesis (authors: Theodora W. Greene, Peter G. M. Wuts, publisher: John Wiley & Sons, Inc., 1999) and the like. Examples of LG include halogen, methanesulfonyloxy, p-toluenesulfonyloxy, trifluoromethanesulfonyloxy, and the like.

Compound (k1) and compound (n1) are available as commercial products.

[Step 1]

Compound (j1) can be manufactured by reacting compound (k1) and compound (n1) in the presence or absence of a suitable base in a suitable solvent.

Examples of the base include organic bases such as triethylamine, diisopropylethylamine, tributylamine, 1.5-diazabicyclo[4.3.0]non-5-ene (DBN), 1,8-diazabicyclo[5.4.0]undec-7-ene (DBU), pyridine, 4-dimethylaminopyridine, picoline, and N-methylmorpholine (NMM) and inorganic bases such as sodium bicarbonate, potassium bicarbonate, sodium carbonate, potassium carbonate, sodium hydroxide, and potassium hydroxide. Preferred examples of the base include triethylamine, diisopropylethylamine, potassium carbonate, sodium hydroxide, and the like.

A solvent is not particularly limited as long as it is a solvent that does not react under the reaction conditions of this step. Examples thereof include alcohol solvents such as methanol, ethanol, 2-propanol (isopropyl alcohol), and tert-butanol; ether solvents such as diethyl ether, diisopropyl ether, tetrahydrofuran, methylcyclopentyl ether, and 1,4-dioxane; aromatic hydrocarbon solvents such as benzene, toluene, chlorobenzene, anisole, and xylene; ester solvents such as ethyl acetate and methyl acetate; aprotic solvents such as acetonitrile, N,N-dimethylformamide, N,N-dimethylacetamide, N-methyl-2-pyrrolidinone, 1,3-dimethyl-2-imidazolidinone, and dimethyl sulfoxide; and mixtures thereof. Preferred examples of solvents include 2-propanol, tetrahydrofuran, toluene, acetonitrile, N,N-dimethylformamide, and the like.

The reaction temperature is generally -80°C to heating under reflux, and preferably 25°C to 90°C.

The reaction time is generally 30 minutes to 48 hours, and preferably 6 to 12 hours.

Manufacturing Method O

The compound of the present disclosure represented by formula (h1) can be manufactured, for example, by the following method.

[Chemical Formula 31]

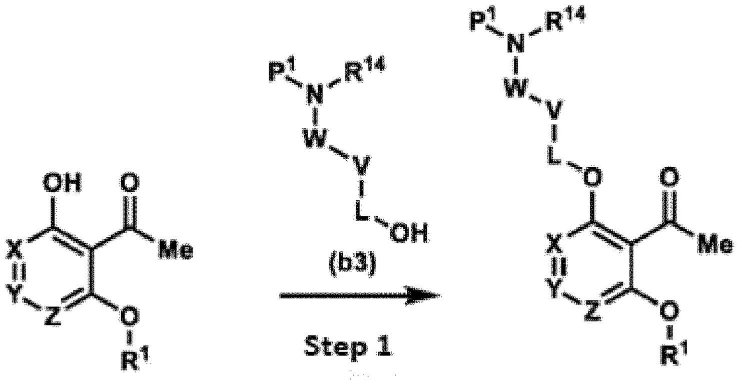


(l1) (h1)

wherein $R^1$, $R^{14}$, X, Y, Z, L, V, and W are defined the same as the description in item 1, and $P^1$ refers to an amino protecting group. Examples of $P^1$ include the amino protecting groups described in Protective Groups in Organic Synthesis (authors: Theodora W. Greene, Peter G. M. Wuts, publisher: John Wiley & Sons, Inc., 1999) and the like.

Compound (l1) and compound (b3) are available as commercial products.

[Step 1]

Compound (h1) can be manufactured from compound (l1) and compound (b3) by the method described in step 2 of Manufacturing Method B or a method in accordance therewith.

Manufacturing Method P

The compound of the present disclosure represented by formula (h1) can be manufactured, for example, by the following method.

[Chemical Formula 32]

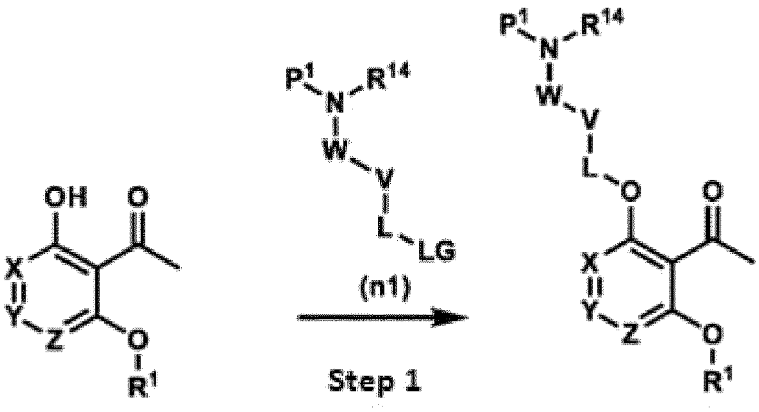


(l1) (h1)

wherein $R^1$, $R^{14}$, X, Y, Z, L, V, and W are defined the same as the description in item 1, $P^1$ refers to an amino protecting group, amd LG refers to a leaving group. Examples of $P^1$ include the amino protecting groups described in Protective

Groups in Organic Synthesis (authors: Theodora W. Greene, Peter G. M. Wuts, publisher: John Wiley & Sons, Inc., 1999) and the like. Examples of LG include halogen, methanesulfonyloxy, p-toluenesulfonyloxy, trifluoromethanesulfonyloxy, and the like.

Compound (l1) and compound (n1) are available as commercial products.

[Step 1]

Compound (h1) can be manufactured from compound (l1) and compound (n1) by the method described in step 2 of Manufacturing Method B or a method in accordance therewith.

Manufacturing Method Q

The compound of the present disclosure represented by formula (h1) can be manufactured, for example, by the following method.

[Chemical Formula 33]

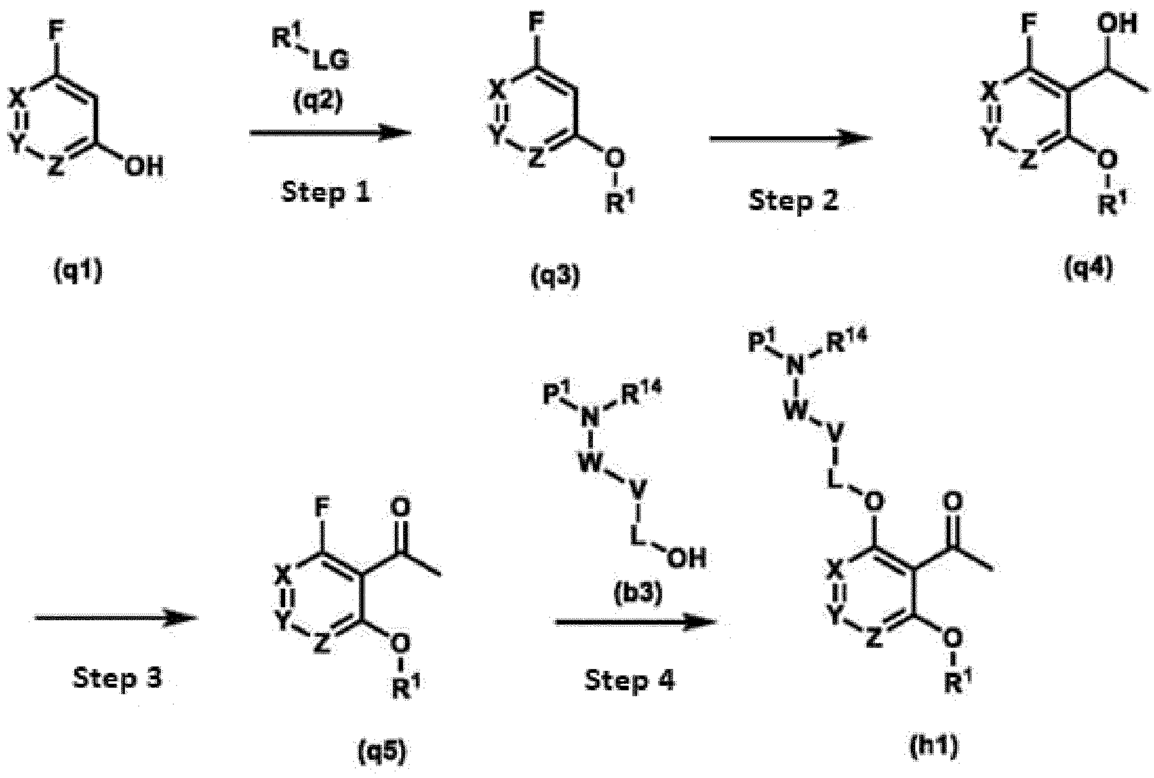


wherein $R^1$, $R^{14}$, X, Y, Z, L, V, and W are defined the same as the description in item 1, $P^1$ refers to an amino protecting group, and LG refers to a leaving group. Examples of $P^1$ include the amino protecting groups described in Protective Groups in Organic Synthesis (authors: Theodora W. Greene, Peter G. M. Wuts, publisher: John Wiley & Sons, Inc., 1999) and the like. Examples of LG include halogen, methanesulfonyloxy, p-toluenesulfonyloxy, trifluoromethanesulfonyloxy, and the like.

Compound (q1), compound (q2) and compound (b3) are available as commercial products.

[Step 1]

Compound (q3) can be manufactured from compound (q1) and compound (q2) by the method described in step 1 of Manufacturing Method N or a method in accordance therewith.

[Step 2]

Compound (q4) can be manufactured by reacting compound (q3) with acetoaldehyde in the presence of a

suitable base in a suitable solvent.

Examples of the base include n-butyllithium, sec-butyllithium, tert-butyllithium, lithium diisopropylamide, lithium tetramethylpiperidide, lithium hexamethyldisilazide, sodium hexamethyldisilazide, potassium hexamethyldisilazide, isopropylmagnesium chloride-lithium chloride complex, and the like.

A solvent is not particularly limited as long as it is a solvent that does not react under the reaction conditions of this step. Examples thereof include ether solvents such as diethyl ether, diisopropyl ether, tetrahydrofuran, methylcyclopentyl ether, and 1,4-dioxane; aromatic hydrocarbon solvents such as benzene, toluene, chlorobenzene, anisole, and xylene; halogen solvents such as methylene chloride, chloroform, and 1,2-dichloroethane; aprotic solvents such as acetonitrile, N,N-dimethylformamide, N,N-dimethylacetamide, N-methyl-2-pyrrolidinone, 1,3-dimethyl-2-imidazolidinone, and dimethyl sulfoxide; and mixtures thereof. Preferred examples of solvents include tetrahydrofuran.

The reaction temperature is generally -80°C to heating under reflux, and preferably -80°C to 25°C.

The reaction time is generally 30 minutes to 48 hours, and preferably 6 to 12 hours.

[Step 3]

Compound (q5) can be manufactured by reacting compound (q4) with a suitable oxidizing agent in the presence or absence of a suitable base in a suitable solvent.

Examples of the oxidizing agent include manganese dioxide, Dess-Martin reagents, dimethyl sulfoxide-oxalyl chloride, dimethyl sulfoxide-trifluoroacetic acid anhydride, sulfur trioxide-pyridine complex, 2,2,6,6-tetramethylpiperidin-1-oxy radical-sodium hypochlorite, and the like.

Examples of the base include organic bases such as triethylamine, diisopropylethylamine, tributylamine, 1.5-diazabicyclo[4.3.0]non-5-ene (DBN), 1,8-diazabicyclo[5.4.0]undec-7-ene (DBU), pyridine, 4-dimethylaminopyridine, picoline, and N-methylmorpholine (NMM) and inorganic bases such as sodium bicarbonate, potassium bicarbonate, sodium carbonate, potassium carbonate, sodium hydroxide, and potassium hydroxide.

A solvent is not particularly limited as long as it is a solvent that does not react under the reaction conditions of this step. Examples thereof include halogen solvents such as methylene chloride, chloroform, and 1,2-dichloroethane; ether solvents such as diethyl ether, diisopropyl ether, tetrahydrofuran, methylcyclopentyl ether, and 1,4-dioxane; aromatic hydrocarbon solvents such as benzene, toluene, chlorobenzene, anisole, and xylene; ester solvents such as ethyl acetate and methyl acetate; aprotic solvents such as acetonitrile, N,N-dimethylformamide, N,N-dimethylacetamide, N-methyl-2-pyrrolidinone, 1,3-dimethyl-2-imidazolidinone, and dimethyl sulfoxide; and mixtures thereof. Preferred examples of solvents include methylene chloride, tetrahydrofuran, toluene, acetonitrile, N,N-dimethylformamide, and the like.

The reaction temperature is generally -80°C to heating under reflux, and preferably -80°C to 25°C.

The reaction time is generally 30 minutes to 48 hours, and preferably 6 to 12 hours.

[Step 4]

Compound (h1) can be manufactured from compound (q5) and compound (b3) by the method described in step 2 of Manufacturing Method B or a method in accordance therewith.

<u>Manufacturing Method R</u>

The compound of the present disclosure represented by formula (i2) can be manufactured, for example, by the following method.

[Chemical Formula 34]

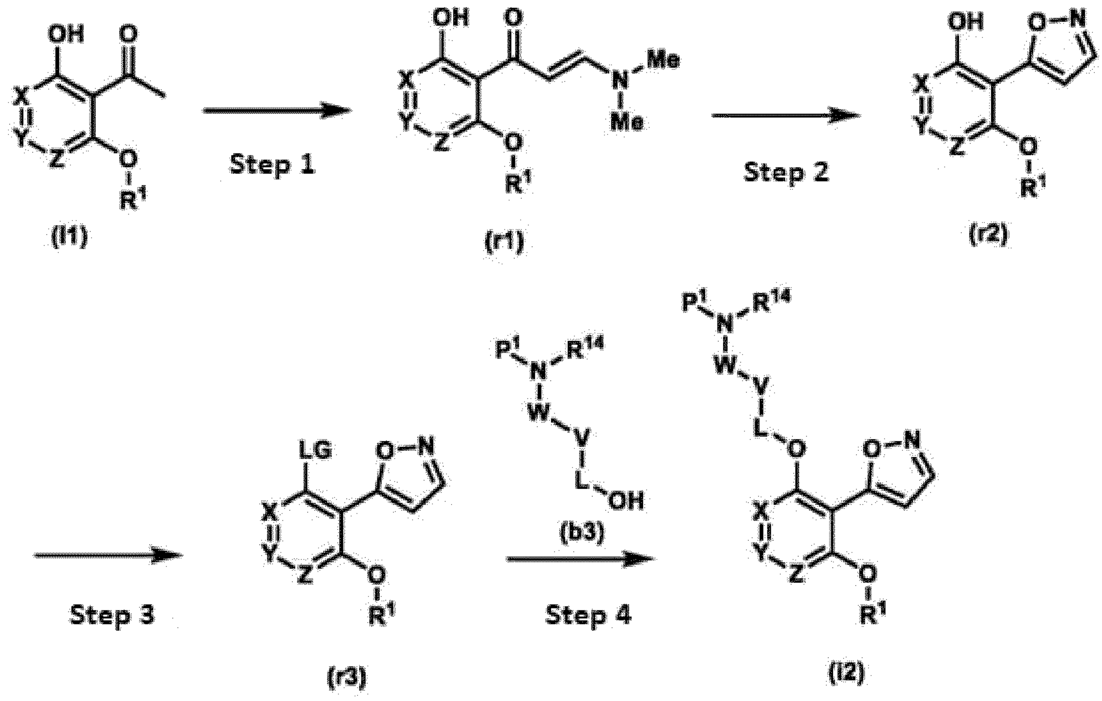


wherein $R^1$, $R^{14}$, X, Y, Z, L, V, and W are defined the same as the description in item 1, $P^1$ refers to an amino protecting group, and LG refers to a leaving group. Examples of $P^1$ include the amino protecting groups described in Protective Groups in Organic Synthesis (authors: Theodora W. Greene, Peter G. M. Wuts, publisher: John Wiley & Sons, Inc., 1999) and the like.

Examples of LG include halogen, methanesulfonyloxy, p-toluenesulfonyloxy, trifluoromethanesulfonyloxy, and the like.

Compound (I1) and compound (b3) are available as commercial products.

[Step 1]

Compound (r1) can be manufactured from compound (I1) by the method described in step 1 of Manufacturing Method F or a method in accordance therewith.

[Step 2]

Compound (r2) can be manufactured from compound (r1) by the method described in step 2 of Manufacturing Method F or a method in accordance therewith.

[Step 3]

Compound (r3) can be manufactured by reacting compound (r2) with a suitable halogenating agent or sulfonylating agent in the presence or absence of a suitable base in a suitable solvent or without a solvent.

Examples of the halogenating agent or sulfonylating agent include thionyl chloride, phosphorus oxychloride, oxalyl chloride, phosphorus tribromide, methanesulfonyl chloride, N-phenylbis(trifluoromethane sulfonimide), trifluoromethanesulfonic anhydride, and the like.

Examples of the base include organic bases such as triethylamine, diisopropylethylamine, tributylamine, 1.5-diazabicyclo[4.3.0]non-5-ene (DBN), 1,8-diazabicyclo[5.4.0]undec-7-ene (DBU), pyridine, 4-dimethylaminopyridine, picoline, and N-methylmorpholine (NMM) and inorganic bases such as sodium bicarbonate, potassium bicarbonate, sodium carbonate, potassium carbonate, sodium hydroxide, and potassium hydroxide.

A solvent is not particularly limited as long as it is a solvent that does not react under the reaction conditions of this step. Examples thereof include halogen solvents such as methylene chloride, chloroform, and 1,2-dichloroethane; ether solvents such as diethyl ether, diisopropyl ether, tetrahydrofuran, methylcyclopentyl ether, and 1,4-dioxane; aromatic

hydrocarbon solvents such as benzene, toluene, chlorobenzene, anisole, and xylene; ester solvents such as ethyl acetate and methyl acetate; aprotic solvents such as acetonitrile, N,N-dimethylformamide, N,N-dimethylacetamide, N-methyl-2-pyrrolidinone, 1,3-dimethyl-2-imidazolidinone, and dimethyl sulfoxide; and mixtures thereof. Preferred examples of solvents include methylene chloride, tetrahydrofuran, toluene, acetonitrile, N,N-dimethylformamide, and the like.

The reaction temperature is generally -80°C to heating under reflux, and preferably 0°C to heating under reflux.

The reaction time is generally 30 minutes to 48 hours, and preferably 30 minutes to 12 hours.

[Step 4]

Compound (i2) can be manufactured by a reaction of compound (r3) and compound (b3) catalyzed by a suitable palladium source and a ligand in the presence of a suitable base in a suitable solvent.

Examples of the base include organic bases such as triethylamine, diisopropylethylamine, tributylamine, pyridine, 4-dimethylaminopyridine, picoline, and N-methylmorpholine (NMM) and inorganic bases such as sodium carbonate, potassium carbonate, cesium carbonate, sodium hydroxide, and potassium hydroxide. Preferred examples of the base include diisopropylethylamine, cesium carbonate, and the like.

Examples of the palladium source include salts of palladium chloride or palladium acetate, zerovalent palladium complexes such as tris(dibenzylideneacetone)dipalladium(0) chloroform adduct and the like.

Examples of ligands include phosphine ligands such as 4,5-bis(diphenylphosphino)-9,9-dimethylxanthene (xantphos), 2,2'-bis(diphenylphosphino)-1,1'-binaphthyl (BINAP), 2-dicyclohexylphosphino-2',6'-dimethoxybiphenyl (SPhos), and 2-dicyclohexylphosphino-2',4',6'-triisopropylbiphenyl (XPhos). Preferred examples of ligands include 4,5-bis(diphenylphosphino)-9,9-dimethylxanthene (xantphos).

A solvent is not particularly limited as long as it is a solvent that does not react under the reaction conditions of this step. Examples thereof include halogen solvents such as methylene chloride, chloroform, and 1,2-dichloroethane; ether solvents such as diethyl ether, diisopropyl ether, tetrahydrofuran, methylcyclopentyl ether, and 1,4-dioxane; aromatic hydrocarbon solvents such as benzene, toluene, chlorobenzene, anisole, and xylene; ester solvents such as ethyl acetate and methyl acetate; aprotic solvents such as acetonitrile, N,N-dimethylformamide, N,N-dimethylacetamide, N-methyl-2-pyrrolidinone, 1,3-dimethyl-2-imidazolidinone, and dimethyl sulfoxide; and mixtures thereof. Preferred examples of solvents include tetrahydrofuran, 1,4-dioxane, toluene, acetonitrile, N,N-dimethylformamide, and the like.

The reaction temperature is generally 0°C to heating under reflux, and preferably 50°C to heating under reflux.

The reaction time is generally 5 minutes to 72 hours, and preferably 2 to 12 hours.

In the manufacturing method described above, the starting material and intermediates for which the manufacturing method is not described are available as commercial products, or can be synthesized from a commercially available product by a method known to those skilled in the art or a method in accordance therewith.

Even without an explicit description of a specific use of a protecting group in each reaction of the manufacturing

methods described above, a protecting group can be used as needed. A compound of interest can be obtained by protecting a portion other than the reaction point and deprotecting the portion after the completion of a reaction or a series of reactions as needed if one of the functional groups other than the reaction point is altered under the described reaction condition or if the absence of a protecting group is unsuitable for performing the described method.

As the protecting group, the protecting group described in Protective Groups in Organic Synthesis (authors: Theodora W. Greene, Peter G. M. Wuts, publisher: John Wiley & Sons, Inc., 1999) or the like can be used. Specific examples of amino protecting group include benzyloxylcarbonyl, tert-butoxycarbonyl, acetyl, benzyl, and the like. Specific examples of a protecting group of a hydroxyl group include trialkylsilyl such as trimethylsilyl and tert-butyldimethylsilyl, acetyl, benzyl, and the like.

A method that is commonly used in synthetic organic chemistry (see, for example, the aforementioned Protective Groups in Organic Synthesis) or a method in accordance therewith can be used for the introduction or removal of a protecting group.

As used herein, protecting groups, condensing agents, or the like may be denoted by the nomenclature of IUPAC-IUB (Biochemical Nomenclature Committees) that is commonly used in the art. It should be noted that compound names used herein do not necessarily follow the IUPAC nomenclature.

The intermediates or compounds of interest in the manufacturing methods described above can be guided into another compound in the present disclosure by appropriately converting its functional group **(e.g.,** various convertions using amino, hydroxyl group, carbonyl, halogen, or the like while protecting or deprotecting a functional group as needed). A functional group can be converted by a common method that is routinely used (see, for example, Comprehensive Organic Transformations, R. C. Larock, John Wiley & Sons Inc. (1999), etc.).

The intermediates or compounds of interest in the manufacturing methods described above can be isolated and purified by a purification method that is commonly used in organic synthetic chemistry (e.g., neutralization, filtration, extraction, washing, drying, concentration, recrystallization, various chromatographies). Intermediates can also be used in the subsequent reaction without any particular purification.

Examples of "pharmaceutically acceptable salt" include acid addition salts and base addition salts. Examples of acid addition salts include inorganic acid salts such as hydrochloric acid salt, hydrobromic acid salt, sulfuric acid salt, hydroiodic acid salt, nitric acid salt, and phosphoric acid salt, and organic acid salts such as citric acid salt, oxalic acid salt, phthalic acid salt, fumaric acid salt, maleic acid salt, succinic acid salt, malic acid salt, acetic acid salt, formic acid salt, propionic acid salt, benzoic acid salt, trifluoroacetic acid salt, methanesulfonic acid salt, benzenesulfonic acid salt, p-toluenesulfonic acid salt, and camphorsulfonic acid salt. Examples of base addition salts include inorganic base salts such as sodium salt, potassium salt, calcium salt, magnesium salt, barium salt, and aluminum salt, and organic base salts such as trimethylamine, triethylamine, pyridine, picoline, 2,6-lutidine, ethanolamine, diethanolamine, triethanolamine, tromethamine [tris(hydroxymethyl)methylamine], tert-butylamine, cyclohexylamine, dicyclohexylamine, and N-N-dibenzylethylamine, and the like. Furthermore, examples of "pharmaceutically acceptable salt" include amino acid salts of a basic or acidic amino acid such as arginine, lysine, ornithine, aspartic acid, and glutamic acid.

Salts that are suitable for a staring material and intermediate and salts that are acceptable as a raw material of a pharmaceutical product are conventionally used non-toxic salts. Such salts can be, for example, acid addition salts such as organic acid salts **(e.g.,** acetic acid salt, trifluoroacetic acid salt, maleic acid salt, furamic acid salt, citric acid salt, tartaric acid salt, methanesulfonic acid salt, benzenesulfonic acid salt, formic acid salt, toluenesulfonic acid salt, etc.) and inorganic acid salts **(e.g.,** hydrochloric acid salt, hydrobromic acid salt, hydroiodic acid salt, sulfuric acid salt, nitric acid salt, phosphoric acid salt, etc.), salts of amino acid **(e.g.,** arginine, asparaginic acid, glutamic acid, etc.), metal salts such as alkali metal salts **(e.g.,** sodium salt, potassium salt, etc.) and alkali earth metal salts **(e.g.,** calcium salt, magnesium salt, etc.), ammonium salts, organic base salts **(e.g.,** trimethylamine salt, triethylamine salt, pyridine salt, picoline salt, dicyclohexylamine salt, N,N'-dibenzylethylenediamine salt, etc.), and the like. Those skilled in the art can also appropriately select other salts.

Deuterated compounds prepared by converting any one or more of $^1H$ of compounds represented by formulas (1) to (5) to $^2H$(D) are also encompassed by the compounds represented by formulas (1) to (5) in the present disclosure.

The present disclosure encompasses the compounds represented by formulas (1) to (5) and pharmaceutically acceptable salts thereof. The compound of the present disclosure can also be in a form of a hydrate and/or solvate of various solvents (ethanolate, etc.) Thus, such hydrates and/or solvates are also encompassed by the compound of the present disclosure.

The compound of the present disclosure also encompasses enantiomers based on an optically-active center, atropisomers based on axial or planar chirality resulting from restriction of intramolecular rotation, other stereoisomers, tautomers, geometric isomers, all other possible isomers, crystalline forms in any form, and mixtures thereof.

In particular, an enantiomer and an atropisomer can be obtained as a racemate and an optically-active form if an optically-active starting material or intermediate is used, respectively. If necessary, a corresponding raw material, intermediate, or final product racemate can be physically or chemically resolved, during an appropriate step of the manufacturing method described above, into their optical enantiomers by a known separation method, such as a method using an optically active column or a fractional crystallization method. Examples of the resolution method include a diastereomer method for reacting a racemate with an optically-active resolving agent to synthesize two types of diastereomers, and utilizing the difference in physical properties for resolving through a method such as a fractional crystallization method.

If it is desirable to obtain a pharmaceutically acceptable salt of the compound of the present disclosure, a compound represented by formula (1) to (5), when obtained in a form of a pharmaceutically acceptable salt, can be directly purified, or when obtained in a free form, a salt may be formed through a common method by dissolving or suspending the compound in a suitable organic solvent and adding an acid or a base.

A liposome encapsulating the compound of the present disclosure can be manufactured, for example, by the following method.

[Step 1]

A membrane constituting component such as a phospholipid or cholesterol is dissolved in an organic solvent such as chloroform, and the organic solvent is evaporated within a flask to form a thin film of a lipid mixture on the inner wall of the flask. Alternatively, a lipid mixture can be obtained as a lyophilized product by dissolving the component in t-butyl alcohol or the like and then lyophilizing the solution. A membrane constituting component such as phospholipid or cholesterol can also be dissolved in an organic solvent and then a powderized lipid mixture can be obtained using an instantaneous vacuum dryer CRUX (Hosokawa Micron Corporation), or this can be obtained from Nippon Fine Chemical Co., Ltd. under the name Presome®.

[Step 2]

A crude liposome dispersion is obtained by adding and dispersing an internal aqueous phase such as an

aqueous ammonium sulfate solution to the lipid mixture obtained in step 1.

[Step 3]

The crude liposome dispersion obtained in step 2 is passed through a filter to attain a desired particle size by using an extruder. Alternatively, the crude liposome dispersion obtained in step 2 is discharged from a nozzle at a high pressure by using a high pressure homogenizer to attain a desire particle size. While the particle size of liposomes is not particularly limited, the particle size is, for example, 10 nm to 200 nm, preferably 30 nm to 150 nm, more preferably 40 nm to 140 nm, still more preferably 50 to 120 nm, and most preferably 60 to 100 nm. The particle size of liposomes is a mean value measured by dynamic light scattering, and can be measured using, for example, Zetasizer Nano ZS (Malvern Instruments).

[Step 4]

For the liposome solution obtained in step 3, the outer aqueous phase is replaced by gel filtration, dialysis, tangential flow filtration, ultrafiltration, or the like.

[Step 5]

The liposome solution obtained in step 4 with the outer aqueous phase replaced is incubated with a compound to be encapsulated to encapsulate the compound in the liposome.

[Step 6]

The resulting liposome encapsulating a compound is subjected to gel filtration, dialysis, tangential flow filtration, ultrafiltration, or the like to remove compounds that are not encapsulated. If a desired encapsulation ratio is achieved in step 5, step 6 can be omitted.

The compound of the present disclosure can be used concomitantly with another drug in order to enhance the effect thereof. Specifically, the compound of the present disclosure can be used concomitantly with a drug such as a hormonal therapy agent, a chemotherapeutic agent, an immunotherapeutic agent, or an agent inhibiting a cell growth factor and its receptor action. Hereinafter, a drug that can be concomitantly used with the compound of the present disclosure is abbreviated as the concomitantly used drug.

Although the compound of the present disclosure exhibits excellent anticancer action when used as a single agent, the effect thereof can be further enhanced, or the QOL of a patient can be improved by concomitantly using one or several of the concomitantly used drugs described above (combined use of multiple drugs).

Examples of "hormonal therapy agent" include fosfestrol, diethylstilbestrol, chlorotrianisene, medroxyprogesterone acetate, megestrol acetate, chlormadinone acetate, cyproterone acetate, danazol, dienogest, asoprisnil, allylestrenol, gestrinone, nomegestol, tadenan, mepartricin, raloxifene, ormeroxifene, levormeloxifene, antiestrogens (e.g., tamoxifen citrate, toremifene citrate, and the like), pill formulations, mepitiostane, testolactone, aminoglutethimide, LH-RH derivatives (LH-RH agonists (e.g., goserelin acetate, buserelin, leuprorelin, and the like), and LH-RH antagonists), droloxifene, epitiostanol, ethinylestradiol sulfonate, aromatase inhibitors (e.g., fadrozole hydrochloride, anastrozole, letrozole, exemestane, vorozole, formestane, and the like), antiandrogens (e.g., flutamide, enzalutamide, apalutamide, bicalutamide, nilutamide, and the like), adrenocortical hormone agents (e.g., dexamethasone, prednisolone, betamethasone, triamcinolone, and the like), androgen synthesis inhibitors (e.g., abiraterone and the like), retinoids, drugs that slow the metabolism of retinoids (e.g., liarozole and the like), and the like.

For example, an alkylating agent, antimetabolite, anticancer antibiotic, plant derived anticancer agent, molecularly targeted therapy agent, immunomodulator, other chemotherapeutic agent, or the like can be used as a "chemotherapeutic agent". Representative examples thereof are described below.

Examples of "alkylating agents" include nitrogen mustard, nitrogen mustard n-oxide hydrochloride, chlorambucil, cyclophosphamide, ifosfamide, thiotepa, carboquone, improsulfan tosylate, busulfan, nimustine hydrochloride, mitobronitol, melphalan, dacarbazine, ranimustine, estramustine phosphate sodium, triethylenemelamine, carmustine, lomustine, streptozocin, pipobroman, etoglucide, carboplatin, cisplatin, miboplatin, nedaplatin, oxaliplatin, altretamine, ambamustine, dibrospidium chloride, fotemustine, prednimustine, pumitepa, ribomustin, temozolomide, treosulfan, trofosfamide, zinostatin stimalamer, adozelesin, cystemustine, bizelesin, trabectedin, DDS formulations thereof, and the like.

Examples of "antimetabolite" include mercaptopurine, 6-mercaptopurine riboside, thioinosine, methotrexate, pemetrexed, enocitabine, cytarabine, cytarabine ocfosfate, ancitabine hydrochloride, 5-FU based agents (e.g., fluorouracil, tegafur, UFT, doxifluridine, carmofur, galocitabine, emitefur, capecitabine, and the like), aminopterin, nelzarabine,

leucovorin calcium, tabloid, butocin, calcium folinate, calcium levofolinate, cladribine, emitefur, fludarabine, gemcitabine, hydroxycarbamide, pentostatin, piritrexim, idoxuridine, mitoguazone, tiazofurin, ambamustine, bendamustine, DDS formulations thereof, and the like.

Examples of "anticancer antibiotic" include actinomycin D, actinomycin C, mitomycin C, chromomycin A3, bleomycin hydrochloride, bleomycin sulfate, peplomycin sulfate, daunorubicin hydrochloride, doxorubicin hydrochloride, aclarubicin hydrochloride, pirarubicin hydrochloride, epirubicin hydrochloride, neocarzinostatin, mithramycin, sarkomycin, carzinophilin, mitotane, zorubicin hydrochloride, mitoxantrone hydrochloride, idarubicin hydrochloride, eribulin, DDS formulations thereof, and the like.

Examples of "plant derived anticancer agent" include etoposide, etoposide phosphate, vinblastine sulfate, vincristine sulfate, vindesine sulfate, teniposide, paclitaxel, docetaxel, DJ-927, vinorelbine, irinotecan, topotecan, DDS formulations thereof, and the like.

Examples of "molecularly targeted therapy agent" include imatinib, gefitinib, erlotinib, sorafenib, dasatinib, sunitinib, nilotinib, lapatinib, pazopanib, ruxolitinib, crizotinib, vemurafenib, vandetanib, ponatinib, cabozantinib, tofacitinib regorafenib, bosutinib, axitinib, dabrafenib, trametinib, nintedanib, idelalisib, ceritinib, lenvatinib, palbociclib, alectinib, afatinib, osimertinib, ribociclib, abemaciclib, brigatinib, neratinib, copanlisib, cobimetinib, ibrutinib, acalabrutinib, encorafenib, binimetinib, baricitinib, fostamatinib, lorlatinib, erdafitinib, entrectinib, dacomitinib, sirolimus, everolimus, temsirolimus, olaparib, rucaparib, niraparib, venetoclax, azacitidine, decitabine, vorinostat, panobinostat, romidepsin, bortezomib, carfilzomib, larotrectinib, ixazomib, and the like.

Examples of "immunomodulator" include lenalidomide, pomalidomide, and the like.

Examples of "other chemotherapeutic agent" include sobuzoxane and the like.

Examples of "immunotherapeutic agent (BRM)" include picibanil, krestin, sizofiran, lentinan, ubenimex, interferon, interleukin, macrophage colony stimulating factor, granulocyte-colony stimulating factor, erythropoietin, lymphotoxin, BCG vaccine, corynebacterium parvum, levamisole, polysaccharide K, procodazole, anti-CTLA4 antibody, anti-PD-1 antibody, anti-PD-L1 antibody, and Toll-like Receptor agonist (e.g., TLR7 agonist, TLR8 agonist, TLR9 agonist, and the like).

The cell growth factor in an agent inhibiting a cell growth factor and its receptor action can be any substance, as long as it is a substance that promotes cell growth. A cell growth factor is generally a peptide having a molecular weight of 20,000 or less and exerting action at a low concentration by binding with a receptor. Specific examples thereof include EGF (epidermal growth factor) or substances having substantially the same activity as EGF **(e.g.,** TGF-alpha and the like), insulin or substances having substantially the same activity as insulin **(e.g.,** insulin, IGF (insulin-like growth factor)-1, IGF-2, and the like), FGF (fibroblast growth factor) or substances having substantially the same activity as FGF **(e.g.,** acidic FGF, basic FGF, KGK (keratinocyte growth factor), FGF-10, and the like), and other cell growth factors **(e.g.,** CSF (colony stimukating factor), EPO (erythropoietin), IL-2 (interleukin-2), NGF (nerve growth factor), PDGF (platelet-derived growth factor), TGF-beta (transforming growth factor beta), HGF (hepatocyte growth factor), VEGF (vascular endothelial growth factor), heregulin, angiopoietin, and the like).

The dosing period of the compound of the present disclosure and a concomitantly used drug is not limited. They can be administered simultaneously or separately to a target of administration. The compound of the present disclosure and a concomitantly used drug can also be prepared as a combined drug. The amount of concomitantly used drug to be administered can be appropriately selected based on clinically used doses. The blend ratio of the compound of the present disclosure and a concomitantly used drug can be appropriately selected depending on the subject of administration, route of administration, target disease, symptom, combination, or the like. If, for example, the subject of administration is a human, 0.01 to 100 parts by weight of concomitantly used drug can be used with respect to 1 part by weight of the compound of the present disclosure. They can also be used in combination with an agent (concomitantly used drug) such as an antiemetic, sleep inducing agent, or anticonvulsive in order to suppress side effects thereof.

In the case of solid tumor, "exhibiting therapeutic resistance to an immune checkpoint inhibitor" means a state in which tumor growth cannot be suppressed even when an immune checkpoint inhibitor is administered. Exhibiting resistance to an anti-PD-1 antibody means a state in which tumor growth cannot be suppressed even when the anti-PD-1 antibody described above is administered. Exhibiting resistance to an anti-PD-L1 antibody means a state in which tumor growth cannot be suppressed even when the anti-PD-L1 antibody described above is administered. In the case of hematologic malignancy, it means a state in which the increase in the number of cancer cells in the blood cannot be suppressed even when an immune checkpoint inhibitor is administered. Exhibiting resistance to an anti-PD-1 antibody means a state in which the increase in the number of cancer cells in the blood cannot be suppressed even when the anti-PD-1 antibody described above is administered. Exhibiting resistance to an anti-PD-L1 antibody means a state in which the increase in the number of cancer cells in the blood cannot be suppressed even when the anti-PD-L1 antibody described above is administered.

An anti-PD-1 antibody is a protein that recognizes a PD-1 molecule, has a Y-shaped four-chain structure (two polypeptide chains including two light and heavy chains), and is a molecule that selectively recognizes one molecule by a Fab site. The anti-PD-1 antibody is produced by producing a hybridoma obtained by cell fusion between B cells producing

an antibody and myeloma and then purifying the antibody secreted into the culture supernatant.

The M1-M2 ratio of macrophages refers to a ratio of a cell group defined by a marker of M1 macrophages **(e.g.,** CD86, STAT1, IL-1beta, iNOS, CXCL9, CXCL10, and the like) and a cell group defined by a marker of M2 macrophages **(e.g.,** Mrc1, Arg1, CD163, and the like). As the value is higher, the intratumoral environment more easily becomes an inflammatory environment, which means that the anti-PD-1/PD-L1 antibodies easily exert the effect.

Tumor Mutation Burden-High (TMB-High) solid tumors refer to cancers with a TMB score of 10 mut/Mb or more as measured in FoundationOne CDx cancer genome profile.

An anti-PD-1 antibody-responsive patient refers to a state in which tumor growth is suppressed by administering an anti-PD-1 antibody. The state in which tumor growth is suppressed refers to any one of stable (SD), partial response (PR), and complete response (CR) states in RECIST criteria. In particular, a state in which the effect of suppressing tumor growth lasts for 6 months (24 weeks) or longer is referred to as Durable Clinical Benefit (DCB).

The anti-PD-1 antibody may be administered 3 days, 1 week, 2 weeks, 3 weeks, 4 weeks, 5 weeks, or 6 weeks after the CHK1 inhibitor and the immune checkpoint inhibitor are administered simultaneously.

The M1-M2 ratio of macrophages of a cancer patient exhibiting therapeutic resistance to an immune checkpoint inhibitor is preferably 21 or less, 20 or less, 19 or less, 18 or less, 17 or less, 16 or less, 15 or less, 14 or less, 13 or less, 12 or less, 11 or less, 10 or less, **9.9** or less, **9.8 or** less, **9.7** or less, **9.6** or less, **9.5** or less, **9.4** or less, **9.3** or less, 9.2 or less, **9.1** or less, 9.0 or less, 8.9 or less, **8.8** or less, **8.7** or less, **8.6** or less, **8.5** or less, **8.4 or** less, **8.3** or less, **8.2** or less, **8.1** or less, **8.0** or less, **7.9** or less, 7.8 or less, 7.7 or less, 7.6 or less, 7.5 or less, 7.4 or less, 7.3 or less, 7.2 or less, 7.1 or less, 7.0 or less, 6.9 or less, 6.8 or less, 6.7 or less, 6.6 or less, 6.5 or less, 6.4 or less, 6.3 or less, 6.2 or less, **6.1** or less, 6.0 or less, 5.9 or less, 5.8 or less, 5.7 or less, 5.6 or less, 5.5 or less, 5.4 or less, 5.3 or less, 5.2 or less, 5.1 or less, 5.0 or less, 4.9 or less, 4.8 or less, 4.7 or less, 4.6 or less, 4.5 or less, 4.4 or less, 4.3 or less, 4.2 or less, 4.1 or less, 4.0 or less, 3.9 or less, 3.8 or less, **3.7** or less, 3.6 or less, 3.5 or less, 3.4 or less, 3.3 or less, 3.2 or less, 3.1 or less, 3.0 or less, 2.9 or less, 2.8 or less, 2.7 or less, 2.6 or less, 2.5 or less, 2.4 or less, 2.3 or less, 2.2 or less, 2.1 or less, 2.0 or less, 1.9 or less, 1.8 or less, 1.7 or less, 1.6 or less, 1.5 or less, 1.4 or less, 1.3 or less, 1.2 or less, 1.1 or less, 1.0 or less, 0.9 or less, 0.8 or less, 0.7 or less, 0.6 or less, 0.5 or less, 0.4 or less, 0.3 or less, 0.2 or less, or 0.1 or less.

The number of CD8 positive cells in tumor tissue of a cancer patient exhibiting therapeutic resistance to an immune checkpoint inhibitor is preferably 0.135 or less, 0.130 or less, 0.125 or less, 0.120 or less, 0.115 or less, 0.110 or less, 0.095 or less, 0.090 or less, 0.085 or less, 0.080 or less, 0.075 or less, 0.070 or less, 0.065 or less, 0.060 or less, 0.055 or less, 0.050 or less, 0.045 or less, 0.040 or less, 0.035 or less, 0.030 or less, 0.025 or less, 0.020 or less, 0.015 or less, or 0.010 or less.

As used herein, **"or"** is used when "at least one or more" of the listed matters in the sentence can be employed. When explicitly described herein as "within the range of two values", the range also includes the two values themselves.

Reference literatures such as scientific literatures, patents, and patent applications cited herein are incorporated herein by reference to the same extent that the entirety of each document is specifically described.

The present disclosure has been described while showing preferred embodiments to facilitate understanding. While the present disclosure is described hereinafter based on the Examples, the above descriptions and the following Examples are provided for the sole purpose of exemplification, not limitation of the present disclosure. Thus, the scope of the present disclosure is not limited to the embodiments and Examples that are specifically described herein and is limited only by the scope of claims.

[Examples]

The present disclosure is described more specifically with the Reference Examples, Examples, and Test Example hereinafter, but the present invention is not limited thereto.

The present specification may use the following abbreviations.

Ts: p-toluenesulfonyl
THF: tetrahydrofuran
TFA: trifluoroacetic acid
DMF: N,N-dimethylformamide
DMSO: dimethyl sulfoxide
MeCN: acetonitrile
Me: methyl
Boc: tert-butoxycarbonyl
Dess-Martin reagent: Dess-Martin periodinane(1,1,1-triacetoxy-1,1-dihydro-1,2-benzoiodoxol-3-(1H)-one)

NMR (Nuclear Magnetic Resonance) data used for identifying a compound was obtained with JNM-ECS 400 NMR spectrometer (400 MHz) (JEOL Ltd.)

As symbols used in NMR, s refers to singlet, d refers to doublet, dd refers to doublet of doublets, t refers to triplet, td refers to doublet of triplets, q refers to quartet, m refers to multiplet, br refers to broad, brs refers to broad singlet, brm refers to broad multiplet, and J refers to a coupling constant.

LC/MS (Liquid Chromatography-Mass Spectrometry) analysis conditions used in identification of compounds are as follows. Among the observed mass spectrometry values [MS (m/z)], a value corresponding to monoisotopic mass (precise mass consisting of only the primary isotope) is indicated by $[M+H]^+$, $[M-H]^-$, $[M+2H]^{2+}$, or the like, and the time of retention is indicated by Rt (minutes).

Measurement condition A

Detector: ACQUITY® SQ detector (Waters)
HPLC: ACQUITY UPLC® system
Column: Waters ACQUITY UPLC® BEH C18 (1.7 μm, 2.1 mm × 30 mm)
Solvent: solution A: 0.06% formic acid/$H_2O$, solution B: 0.06% formic acid/MeCN
Gradient condition: 0.0-1.3 min Linear gradient from B 2% to 96%
Flow rate: 0.8 mL/min
UV: 220 nm and 254 nm
Column temperature: 40°C

Measurement condition B

Detector: APCI 6120 Quadrupole LC/MS (Agilent Technologies) HPLC: Agilent Technologies 1260 Infinity® system
Column: Agilent Technologies® ZORBAX SB-C18 (1.8 μm, 2.1 mm × 50 mm)
Solvent: solution A: 0.1% formic acid/$H_2O$, solution B: MeCN
Gradient condition: 0.0-5.0 min Linear gradient from B 5% to 90%
Flow rate: 0.6 mL/min
UV: 210 nm, 254 nm, and 280 nm
Column temperature: 40°C

Measurement condition C

Detector: Shimadzu LCMS-2020
Column: L-column-2 ODS (4.6 mm × 35 mm)
Gradient condition: $MeCN/H_2O/HCO_2H$ = 10/90/0.1 → 100/0/0.1 (0-2 min), 100/0/0.1 (2-4 min)
Flow rate: 2 mL/min
Column temperature: 40°C

Reference Example 1:

tert-butyl 3-{[(4-acetyl-5-methoxypyridin-3-yl)oxy]methyl}-3-fluoroazetidine-1-carboxylate

[Chemical Formula 35]

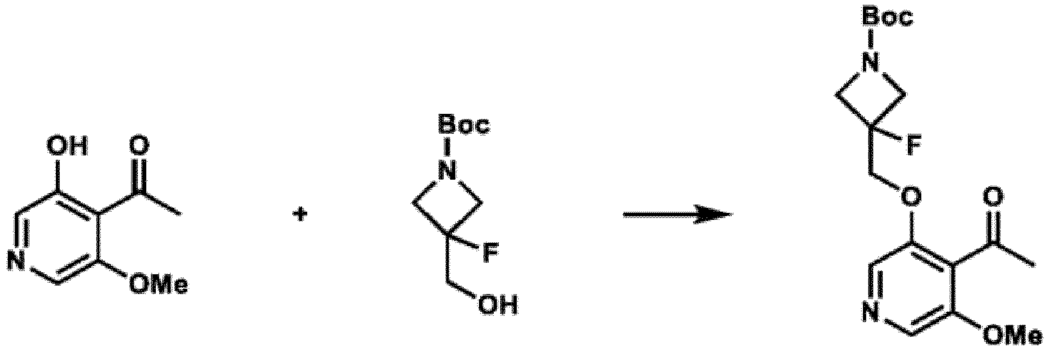

Reference Example 1

Manufacture of tert-butyl 3-{[(4-acetyl-5-methoxypyridin-3-yl)oxy]methyl}-3-fluoroazetidine-1-carboxylate (Reference Example 1)

tert-butyl 3-fluoro-3-(hydroxymethyl)azetidine-1-carboxylate (1.85 g) and triphenylphosphine (3.15 g) were added to a THF (50.0 mL) solution of 1-(3-hydroxy-5-methoxy-4-pyridinyl)ethanone (1.00 g) at room temperature, and the mixture was cooled to 0°C. Diisopropyl azodicarboxylate (2.5 mL) was added and stirred for 12 hours at 0°C. Saturated saline was added to the reaction solution, which was extracted with ethyl acetate. The organic layer was dried with anhydrous sodium sulfate then filtered, and the solvent was evaporated under reduced pressure. The residue was purified by silica gel column chromatography (hexane/ethyl acetate) to obtain Reference Example 1 (720 mg).
LC-MS; $[M+H]^+$ 355.2/Rt (minutes) 0.886 (measurement condition A)

Reference Example 2:

tert-butyl 3-{[(3-acetyl-2-methoxypyridin-4-yl)oxy]methyl}-3-(hydroxymethyl)azetidine-1-carboxylate

[Chemical Formula 36]

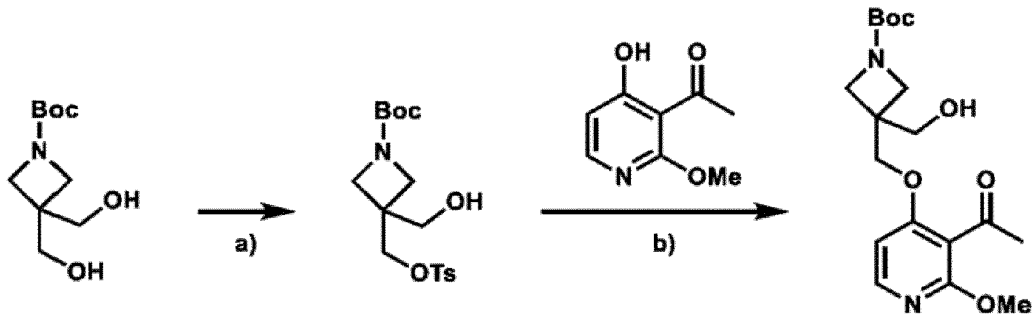


Reference Example 2

a) Manufacture of tert-butyl 3-(hydroxymethyl)-3-{[(4-methylbenzene-1-sulfonyl)oxy]methyl}azetidine-1-carboxylate

Pyridine (5.60 mL), trimethylamine hydrochloride (0.13 g), and para-toluenesulfonyl chloride (1.45 g) were added to a methylene chloride (20.0 mL) solution of tert-butyl 3,3-bis(hydroxymethyl)azetidine-1-carboxylate (1.50 g) at 0°C, and the mixture was stirred for 12 hours at room temperature. Saturated saline was added to quench the reaction solution, and the mixture was then extracted with ethyl acetate. The organic layer was dried with anhydrous sodium sulfate and then filtered, and the solvent was evaporated under reduced pressure to obtain the title compound (2.30 g). The resulting residue was used in the subsequent reaction without purification. LC-MS; $[M+H]^+$ 372.1/Rt (minutes) 0.928 (measurement

condition A)

b) Manufacture of tert-butyl 3-{[(3-acetyl-2-methoxypyridin-4-yl)oxy]methyl}-3-(hydroxymethyl)azetidine-1-carboxylate

(Reference Example 2)

1-(4-hydroxy-2-methoxy-3-pyridinyl)ethanone (1.00 g) and cesium carbonate (7.80 g) were added to a DMF (10.0 mL) solution of tert-butyl 3-(hydroxymethyl)-3-{[(4-methylbenzene-1-sulfonyl)oxy]methyl}azetidine-1-carboxylate (2.30 g), and the mixture was stirred for 12 hours at room temperature. Saturated saline was added to the reaction solution, which was extracted with ethyl acetate. The organic layer was dried with anhydrous sodium sulfate and then filtered, and the solvent was evaporated under reduced pressure. The residue was purified by silica gel column chromatography (hexane/ethyl acetate) to obtain Reference Example 2 (1.01 g).
LC-MS; $[M+H]^+$ 367.3/Rt (minutes) 0.796 (measurement condition A)

Reference Example 3:

tert-butyl(3-{[3-(3-amino-1H-pyrazol-5-yl)-4-methoxypyridin-2-yl]oxy}propyl)carbamate

[Chemical Formula 37]

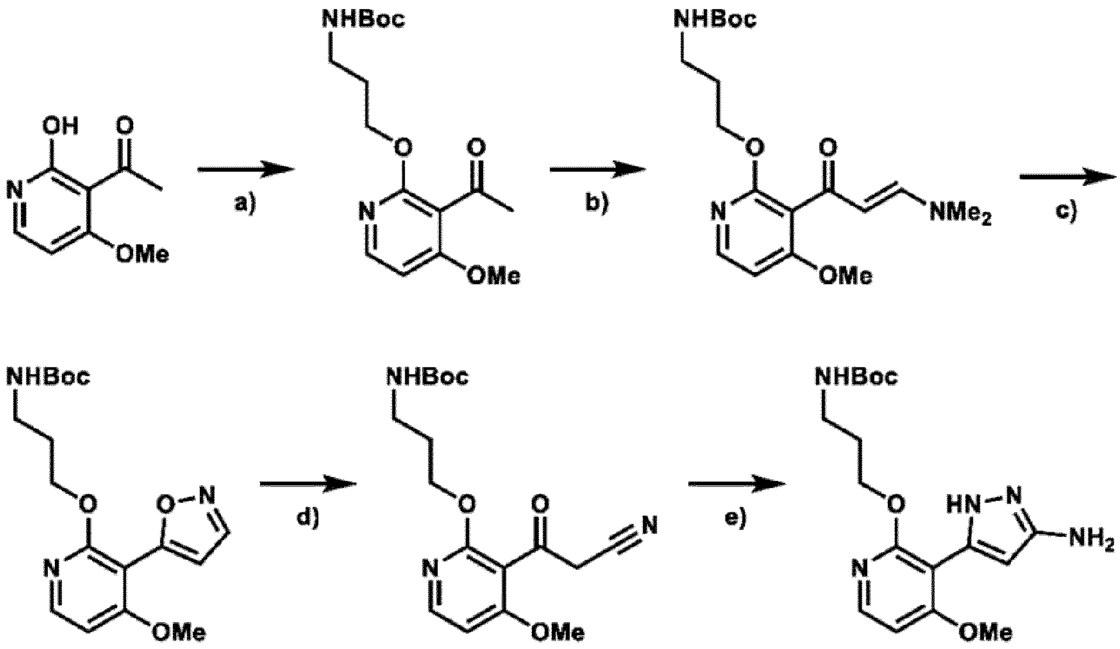


Reference Example 3

a) Manufacture of tert-butyl {3-[(3-acetyl-4-methoxypyridin-2-yl)oxy]propyl}carbamate

Cesium carbonate (7.80 g) and 3-(Boc-amino)propylbromide (4.27 g) were added to a DMF (40.0 mL) solution of 1-(2-hydroxy-4-methoxypyridin-3-yl)ethanone (2.00 g) at 0°C, and the mixture was stirred for 12 hours at room temperature. Saturated saline was added to the reaction solution, which was extracted with ethyl acetate. The organic layer was dried with anhydrous sodium sulfate and then filtered, and the solvent was evaporated under reduced pressure. The residue was purified by silica gel column chromatography (hexane/ethyl acetate) to obtain the title compound (2.25 g).

LC-MS; $[M+H]^+$ 325.2/Rt (minutes) 0.959 (measurement condition A)
1H-NMR($CDCl_3$) $\delta$: 8.06 (1H, d, J = 6.0 Hz), 6.55 (1H, d, J = 6.0 Hz), 4.97 (1H, brs), 4.40 (2H, t, J = 6.4 Hz), 3.86 (3H, s), 3.27-3.24 (2H, m), 2.49 (3H, s), 1.96-1.90 (2H, m), 1.44 (9H, s).

b) Manufacture of tert-butyl [3-({3-[(2E)-3-(dimethylamino)prop-2-enyl]-4-methoxypyridin-2-yl}oxy)propyl]carbamate

N,N-dimethylformamide dimethylacetal (10.0 mL) was added to a DMF (10.0 mL) solution of tert-butyl {3-[(3-acetyl-4-methoxypyridin-2-yl)oxy]propyl}carbamate (2.25 g). The mixture was stirred for 24 hours at 115°C. After allowing the reaction solution to cool, the solvent thereof was evaporated under reduced pressure to obtain the title compound (4.30 g) as a crude product.
LC-MS; $[M+H]^+$ 380.3/Rt (minutes) 0.722 (measurement condition A)

c) Manufacture of tert-butyl(3-{[4-methoxy-3-(1,2-oxazol-5-yl)pyridin-2-yl]oxy}propyl)carbamate

Hydroxyamine hydrochloride (5.49 g) was added to an ethanol (30.0 mL) solution of tert-butyl [3-({3-[(2E)-3-(dimethylamino)prop-2-enyl]-4-methoxypyridin-2-yl}oxy)propyl]carbamate (4.30 g). The mixture was stirred for 2 hours at 65°C. After allowing the reaction solution to cool, the reaction solution was added to an aqueous saturated sodium bicarbonate solution and quenched. The resulting aqueous solution was extracted twice with ethyl acetate. The resulting organic layer was washed with saturated saline, dried with anhydrous sodium sulfate and then filtered, and the solvent was evaporated under reduced pressure. The residue was purified by silica gel column chromatography (hexane/ethyl acetate) to obtain the title compound (1.63 g).

LC-MS; $[M+H]^+$ 350.2/Rt (minutes) 0.722 (measurement condition A)
1H-NMR($CDCl_3$) $\delta$: 8.32 (1H, d, J = 1.6 Hz), 8.12 (1H, d, J = 6.0 Hz), 6.64 (1H, d, J = 6.0 Hz), 6.59 (1H, d, J = 1.6 Hz), 4.87 (1H, brs), 4.45 (2H, t, J = 6.4 Hz), 3.92 (3H, s), 3.28-3.24 (2H, m), 1.99-1.93 (2H, m), 1.44 (9H, s).

d) Manufacture of tert-butyl(3-{[3-(cyanoacetyl)-4-methoxypyridin-2-yl]oxy}propyl)carbamate

Potassium hydroxide (0.29 g) was added to a mixture of ethanol (20.0 mL) and water (5.00 mL) of tert-butyl(3-{[4-methoxy-3-(1,2-oxazol-5-yl)pyridin-2-yl]oxy}propyl)carbamate (1.63 g). The mixture was stirred for 5 hours at room temperature. The reaction solution was concentrated under reduced pressure. The residue was added with saturated saline for quenching. The resulting aqueous solution was extracted twice with ethyl acetate. The resulting organic layer was washed with saturated saline, dried with anhydrous sodium sulfate and then filtered, and the solvent was evaporated under reduced pressure. The residue was purified by silica gel column chromatography (hexane/ethyl acetate) to obtain the title compound (1.50g).

LC-MS; $[M+H]^+$ 350.2/Rt (minutes) 0.872 (measurement condition A)
1H-NMR($CDCl_3$) $\delta$: 8.14 (1H, d, J = 6.0 Hz), 6.59 (1H, d, J = 6.0 Hz), 4.94 (1H, brs), 4.44 (2H, t, J = 6.0 Hz), 3.92 (3H, s), 3.29-3.23 (2H, m), 1.99-1.93 (2H, m), 1.43 (9H, s).

e) Manufacture of tert-butyl(3-{[3-(3-amino-1H-pyrazol-5-yl)-4-methoxypyridin-2-yl]oxy}propyl)carbamate (Reference Example 3)

Acetic acid (3.28 mL) and hydrazine monohydrate (2.78 mL) were added to an ethanol (20.0 mL) solution of tert-butyl(3-{[3-(cyanoacetyl)-4-methoxypyridin-2-yl]oxy}propyl)carbamate (1.99 g) at 0°C. The mixture was stirred for 24 hours at 90°C. After allowing the reaction solution to cool, the reaction solution was added to an aqueous saturated sodium bicarbonate solution and quenched. The resulting aqueous solution was extracted twice with chloroform. The resulting organic layer was washed with saturated saline, dried with anhydrous sodium sulfate, and then filtered, and the solvent was evaporated under reduced pressure. The residue was purified by silica gel column chromatography (chloroform/-methanol) to obtain Reference Example 3 (1.60 g).

LC-MS; $[M+H]^+$ 364.0/Rt (minutes) 0.684 (measurement condition A)
1H-NMR (DMSO-D6) $\delta$: 11.61-10.91 (1H, brs), 8.01 (1H, d, J = 12.0 Hz), 6.93-6.89 (1H, m), 6.84 (1H, d, J = 6.0 Hz), 5.93 (1H, brs), 4.50 (1H, brs), 4.32 (2H, t, J = 6.4 Hz), 3.89 (3H, s), 3.09-3.04 (2H, m), 1.86-1.79 (2H, m), 1.37 (9H, s).

<u>Reference Example 4:</u>

tert-butyl(3-{[3-(3-amino-1H-pyrazol-5-yl)-6-chloro-4-methoxypyridin-2-yl]oxy}propyl)carbamate

[Chemical Formula 38]

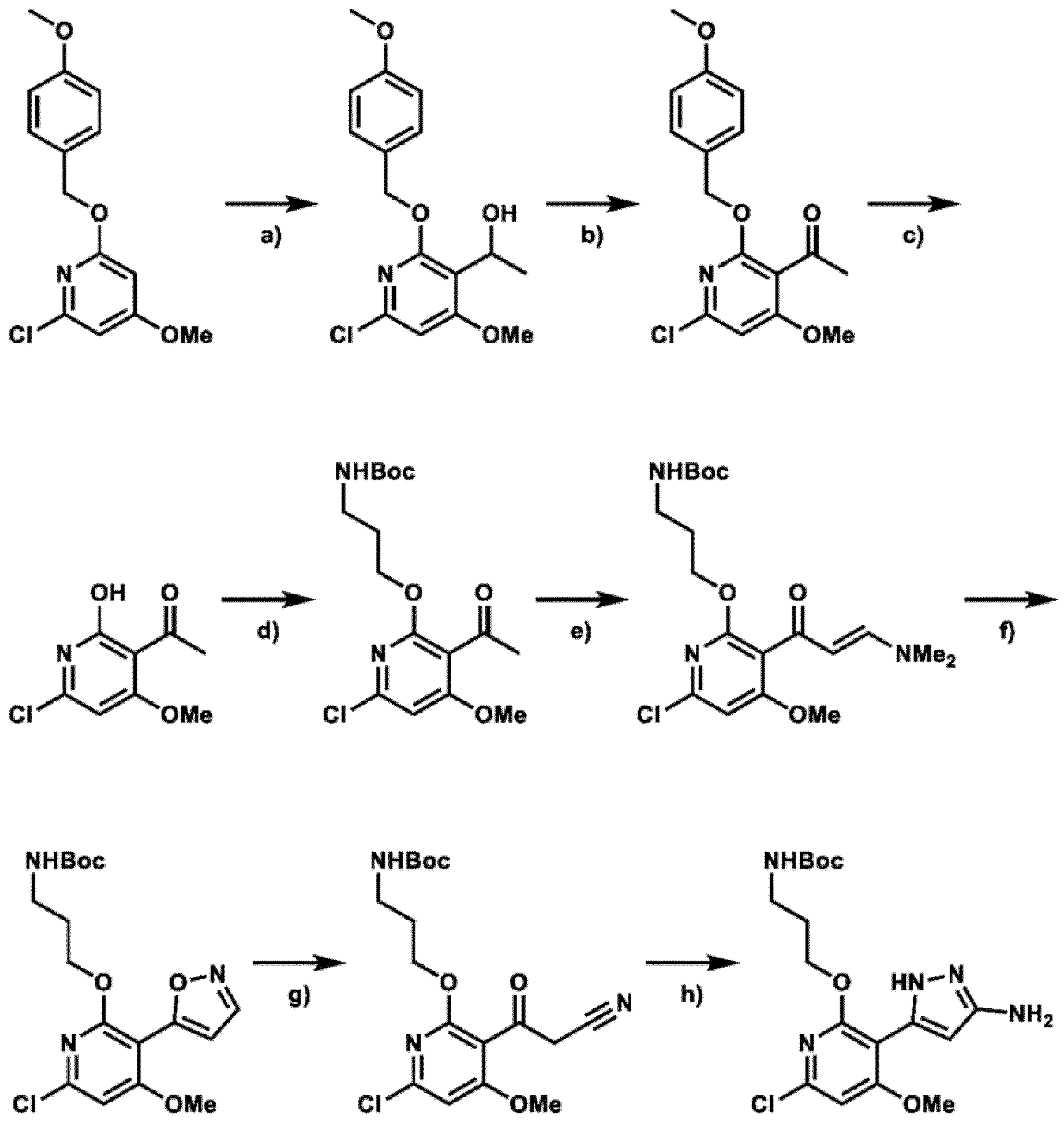


Reference Example 4

a) Manufacture of 1-{6-chloro-4-methoxy-2-[(4-methoxyphenyl)methoxy]pyridin-3-yl}ethan-1-ol

A THF (100 mL) solution of 2-chloro-4-methoxy-6-[(4-methoxybenzyl)oxy]pyridine (10.0 g) was cooled to -78°C. 2.8 mol/L n-butyllithium (15.3 mL) was added, and the mixture was stirred for 3 hours at -78°C. Acetamide (6.30 mL) was added, and the mixture was stirred for 6 hours at -78°C. The reaction solution was added with an aqueous saturated sodium bicarbonate solution and quenched, which was extracted with ethyl acetate. The organic layer was dried with anhydrous sodium sulfate and then filtered, and the solvent was evaporated under reduced pressure. The residue was purified by silica gel column chromatography (hexane/ethyl acetate) to obtain the title compound (4.25 g).

LC-MS; $[M+H]^+$ 324.2/Rt (minutes) 1.118 (measurement condition A)
1H-NMR ($CDCl_3$) δ: 7.36 (2H, d, J = 8.8 Hz), 6.89 (2H, d, J = 8.8 Hz), 6.55 (1H, s), 5.32 (2H, s), 5.16-5.11 (1H, m), 3.85 (3H, s), 3.80 (3H, s), 3.30 (1H, s), 1.41 (3H, d, J = 6.8 Hz). 13C-NMR ($CDCl_3$) &: 165.2, 159.6, 147.6, 130.1, 128.6, 114.1, 112.6, 101.8, 68.7, 62.9, 56.3, 55.4, 23.1.

b) Manufacture of 1-{6-chloro-4-methoxy-2-[(4-methoxyphenyl)methoxy]pyridin-3-yl}ethan-1-one

A Dess-Martin reagent (6.19 g) was added to a methylene chloride (100 mL) solution of 1-{6-chloro-4-methoxy-2-[(4-methoxyphenyl)methoxy]pyridin-3-yl}ethan-1-ol (3.15 g). The mixture was stirred for 12 hours at room temperature. The reaction solution was added with an aqueous saturated sodium bicarbonate solution and quenched. The resulting aqueous solution was extracted twice with ethyl acetate. The resulting organic layer was washed with saturated saline, dried with anhydrous sodium sulfate, and then filtered, and the solvent was evaporated under reduced pressure. The residue was purified by silica gel column chromatography (hexane/ethyl acetate) to obtain the title compound (2.50 g). LC-MS; $[M+H]^+$ 322.2/Rt (minutes) 1.081 (measurement condition A)

c) Manufacture of 1-(6-chloro-2-hydroxy-4-methoxypyridin-3-yl)ethan-1-one

TFA (6.00 mL) was added to a methylene chloride (30.0 mL) solution of 1-{6-chloro-4-methoxy-2-[(4-methoxyphenyl)methoxy]pyridin-3-yl}ethan-1-one (2.50 g). The mixture was stirred for 2 hours at room temperature. The solvent was evaporated under reduced pressure, and the residue was purified by amine silica gel column chromatography (chloroform/methanol) to obtain the title compound (2.00 g). LC-MS; $[M+H]^+$ 202.1/Rt (minutes) 0.773 (measurement condition A)

d) Manufacture of tert-butyl {3-[(3-acetyl-6-chloro-4-methoxypyridin-2-yl)oxy]propyl}carbamate

Cesium carbonate (9.70 g) and 3-(Boc-amino)propylbromide (5.63 g) were added to a DMF (50.0 mL) solution of 1-(6-chloro-2-hydroxy-4-methoxypyridin-3-yl)ethan-1-one (3.00 g) at 0°C. The mixture was stirred for 12 hours at room temperature. Saturated saline was added to the reaction solution, which was extracted with ethyl acetate. The organic layer was dried with anhydrous sodium sulfate and then filtered, and the solvent was evaporated under reduced pressure. The residue was purified by silica gel column chromatography (hexane/ethyl acetate) to obtain the title compound (1.01 g). LC-MS; $[M+H]^+$ 359.3/Rt (minutes) 1.123 (measurement condition A)

e) Manufacture of tert-butyl [3-({6-chloro-3-[(2E)-3-(dimethylamino)prop-2-enyl]-4-methoxypyridin-2-yl}oxy)propyl]carbamate

N,N-dimethylformamide dimethylacetal (3.00 mL) was added to a DMF (10.0 mL) solution of tert-butyl {3-[(3-acetyl-6-chloro-4-methoxypyridin-2-yl)oxy]propyl}carbamate (840 mg). The mixture was stirred for 24 hours at 115°C. After allowing the reaction solution to cool, the solvent of the reaction solution was evaporated under reduced pressure to obtain the title compound (0.97 g) as a crude product.
LC-MS; $[M+H]^+$ 414.4/Rt (minutes) 0.944 (measurement condition A)

f) Manufacture of tert-butyl(3-{[6-chloro-4-methoxy-3-(1,2-oxazol-5-yl)pyridin-2-yl]oxy}propyl)carbamate

Hydroxyamine hydrochloride (1.70 g) was added to an ethanol (30.0 mL) solution of tert-butyl [3-({6-chloro-3-[(2E)-3-(dimethylamino)prop-2-enyl]-4-methoxypyridin-2-yl}oxy)propyl]carbamate (0.97 g). The mixture was stirred for 2 hours at 65°C. After allowing the reaction solution to cool, the reaction solution was added to an aqueous saturated sodium bicarbonate solution and quenched. The resulting aqueous solution was extracted twice with ethyl acetate. The resulting organic layer was washed with saturated saline, dried with anhydrous sodium sulfate, and then filtered, and the solvent was evaporated under reduced pressure. The residue was purified by silica gel column chromatography (hexane/ethyl acetate) to obtain the title compound (677 mg).
LC-MS; $[M+H]^+$ 384.3/Rt (minutes) 1.132 (measurement condition A)

g) Manufacture of tert-butyl(3-{[6-chloro-3-(cyanoacetyl)-4-methoxypyridin-2-yl]oxy}propyl)carbamate

Potassium hydroxide (100 mg) was added to a mixture of ethanol (20.0 mL) and water (5.00 mL) of tert-butyl(3-{[6-chloro-4-methoxy-3-(1,2-oxazol-5-yl)pyridin-2-yl]oxy}propyl)carbamate (677 mg). The mixture was stirred for 2 hours at room temperature. The reaction solution was concentrated under reduced pressure. The residue was added to saturated saline and quenched. The resulting aqueous solution was extracted twice with ethyl acetate. The resulting organic layer was washed with saturated saline, dried with anhydrous sodium sulfate, and then filtered, and the solvent was evaporated under reduced pressure. The residue was purified by silica gel column chromatography (hexane/ethyl acetate) to obtain the title compound (470 mg).
LC-MS; $[M+H]^+$ 384.3/Rt (minutes) 1.064 (measurement condition A)

h) Manufacture of tert-butyl(3-{[3-(3-amino-1H-pyrazol-5-yl)-6-chloro-4-methoxypyridin-2-yl]oxy}propyl)carbamate (Reference Example 4)

Acetic acid (0.71 mL) and hydrazine monohydrate (0.77 mL) were added to an ethanol (20.0 mL) solution of tert-butyl(3-{[6-chloro-3-(cyanoacetyl)-4-methoxypyridin-2-yl]oxy}propyl)carbamate (470 mg) at 0°C. The mixture was stirred for 24 hours at 90°C. After allowing the reaction solution to cool, the reaction solution was added to an aqueous saturated sodium bicarbonate solution and quenched. The resulting aqueous solution was extracted twice with chloroform. The resulting organic layer was washed with saturated saline, washed with anhydrous sodium sulfate, and then filtered, and the solvent was evaporated under reduced pressure. The residue was purified by silica gel column chromatography (chloroform/methanol) to obtain Reference Example 4 (350 mg). LC-MS; $[M+H]^+$ 398.3/Rt (minutes) 0.911 (measurement condition A)

Reference Example 5:

1-(4-hydroxy-6-methoxypyrimidin-5-yl)ethan-1-one

[Chemical Formula 39]

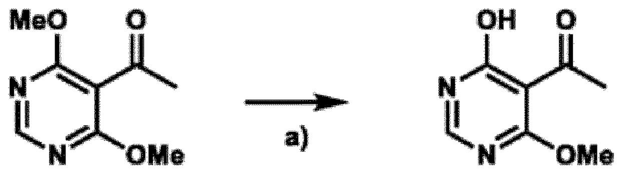


Reference Example 5

a) Manufacture of 1-(4-hydroxy-6-methoxypyrimidin-5-yl)ethan-1-one

A dichloromethane solution (1.0 mol/L, 54.9 mL) of boron tribromide was added to a dichloromethane (30.0 mL) solution of 1-(4,6-dimethoxypyrimidin-5-yl)ethan-1-one (2.00 g) at -60°C. The mixture was stirred for 3 hours at -50°C or lower. The reaction solution was added with saturated saline and quenched, and then extracted with chloroform. The organic layer was washed with saturated saline, dried with anhydrous sodium sulfate, and then filtered, and the solvent was evaporated under reduced pressure. The residue was purified by silica gel column chromatography (methanol/-chloroform) to obtain the title compound (220 mg).

LC-MS; $[M+H]^+$ 169.1/Rt (minutes) 0.367 (measurement condition A)
1H-NMR ($CDCl_3$) $\delta$: 14.31 (1H, brs), 8.36 (1H, s), 4.10 (3H, s), 2.65 (3H, s).

Reference Example 6:

tert-butyl(3-{[4-acetyl-5-(fluoromethoxy)pyridin-3-yl]oxy}propyl)carbamate

[Chemical Formula 40]

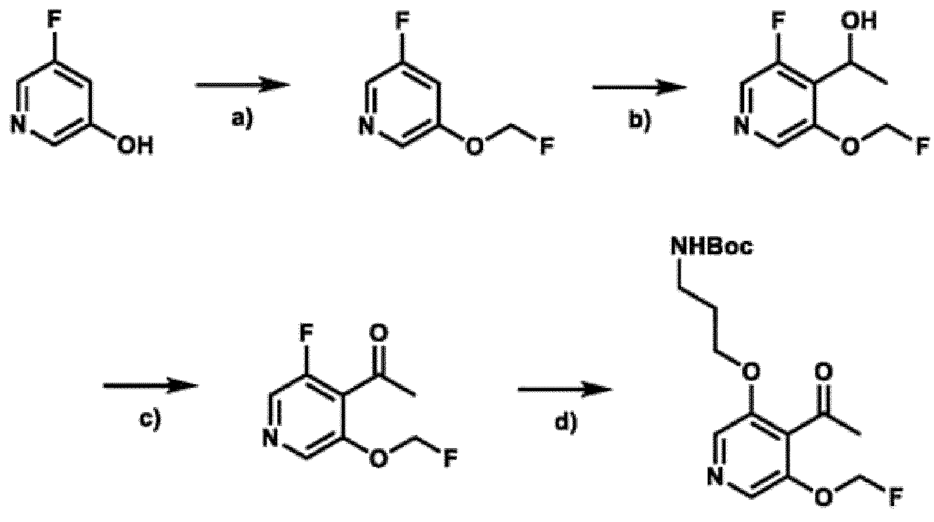


Reference Example 6

a) Manufacture of 3-fluoro-5-(fluoromethoxy)pyridine

Cesium carbonate (4.32 g) and fluoromethyl 4-methylbenzenesulfonate (2.17 g) were added to a DMF (30.0 mL) solution of 5-fluoropyridin-3-ol (1.00 g) at room temperature. The mixture was heated and stirred for 8 hours at 80°C. Water was added to the reaction solution, which was extracted with diethyl ether. The organic layer was dried with anhydrous sodium sulfate and then filtered, and the solvent was evaporated under reduced pressure. The residue was purified by silica gel column chromatography (hexane/ethyl acetate) to obtain the title compound (538 mg).
LC-MS; $[M+H]^+$ 146.0/Rt (minutes) 0.574 (measurement condition A)

b) Manufacture of 1-[3-fluoro-5-(fluoromethoxy)pyridin-4-yl]ethan-1-ol

A THF (15 mL) solution of N,N-diisopropylethylamine (0.687 mL) was cooled to -78°C, and n-butyllithium (1.58 moL/L, 3.05 mL) was added. The mixture was stirred for 15 minutes at 0°C. The reaction solution was cooled again to -78°C, and 3-fluoro-5-(fluoromethoxy)pyridine (538 mg) was added. After stirring for 1 hour, acetaldehyde (0.419 mL) was added. The reaction solution was gradually warmed to room temperature and stirred overnight. Water was then added to the reaction solution, which was extracted with ethyl acetate. The organic layer was dried with anhydrous sodium sulfate and then filtered, and the solvent was evaporated under reduced pressure. The residue was purified by silica gel column chromatography (hexane/ethyl acetate) to obtain the title compound (475 mg).

LC-MS; $[M+H]^+$ 190.1/Rt (minutes) 0.494 (measurement condition A)
1H-NMR ($CDCl_3$) $\delta$: 8.30 (1H, d, J = 1.8 Hz), 8.26 (1H, s), 5.85 (1H, dd, J = 6.7, 3.1 Hz), 5.71 (1H, dd, J = 7.2, 3.2 Hz), 5.31-5.22 (1H, m), 2.60 (1H, dd, J = 9.8, 1.8 Hz), 1.59 (3H, d, J = 6.7 Hz).

c) Manufacture of 1-[3-fluoro-5-(fluoromethoxy)pyridin-4-yl]ethan-1-one

A dichloromethane (10.0 mL) solution of 1-[3-fluoro-5-(fluoromethoxy)pyridin-4-yl]ethan-1-ol (475 mg) was cooled with ice, and a Dess-Martin reagent (1.60 g) was added. The mixture was stirred overnight at room temperature. An aqueous sodium thiosulfate solution and aqueous saturated sodium bicarbonate solution were added to the reaction solution, which was extracted with chloroform. The resulting organic layer was dried with anhydrous sodium sulfate, and then filtered, and the solvent was evaporated under reduced pressure. The residue was purified by silica gel column chromatography (hexane/ethyl acetate) to obtain the title compound (407 mg).
LC-MS; $[M+H]^+$ 188.0/Rt (minutes) 0.618 (measurement condition A)

d) Manufacture of tert-butyl(3-{[4-acetyl-5-(fluoromethoxy)pyridin-3-yl]oxy}propyl)carbamate

tert-butyl(3-hydroxypropyl)carbamate (762 mg) was added to a DMF (10.0 mL) solution of 1-[3-fluoro-5-(fluoromethoxy)pyridin-4-yl]ethan-1-one (407 mg) and cesium carbonate (1.42 g), and the mixture was heated and stirred for 8 hours at 80°C. Water was added to the reaction solution, which was extracted with diethyl ether. The organic layer was dried with anhydrous sodium sulfate and then filtered, and the solvent was evaporated under reduced pressure. The residue was purified by silica gel column chromatography (hexane/ethyl acetate) to obtain the title compound (67 mg).

LC-MS; $[M+H]^+$ 343.2/Rt (minutes) 0.832 (measurement condition A)
1H-NMR (CDCl3) δ: 8.22 (1H, d, J = 1.8 Hz), 8.16 (1H, s), 5.68 (2H, d, J = 53.6 Hz), 4.70 (1H, brs), 4.15 (2H, t, J = 6.1 Hz), 3.32-3.21 (2H, m), 2.50 (3H, s), 2.00-1.93 (2H, m), 1.41 (9H, s).

Reference Example 7:

tert-butyl(3-{[4-methoxy-3-(1,2-oxazol-5-yl)pyridin-2-yl]oxy}propyl)carbamate

[Chemical Formula 41]

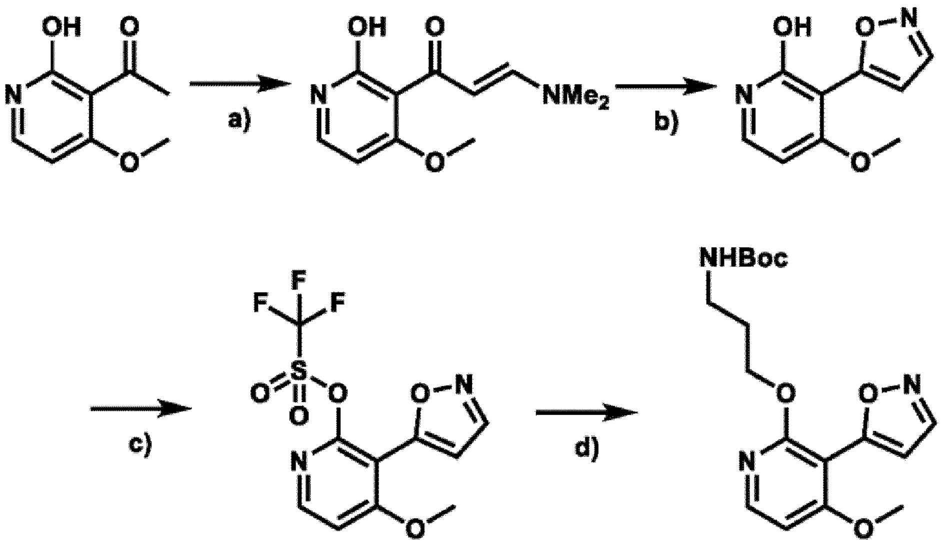


Reference Example 7

a) Manufacture of (2E)-3-(dimethylamino)-1-(2-hydroxy-4-methoxypyridin-3-yl)prop-2-en-1-one

tert-butoxybis(dimethylamino)methane (5.21 g) was added to a mixture of 1-(2-hydroxy-4-methoxypyridin-3-yl)ethanone (2.50 g) and toluene (6.23 ml). The mixture was heated and stirred for 6 hours at 55°C. After allowing the mixture to cool, toluene (18.6 ml) was added dropwise. The resulting crystal was collected by filtration and washed with toluene. The crystal was vacuum dried to obtain the title compound (2.87 g). The filtrate was concentrated under reduced pressure. The crystal deposited out from an ethanol/toluene mixture was washed with an ethanol/toluene mixture and vacuum dried to obtain a second crop (0.20 g) of the title compound.

LC-MS; $[M+H]^+$ 223.1/Rt (minutes) 1.564
1H-NMR (DMSO-D6) δ: 11.25 (1H, br s), 7.39 (1H, d, J = 6.7 Hz), 7.11 (1H, br s), 6.21 (1H, d, J = 7.9 Hz), 5.00 (1H, d, J = 11.0 Hz), 3.73 (3H, s), 3.00 (3H, br s), 2.75 (3H, br s).

b) Manufacture of 4-methoxy-3-(1,2-oxazol-5-yl)pyridin-2-ol

Hydroxyamine hydrochloride (1.56 g) was added to a mixture of (2E)-3-(dimethylamino)-1-(2-hydroxy-4-methoxypyridin-3-yl)prop-2-en-1-one (2.50 g) and ethanol (37.5 ml). The mixture was stirred for 16 hours at room temperature. Water (37.5 ml) was added to the reaction mixture. The mixture was concentrated under reduced pressure until reaching 28.5 g. Water (9.0 ml) was then further added. The mixture was stirred at 0°C. The resulting crystal was collected by filtration, washed with cold water, and vacuum dried to obtain the title compound (1.53 g).

LC-MS; $[M+H]^+$ 193.1/Rt (minutes) 0.928
1H-NMR (DMSO-D6) δ: 11.83 (1H, br s), 8.51 (1H, d, J = 1.8 Hz), 7.61 (1H, d, J = 7.3 Hz), 6.83 (1H, d, J = 1.8 Hz), 6.38 (1H, d, J = 7.3 Hz), 3.93 (3H, s).

c) Manufacture of 4-methoxy-3-(1,2-oxazol-5-yl)pyridin-2-yl trifluoromethanesulfonate

Trifluoromethanesulfonic acid anhydride (2.16 g) was added dropwise to a mixture of 4-methoxy-3-(1,2-oxazol-5-yl)pyridin-2-ol (1.23 g) and pyridine (6.1 ml) at 0°C. The reaction mixture was warmed, and stirred for 16 hours at room temperature. The reaction mixture was cooled to 0°C. Water (16 ml) was added dropwise. The mixture was stirred for 1.5 hours at 0°C. The resulting crystal was collected by filtration, washed with a water/pyridine mixture and then water, and vacuum dried to obtain the title compound (1.91 g).

LC-MS; $[M+H]^+$ 325.0/Rt (minutes) 4.781
1H-NMR ($CDCl_3$) δ: 8.37 (1H, d, J = 1.8 Hz), 8.32 (1H, d, J = 6.1 Hz), 7.01 (1H, d, J = 6.1 Hz), 6,67 (1H, d, J = 1.8 Hz), 4.00 (3H, s).

d) Manufacture of tert-butyl(3-{[4-methoxy-3-(1,2-oxazol-5-yl)pyridin-2-yl]oxy}propyl)carbamate

Diisopropylethylamine (72 mg) was added to a mixture of 4-methoxy-3-(1,2-oxazol-5-yl)pyridin-2-yl trifluoromethanesulfonate (50 mg), tert-butyl(3-hydroxypropyl)carbamate (54 mg), 4,5-bis(diphenylphosphino)-9,9-dimethylxanthene (18 mg), tris(dibenzylideneacetone)dipalladium(0) chloroform adduct (16 mg), and toluene (1.5 ml). The mixture was heated and stirred for 4 hours at 100°C. After allowing the reaction mixture to cool, the insoluble matter was filtered out with celite, and the mixture was washed with ethyl acetate. The filtrate was concentrated, and the residue was purified by silica gel column chromatography (hexane/ethyl acetate) to obtain the title compound (30 mg).
LC-MS; $[M+H]^+$ 350.2/Rt (minutes) 4.647

Reference Examples 8 to 41:

The compounds in Reference Examples 8 to 41 shown in the following table were obtained by the similar method to the method described in Reference Examples 1-7 by using a corresponding raw material compound.

[Table 1-1]

| Reference Example | Structural formula | LC-MS; [M+H]+ Time of retention (minutes) | Measurement condition |
|---|---|---|---|
| 8 | 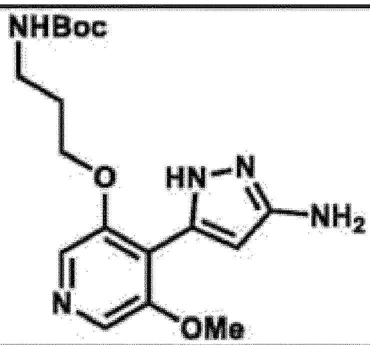 | 364.3/0.724 | A |
| 9 | 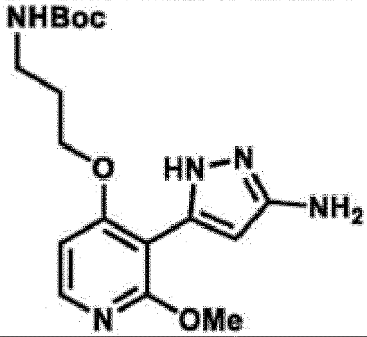 | 364.3/0.722 | A |
| 10 | 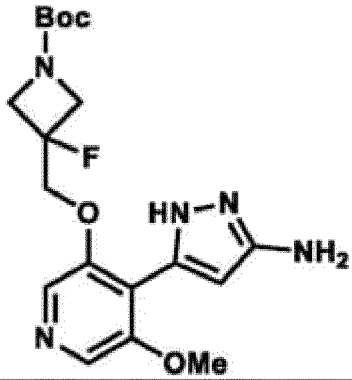 | 394.3/0.660 | A |
| 11 | 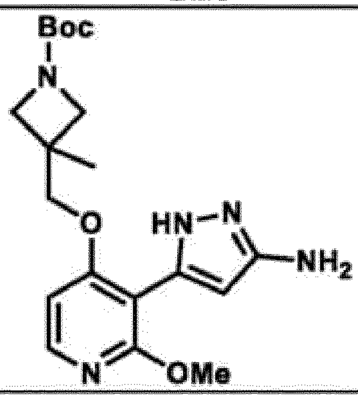 | 390.6/0.714 | A |

[Table 1-2]

| | | | |
|---|---|---|---|
| １２ | 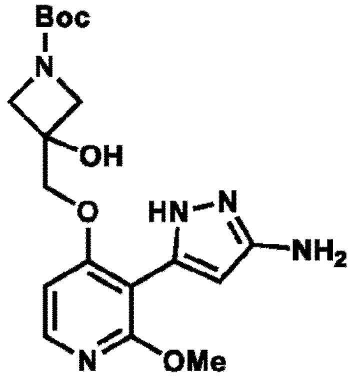 | ３９２．４／０．６３２ | Ａ |
| １３ | 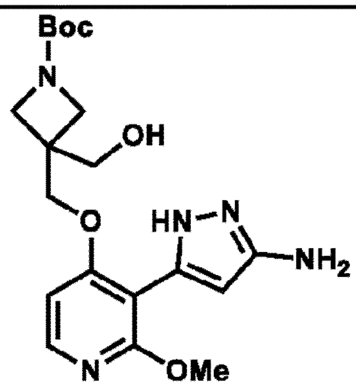 | ４０６．４／０．６５８ | Ａ |
| １４ | 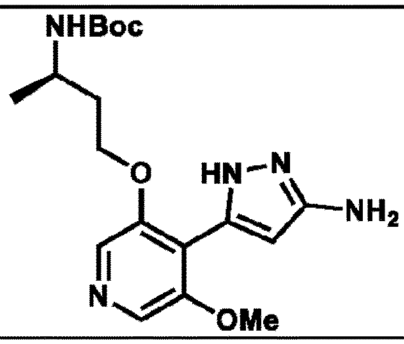 | ３７８．３／０．６１０ | Ａ |
| １５ | 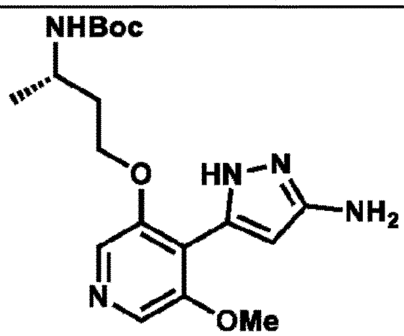 | ３７８．３／０．６１６ | Ａ |

[Table 1-3]

| | | | |
|---|---|---|---|
| １６ | 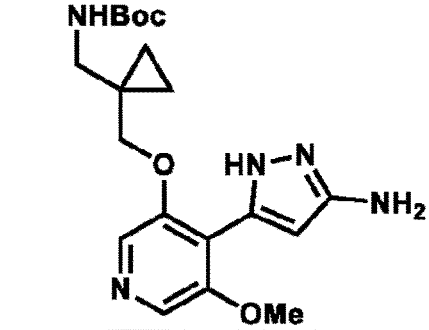 | ３９０．３／０．６３３ | Ａ |
| １７ | 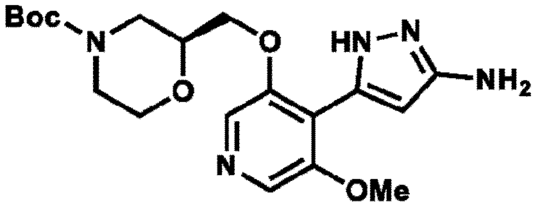 | ４０６．３／０．６２４ | Ａ |
| １８ | 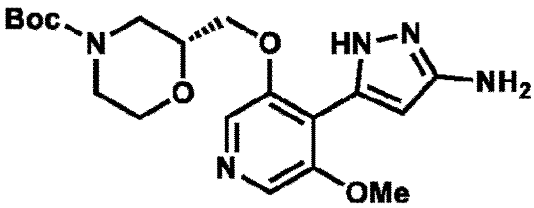 | ４０６．４／０．６３８ | Ａ |
| １９ | 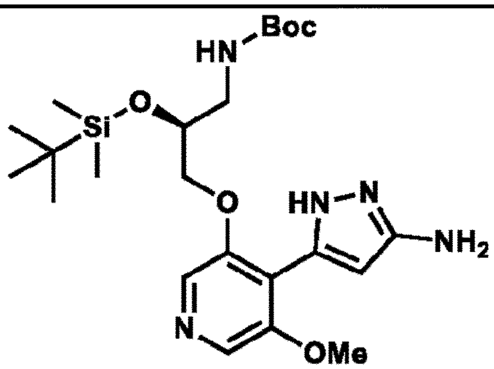 | ４９４．５／０．９８１ | Ａ |
| ２０ | 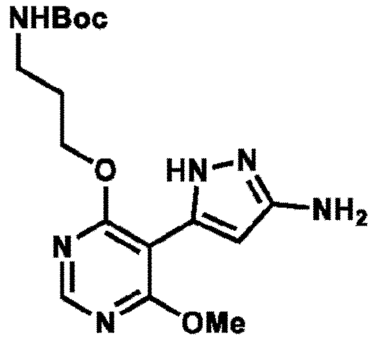 | ３６５．３／０．６７８ | Ａ |

[Table 1-4]

| | | | |
|---|---|---|---|
| ２１ | 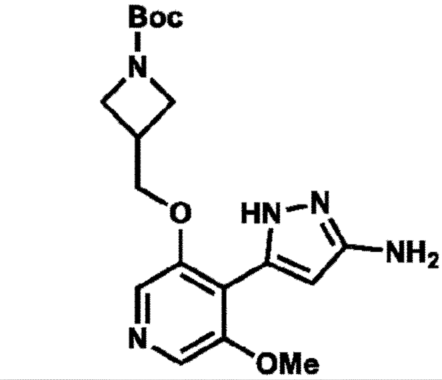 | ３７６．３／０．６１１ | A |
| ２２ | 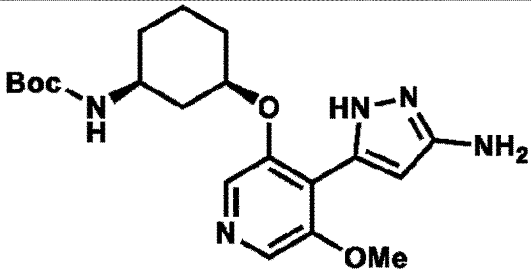 | ４０４．４／０．６８８ | A |
| ２３ | 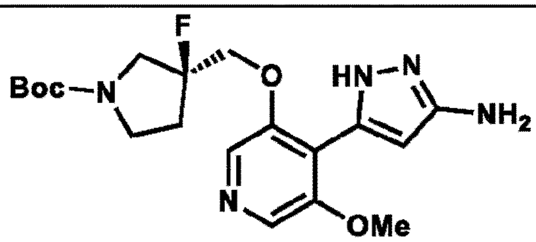 | ４０８．３／０．６８７ | A |
| ２４ | 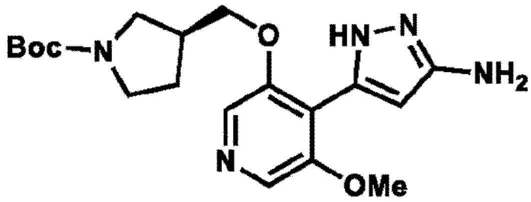 | ３９０．３／０．６７４ | A |
| ２５ | 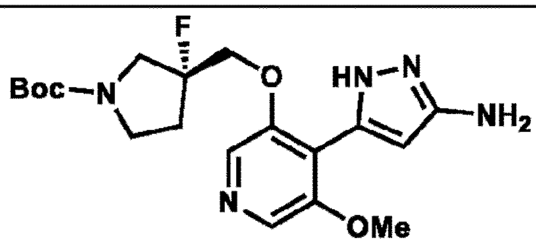 | ４０８．３／０．６９８ | A |

[Table 1-5]

| | | | |
|---|---|---|---|
| ２６ | 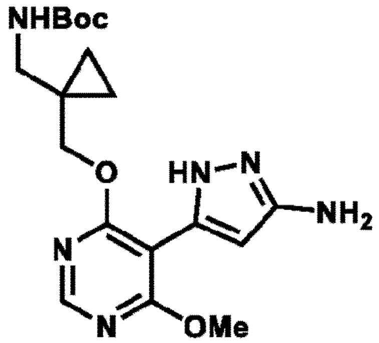 | ３９１．３／０．７７７ | A |
| ２７ | 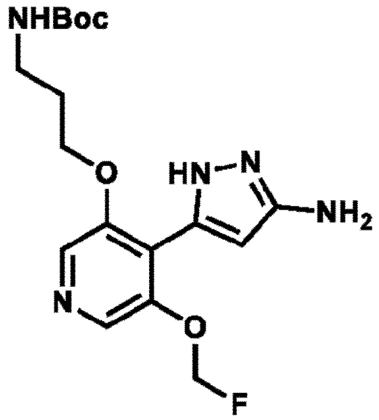 | ３８２．３／０．６６０ | A |
| ２８ | 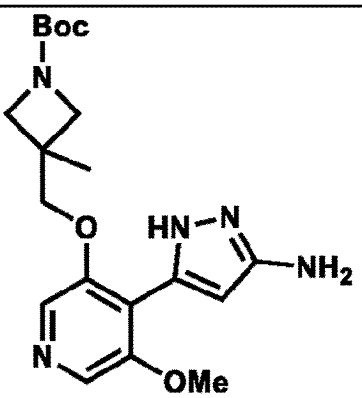 | ３９０．３／０．６６０ | A |
| ２９ | 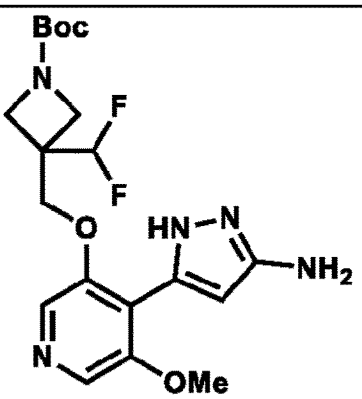 | ４２６．３／０．７２１ | A |

[Table 1-6]

| | | | |
|---|---|---|---|
| ３０ | 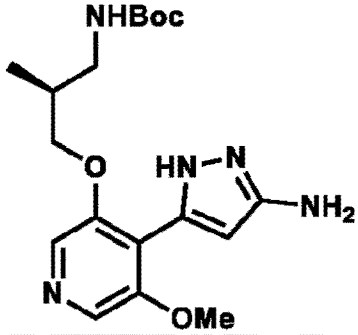 | ３７８／１．１７４ | C |
| ３１ | 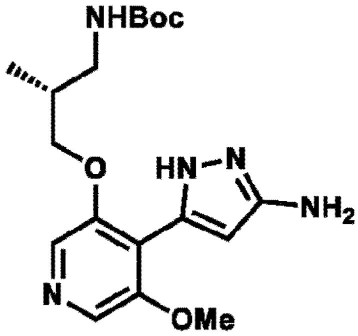 | ３７８／１．１７５ | C |
| ３２ | 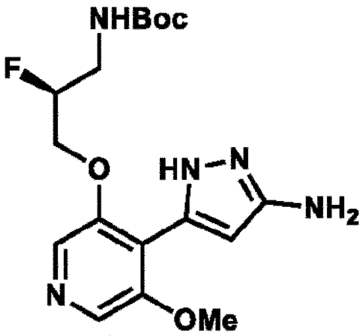 | ３８２／１．１５５ | C |
| ３３ | 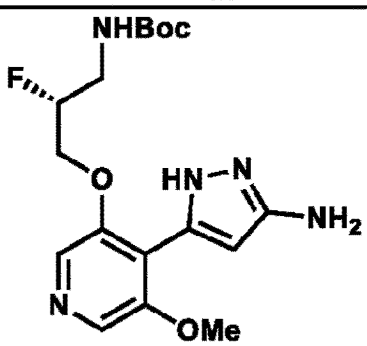 | ３８２／１．１５４ | C |

[Table 1-7]

| | | | |
|---|---|---|---|
| ３４ | 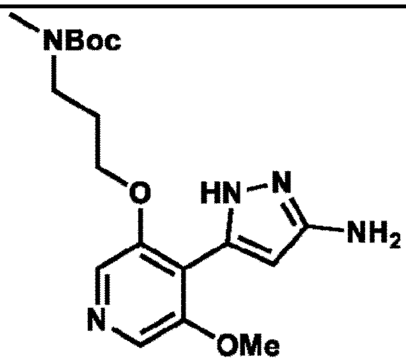 | ３７８／１．１９４ | Ｃ |
| ３５ | 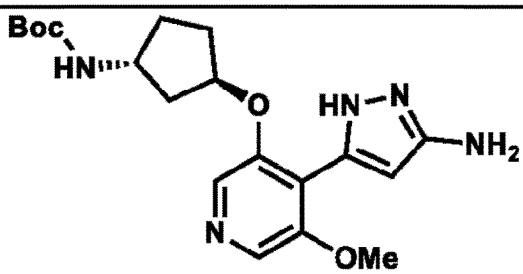 | ３９０／１．１７７ | Ｃ |
| ３６ | 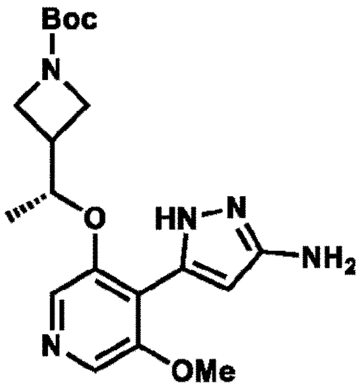 | ３９０／１．２２２ | Ｃ |
| ３７ | 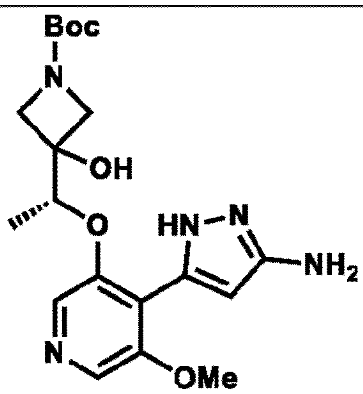 | ４０６／１．１４２ | Ｃ |

[Table 1-8]

| | | | |
|---|---|---|---|
| 3 8 | 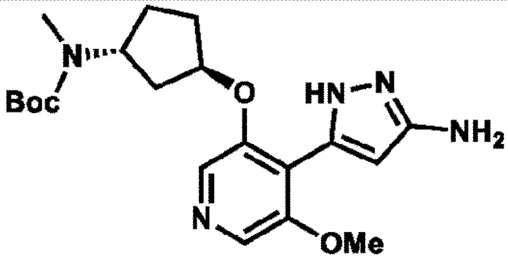 | 4 0 4 ／1. 2 9 0 | C |
| 3 9 | 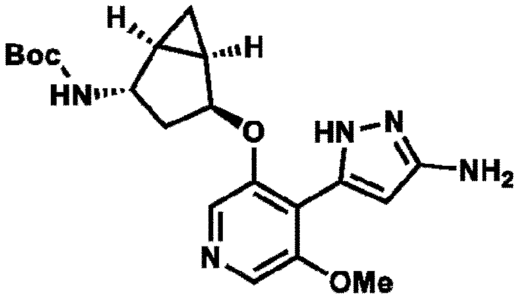 | 4 0 2 ／1. 2 2 8 | C |
| 4 0 | 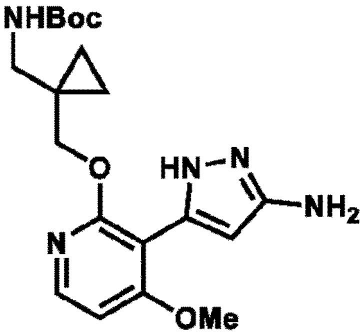 | 3 9 0 ／1. 3 4 4 | C |
| 4 1 | 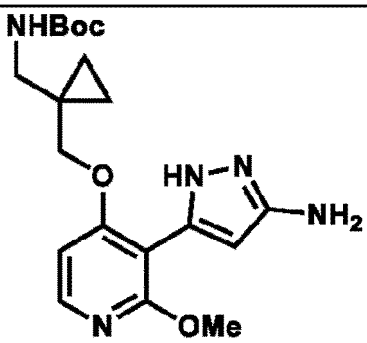 | 3 9 0 ／1. 3 1 5 | C |

Reference Example 42:

tert-butyl {3-[(3-{3-[(5-cyanopyrazin-2-yl)amino]-1H-pyrazol-5-yl}-4-methoxypyridin-2-yl)oxy]propyl}carbamate

[Chemical Formula 42]

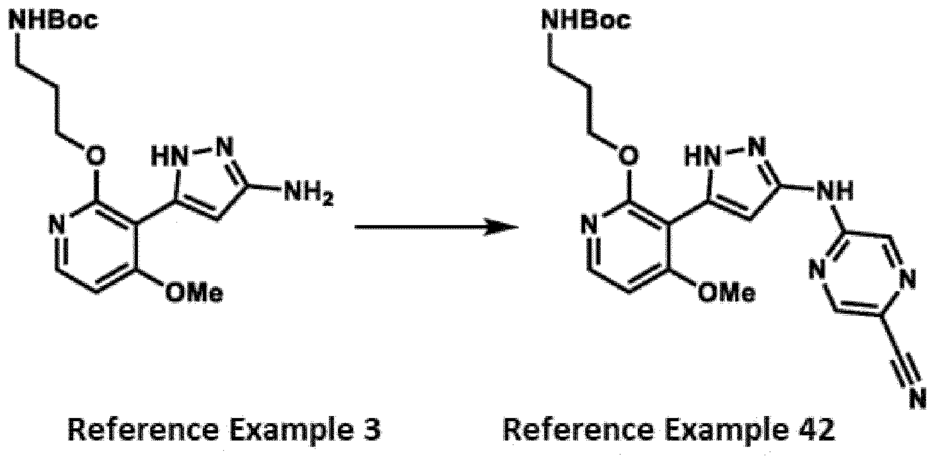

Reference Example 3 Reference Example 42

4-ethylmorpholine (1.00 mL) and 5-chloropyrazine-2-carbonitrile (461 mg) were added to a DMSO (10.0 mL) solution of tert-butyl(3-{[3-(3-amino-1H-pyrazol-5-yl)-4-methoxypyridin-2-yl]oxy}propyl)carbamate (600 mg) at room temperature. The mixture was stirred for 12 hours at 80°C. Saturated saline was added to the reaction solution, which was extracted with ethyl acetate. The organic layer was dried with anhydrous sodium sulfate and then filtered, and the solvent was evaporated under reduced pressure. The residue was purified by silica gel column chromatography (hexane/ethyl acetate) to obtain the title compound (1.01 g).

LC-MS; $[M+H]^+$ 467.3/Rt (minutes) 0.885 (measurement condition A)
1H-NMR (DMSO-D6) $\delta$: 12.4 (1H, s), 10.7 (1H, s), 8.61 (1H, s), 8.50 (1H, brs), 8.07 (1H, d, J = 5.2 Hz), 7.06 (1H, brs), 6.90 (1H, d, J = 6.0 Hz), 4.34 (2H, t, J = 6.4 Hz), 3.93 (3H, s), 3.14-3.08 (2H, m), 1.87-1.84 (2H, m), 1.35 (9H, s).

<u>Reference Examples 43 to 78:</u>

The compounds in Reference Examples 43 to 78 shown in the following table were obtained by the similar method to the method described in Reference Example 42 by using a corresponding raw material compound.

[Table 2-1]

| Reference Example | Structural formula | LC−MS ; [M+H] + Time of retention (minutes) | Measurement condition |
|---|---|---|---|
| 43 | 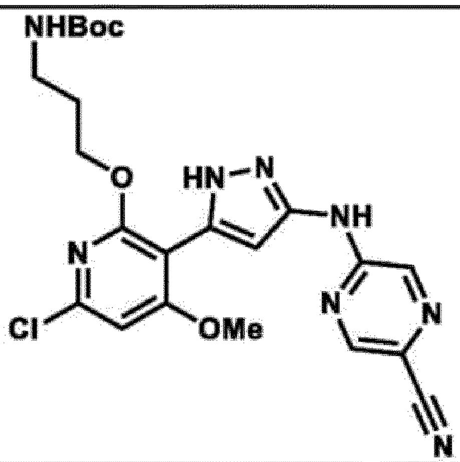 | 501. 4／1. 123 | A |
| 44 | 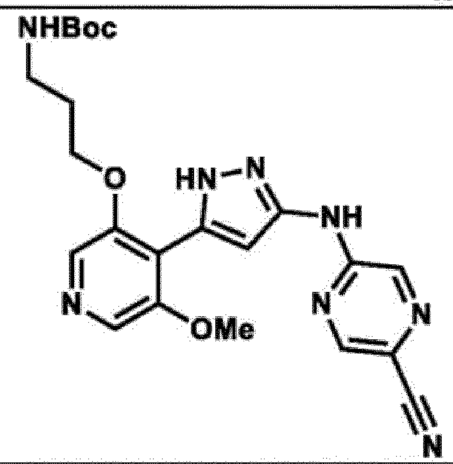 | 467. 3／0. 898 | A |
| 45 | 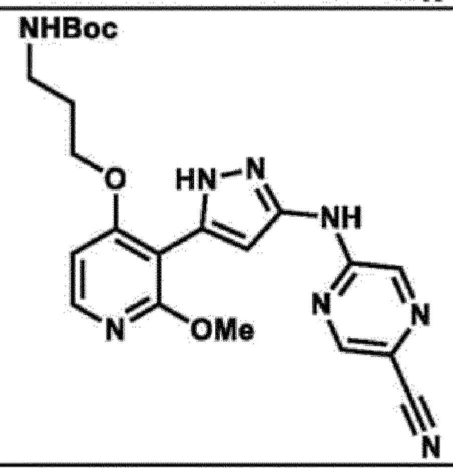 | 467. 3／0. 885 | A |

[Table 2-2]

| | | | |
|---|---|---|---|
| ４６ | 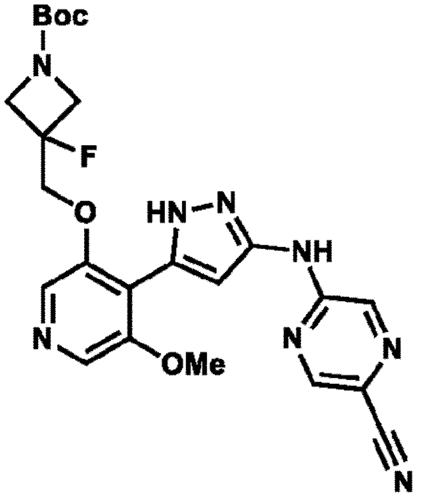 | ４９７．３／０．８５３ | Ａ |
| ４７ | 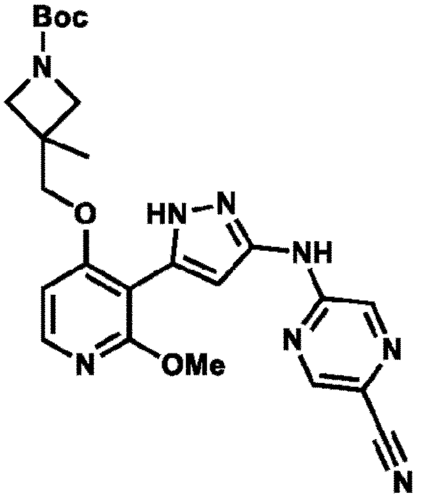 | ４９３．４／０．９７２ | Ａ |
| ４８ | 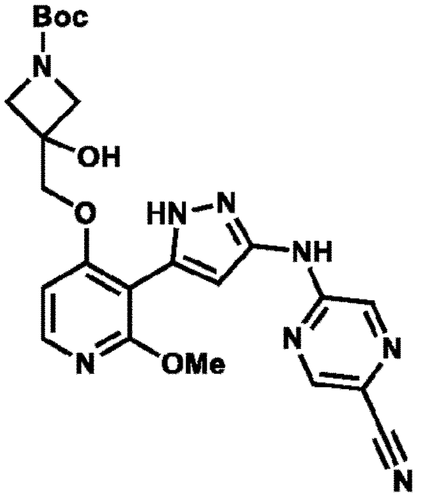 | ４９５．４／０．８４９ | Ａ |

[Table 2-3]

| | | | |
|---|---|---|---|
| ４９ | 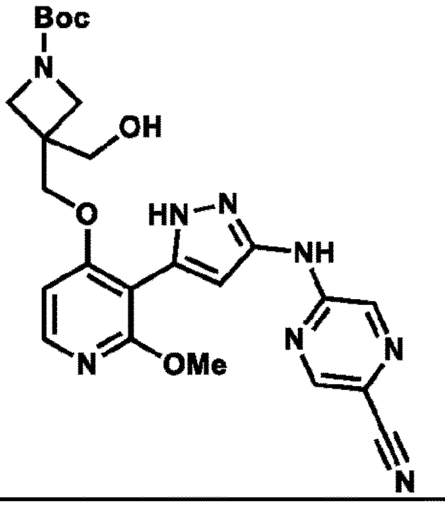 | ５０９．４／０．８４６ | A |
| ５０ | 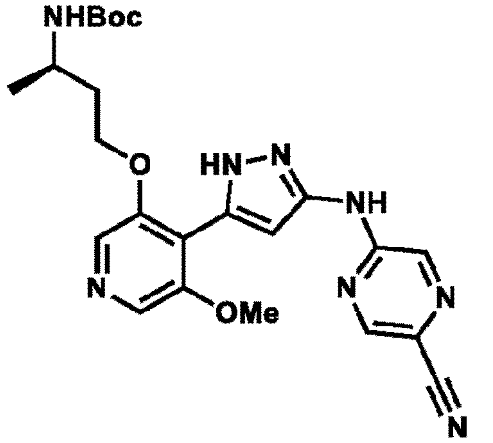 | ４８１．４／０．８２６ | A |
| ５１ | 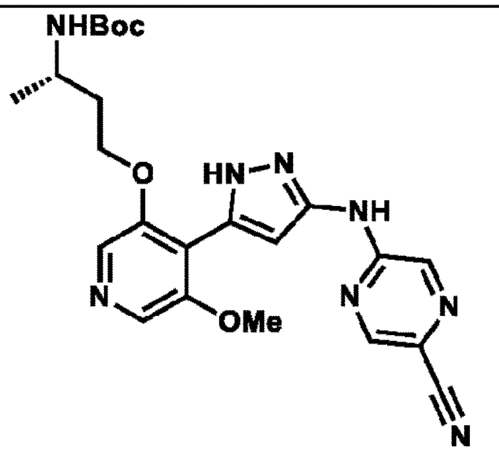 | ４８１．４／０．８２７ | A |

[Table 2-4]

| | | | |
|---|---|---|---|
| 52 | 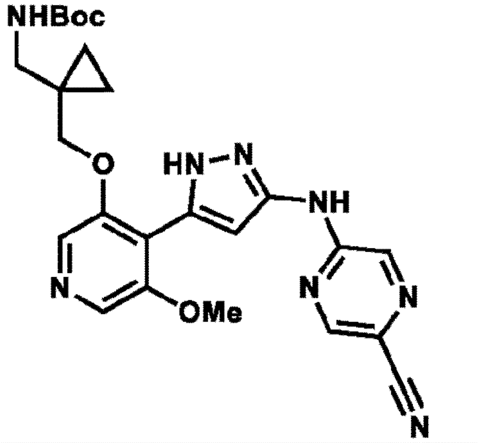 | 494.4/0.828 | A |
| 53 | 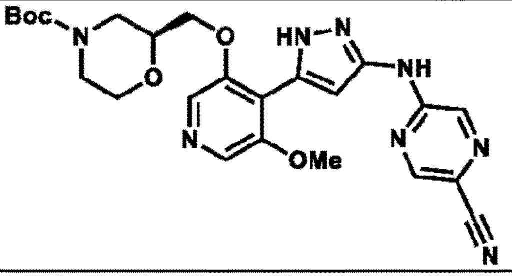 | 509.4/0.839 | A |
| 54 | 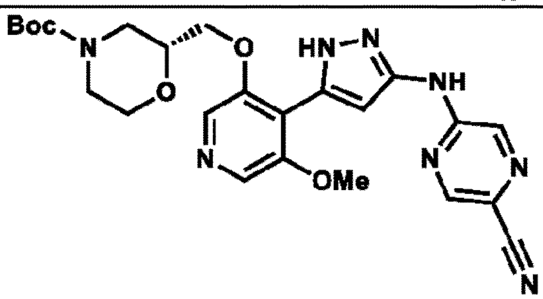 | 509.4/0.835 | A |
| 55 | 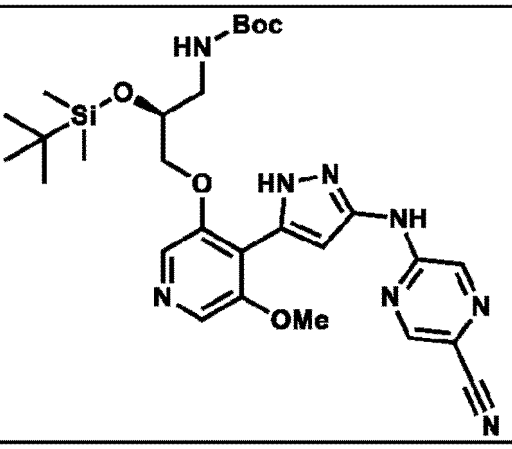 | 597.5/1.154 | A |

[Table 2-5]

| | | | |
|---|---|---|---|
| ５６ | 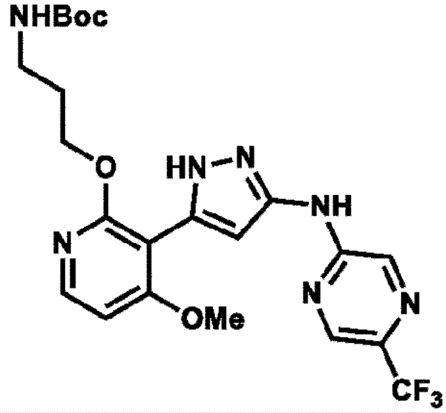 | ５１０．４／１．０１７ | Ａ |
| ５７ | 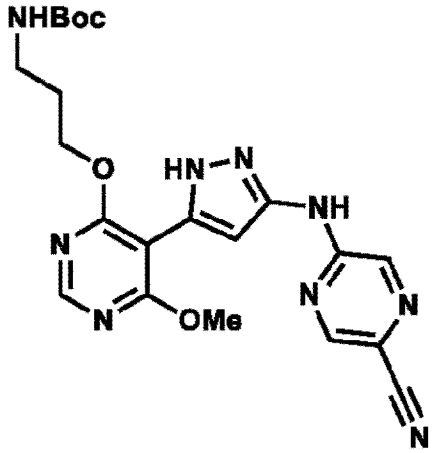 | ４６８．４／０．９２７ | Ａ |
| ５８ | 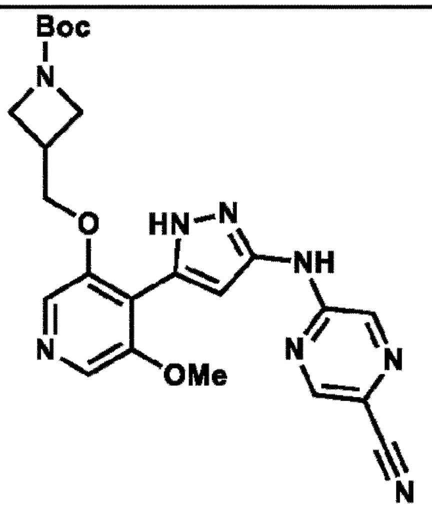 | ４７９．４／０．８１９ | Ａ |

[Table 2-6]

| | | | |
|---|---|---|---|
| 59 | 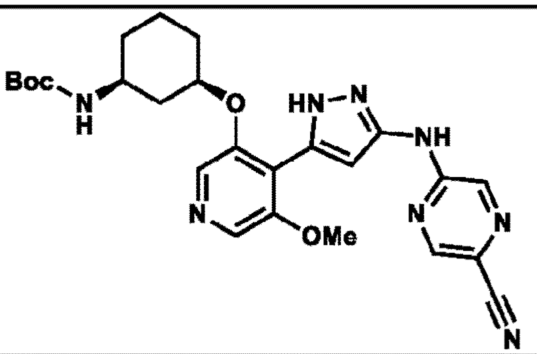 | 507.4/0.872 | A |
| 60 | 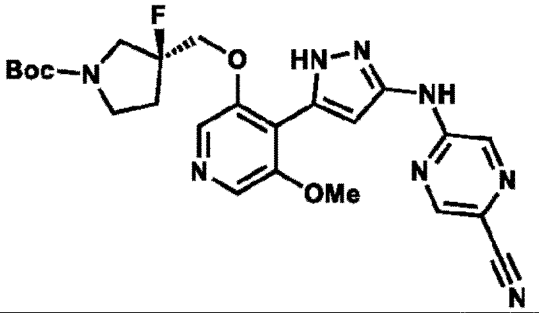 | 511.4/0.851 | A |
| 61 | 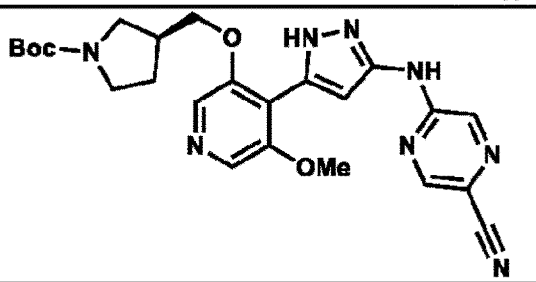 | 493.4/0.869 | A |
| 62 | 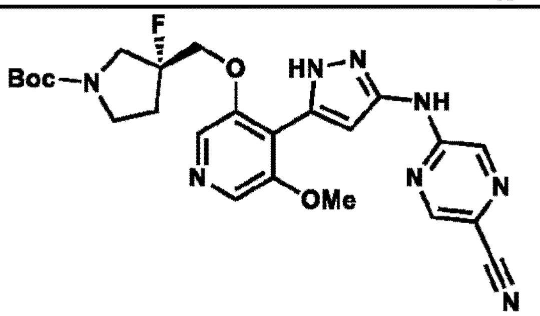 | 511.4/0.859 | A |

[Table 2-7]

| | | | |
|---|---|---|---|
| ６３ | 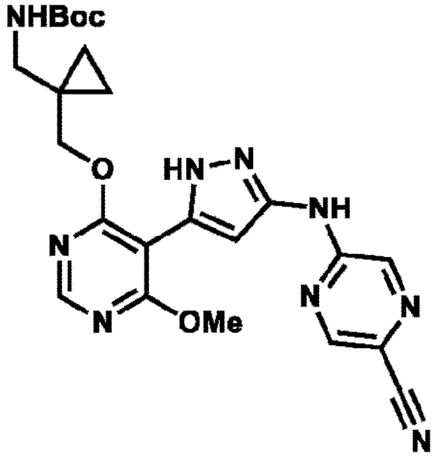 | ４９４．４／０．９８４ | A |
| ６４ | 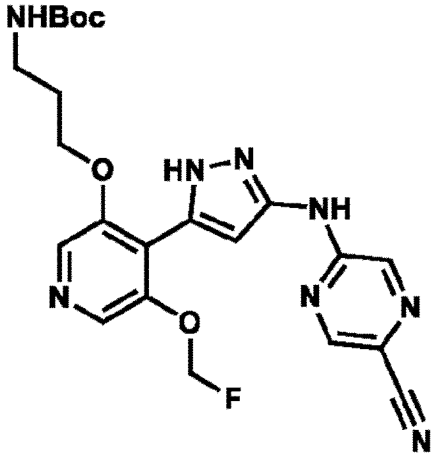 | ４８５．４／０．８４６ | A |
| ６５ | 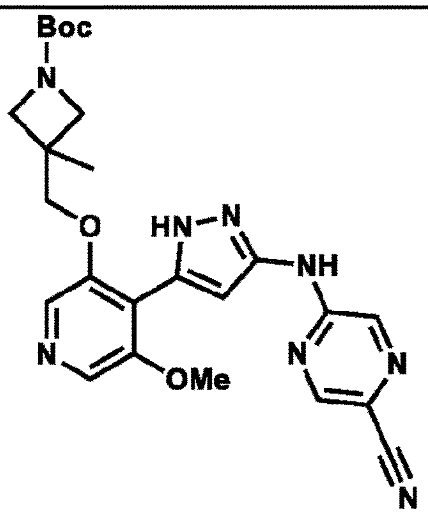 | ４９３．４／０．８６３ | A |

[Table 2-8]

| | | | |
|---|---|---|---|
| ６６ | 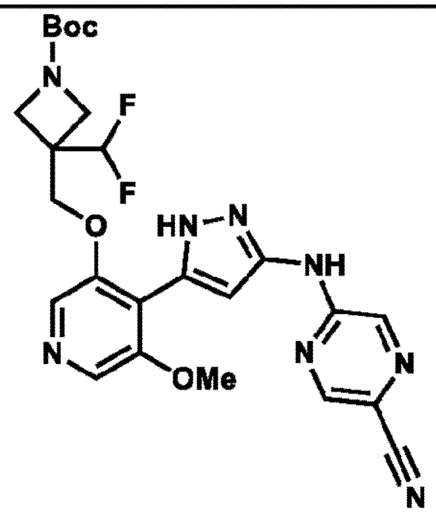 | ５２９．４／０．８８９ | A |
| ６７ | 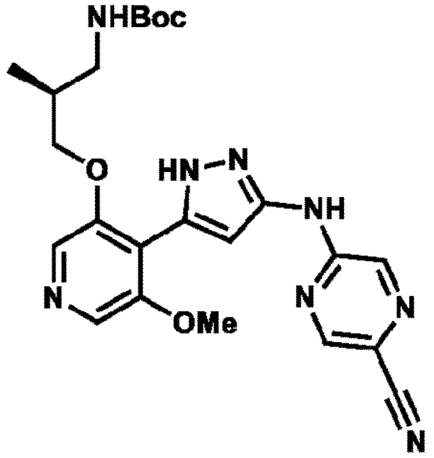 | ４８１．５／０．８６５ | A |
| ６８ | 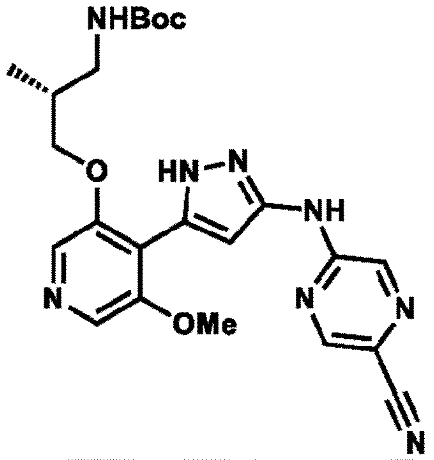 | ４８１．５／０．８５７ | A |

[Table 2-9]

| | | | |
|---|---|---|---|
| ６９ | 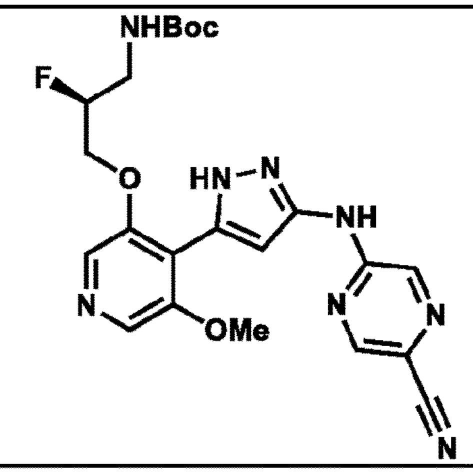 | ４８５．４／０．８３６ | Ａ |
| ７０ | 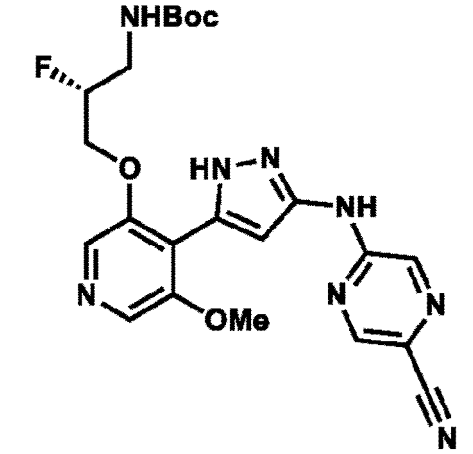 | ４８５．４／０．８１４ | Ａ |
| ７１ | 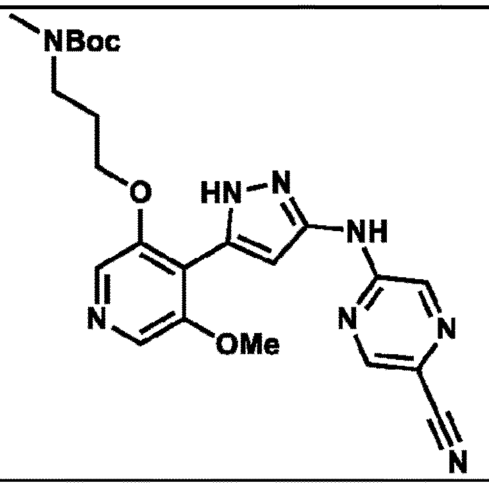 | ４８１．４／０．８４８ | Ａ |

[Table 2-10]

| | | | |
|---|---|---|---|
| ７２ | 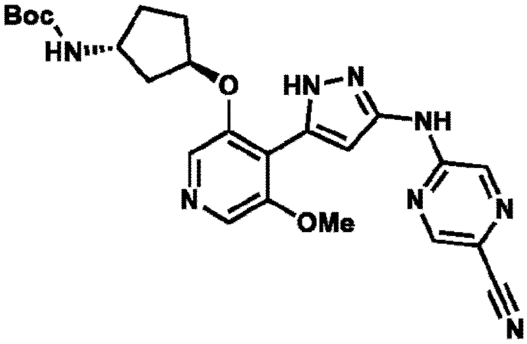 | ４９３．４／０．８５０ | A |
| ７３ | 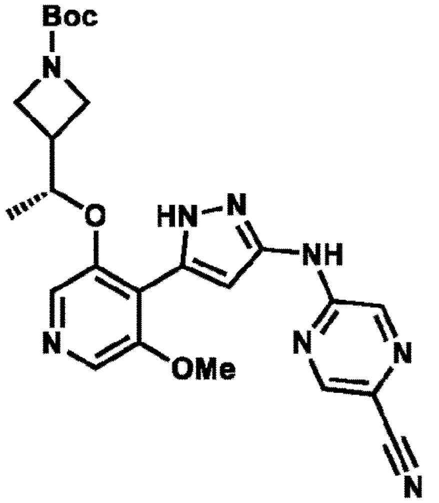 | ４９３．４/０．８５１ | A |
| ７４ | 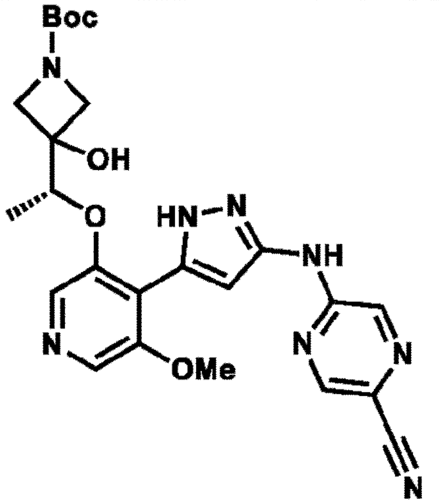 | ５０９．４／０．８０３ | A |

[Table 2-11]

| | | | |
|---|---|---|---|
| ７５ | 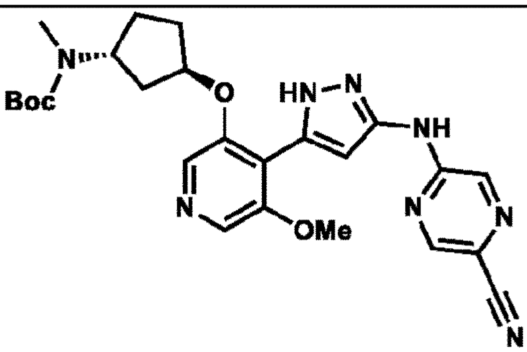 | ５０７．５／０．９２８ | Ａ |
| ７６ | 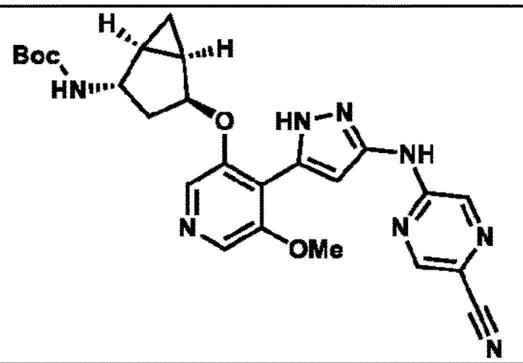 | ５０５．５／０．８９２ | Ａ |
| ７７ | 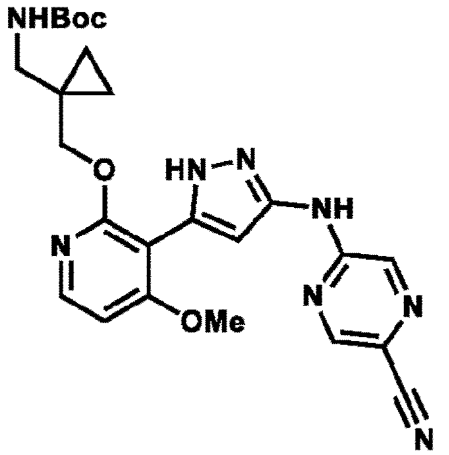 | ４９３．４／０．９５５ | Ａ |

[Table 2-12]

| | | | |
|---|---|---|---|
| 78 | 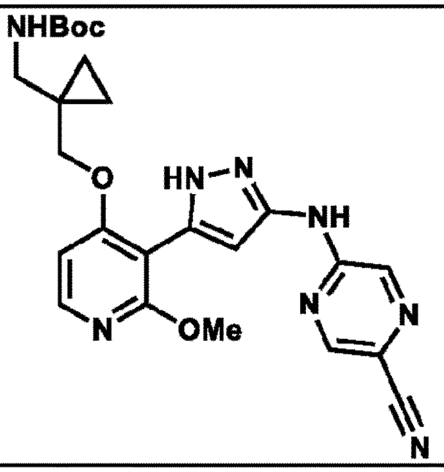 | 493.5/0.943 | A |

Reference Example 79:

tert-butyl {3-[(3-{3-[(5-chloropyrazin-2-yl)amino]-1H-pyrazol-5-yl}-4-methoxypyridin-2-yl)oxy]propyl}carbamate

[Chemical Formula 43]

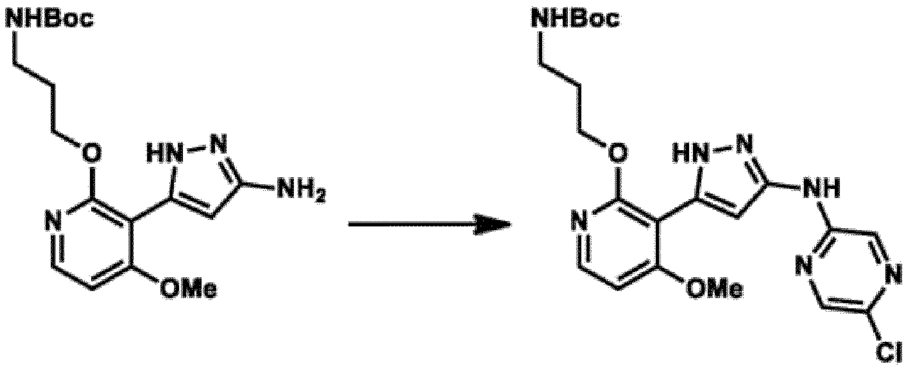

Reference Example 3 Reference Example 79

2,5-dichloropyrazine (32.9 μL), tris(dibenzylideneacetone)dipalladium(0) (15.1 mg), 4,5-bis(diphenylphosphino)-9,9-dimethylxanthene (19.1 mg), and cesium carbonate (108 mg) were added to a 1,4-dioxane (825 μL) solution of tert-butyl(3-{[3-(3-amino-1H-pyrazol-5-yl)-4-methoxypyridin-2-yl]oxy}propyl)carbamate (60 mg) at room temperature. The mixture was stirred for 2 hours at 150°C under microwave irradiation. Water was added to the reaction solution, which was extracted with ethyl acetate. The organic layer was washed with saturated saline, dried with anhydrous sodium sulfate, and then filtered, and the solvent was evaporated under reduced pressure. The residue was purified by silica gel column chromatography (hexane/ethyl acetate) to obtain the title compound.
LC-MS; $[M+H]^+$ 476.3/Rt (minutes) 0.969 (measurement condition A)

Example 1:

5-({5-[2-(3-aminopropoxy)-4-methoxypyridin-3-yl]-1H-pyrazol-3-yl}amino)pyrazine-2-carbonitrile

[Chemical Formula 44]

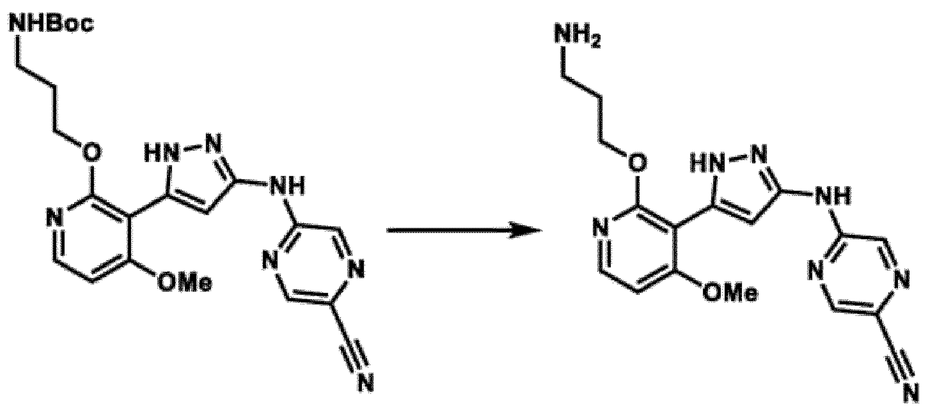

Reference Example 42 Example 1

TFA (2.00 mL) was added to a methylene chloride (20.0 mL) solution of tert-butyl {3-[(3-{3-[(5-cyanopyrazin-2-yl)amino]-1H-pyrazol-5-yl}-4-methoxypyridin-2-yl)oxy]propyl}carbamate (200 mg) obtained in Reference Example 42 at room temperature. The mixture was stirred for 1 hour at room temperature. The solvent was evaporated under reduced pressure. The residue was purified by amine silica gel column chromatography (ethyl acetate/methanol) to obtain Example 1 (112 mg).

LC-MS; $[M+H]^+$ 367.2/Rt (minutes) 0.671 (measurement condition A)
1H-NMR (DMSO-D6) $\delta$: 8.65 (1H, s), 8.50 (1H, brs), 8.06 (1H, d, J = 6.4 Hz), 7.04 (1H, brs), 6.89 (1H, d, J = 6.4 Hz), 4.41 (2H, t, J = 6.0 Hz), 3.92 (3H, s), 2.75 (1H, t, J = 6.8 Hz), 1.85-1.76 (2H, m).

Examples 2 to 38:

The compounds in Examples 2 to 38 shown in the following table were obtained by the similar method to Example 1 by using a corresponding raw material compound.

[Table 3-1]

| Example | Structural formula | LC−MS; [M+H]⁺ Time of retention (minutes) ¹H−NMR |
|---|---|---|
| 2 | 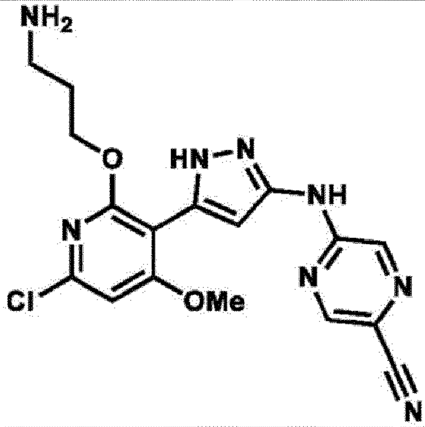 | 401.3/0.636 (Measurement condition A) ¹H-NMR (DMSO-D6) δ: 8.65 (1H, s), 8.48 (1H, brs), 7.05-6.99 (2H, m), 4.38 (2H, t, J = 6.0 Hz), 3.96 (3H, s), 2.75 (1H, t, J = 6.0 Hz), 1.85-1.78 (2H, m). |
| 3 | 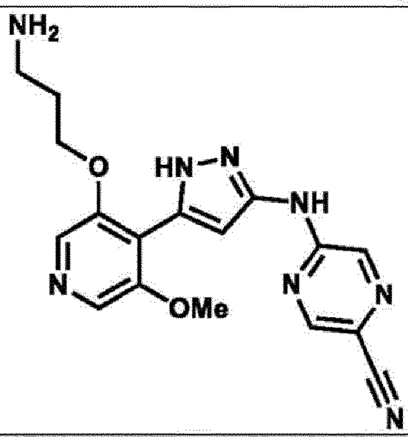 | 367.3/0.673 (Measurement condition A) ¹H-NMR (DMSO-D6) δ: 8.67 (1H, s), 8.48 (1H, brs), 8.19 (2H, d, J = 6.0 Hz), 7.22 (1H, brs), 4.28 (2H, t, J = 6.0 Hz), 3.98 (3H, s), 2.79 (2H, t, J = 6.0 Hz), 1.89-1.85 (2H, m). |
| 4 | 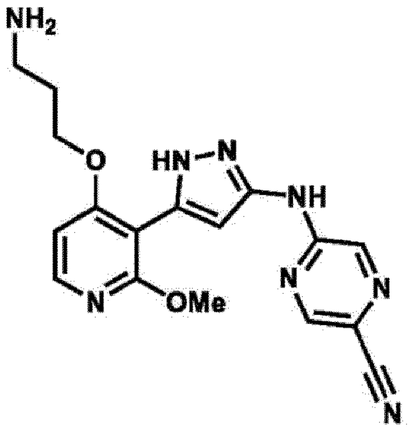 | 367.3/0.610 (Measurement condition A) ¹H-NMR (DMSO-D6) δ: 8.66 (1H, s), 8.49 (1H, brs), 8.05 (1H, d, J = 6.0 Hz), 7.07 (1H, brs), 6.91 (1H, d, J = 5.6 Hz), 4.23 (2H, t, J = 6.0 Hz), 3.93 (3H, s), 3.33 (2H, brs), 2.77 (2H, t, J = 6.0 Hz), 1.88-1.82 (2H, m). |

[Table 3-2]

| | | |
|---|---|---|
| 5 | 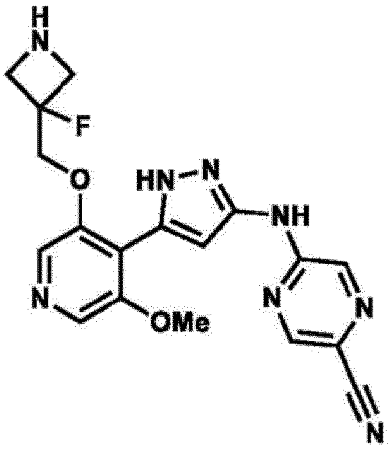 | 397.3/0.528 (Measurement condition A) $^1$H-NMR (DMSO-D6) δ: 13.05 (1H, brs), 10.80 (1H, brs), 8.60 (1H, d, J = 1.2 Hz), 8.47 (1H, brs), 8.27 (1H, d, J = 11.6 Hz), 7.21 (1H, brs), 4.55 (2H, d, J = 18.8 Hz), 4.01 (3H, s), 3.76 (1H, dd, J = 9.6, 20.8 Hz), 3.56 (2H, t, J = 10.4, 12.8 Hz). |
| 6 | 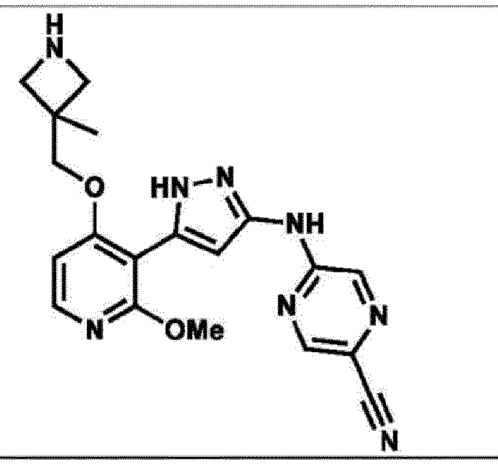 | 393.3/0.568 (Measurement condition A) $^1$H-NMR (DMSO-D6) δ: 8.62 (1H, s), 8.52 (1H, brs), 8.07 (1H, d, J = 6.4 Hz), 7.12 (1H, brs), 6.97 (1H, d, J = 5.6 Hz), 4.17 (2H, s), 3.98 (3H, s), 3.52 (2H, d, J = 8.0 Hz), 3.35 (2H, d, J = 8.0 Hz), 1.23 (3H, s). |

[Table 3-3]

| | | |
|---|---|---|
| 7 | 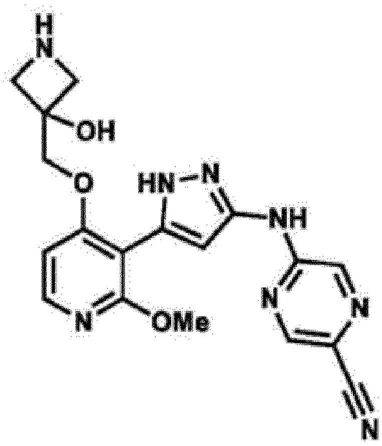 | 395. 3/0. 544<br>(Measurement condition A)<br><br>$^1$H-NMR (DMSO-D6) δ: 14.19 (1H, brs), 10.71 (1H, brs), 8.71 (1H, d, J = 1.2 Hz), 8.60 (1H, brs), 8.14 (1H, d, J = 6.0 Hz), 7.26 (1H, s), 7.07 (1H, d, J = 6.0 Hz), 6.03 (1H, brs), 4.26 (2H, s), 4.05 (3H, s), 3.76 (2H, d, J = 8.8 Hz), 3.48 (2H, d, J = 8.4 Hz), 3.38 (1H, brs). |
| 8 | 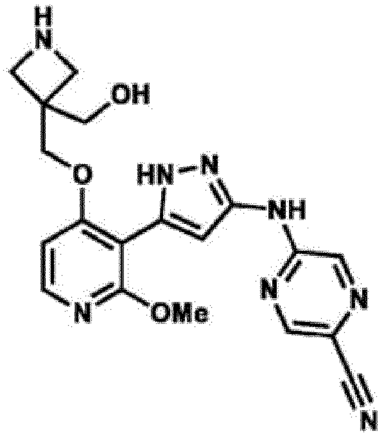 | 409. 3/0. 545<br>(Measurement condition A)<br><br>$^1$H-NMR (DMSO-D6) δ: 13.89 (1H, brs), 8.63 (1H, d, J = 1.2 Hz), 8.51 (1H, brs), 8.07 (1H, d, J = 6.0 Hz), 7.13 (1H, brs), 6.98 (1H, d, J = 6.0 Hz), 4.93 (1H, brs), 4.28 (2H, s), 3.98 (3H, s), 3.61-3.56 (4H, m), 3.31-3.16 (2H, m). |
| 9 | 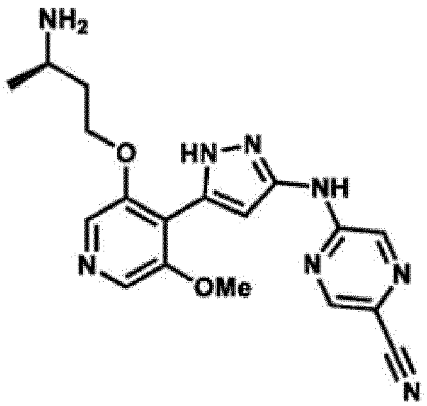 | 381. 3/0. 497<br>(Measurement condition A)<br><br>$^1$H-NMR (DMSO-D6) δ: 8.65 (1H, d, J = 1.2 Hz), 8.49 (1H, brs), 8.20 (2H, d, J = 9.6 Hz), 7.19 (1H, brs), 4.34-4.22 (2H, m), 3.98 (3H, s), 3.06-3.01 (1H, m), 1.84-1.71 (2H, m), 1.03 (3H, d, J = 6.8 Hz). |

[Table 3-4]

| | | |
|---|---|---|
| 10 | 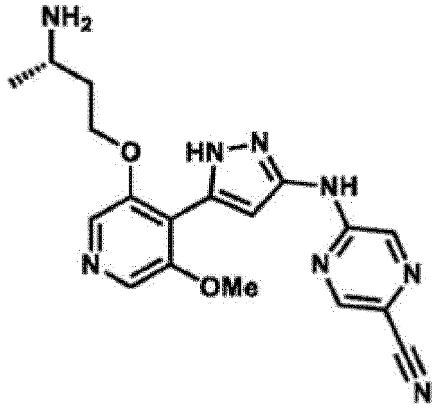 | 381.3/0.546 (Measurement condition A) $^1$H-NMR (DMSO-D6) δ: 8.55 (1H, d, J = 2.0 Hz), 8.39 (1H, brs), 8.10 (2H, d, J = 10.0 Hz), 7.09 (1H, brs), 4.24-4.13 (2H, m), 3.88 (3H, s), 2.96-2.92 (1H, m), 1.88-1.61 (2H, m), 0.94 (3H, d, J = 6.0 Hz) |
| 11 | 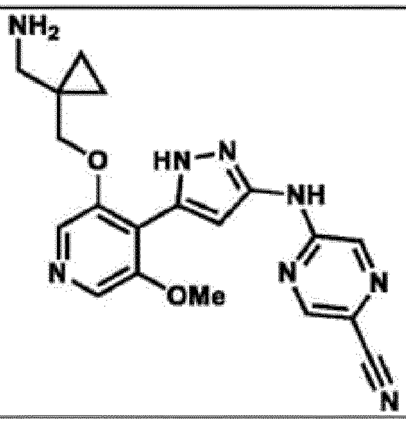 | 393.3/0.594 (Measurement condition A) $^1$H-NMR (DMSO-D6) δ: 8.63 (1H, d, J = 1.2 Hz), 8.49 (1H, brs), 8.17 (2H, d, J = 11.6 Hz), 7.31 (1H, s), 4.13 (2H, s), 3.99 (3H, s), 2.72 (2H, s), 0.57-0.50 (4H, m) |
| 12 | 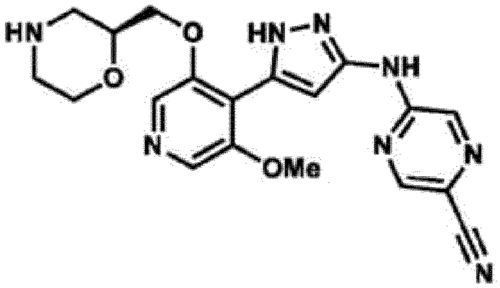 | 409.3/0.561 (Measurement condition A) $^1$H-NMR (DMSO-D6) δ: 12.58 (1H, s), 10.82 (1H, s), 8.71 (1H, d, J = 1.2 Hz), 8.47 (1H, brs), 8.20 (2H, d, J = 3.6 Hz), 7.24 (1H, brs), 4.18 (2H, d, J = 4.8 Hz), 3.99 (3H, s), 3.78-3.75 (2H, m), 3.52-3.46 (1H, m), 2.96-2.93 (1H, m), 2.70-2.56 (4H, m). |

[Table 3-5]

| | | |
|---|---|---|
| 1 3 | 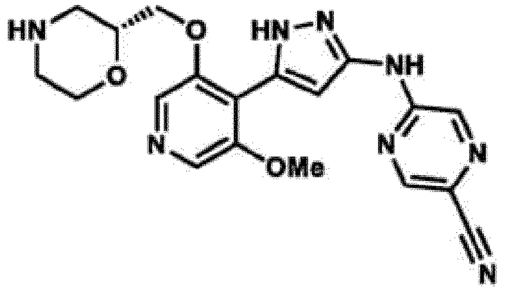 | 4 0 9. 3／0. 4 9 7<br>**(Measurement condition A)**<br><br>$^1$H-NMR (DMSO-D6) δ : 12.58 (1H, s), 10.82 (1H, s), 8.71 (1H, d, J = 1.2 Hz), 8.46 (1H, brs), 8.20 (2 H, d, J = 3.7 Hz), 7.23 (1H, brs), 4.18 (2H, d, J = 4.9 Hz), 3.99 (3H, s), 3.83-3.76 (2H, m), 3.50 (1H, td, J = 10.8, 2.8 Hz), 2.95 (1H, dd, J = 12.2, 1.8 Hz), 2.70-2.56 (3H, m). |
| 1 4 | 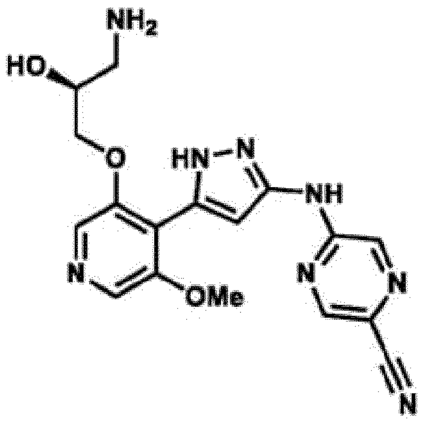 | 3 8 3. 2／0. 4 5 2<br>**(Measurement condition A)**<br><br>$^1$H-NMR (DMSO-D6) δ : 8.74 (1H, d, J = 1.2 Hz), 8.56 (1H, brs), 8.29 (1H, s), 8.27 (1H, s), 7.30 (1H, brs), 4.30 (1H, dd, Jgem = 9.5, J = 4.6 Hz), 4.22 (1H, dd, Jgem = 9.5, J = 5.8 Hz), 4.05 (3H, s), 3.92-3.87 (1H, m), 2.82 (1H, dd, Jgem = 12.8, J = 5.5 Hz), 2.76 (1H, dd, Jgem = 12.8, J = 6.1 Hz). |

[Table 3-6]

| | | |
|---|---|---|
| 15 | 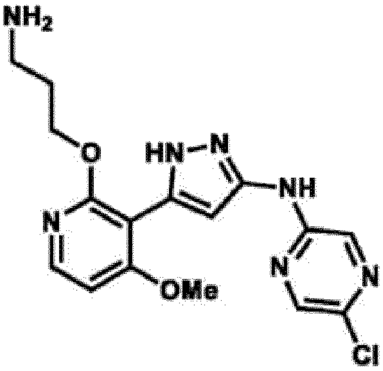 | 376/0.578 (Measurement condition A) 1H-NMR (DMSO-D6) δ: 9.99 (1H, s), 8.51 (1H, d, J = 1.2 Hz), 8.31 (1H, d, J = 1.2 Hz), 8.12 (1H, d, J = 6.1 Hz), 6.95 (1H, d, J = 6.1 Hz), 6.91 (1H, s), 4.47 (2H, t, J = 6.1 Hz), 3.99 (3H, s), 2.81 (2H, t, J = 6.7 Hz), 1.92-1.85 (2H, m). |
| 16 | 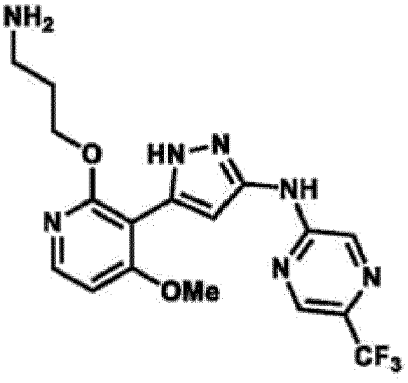 | 410/0.637 (Measurement condition A) 1H-NMR (CD3OD) δ: 8.58 (1H, s), 8.55 (1H, s), 8.13 (1H, d, J = 5.5 Hz), 7.05 (1H, s), 6.94 (1H, d, J = 6.1 Hz), 4.57 (2H, t, J = 6.1 Hz), 4.07 (3H, s), 2.91 (2H, t, J = 6.7 Hz), 2.05 (2H, td, J = 9.6, 5.5 Hz). |
| 17 | 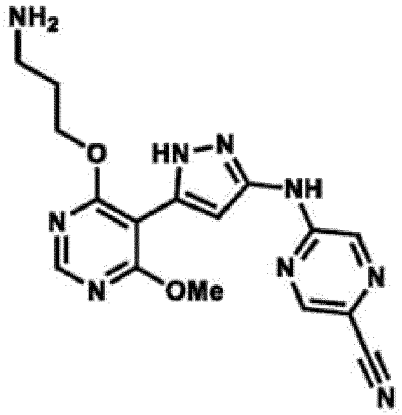 | 368.2/0.558 (Measurement condition A) 1H-NMR (DMSO-D6, 65° C) δ: 8.61 (1H, s), 8.53 (1H, s), 8.44 (1H, s), 7.06 (1H, s), 4.55 (2H, t, J = 6.1 Hz), 4.05 (3H, s), 2.78 (2H, t, J = 6.4 Hz), 1.90-1.84 (2H, m). |

[Table 3-7]

| | | |
|---|---|---|
| １８ | 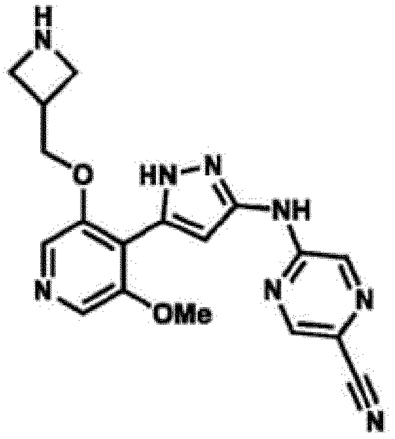 | ３７９．２／０．５２５ (Measurement condition A) $^1$H-NMR (DMSO-D6) δ: 14.25 (1H, brs), 8.64 (1H, d, J = 1.2 Hz), 8.50 (1H, brs), 8.24 (1H, s), 8.22 (1H, s), 7.29 (1H, brs), 4.39 (2H, d, J = 3.7 Hz), 4.02 (3H, s), 3.85 (2H, t, J = 7.9 Hz), 3.29 (2H, dd, J = 7.6, 4.0 Hz), 2.98-2.92 (1H, m). |
| １９ | 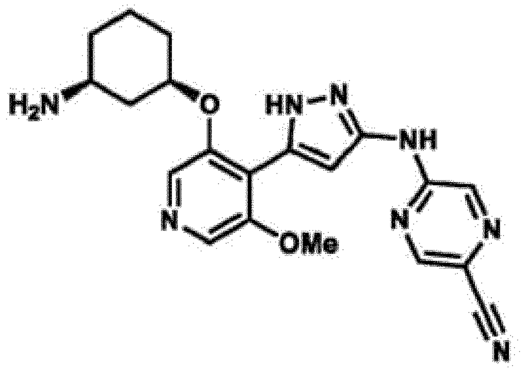 | ４０７．３／０．５９７ (Measurement condition A) $^1$H-NMR (DMSO-D6) δ: 8.65 (1H, s), 8.51 (1H, brs), 8.23 (1H, s), 8.15 (1H, s), 7.22 (1H, brs), 4.59 (1H, m), 3.97 (3H, s), 2.76 (1H, m), 2.16 (1H, d, J = 11.6 Hz), 1.99 (1H, d, J = 10.4 Hz), 1.79-1.67 (2H, m), 1.41-1.23 (3H, m), 1.11-1.03 (1H, m). |
| ２０ | 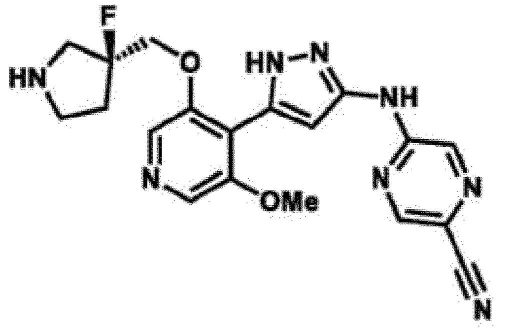 | ４１１．３／０．５６２ (Measurement condition A) $^1$H-NMR (DMSO-D6) δ: 12.61 (1H, brs), 10.85 (1H, brs), 8.58 (1H, d, J = 1.2 Hz), 8.46 (1H, brs), 8.23 (1H, s), 8.22 (1H, s), 7.27 (1H, brs), 4.54-4.38 (2H, m), 4.00 (3H, s), 3.24-3.15 (1H, m), 3.03-2.93 (2H, m), 2.85-2.79 (1H, m), 2.12-1.87 (2H, m). |

[Table 3-8]

| | | |
|---|---|---|
| 21 | 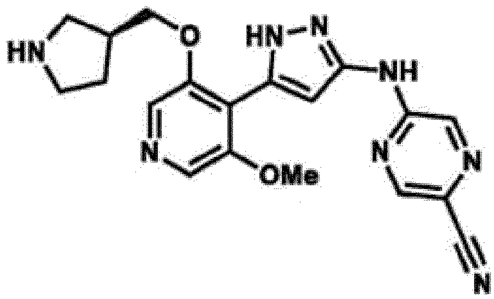 | 393.3/0.603 (Measurement condition A) $^1$H-NMR (DMSO-D6) δ: 13.17 (1H, brs), 8.63 (1H, d, J = 1.8 Hz), 8.47 (1H, brs), 8.19 (1H, s), 8.18 (1H, s), 7.26 (1H, brs), 4.20 (1H, dd, J = 9.2, 5.5 Hz), 4.09 (1H, dd, J = 8.9, 6.4 Hz), 3.99 (3H, s), 2.98 (1H, dd, J = 10.4, 7.3 Hz), 2.87 (1H, m), 2.76-2.70 (2H, m), 2.53 (1H, m), 1.92 (1H, m), 1.48 (1H, m). |
| 22 | 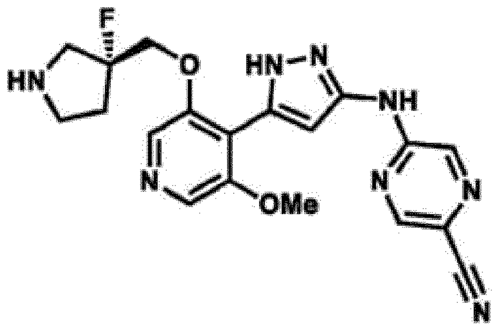 | 411.3/0.559 (Measurement condition A) $^1$H-NMR (DMSO-D6) δ: 12.61 (1H, brs), 10.85 (1H, brs), 8.57 (1H, d, J = 1.2 Hz), 8.45 (1H, brs), 8.23 (1H, s), 8.22 (1H, s), 7.27 (1H, brs), 4.54-4.38 (2H, m), 4.00 (3H, s), 3.24-3.15 (1H, m), 3.03-2.93 (2H, m), 2.85-2.79 (1H, m), 2.12-1.87 (2H, m). |

[Table 3-9]

| | | |
|---|---|---|
| 23 | 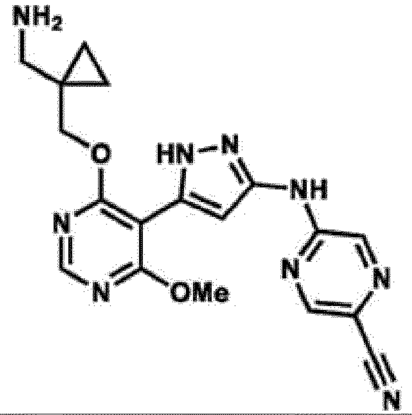 | 394.3/0.594<br>(Measurement condition A)<br><br>$^1$H-NMR (DMSO-D6) δ: 8.64 (1H, s), 8.48 (1H, brs), 8.43 (1H, s), 7.24 (1H, brs), 4.40 (2H, s), 4.05 (3H, s), 2.72 (2H, s), 0.56 (4H, m) |
| 24 | 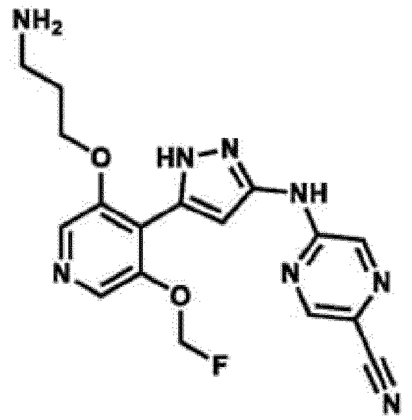 | 385/0.570<br>(Measurement condition A)<br><br>1H-NMR (DMSO-D6) δ: 8.68 (1H, d, J = 1.2 Hz), 8.48 (1H, s), 8.33 (1H, s), 8.27 (1H, s), 7.18 (1H, br s), 5.99 (2H, d, J = 53.2 Hz), 4.30 (2H, t, J = 5.8 Hz), 2.83 (2H, t, J = 6.4 Hz), 1.94-1.88 (2H, m). |
| 25 | 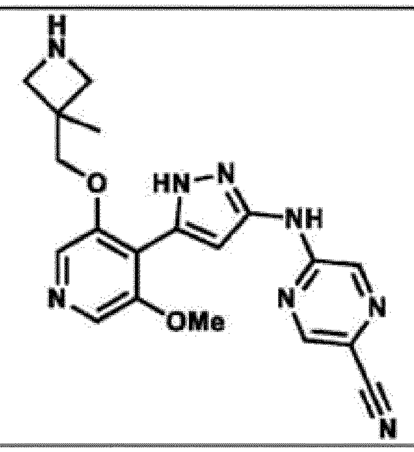 | 393/0.573<br>(Measurement condition A)<br><br>1H-NMR (DMSO-D6) δ: 8.59 (1H, d, J = 1.2 Hz), 8.47 (1H, br s), 8.21 (1H, s), 8.19 (1H, s), 7.27 (1H, br s), 4.18 (2H, s), 3.99 (3H, s), 3.51 (2H, d, J = 7.9 Hz), 3.35 (2H, d, J = 7.9 Hz), 1.20 (3H, s). |

[Table 3-10]

| | | |
|---|---|---|
| 26 | 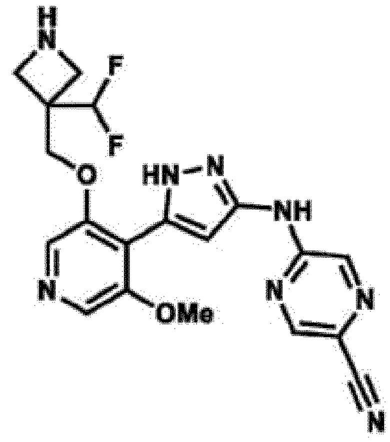 | 429／0.567<br>(Measurement condition A)<br><br>1H-NMR (DMSO-D6) δ: 10.78 (1H, br s), 8.61 (1H, d, J = 1.2 Hz), 8.49 (1H, br s), 8.30 (1H, s), 8.26 (1H, s), 7.21 (1H, s), 6.27 (1H, t, J = 56.4 Hz), 4.48 (2H, s), 4.02 (3H, s), 3.80 (2H, d, J = 8.5 Hz), 3.35 (2H, d, J = 8.5 Hz). |
| 27 | 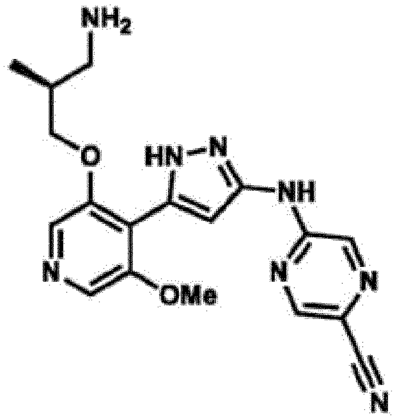 | 381／0.622<br>(Measurement condition A)<br><br>1H-NMR (DMSO-D6) δ: 8.63 (1H, d, J = 1.2 Hz), 8.46 (1H, br s), 8.21 (1H, s), 8.19 (1H, s), 7.26 (1H, br s), 4.19-4.07 (2H, m), 3.98 (3H, s), 2.67 (2H, d, J = 6.1 Hz), 2.02-1.90 (1H, m), 1.01 (3H, d, J = 6.1 Hz). |
| 28 | 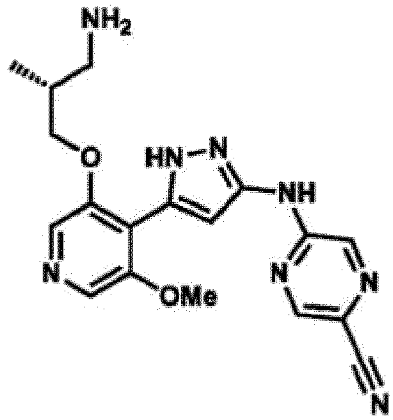 | 381／0.573<br>(Measurement condition A)<br><br>1H-NMR (DMSO-D6) δ: 8.63 (1H, s), 8.46 (1H, br s), 8.20 (1H, s), 8.19 (1H, s), 7.26 (1H, br s), 4.18-4.07 (2H, m), 3.98 (3H, s), 2.66 (2H, d, J = 6.1 Hz), 2.01-1.91 (1H, m), 1.01 (3H, d, J = 7.3 Hz). |

[Table 3-11]

| | | |
|---|---|---|
| 29 | 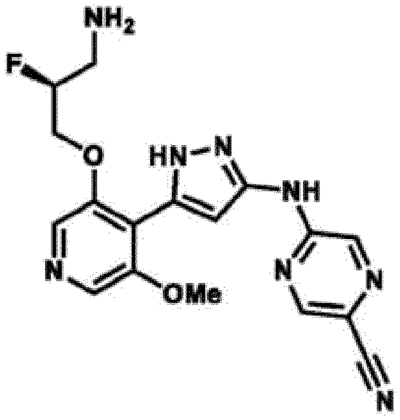 | 385/0.543<br>(Measurement condition A)<br><br>1H-NMR (DMSO-D6) δ: 10.81 (1H, br s), 8.65 (1H, d, J = 1.2 Hz), 8.48 (1H, br s), 8.22 (2H, s), 7.22 (1H, br s), 4.92-4.72 (1H, m), 4.54-4.33 (2H, m), 3.99 (3H, s), 2.95 (1H, d, J = 6.1 Hz), 2.90 (1H, dd, J = 5.8, 1.5 Hz). |
| 30 | 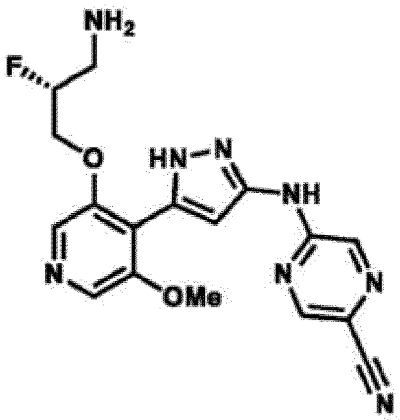 | 385/0.548<br>(Measurement condition A)<br><br>1H-NMR (DMSO-D6) δ: 8.65 (1H, d, J = 1.2 Hz), 8.48 (1H, br s), 8.22 (2H, s), 7.22 (1H, br s), 4.93-4.71 (1H, m), 4.53-4.33 (2H, m), 3.99 (3H, s), 2.95 (1H, d, J = 6.1 Hz), 2.90 (1H, dd, J = 5.8, 1.5 Hz). |
| 31 | 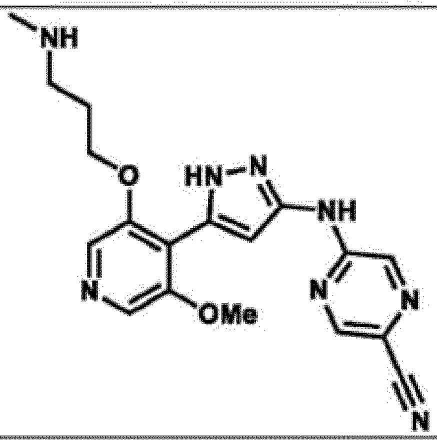 | 381/0.527<br>(Measurement condition A)<br><br>1H-NMR (DMSO-D6) δ: 8.66 (1H, d, J = 1.2 Hz), 8.50 (1H, br s), 8.19 (2H, s), 7.24 (1H, br s), 4.28 (2H, t, J = 5.8 Hz), 3.99 (3H, s), 2.69 (2H, t, J = 6.1 Hz), 2.31 (3H, s), 1.98-1.89 (2H, m). |

[Table 3-12]

| | | |
|---|---|---|
| 32 | 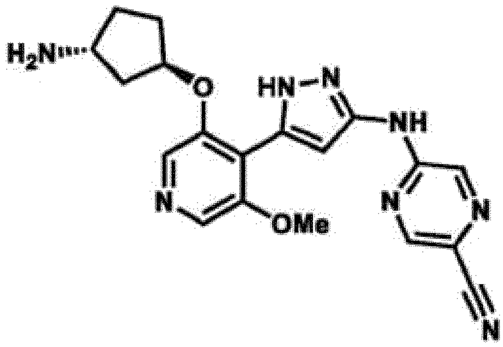 | 393／0．576 (Measurement condition A) 1H-NMR (DMSO-D6) δ: 12.52 (1H, br s), 8.66 (1H, d, J = 1.2 Hz), 8.47 (1H, br s), 8.15 (1H, s), 8.13 (1H, s), 7.24 (1H, br s), 5.15-5.07 (1H, m), 3.98 (3H, s), 3.56-3.47 (1H, m), 2.30-2.18 (1H, m), 2.08-1.90 (2H, m), 1.78-1.64 (2H, m), 1.38-1.26 (1H, m). |
| 33 | 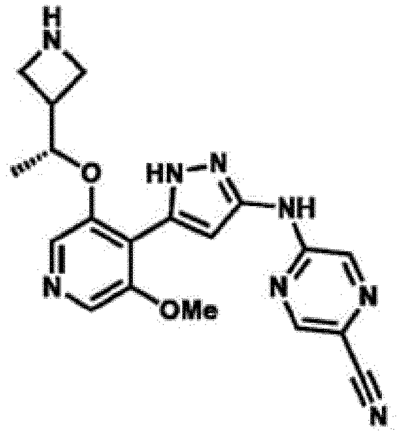 | 393／0．608 (Measurement condition A) 1H-NMR (DMSO-D6) δ: 8.65 (1H, d, J = 1.2 Hz), 8.51 (1H, br s), 8.25 (1H, s), 8.18 (1H, s), 7.18 (1H, br s), 5.02-4.92 (1H, m), 3.99 (3H, s), 3.83-3.77 (2H, m), 3.39-3.35 (1H, m), 3.20-3.14 (1H, m), 2.82-2.72 (1H, m), 1.23 (3H, d, J = 6.1 Hz). |

[Table 3-13]

| | | |
|---|---|---|
| 34 | 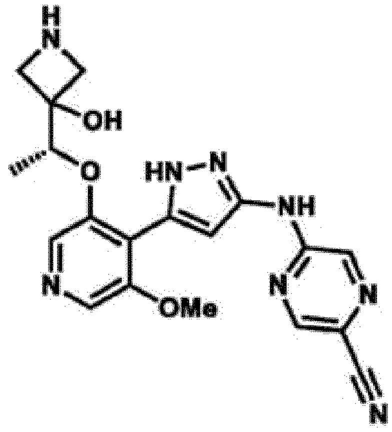 | 409/0.626 (Measurement condition A) 1H-NMR (DMSO-D6) δ: 10.65 (1H, br s), 8.65 (1H, d, J = 1.2 Hz), 8.52 (1H, br s), 8.26 (1H, s), 8.19 (1H, s), 7.20 (1H, br s), 5.98 (1H, br s), 4.80 (1H, d, J = 6.1 Hz), 4.77 (1H, d, J = 6.1 Hz), 4.00 (3H, s), 3.69 (1H, d, J = 7.9 Hz), 3.65 (1H, d, J = 7.9 Hz), 3.52 (1H, d, J = 8.5 Hz), 3.35 (1H, d, J = 8.5 Hz), 1.26 (3H, d, J = 6.1 Hz). |
| 35 | 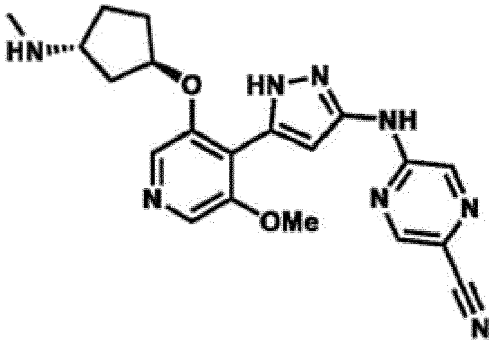 | 407/0.642 (Measurement condition A) 1H-NMR (DMSO-D6) δ: 12.52 (1H, br s), 8.63 (1H, s), 8.48 (1H, br s), 8.15 (1H, s), 8.14 (1H, s), 7.23 (1H, br s), 5.12-5.05 (1H, m), 3.97 (3H, s), 3.19-3.11 (1H, m), 2.20 (3H, s), 2.19-2.09 (1H, m), 2.08-1.99 (1H, m), 1.99-1.89 (1H, m), 1.82-1.71 (2H, m), 1.47-1.36 (1H, m). |

[Table 3-14]

| | | |
|---|---|---|
| 36 | 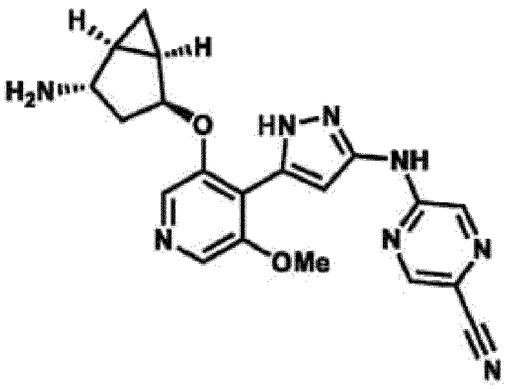 | 405/0.584<br>(Measurement condition A)<br><br>1H-NMR (DMSO-D6) δ: 12.50 (1H, br s), 8.61 (1H, s), 8.50 (1H, br s), 8.28 (1H, s), 8.16 (1H, s), 7.26 (1H, br s), 5.52-5.43 (1H, m), 3.98 (3H, s), 3.35-3.34 (1H, m), 3.33-3.32 (1H, m), 1.87 (1H, dd, J = 13.4, 7.9 Hz), 1.82-1.74 (1H, m), 1.61-1.51 (1H, m), 1.41-1.31 (1H, m), 0.62-0.56 (1H, m), 0.52-0.45 (1H, m). |
| 37 | 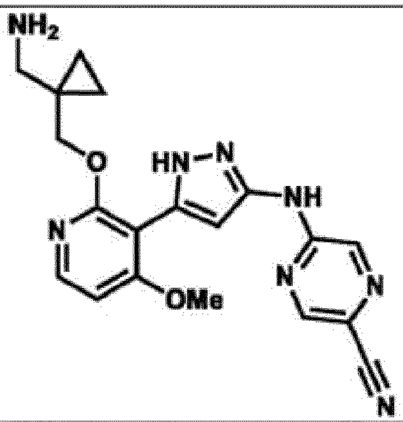 | 393/0.654<br>(Measurement condition A)<br><br>1H-NMR (DMSO-D6) δ: 8.64 (1H, s), 8.50 (1H, br s), 8.04 (1H, d, J = 5.5 Hz), 7.14 (1H, br s), 6.90 (1H, d, J = 6.1 Hz), 4.27 (2H, s), 3.94 (3H, s), 2.80 (2H, s), 0.63-0.51 (4H, m). |
| 38 | 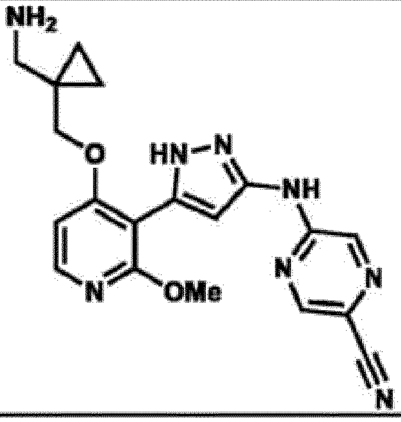 | 393/0.682<br>(Measurement condition A)<br><br>1H-NMR (DMSO-D6) δ: 8.62 (1H, d, J = 1.2 Hz), 8.49 (1H, br s), 8.03 (1H, d, J = 5.5 Hz), 7.18 (1H, br s), 6.87 (1H, d, J = 6.1 Hz), 4.09 (2H, s), 3.95 (3H, s), 2.70 (2H, s), 0.58-0.47 (4H, m). |

X ray powder diffraction in Examples 39 to 44 was measured under the conditions set forth below. The resulting diffraction patterns (XRD spectrum) are shown in Figures 1 to 6. Examples 39 to 41 are crystals of various salts of the compound of Example 1, and Examples 42 to 44 are crystalline polymorphisms of the compound of Example 1.

A crystalline form can be identified with a determination based on a characteristic diffraction peak of each crystal shown in the diffraction diagrams of Figures 1 to 6.

The primary diffraction peak and characteristic diffraction peak identified from the diffraction patterns in Figures **1** to **6** are each listed below. The diffraction peak value in diffraction angle 2θ(°) described in the following Examples can have a slight measurement error depending on the measuring equipment, measurement condition, or the like. Specifically, the

measurement error can be within a range of ± 0.2, preferably ± 0.1.

X-ray powder diffraction measurement method:

Detector: Spectris Power X-ray diffraction system Empyrian
X-ray tube: CuKα (wavelength: 1.54 angstroms)
Tube voltage: 45kV
Tube current: 40 mA
Measurement range: 4 to 40° (2θ)
Step width: 0.013 degrees
Integration time: 100 seconds/step

Example 39:

5-({5-[2-(3-aminopropoxy)-4-methoxypyridin-3-yl]-1H-pyrazol-3-yl}amino)pyrazine-2-carbonitrile hydrochloride

Methanol (0.6 mL) was added to the compound (30.0 mg) of Example 1, and a 5 to 10% (W/V) hydrochlorica cid-methanol solution (60 μL) was added. The mixture was stirred for 2 hours at a set temperature of 60°C. After allowing the mixture to cool, the mixture was left standing overnight. The deposited solid was filtered out and dried to obtain the title compound as a crystal (form I).

[Form I] The X ray powder diffraction pattern is shown in Figure 1.
Primary diffraction peaks: 2θ(°) = 7.2, 8.8, 9.8, 10.2, 10.7, 16.7, 18.5, 26.2, 27.0
Characteristic diffraction peaks: 2θ(°) = 7.2, 8.8, 9.8, 10.2, 10.7

Example 40:

5-({5-[2-(3-aminopropoxy)-4-methoxypyridin-3-yl]-1H-pyrazol-3-yl}amino)pyrazine-2-carbonitrile phosphate

13.4 mg/mL phosphoric acid-methanol solution (1200 μL) was added to the compound (30.0 mg) of Example 1. The mixture was stirred for 4 hours at a set temperature of 60°C. After allowing the mixture to cool, the mixture was left standing overnight. The deposited solid was filtered out and dried to obtain the title compound as a crystal (form II).

[Form II] The X ray powder diffraction pattern is shown in Figure 2.
Primary diffraction peaks: 2θ(°) = 6.8, 7.5, 11.7, 11.9, 13.0, 16.4, 19.3, 20.4, 22.7, 24.3
Characteristic diffraction peaks: 2θ(°) = 6.8, 7.5, 11.7, 11.9, 13.0

Example 41:

5-({5-[2-(3-aminopropoxy)-4-methoxypyridin-3-yl]-1H-pyrazol-3-yl}amino)pyrazine-2-carbonitrile tosylate

Methanol (0.6 mL) was added to the compound (30.0 mg) of Example 1, and 26.0 mg/mL tosic acid-methanol solution (0.6 mL) was further added. The mixture was stirred for 2 hours at a set temperature of 60°C. After allowing the mixture to cool, the mixture was left standing overnight. The deposited solid was filtered out and dried to obtain the title compound as a crystal (form III).

[Form III] The X ray powder diffraction pattern is shown in Figure 3.
Primary diffraction peaks: 2θ(°) = 6.0, 9.0, 12.1, 14.4, 16.2, 17.0, 22.8, 26.3
Characteristic diffraction peaks: 2θ(°) = 6.0, 9.0, 12.1, 14.4, 16.2, 17.0

Example 42:

5-({5-[2-(3-aminopropoxy)-4-methoxypyridin-3-yl]-1H-pyrazol-3-yl}amino)pyrazine-2-carbonitrile

[Form IV] The X ray powder diffraction pattern is shown in Figure 4.
From Example 1, the title compound was obtained as a crystal (form IV).
Primary diffraction peaks: 2θ(°) = 9.3, 10.2, 10.7, 13.6, 16.7, 17.1, 17.8, 18.6, 26.1, 26.4
Characteristic diffraction peaks: 2θ(°) = 9.3, 10.2, 10.7, 16.7, 26.1, 26.4

Example 43:

5-({5-[2-(3-aminopropoxy)-4-methoxypyridin-3-yl]-1H-pyrazol-3-yl}amino)pyrazine-2-carbonitrile

[Form V] The X ray powder diffraction pattern is shown in Figure 5.
A tetrahydrofuran/water (10/1, 0.5 mL) solution of the compound (5 mg) of Example 1 was added and heated for 1 hour at a set temperature of 105°C. After allowing the mixture to cool, the mixture was sealed and left standing for 4 days, and then opened and left standing for 3 days. The solvent was evaporated. From the deposited solid, the title compound was obtained as a crystal (form V).
Primary diffraction peaks: 2θ(°) = 7.9, 8.7, 12.2, 13.1, 15.9, 17.6, 19.9, 21.9, 22.8, 26.6
Characteristic diffraction peaks: 2θ(°) = 7.9, 8.7, 12.2, 13.1, 15.9, 26.6

Example 44:

5-({5-[2-(3-aminopropoxy)-4-methoxypyridin-3-yl]-1H-pyrazol-3-yl}amino)pyrazine-2-carbonitrile

[Form VI] The X ray powder diffraction pattern is shown in Figure 6.
An acetone/water (10/1, 0.5 mL) solution of the compound (5 mg) of Example 1 was added. The mixture was heated for 1 hour at a set temperature of 105°C. After allowing the mixture to cool, the mixture was sealed and left standing for 4 days, and then opened and left standing for 3 days. The solvent was evaporated. From the deposited solid, the title compound was obtained as a crystal (form VI).
Primary diffraction peaks: 2θ(°) = 5.3, 5.7, 7.0, 7.3, 7.8, 8.4, 9.3, 10.5, 11.5, 14.1
Characteristic diffraction peaks: 2θ(°) = 5.3, 5.7, 7.0, 7.3, 8.4, 9.3

Test Example

Test Example 1: CHK1 inhibitory activity test

IMAP TR-FRET Screening Express Kit (R8160) was obtained from Molecular Devices. CHK1 kinase (02-117, Carna Bio), FAM-labeled CHK1tide (R7185, Molecular Devices), and ATP were diluted with an assay buffer so that the final concentration would be 4 μg/mL, 2 μM, and 20 μM, respectively. FAM-PKAtide (R7255, Molecular Devices) and FAM-Phospho-PKAtide (R7304, Molecular Devices) were admixed after dilution, and calibration standard systems for phosphorylation levels from 0 to 100% were created. 5 μL of a compound dissolved in a 0.4% DMSO solution was added to a 384 well plate. A compound study group to which 5 μL of each of CHK1, CHK1tide, and ATP was added, and a standard group to which 20 μL of standard was added were created, and subjected to a kinase reaction for 3 hours at 30°C. Subsequently, 60 μL of Binding Reagent (80% Buffer A, 20% Buffer B, 1:600 Binding Reagent, 1:400 Tb-Donor) was added for a binding reaction for 2 hours at room temperature. SpectraMax Paradigm (Molecular Devices) was used to obtain fluorescence intensity of 520 nm or 490 nm as of 340 nm excitation. The standard was used to compute the phosphorylation level of CHK1tide, and the kinase activity was found by using the equation described below while assuming the phosphorylation level of the DMSO treated group as 100%, and the $IC_{50}$ value corresponding to the concentration of the evaluated compound indicating kinase activity of 50% was calculated.

$$\text{Kinase inhibition (\%)} = 100 - A/B \times 100$$

A: signal in the presence of evaluated compound
B: signal in negative control (DMSO treated group)

The test shown in Test Example 1 was conducted on the compound obtained in the Examples and prexasertib

purchased from MedChemExpress (the supplier is the same in the following Test Examples). The $IC_{50}$ values (nM) for the test results for each tested substance are shown in the following table.

[Table 4-1]

| Example | $IC_{50}$(nM) | CHK1 inhibition % at 0.1 nM |
|---|---|---|
| Prexasertib | 0.54 | 11 |
| 1 | <0.1 | 55 |
| 2 | 0.22 | 31 |
| 3 | 0.22 | 28 |
| 4 | 0.23 | 25 |
| 5 | 0.33 | 15 |
| 6 | 0.27 | 15 |
| 7 | 0.21 | 29 |
| 8 | <0.1 | 51 |
| 9 | 0.24 | 24 |
| 10 | 0.30 | 20 |
| 11 | 0.18 | 35 |
| 12 | 0.34 | 21 |
| 13 | 0.74 | 6 |
| 14 | 0.22 | 30 |
| 15 | 0.26 | 29 |
| 16 | 3.04 | <5 |
| 17 | 0.30 | 24 |
| 18 | 0.51 | <5 |
| 19 | 0.26 | 20 |
| 20 | 0.34 | 6 |
| 21 | 0.29 | 15 |
| 22 | 0.24 | 22 |
| 23 | 0.79 | <5 |
| 24 | 0.30 | 23 |
| 25 | 0.74 | <5 |
| 26 | 0.14 | 42 |
| 27 | 3.46 | <5 |
| 28 | 0.50 | <5 |
| 29 | 0.60 | <5 |
| 30 | 0.26 | 28 |
| 31 | 0.75 | <5 |
| 32 | 0.76 | <5 |

[Table 4-2]

| | | |
|---|---|---|
| 33 | 0.69 | <5 |
| 34 | 1.82 | <5 |

(continued)

| | | |
|---|---|---|
| 35 | 0.62 | <5 |
| 36 | 0.73 | <5 |
| 37 | 0.32 | 18 |
| 38 | 0.70 | <5 |

As shown in the above table, the compounds of Examples 1, 2, 3, 4, 5, 6, 7, 8, 9, 10, 11, 12, 14, 15, 17, 18, 19, 20, 21, 22, 24, 26, 28, 30, and 37 exhibited a more potent effect of suppressing CHK1 activity than prexasertib. In particular, the compounds of Examples 1 and 8 have a significant effect of especially potently suppressing CHK1 activity.

Test Example 2: Cell growth suppression experiment

ES-2 cells were obtained from the American Type Culture Collection (ATCC). These cells were cultured at 37°C in the presence of 5%$CO_2$ in a 10% fetal bovine serum, 1% penicillin/streptomycin containing McCoy's 5a medium.

500 cells were seeded for each well in a 384-well plate, and the evaluated compound was added so that the final concentration of DMSO would be 0.1% to culture the cells for 2 days. After completion of the culture, the cell viability rate was calculated using CellTiter-Glo® 3D Reagent (Promega, G968B). The $IC_{50}$ values corresponding to the concentration of the evaluated compound indicating cell growth suppression ratio of 50% was calculated from the viability rate curve. ES-2 cells are known to exhibit cisplatin resistance.

The test shown in Test Example 2 was conducted on the compounds obtained in Examples and prexasertib. The concentrations at which 50% of cell growth is suppressed of each test compound ($IC_{50}$ value; nM) are shown in the following table. The results of cell growth suppression test on representative compounds of the present disclosure are listed in the following table based on the ranking of I, II, III, IV, and V (1000 nM < I, 1000 nM > II > 300 nM, 300 nM > III > 100 nM, 100 nM > IV > 30 nM, 30 nM > V).

[Table 5]

| Examples | $IC_{50}$ (nM) | Examples | $IC_{50}$ (nM) |
|---|---|---|---|
| Prexasertib | V | 20 | V |
| 1 | V | 21 | V |
| 2 | IV | 22 | V |
| 3 | V | 23 | V |
| 4 | V | 24 | II |
| 5 | V | 25 | V |
| 6 | V | 26 | V |
| 7 | IV | 27 | V |
| 8 | IV | 28 | V |
| 9 | IV | 29 | V |
| 10 | IV | 30 | V |
| 11 | V | 31 | V |
| 12 | III | 32 | V |
| 13 | II | 33 | V |
| 14 | II | 34 | I |
| 15 | V | 35 | V |
| 16 | II | 36 | V |
| 17 | V | 37 | V |
| 18 | IV | 38 | V |
| 19 | V | | |

The compounds of Examples 1, 3, 4, 5, 6, 11, 15, 17, 19, 20, 21, 22, 23, 25, 26, 27, 28, 29, 30, 31, 32, 33, 35, 36, 37, and 38 exhibited an excellent cell growth suppression effect that is equivalent to that of prexasertib as shown in the above table.

Test Example 3: hERG current blocking test

The test compounds were added to cultured hERG (human Ether-a-go-go Related Gene) gene stably expressing CHO cell line cells to achieve 0.27 to 100 μM. The hERG current under electric potential stimulation was measured using Qube384 (Sophion Bioscience) to calculate the concentration at which each test compound suppresses 50% hERG current ($IC_{50}$ value; μM).

A test shown in Test Example 3 was conducted on the compounds obtained in the Examples and prexasertib. A value from dividing $IC_{50}$ of hERG obtained in Test Example 3 by $IC_{50}$ of CHK1 inhibition obtained in Test Example 1 was calculated as hERG/CHK1 for the compound of each Example. The results are shown in the following table.

[Table 6-1]

| Examples | hERG inhibition $IC_{50}$ (μM) | hERG/CHK1 |
|---|---|---|
| Prexasertib | 3.3 | 6111 |
| 1 | 11.5 | >115000 |
| 2 | 10.7 | 48636 |
| 3 | 87.4 | 397273 |
| 4 | 13.6 | 59130 |
| 5 | 2.7 | 8182 |
| 6 | 0.4 | 1481 |
| 7 | 72.8 | 346667 |
| 8 | >100 | >1000000 |
| 9 | 18.7 | 77917 |
| 10 | 21.6 | 72000 |
| 11 | 5.1 | 28333 |
| 12 | 3.1 | 9118 |
| 13 | <2.7 | <3649 |
| 14 | >100 | >454545 |
| 15 | <2.7 | <10385 |
| 16 | <2.7 | <888 |
| 17 | 12.6 | 42000 |
| 18 | 18.9 | 37059 |
| 19 | 11.3 | 43462 |
| 20 | 3.8 | 11176 |
| 21 | 10.3 | 35517 |
| 22 | 3.0 | 12500 |
| 23 | <2.7 | <6923 |
| 24 | 34.5 | 115000 |
| 25 | 4.7 | 6351 |
| 26 | <2.7 | 19286 |
| 27 | 13.9 | 4017 |
| 28 | 16.7 | 33400 |

(continued)

| Examples | hERG inhibition $IC_{50}$ (μM) | hERG/CHK1 |
|---|---|---|
| 29 | 11.2 | 18667 |
| 30 | 10.4 | 40000 |
| 31 | 6.9 | 9200 |

[Table 6-2]

| | | |
|---|---|---|
| 32 | 56.4 | 74211 |
| 33 | 14.5 | 21014 |
| 34 | >100 | >54945 |
| 35 | 8.7 | 14032 |
| 36 | 22.0 | 30137 |
| 37 | <2.7 | <8438 |
| 38 | <2.7 | <4500 |

As shown in the above table, the compounds of Examples 1, 3, 4, 7 to 10, 14, 24, 32, and 34 were demonstrated as having a deviation of 50000-fold or greater between $IC_{50}$ of CHK1 inhibition and $IC_{50}$ of hERG. In particular, the compounds of Examples 1, 3, 7, 8, 14, and 24 were demonstrated as having a deviation of 100000-fold or greater between $IC_{50}$ of CHK1 inhibition and $IC_{50}$ of hERG.

These six compounds exhibit a deviation of 18-fold or greater relative to prexasertib and have a different effect of having high safety.

Test Example 4: CYP3A4 MBI and enzyme inactivation clearance evaluation

Cytochrome P450 (hereinafter CYP) is the most important group of enzymes associated with drug metabolism. Many of the pharmacokinetic interactions are based on inhibition of CYP activity. CYP includes a plurality of molecular species. In particular, CYP3A4 has the highest ratio of involvement in the metabolism of a drug in an oxidation reaction by CYP and accounts for the majority of CYP in the liver.

The mode of CYP inhibition is roughly divided into "reversible inhibition" and "irreversible inhibition (mechanism-based inhibition: MBI)". It is known that CYP inhibition based on MBI in particular has the possibility of inducing not only drug interactions but also a severe side effect (hepatotoxicity, etc.) (Curr Opin Drug Discov Devel. 2010 January, 13(1), 66-77, Therapeutics and Clinical Risk Management, 2005, 1(1), 3-13).

CYP3A4 MBI and enzyme inactivation clearance were evaluated using the compound of the Example and prexasertib.

The effect and mode of inhibition of the test compound on CYP3A4 were evaluated using a human liver microsome as an enzyme source and midazolam or testosterone as a substrate. A substrate-derived metabolite of the test compound added groups (4 concentrations) and a substrate-derived metabolite of a no addition group were measured by LC-MS/MS after 30 minutes of metabolic reaction at 37°C, and the inhibition rate was calculated from the ratio thereof. The $IC_{50}$ value was calculated from a concentration plot. When an MBI action was found in a test compound, since it is known that $IC_{50}$ decreases by starting a reaction from adding a substance after pre-incubation in the presence of NADPH (cofactor) (Xenobiotica, 2009, 39(2), 99-112), the compound was determined as having an MBI action when the change in the $IC_{50}$ value due to pre-incubation was 2-fold or greater.

When an MBI action was found, $k_{inact}$ (maximum enzyme inactivation rate constant) and $K_I$ (concentration of inhibitor resulting in 50% of maximum enzyme inactivation rate) were calculated by nonlinear least squares method. Furthermore, enzyme inactivation clearance was calculated in accordance with the method described in Drug Metabolism and Disposition, 2011, 39 (7), 1247-1254 ($CL_{int} = k_{inact}/K_I$ (ml/min/mmol) × CYP contents (pmol/mg protein)).

The enzyme inactivation clearance and the factor of change in the $IC_{50}$ value by pre-incubation that can be an indicator of MBI action of the compound of each Example and prexasertib on CYP3A4 are shown in the following table.

[Table 7-1]

| Example | Factor of change in $IC_{50}$ value | Enzyme inactivation clearance (μL/min/mg protein) |
|---|---|---|
| Prexasertib | 2.6 | 0.019 |
| 1 | Not Detected | - |
| 2 | Not Detected | - |
| 3 | 3.1 | 0.036 |
| 4 | Not Detected | - |
| 5 | 2.9 | 0.717 |
| 6 | Not Detected | - |
| 7 | Not Detected | - |
| 8 | Not Detected | - |
| 9 | 4.5 | 0.150 |
| 10 | 3.3 | 0.091 |
| 11 | 3.5 | 1.040 |
| 12 | 2.7 | 0.287 |
| 13 | 3.6 | 0.484 |
| 14 | Not Detected | - |
| 17 | 10.9 | 0.766 |
| 18 | 3.9 | 0.352 |
| 19 | 8.4 | 1.157 |
| 20 | 3.7 | 1.112 |
| 21 | 2.5 | 0.074 |
| 22 | 2.1 | 0.349 |
| 23 | 2.4 | 0.433 |
| 25 | 2.6 | 0.722 |
| 26 | 2.2 | >100 |
| 27 | 3.6 | 0.679 |
| 28 | 3.5 | 0.188 |
| 29 | Not Detected | 0.054 |
| 30 | Not Detected | - |

[Table 7-2]

| | | |
|---|---|---|
| 31 | Not Detected | - |
| 32 | 3.4 | 3.831 |
| 33 | 2.3 | 0.887 |
| 34 | Not Detected | 0.002 |
| 35 | 2.9 | 0.192 |
| 36 | 3.9 | 0.430 |
| 37 | Not Detected | - |
| 38 | Not Detected | - |

The inventors newly found that prexasertib has a risk of inducing a severe side effect such as hepatotoxicity by

inhibiting CYP3A4 based on MBI.

As shown in the above table, it was revealed that, although prexasertib exhibits inhibition of CYP3A4 based on MBI, the compounds of Examples 1, 2, 4, 6 to 8, 14, 29 to 31, 34, 37, and 38 do not exhibit inhibition of CYP3A4 based on MBI. In view of this result, the compounds of Examples 1, 2, 4, 6 to 8, 14, 29 to 31, 34, 37, and 38 have a different effect of inducing not only drug interactions but also having high safety with reduced risk of severe side effect such as hepatotoxicity. In particular, the compound represented by formula (3) exhibits a particularly high safety and having a different effect.

Test Example 5. Human bone marrow cell colony formation test (hemotoxicological evaluation test)

Human bone marrow CD34 positive hematopoietic stem cells were obtained from Lonza K.K. The frozen cells were thawed in a medium prepared by mixing a Methocult Express medium and a 10% fetal bovine serum with 1% penicillin/streptomycin containing RPMI 1640 medium at 9:1. 5000 cells were seeded per well in a 6 well plate and cultured overnight at 37°C in the presence of 5% $CO_2$. The evaluated compound was added so that the final concentration of DMSO would be 0.1% and 100 nM, and the cells were cultured for 48 hours. The cells were retrieved and washed with PBS. 1/6 of the amount of cells was seeded in a 12 well plate and cultured for about 2 weeks at 37°C in the presence of 5% $CO_2$. After completion of culture, the number of colonies formed was calculated using Thiazolyl blue tetrazolium bromide (MTT) .

The test shown in Test Example 5 was conducted for a representative compound obtained in the Examples and prexasertib. The results are shown in the following table.

[Table 8]

| | Compound concentration (nM) | Colony count (/well) | |
|---|---|---|---|
| | | mean | S.D. |
| DMSO | - | 34 | 17 |
| Prexasertib | 100 | 19 | 9 |
| Example 1 | 100 | 34 | 24 |

As shown in the above table, it was revealed that, although 100 nM of prexasertib exhibits an effect of suppressing human bone marrow cell colony formation, 100 nM of Example 1 does not exhibit an effect of suppressing human bone marrow colony formation. In view of the results, the compound of Example 1 has a different effect of having high safety with respect to hemotoxicity.

Test Example 6. Liposome encapsulation test

Liposome encapsulation tests were conducted for representative Example compounds and prexasertib.

6.48 g of hydrogenated soybean phosphatidylcholine (COATSOME NC-21E, NOF Corporation), 2.32 g of cholesterol (Sigma) and 2.06 g of distearoylphosphatidylethanolamine-methoxypolyethylene glycol 2000 (SUNBRIGHT DSPE-020CN, NOF Corporation) were dissolved in 560 mL of t-butyl alcohol heated to 65°C. The solution was frozen with dry ice/acetone bath and t-butyl alcohol was then removed by evaporation under reduced pressure to obtain a lipid mixture.

200 mL of 250 mM ammonium sulfate solution was added to the lipid mixture. The resulting mixture was heated to 65°C and dispersed with a homogenizer (ULTRA-TURRAX, IKA) to obtain a crude liposome dispersion. The crude liposome dispersion was further dispersed with a high pressure homogenizer (Nano-Mizer NM2, Yoshida Kikai) at a pressure of 100 MPa to obtain a liposome with a mean particle size (Z-average) of about 80 nm. A dialysis cassette (Slide-A-Lyzer G2 Dialysis Cassettes 20K MWCO, Thermo Scientific) was used to replace the outer aqueous phase of liposome with 10 mM L-histidine buffer/10% sucrose solution (pH 6.5) to obtain an empty liposome solution. The solution was filtered through a 0.22 μm membrane filter, and 10 mM L-histidine buffer/10% sucrose solution (pH 6.5) was added to adjust the total lipid concentration to be 75 mM (75 μmol/mL) or 50 mM (50 μmol/mL). The prepared amount was increased or decreased appropriately.

2 to 3 mg of the test compound was weighed out. 1 mL of empty liposome solution with a total lipid concentration of 50 mM was added. An aqueous 1 mol/L hydrochloric acid or 1 mol/L sodium hydroxide solution was added to adjust the pH to 5.5 to 7. The mixture was heated for 10 to 30 minutes in a 65°C water bath and then cooled with ice. Insolubles, when present, were removed by centrifugation for 5 minutes at 15,000× g.

The liposome encapsulation ratio was calculated as follows.

100 μL of liposome solution was passed through an ultrafiltration filter (Amicon Ultra, 100K, 0.5 mL, Merck), and centrifuged at 4°C at 15,000 × g for 10 minutes. The concentration of the compound in the filtrate after ultrafiltration was

measured by HPLC as the unencapsulated compound concentration.

The liposome solution was diluted with a trifluoroacetic acid/water/methanol mixture (0.1/25/75) and was left standing for 10 minutes or longer at 5°C. The solution was centrifuged for 5 minutes at 15,000× g to remove insolubles. The compound concentration in the supernatant was measured by HPLC as the compound concentration in the liposome solution.

The encapsulation ratio and the encapsulation efficiency were calculated by the following equations.

Encapsulation ratio (%) = (compound concentration in liposome solution - unencapsulated compound concentration) × 100/compound concentration in liposome solution Encapsulation efficiency (%) = (compound concentration in liposome solution - unencapsulated compound concentration) × 100/concentration upon compound introduction

HPLC measurement conditions are the following.

HPLC conditions

Column: Acquity UPLC BEH C18, 1.7 μm, 50 x 2.1 mm
Column temperature: 40°C
Mobile phase: A: 0.1% trifluoroacetic acid containing water B: acetonitrile
A/B (min): 95/5 (0) → 0/100 (3.5) → 0/100 (4) → 95/5 (4.01) → 95/5 (5)
Flow rate: 0.8 mL/min
Detection: UV visible detector, measured wavelength 254 nm Injected volume: 3 μL or 5 μL

[Table 9]

| Compound | Concentration upon compound introduction (mg/mL) | Compound concentration in liposome solution (mg/mL) | Encapsulation ratio (%) | Encapsulation efficiency (%) |
|---|---|---|---|---|
| Prexasertib | 2 | 2.16 | 99.8 | 107.7 |
| Example 1 | 3 | 2.70 | 97.1 | 87.4 |
| Example 3 | 2 | 1.86 | 99.3 | 92.3 |
| Example 4 | 2 | 1.73 | 99.9 | 86.6 |
| Example 5 | 3 | 2.97 | 100.0 | 99.1 |
| Example 6 | 3 | 3.01 | 99.9 | 100.3 |

It was confirmed that prexasertib and Examples 1, 3, 4, 5, and 6 can achieve a high encapsulation efficiency of 80% or higher. A significant increase in the liquid viscosity was found when pH was adjusted by adding 1 mol/L hydrochloric acid during the liposome encapsulation procedure in Example 4. The increase in viscosity can be an issue in large-scale liposome manufacture. Meanwhile, an increase in viscosity was not found during the liposome encapsulation procedure in Examples 1, 3, 5, and 6. Thus, the compounds represented by formulas (3) and (4) exhibited a particularly superb liposome encapsulation operability.

Test Example 6A. Liposome encapsulation test of Example 1

A liposome encapsulation test was conducted on the compound of Example 1.

11.08 g of Presome ACD-1 (pre-prepared mixture consisting of hydrogenated soybean phosphatidylcholine, cholesterol, and distearoylphosphatidylethanolamine-methoxypolyethylene glycol 2000 at a mass ratio of 3:1:1, Nippon Fine Chemical Co., Ltd.) was weighed out, and 200 mL of 250 mM ammonium sulfate solution was added. The resulting mixture was heated to 65°C and dispersed with a homogenizer (ULTRA-TURRAX, IKA) to obtain a crude liposome dispersion. The crude liposome dispersion was further dispersed with a high pressure homogenizer (Nano-Mizer NM2, Yoshida Kikai) at a pressure of 100 MPa to obtain a liposome with a mean particle size (Z-average) of 80 nm. A dialysis cassette (Slide-A-Lyzer G2 Dialysis Cassettes 20K MWCO, Thermo Scientific) was used to replace the outer aqueous

phase of liposome with 10 mM L-histidine buffer/10% sucrose solution (pH 6.5). The mixture was filtered through a 0.22 μm membrane filter to obtain an empty liposome solution with a total lipid concentration of 75 mM (75 μmol/mL).

The compound of Example 1 was weighed out. About 0.4 mL of 10 mM L-histidine buffer/10% sucrose solution (pH 6.5), about 0.1 mL of 0.1 mol/L hydrochloric acid, and 1 mL of the empty liposome solution were added, and the pH was adjusted to 6 to 6.5 by adding 1 mol/L hydrochloric acid. The mixture was heated for 30 minutes in a 65°C water bath and then cooled with ice. The mixture was then filtered through a 0.22 μm membrane filter.

The liposome encapsulation ratio was calculated as follows.

Unencapsulated compound concentration: The compound concentration was measured by HPLC for a solution prepared by adding 100 μL of aqueous 4% phosphoric acid solution to 100 μL of liposome solution. HPLC measurement conditions are the following.

HPLC conditions

Column: MonoSelect nPEC (GL Sciences Inc.)
Column temperature: 30°C
Mobile phase: A: 0.1% trifluoroacetic acid containing water B: acetonitrile
A/B (min): 95/5 (0) → 95/5 (1) → 70/30 (6) → 70/30 (7) → 95/5 (7.01) → 95/5 (10)
Flow rate: 1 mL/min
Detection: UV visible detector, measured wavelength 254 nm
Injected volume: 2 μL

Compound concentration in liposome solution: The liposome solution was diluted with a trifluoroacetic acid/water/methanol mixture (0.1/25/75) and was left standing for 10 minutes or longer at 5°C. The mixture was centrifuged for 5 minutes at 15,000× g to remove insolubles. The compound concentration in the supernatant was measured by HPLC. HPLC measurement conditions are the following.

HPLC conditions

Column: Acquity UPLC BEH C18, 1.7 μm, 50 x 2.1 mm
Column temperature: 40°C
Mobile phase: A: 0.1% trifluoroacetic acid containing water B: acetonitrile
A/B (min): 95/5 (0) → 0/100 (3.5) → 0/100 (4) → 95/5 (4.01) → 95/5 (5)
Flow rate: 0.8 mL/min
Detection: UV visible detector, measured wavelength 254 nm
Injected volume: 5 μL

The encapsulation ratio and encapsulation efficiency were calculated by the following equations.

Encapsulation ratio (%) = (compound concentration in liposome solution - unencapsulated compound concentration) × 100/compound concentration in liposome solution

Encapsulation efficiency (%) = (compound concentration in liposome solution - unencapsulated compound concentration) × 100/concentration upon compound introduction

[Table 10]

| Compound | Concentration upon compound introduction (mg/mL) | Compound concentration in liposome solution (mg/mL) | Encapsulation ratio (%) | Encapsulation efficiency (%) |
|---|---|---|---|---|
| Example 1 | 2 | 1.94 | 94.1 | 91.3 |
| | 3 | 2.84 | 96.6 | 91.6 |
| | 4 | 3.78 | 97.5 | 92.2 |

It was confirmed that Example 1 can achieve high liposome encapsulation efficiency of 90% or higher.

Test Example 6B. Liposome encapsulation test of Example 1

A liposome encapsulation test was conducted on the compound of Example 1.

11.08 g of Presome ACD-1 (pre-prepared mixture consisting of hydrogenated soybean phosphatidylcholine, cholesterol, and distearoylphosphatidylethanolamine-methoxypolyethylene glycol 2000 at a mass ratio of 3:1:1, Nippon Fine Chemical Co., Ltd.) was weighed out, and 200 mL of 250 mM ammonium sulfate solution was added. The resulting mixture was heated to 65°C and dispersed with a homogenizer (ULTRA-TURRAX, IKA) to obtain a crude liposome dispersion. The crude liposome dispersion was further dispersed with a high pressure homogenizer (Nano-Mizer NM2, Yoshida Kikai) at a pressure of 100 MPa to obtain a liposome with a mean particle size (Z-average) of 81 nm. A dialysis cassette (Slide-A-Lyzer G2 Dialysis Cassettes 20K MWCO, Thermo Scientific) was used to replace the outer aqueous phase of liposome with 10 mM L-histidine buffer/9.4% sucrose solution (pH 6.5). The mixture was filtered through a 0.22 μm membrane filter to obtain an empty liposome solution with a total lipid concentration of 53.1 mg/mL.

120 mg of the compound of Example 1 was weighed out. 18.3 mL of 10 mM L-histidine buffer/9.4% sucrose solution (pH 6.5), 0.3 mL of 1 mol/L hydrochloric acid, and 41.7 mL of the empty liposome solution were added, and the pH was adjusted to 6.5 with 1 mol/L hydrochloric acid or 1 mol/L sodium hydroxide. The solution was heated for 30 minutes in a 50°C water bath and then cooled with ice. The mixture was filtered through a 0.22 μm membrane filter. The concentration of the compound of Example 1 in the liposome solution was 2.00 mg/mL, the encapsulation ratio was 98.0%, and the mean particle size (Z-average) was 84 nm.

The liposome encapsulation ratio was calculated as follows.
Unencapsulated compound concentration: 100 μL of liposome solution, 100 μL of aqueous 4% phosphoric acid solution, and 300 μL of saline were mixed. The mixture was centrifuged for 60 minutes at 100,000× g to remove insolubles. The compound concentration in the supernatant was measured by HPLC. Compound concentration in liposome solution: The liposome solution was diluted with a trifluoroacetic acid/water/methanol mixture (0.1/25/75) and was left standing for 10 minutes or longer at 5°C. The mixture was centrifuged for 5 minutes at 15,000× g to remove the insolubles. The compound concentration in the supernatant was measured by HPLC.
HPLC measurement conditions are the following.

HPLC conditions

Column: Acquity UPLC BEH C18, 1.7 μm, 50 x 2.1 mm
Column temperature: 40°C
Mobile phase: A: 0.1% trifluoroacetic acid containing water B: acetonitrile
A/B (min): 95/5 (0) → 0/100 (3.5) → 0/100 (4) → 95/5 (4.01) → 95/5 (5)
Flow rate: 0.8 mL/min
Detection: UV visible detector, measured wavelength 254 nm
Injected volume: 5 μL

The encapsulation ratio was calculated by the following equation.

Encapsulation ratio (%) = (compound concentration in liposome solution - unencapsulated compound concentration) × 100/compound concentration in liposome solution

A liposome encapsulating the compound of Example 1 was stored at 5°C to study the change in the compound

concentration, encapsulation ratio, and mean particle size. As shown in Table 11, a significant change in the compound concentration, encapsulation ratio, and mean particle size was not found for a liposome encapsulating Example 1, thus revealing that the liposome has excellent storage stability.

[Table 11]

| Compound | Storage period | Compound concentration (mg/mL) | Encapsulation ratio (%) | Mean particle size (Z-average) |
|---|---|---|---|---|
| Example 1 | Initial value | 2.00 | 98.0 | 84 |
| | 1W | 2.07 | 98.5 | - |
| | 2W | 2.00 | 98.7 | - |
| | 4W | 1.90 | 98.9 | - |
| | 2M | 2.10 | 99.3 | - |
| | 3M | 1.94 | 99.0 | - |
| | 6M | 2.06 | 99.5 | 89 |

Test Example 7. Pharmacokinetic test

A prexasertib solution formulation and liposome formulations of Examples 1, 3, and 6 and prexasertib were intravenously administrated to mice to measure the concentration of the test compound in blood.

The test used a solution formulation, which was dissolved in a 10 mM glycine/5% mannitol solution (pH2) or 20% sulfobutylether-β-cyclodextrin containing 10 mM glycine/5% mannitol solution (pH2) and then filtered through a 0.22 μm membrane filter.

The test used a liposome formulation, which was produced from preparing a liposome encapsulating a test compound by the similar method to Test Example 6, replacing an outer aqueous phase of a liposome with 10 mM L-histidine buffer/10% sucrose solution (pH 6.5) by using gel filtration column PD-10 (GE Healthcare), then filtering the liposome through a 0.22 μm membrane filter, and adding a 10 mM L-histidine buffer/10% sucrose solution (pH 6.5) to adjust the concentration.

<Administration test>

The solution formulation or liposome formulation was instantaneously and intradermally administered into 7 week old female BALB/c mice. Blood was collected over time up to 72 hours after administration from the jugular vein under no anesthesia. Blood immediate after collection, to which 4 times the volume of methanol was added, was centrifuged, and the concentration of the test compound in the supernatant that was obtained was quantified by LC-MS/MS.
LC-MS/MS measurement conditions are the following.

HPLC: Prominence system (Shimadzu Corporation)
MS/MS: 4000 QTRAP (SCIEX)
Column: Cadenza CD-C18, 3 μm, 50 × 2 mm (Imtakt Corporation)
Column temperature: 40°C
Mobile phase: A: 0.1% formic acid containing water
B: 0.1% formic acid containing acetonitrile
A/B (min): 90/10 (0) → 10/90 (2.5) → 10/90 (3.5) → 90/10 (3.6) → 90/10 (5.0)
Flow rate: 0.4 mL/min
Detection: ESI (positive mode)
Injected volume: 0.1 to 5 μL

The test results are shown in the following table. In the table, "mean" refers to the mean, and "S.D." refers to the standard deviation. Tables 12, 13, 14, 15, and 16 show the plasma test compound concentrations from 0 hours to 72 hours after administration.

[Table 12]

| Solution formulation of prexasertib 1.0 mg/kg (i.v.) | Plasma compound concentration (ng/mL) | | | | |
|---|---|---|---|---|---|
| | 1 | 2 | 3 | mean | S.D. |
| 0.08hr | 245 | 277 | 160 | 227 | 61 |
| 0.25hr | 138 | 112 | 140 | 130 | 16 |
| 0.5hr | 93.6 | 139 | 90 | 108 | 27 |
| 1.0hr | 51.2 | 44.3 | 64.9 | 53.5 | 11 |
| 6.0hr | N.D. | 9.95 | 5.46 | 5.14 | 5.0 |
| 24.0hr | N.D. | N.D. | N.D. | N.D. | N.A. |
| 48.0hr | N.D. | N.D. | N.D. | N.D. | N.A. |
| 72.0hr | N.D. | N.D. | N.D. | N.D. | N.A. |

[Table 13]

| Liposome formulation of prexasertib 1.0mg/kg(i.v.) | Plasma compound concentration (ng/mL) | | | | |
|---|---|---|---|---|---|
| | 1 | 2 | 3 | mean | S.D. |
| 0.08hr | 28200 | 24800 | 27400 | 26800 | 1778 |
| 0.25hr | 23200 | 26600 | 25200 | 25000 | 1709 |
| 0.5hr | 26200 | 26100 | 23800 | 25367 | 1358 |
| 1.0hr | 20200 | 24600 | 21000 | 21933 | 2344 |
| 6.0hr | 12600 | 13000 | 11200 | 12267 | 945 |
| 24.0hr | 1610 | 2290 | 3630 | 2510 | 1028 |
| 48.0hr | 211 | 320 | 232 | 254 | 58 |
| 72.0hr | 7.98 | 11.3 | 21.8 | 13.7 | 7.2 |

[Table 14]

| Liposome formulation of Example 1 1.0 mg/kg (i.v.) | Plasma compound concentration (ng/mL) | | | | |
|---|---|---|---|---|---|
| | 1 | 2 | 3 | mean | S.D. |
| 0.08hr | 30400 | 31100 | 30800 | 30767 | 351 |
| 0.25hr | 30500 | 29600 | 33300 | 31133 | 1930 |
| 0.5hr | 28700 | 27600 | 27800 | 28033 | 586 |
| 1.0hr | 24800 | 25500 | 24600 | 24967 | 473 |
| 6.0hr | 18300 | 19000 | 16900 | 18067 | 1069 |
| 24.0hr | 7200 | 5940 | 6810 | 6650 | 645 |
| 48.0hr | 2000 | 2000 | 1580 | 1860 | 242.5 |
| 72.0hr | 159 | 22.6 | 194 | 125.2 | 90.6 |

[Table 15]

| Liposome formulation of Example 3 1.0 mg/kg (i.v.) | Plasma compound concentration (ng/mL) | | | | |
|---|---|---|---|---|---|
| | 1 | 2 | 3 | mean | S.D. |
| 0.08hr | 31000 | 29100 | 37000 | 32367 | 4124 |
| 0.25hr | 33700 | 34200 | 30900 | 32933 | 1779 |
| 0.5hr | 25400 | 24600 | 30600 | 26867 | 3258 |

(continued)

| Liposome formulation of Example 3 1.0 mg/kg (i.v.) | Plasma compound concentration (ng/mL) | | | | |
|---|---|---|---|---|---|
| | 1 | 2 | 3 | mean | S.D. |
| 1.0hr | 28600 | 24700 | 26900 | 26733 | 1955 |
| 6.0hr | 15600 | 14800 | 17300 | 15900 | 1277 |
| 24.0hr | 9780 | 8730 | 8710 | 9073 | 612 |
| 48.0hr | 3520 | 2960 | 4940 | 3807 | 1021 |
| 72.0hr | 1340 | 1442 | 1020 | 1267 | 219 |

[Table 16]

| Liposome formulation of Example 6 1.0 mg/kg (i.v.) | Plasma compound concentration (ng/mL) | | | | |
|---|---|---|---|---|---|
| | 1 | 2 | 3 | mean | S.D. |
| 0.08hr | 24700 | 23500 | 28800 | 25667 | 2779 |
| 0.25hr | 25800 | 28100 | 25400 | 26433 | 1457 |
| 0.5hr | 20700 | 21400 | 24700 | 22267 | 2136 |
| 1.0hr | 23300 | 20100 | 21900 | 21767 | 1604 |
| 6.0hr | 11100 | 10100 | 12700 | 11300 | 1311 |
| 24.0hr | 1050 | 1050 | 926 | 1009 | 71.6 |
| 48.0hr | 50.1 | 31.4 | 24.8 | 35.4 | 13.1 |
| 72.0hr | N.D. | N.D. | N.D. | N.D. | N.A. |

Table 17 shows the AUC calculated by the trapezoidal method from 0 hours after administration to a point in time (t) at which the plasma test compound concentration was able to be quantified for AUC associated with liposome formulations of Examples 1, 3, 6 and prexasertib.

[Table 17]

| Example | AUCo-t (ng·hr/mL) |
|---|---|
| Prexasertib | 236309 |
| 1 | 447473 |
| 3 | 583953 |
| 6 | 186225 |

The above results confirmed high blood retention upon intravenous administration of the liposome formulations prepared by liposome encapsulation of Example 1 and Example 3. This result shows that 72 hours of sustained exposure was achieved, which is important for maximizing the efficacy of prexasertib reported in a non-clinical trial. Therefore, Example 1 and Example 3 have an excellent pharmacokinetic profile and are very useful as an anticancer agent. It was demonstrated that a nitrogen atom on a pyridine ring, which is a feature of a compound set forth in formula (3), is present at a specific position.

Test Example 8. Drug efficacy evaluation test using tumor-bearing mice transplanted with ES-2 cells

Antitumor effect was evaluated by using prexasertib, a liposome formulation encapsulating prexasertib, and a liposome formulation encapsulating Example 1.

The test used a solution formulation of prexasertib, which was prepared by dissolving prexasertib in a 10 mM glycine/5% mannitol solution (pH2) containing 20% sulfobutylether-β-cyclodextrin and then filtering the mixture through a 0.22 μm membrane filter.

A liposome formulation was prepared by the similar method to Test Example 6. The test compound was weighed

out. An empty liposome solution with a total lipid concentration of 50 mM was added so that the compound concentration would be 2 mg/mL, and 1 mol/L hydrochloric acid was added to adjust the pH to 6 to 7. Alternatively, 10 mM L-histidine buffer/10% sucrose solution (pH 6.5) and hydrochloric acid were added to the test compound to dissolve or disperse the compound, and an empty liposome solution with a total lipid concentration of 75 mM was added so that the compound concentration would be 2 mg/mL, and the pH was adjusted to 6 to 7. The mixture was heated for 10 to 30 minutes in a 65°C water bath, and then cooled with ice. The mixture was left standing overnight or longer in a 5°C refrigerator and then filtered with a 0.22 μm membrane filter. The liposome encapsulation ratio calculated by the method described in Test Example 6 was 99% or higher.

ES-2 cells (ATCC) were intradermally transplanted at $1 \times 10^6$ cells/mouse into the flank region in 4 to 7 week old BALB/c-nu/nu mice (CAnN.Cg-Foxn1nu/CrlCrlj, female, Charles River Laboratories Japan). After confirming engraftment of the ES-2 cells 5 to 14 days post-transplantation, a solution formulation was intravenously administered at a dose of 30 mg/kg or a liposome formulation was intravenously administered at a dose of 3 mg/kg, 7.5 mg/kg, or 20 mg/kg once a week, or the same was subcutaneously administered twice a day for 3 consecutive days (total of 6 administrations in a week). The tumor volume was measured over time from the start of administration to evaluate the tumor volume reduction from administration of the compound. The tumor volume was calculated by the following equation by using the minor axis and major axis of tumor measured with an electronic caliper (Mitutoyo).

$$\text{Tumor volume } [\text{mm}^3] = 0.5 \times (\text{minor axis } [\text{mm}])^2 \times \text{major axis } [\text{mm}]$$

A control administration group administered with only a solvent and a compound of the present disclosure administration group were compared. T/C was calculated from the following equation to evaluate the antitumor effect. The complete tumor regression (CR) individual ratio as of the completion of administration due to administration of a solution formulation or liposome formulation was also recorded. For the control administration group, an empty liposome solution prepared by the similar method to Test Example 6 was used.

T/C (%) = (tumor volume as of completion of administration of the compound of the present disclosure administration group - tumor volume as of the start of administration of the compound of the present disclosure administration group/(tumor volume as of completion of administration of the control administration group - tumor volume as of the start of administration of the control administration group) × 100

Table 18 shows the T/C (%) and CR individual ratio for tumor-bearing mice transplanted with ES-2 cells at each dosage and dosing period for the test compound.

[Table 18]

| Test | Route of administration | Dosage (mg/kg) | Dosage period (days) | Schedule (times/week) | T/C (%) | CR (%) |
|---|---|---|---|---|---|---|
| Solution formulation of prexasertib | Intravenous | 30 (maximum tolerable dose) | 14 | 1 | 73 | 0 |
| Solution formulation of prexasertib | Subcutaneous | 10 (maximum tolerable dose) | 14 | 6 | -5 | 0 |
| Liposome formulation of prexasertib | Intravenous | 3 | 10 | 1 | 64 | 0 |
| Liposome formulation of prexasertib | Intravenous | 7.5 | 10 | 1 | -4 | 0 |
| Liposome formulation of prexasertib | Intravenous | 20 | 14 | 1 | -17 | 33 |
| Liposome formulation of Example 1 | Intravenous | 3 | 10 | 1 | 61 | 0 |
| Liposome formulation of Example 1 | Intravenous | 7.5 | 10 | 1 | 6 | 0 |
| Liposome formulation of Example 1 | Intravenous | 20 | 14 | 1 | -18 | 33 |

The drug efficacy evaluation test using tumor-bearing mice revealed that Example 1 prepared as a liposome formulation, at any of the dosages, exhibited an excellent antitumor effect in the same manner as prexasertib prepared as a liposome formulation. These liposome formulations achieved tumor regression, which cannot be achieved by prexasertib prepared as a solution formulation, in an evaluation test using the dosage of 20 mg/kg. In view of the test results, Example 1 is a promising compound exhibiting an excellent antitumor effect, which is a significant effect.

Test Example 9. Measurement of neutrophil count using tumor-bearing mice transplanted with ES-2 cells

ES-2 cells (ATCC) were intradermally transplanted at $1 \times 10^6$ cells/mouse into the flank region in 4 to 7 week old BALB/c-nu/nu mice (CAnN.Cg-Foxn1nu/CrlCrlj, female, Charles River Laboratories Japan). After confirming survival of the ES-2 cells 5 to 14 days post-transplantation, a liposome formulation encapsulating prexasertib and a liposome formulation encapsulating Example 1 were intravenously administered once at each dose. Blood was collected 72 hours after administration to measure the neutrophil count. An empty liposome solution prepared by the similar method to Test Example 6 was used for comparison in this test.

A control administration group administered with an empty liposome solution and administration groups administered with Example 1 prepared as a liposome formulation or prexasertib prepared as a liposome formulation were compared. The ratio of residual neutrophils was calculated from the following equation to evaluate the safety of each formulation.

Ratio of residual neutrophils (%) = (neutrophil count 72 hours after administration in administration group)/(neutrophil count 72 hours after administration in control administration group) $\times$ 100

Table 19 shows the ratio of residual neutrophils for tumor-bearing mice transplanted with ES-2 cells.

[Table 19]

| Test | Dosage (mg/kg) | Ratio of residual neutrophils (%) |
|---|---|---|
| Empty liposome | - | - |
| Liposome formulation of prexasertib | 3 | 15 |
| Liposome formulation of prexasertib | 7.5 | 4 |
| Liposome formulation of Example 1 | 3 | 61 |
| Liposome formulation of Example 1 | 7.5 | 20 |
| Liposome formulation of Example 1 | 20 | 11 |

Example 1 prepared as a liposome formulation exhibits the same antitumor effect at the same dosage as prexasertib prepared as a liposome formulation. The ratio of residual neutrophils was 15% at the minimum effective dose of 3 mg/kg for the prexasertib prepared as a liposome formulation, with both an antitumor effect and hemotoxicity being simultaneously observed. Meanwhile, the ratio of residual neutrophils was 61% at the minimum effective dose of 3 mg/kg for Example 1 prepared as a liposome formulation, with less hemotoxic side effects. Furthermore, as shown in Test Example 8, Example 1 prepared as a liposome formulation exhibited a higher ratio of residual neutrophils at a dosage of 20 mg/kg, where tumor regression is observed, than administration of prexasertib prepared as a liposome formulation at 7.5 mg/kg. In view of the above, Example 1 is an excellent compound having a significant effect, with both higher safety and efficacy than prexasertib. Likewise, a liposome formulation of Example 1 is an excellent compound having a significant effect, with both high safety and efficacy.

Test Example 10. Human bone marrow cell myeloid lineage differentiation induction test (hemotoxicological evaluation test)

Human bone marrow CD34 positive hematopoietic stem cells were obtained from Lonza K. K. The frozen cells were thawed in a 1% fetal bovine serum containing IMDM medium and suspended in a Complete hemaTox® Myeloid Medium. 1000 cells were seeded per well in a 96-well plate. The evaluated compound was added so that the final concentration of DMSO would be 0.1% and 3 nM, and the cells were cultured for 7 days at 37°C in the presence of 5% $CO_2$. After completion of culture, half of the amount of cell suspension was retrieved from each well. The luminescence was measured using CellTiter-Glo® 3D Reagent (Promega, G968B) to calculate the cell survival percentage by the following equation.

Cell survival percentage (%) = (measurement value of luminescence after 7 days of evaluated compound)/(measurement value of luminescence after 7 days of control group) × 100

The test shown in Test Example 10 was conducted on a representative compound obtained in the Examples and prexasertib.

[Table 20]

| | Compound concentration (nM) | Viable cell ratio (%) | |
|---|---|---|---|
| | | mean | S.D. |
| DMSO | - | 100 | 2 |
| Prexasertib | 3 | 26 | 3 |
| Example 1 | 3 | 82 | 1 |

As shown in the above table, it was revealed that 3 nM of prexasertib exhibited an effect of suppressing myeloid cells induced to differentiate from human bone marrow cells, but 3 nM of Example 1 does not exhibit an effect of suppressing myeloid cells induced to differentiate from human bone marrow cells. In view of the results, Example 1 is a compound with a significant and different effect, having high safety with respect to hemotoxicity, compared to prexasertib.

Test Example 11. Tumor PD test using tumor-bearing mice transplanted with ES-2 cells

Pharmaco Dynamic (PD) responses in tumor were evaluated using prexasertib, liposome formulation encapsulating prexasertib, and liposome formulation encapsulating Example 1.

The test used a solution formulation of prexasertib, which was prepared by dissolving prexasertib in a 10 mM glycine/5% mannitol solution (pH2) containing 20% sulfobutylether-β-cyclodextrin and then filtering the mixture through a 0.22 μm membrane filter.

A liposome formulation was prepared by the similar method to Test Example 6. The test compound was weighed out. An empty liposome solution with a total lipid concentration of 50 mM was added so that the compound concentration would be 2 mg/mL, and 1 mol/L hydrochloric acid was added to adjust the pH to 6 to 7. Alternatively, 10 mM L-histidine buffer/10% sucrose solution (pH 6.5) and hydrochloric acid were added to the test compound to dissolve or disperse the compound, and an empty liposome solution with a total lipid concentration of 75 mM was added so that the compound concentration would be 2 mg/mL, and the pH was adjusted to 6 to 7. The solution was heated for 10 to 30 minutes in a 65°C water bath, and then cooled with ice. The solution was left standing overnight or longer in a 5°C refrigerator and then filtered with a 0.22 μm membrane filter. The liposome encapsulation ratio calculated by the method described in Test Example 6 was 99% or higher.

Ovarian cancer ES-2 cells (ATCC) were intradermally transplanted at $1 \times 10^6$ cells/mouse into the flank region in 4 to 7 week old BALB/c-nu/nu mice (CAnN.Cg-Foxn1nu/CrlCrlj, female, Charles River Laboratories Japan). After confirming engraftment of the ES-2 cells 5 to 14 days post-transplantation, a formulation was administered under the following conditions for each test:
Test A: a solution formulation was intravenously administered at a single dose of 30 mg/kg or a liposome formulation was intravenously administered at a single dose of 7.5 mg/kg, 3 mg/kg, 1 mg/kg, or 0.3 mg/kg. Test B: a solution formulation was intravenously administered at a single dose of 30 mg/kg or a liposome formulation was intravenously administered at a single dose of 7.5 mg/kg, 3 mg/kg, 1 mg/kg, or 0.3 mg/kg. Test C: a liposome formulation was intravenously administered at a single dose of 20 mg/kg, 7.5 mg/kg, 3 mg/kg, 1 mg/kg, or 0.3 mg/kg. After administration, tumor was harvested after 1, 3, or 6 days to evaluate PD responses of tumor due to compound administration.

PD responses in tumor were evaluated by Western blotting. Each band intensity detected with an anti-γH2AX antibody (05-636, Merck) and anti-Tubulin antibody (3873, Cell Signaling Technology) was calculated using the ImageJ software.

A control administration group administered with only a solvent and a compound of the present disclosure administration group were compared. The relative value of each band intensity was calculated with the following equation to evaluate the PD response in tumor. For the control administration group, an empty liposome solution prepared by the similar method to Test Example 6 was used.

Intensity of γH2AX = {(band intensity of γH2AX in tumor of the compound of the present disclosure administration group)/(band intensity of tubulin in tumor of the compound of the present disclosure administration group)}/{(band intensity of γH2AX in tumor of the control administration group/band intensity of tubulin in tumor of the control administration group)} × 100

Figure 7 to 9 show the intensity of γH2AX for tumor-bearing mice transplanted with ES-2 cells at each dosage and dosing period for the test compound.

In view of the above results, a potent PD response was observed at a dosage of 7.5 mg/kg 3 days after administration, and a PD response that could not be achieved with prexasertib prepared as a solution formulation was achieved from administration of prexasertib prepared as a liposome formulation, as shown in test A of Test Example 11. A potent PD response was observed at a dosage of 7.5 mg/kg 1 day after administration, and a PD response that could not be achieved with prexasertib prepared as a solution formulation was achieved from administration of Example 1 prepared as a liposome formulation, as shown in test B of Test Example 11. A potent PD response was observed at dosages of 7.5 mg/kg and 20 mg/kg 3 days and 6 days after administration from administration of Example 1 prepared as a liposome formulation, as shown in test C of Test Example 11. It was shown that these liposome formulations achieve 72 hours of sustained exposure, which is important for maximizing the efficacy of prexasertib reported in a non-clinical trial as shown in Test Example 7. Therefore, Example 1 has an excellent pharmacokinetic and pharmacodynamic profiles and are very useful as an anticancer agent.

<u>Test Example 12. Drug efficacy evaluation test using peritoneal dissemination tumor-bearing mice transplanted with ES-2 cells</u>

Antitumor effects were evaluated using prexasertib, liposome formulation encapsulating prexasertib, and liposome formulation encapsulating Example 1.

The test used a solution formulation of prexasertib, which was prepared by dissolving prexasertib in a 10 mM glycine/5% mannitol solution (pH2) containing 20% sulfobutylether-β-cyclodextrin and then filtering the mixture through a 0.22 μm membrane filter.

A liposome formulation encapsulating prexasertib was prepared as follows.

11.08 g of Presome ACD-1 (Nippon Fine Chemical Co., Ltd.) and 200 mL of 250 mM ammonium sulfate solution were mixed, and the resulting mixture was dispersed with a homogenizer (ULTRA-TURRAX, IKA) to obtain a crude liposome dispersion. The crude liposome dispersion was further dispersed with a high pressure homogenizer (Nano-Mizer NM2, Yoshida Kikai) at a pressure of 100 MPa to obtain a liposome with a mean particle size (Z-average) of 80 nm. A dialysis cassette (Slide-A-Lyzer G2 Dialysis Cassettes 20K MWCO, Thermo Scientific) was used to replace the outer aqueous phase of liposome with 10 mM L-histidine buffer/10% sucrose solution (pH 6.5). The mixture was filtered through a 0.22 μm membrane filter to obtain an empty liposome solution with a total lipid concentration of 75 mM. 60 mg of prexasertib, 10 mL of 10 mM L-histidine buffer/9.4% sucrose solution (pH6.5), and 20 mL of the empty liposome solution were added, and the pH was adjusted to 6.5. The solution was heated for 30 minutes in a 50°C water bath and then cooled with ice. The mixture was filtered through a 0.22 μm membrane filter. The prexasertib concentration in the liposome solution was 1.97 mg/mL, and the encapsulation ratio was 98%.

The compound concentration in liposome solution and encapsulation ratio were calculated as follows.

Unencapsulated compound concentration: 100 μL of liposome solution, 100 μL of aqueous 4% phosphoric acid solution, and 300 μL of saline were mixed. The mixture was centrifuged for 60 minutes at 100,000× g to remove insolubles. The compound concentration in the supernatant was measured by HPLC. Compound concentration in liposome solution: The liposome solution was diluted with a trifluoroacetic acid/water/methanol mixture (0.1/25/75) and centrifuged at 4°C for 10 minutes at 15,000× g to remove the insolubles. The compound concentration in the supernatant was measured by HPLC.

HPLC measurement conditions are the following.

HPLC conditions

Column: Acquity UPLC BEH C18, 1.7 μm, 50 × 2.1 mm
Column temperature: 40°C
Mobile phase: A: 0.1% trifluoroacetic acid containing water B: acetonitrile
A/B (min): 90/10 (0) → 70/30 (3) → 0/100 (3.5) → 0/100 (4) → 90/10 (4.01) → 90/10 (5)
Flow rate: 0.8 mL/min
Detection: UV visible detector, measured wavelength 254 nm

Injected volume: 5 μL

The encapsulation ratio was calculated by the following equation.

Encapsulation ratio (%) = (compound concentration in liposome solution - unencapsulated compound concentration) x 100/compound concentration in liposome solution

A liposome formulation encapsulating Example 1 was prepared as follows.

11.08 g of Presome ACD-1 (Nippon Fine Chemical Co., Ltd.) and 200 mL of 250 mM ammonium sulfate solution were mixed, and the resulting mixture was dispersed with a homogenizer (ULTRA-TURRAX, IKA) to obtain a crude liposome dispersion. The crude liposome dispersion was further dispersed with a high pressure homogenizer (Nano-Mizer NM2, Yoshida Kikai) at a pressure of 100 MPa to obtain a liposome with a mean particle size (Z-average) of 81 nm. A dialysis cassette (Slide-A-Lyzer G2 Dialysis Cassettes 20K MWCO, Thermo Scientific) was used to replace the outer aqueous phase of liposome with 10 mM L-histidine buffer/9.4% sucrose solution (pH 6.5). The mixture was filtered through a 0.22 μm membrane filter to obtain an empty liposome solution with a total lipid concentration of 53.1 mg/mL. 80 mg of the compound of Example 1, 12.2 mL of 10 mM L-histidine buffer/9.4% sucrose solution (pH6.5), and 27.8 mL of the empty liposome solution were added, and the pH was adjusted to 6.5. The solution was heated for 30 minutes in a 50°C water bath and then cooled with ice. The mixture was filtered through a 0.22 μm membrane filter. The concentration of the compound of Example 1 in the liposome solution was 1.99 mg/mL, and the encapsulation ratio was 97%.

The liposome encapsulation ratio was calculated as follows.

Unencapsulated compound concentration: 100 μL of liposome solution was mixed with 100 μL of aqueous 4% phosphoric acid solution, and the compound concentration in the solution was measured by HPLC. HPLC measurement conditions are the following.

HPLC conditions

Column: MonoSelect nPEC (GL Sciences Inc.)
Column temperature: 30°C
Mobile phase: A: 0.1% trifluoroacetic acid containing water B: acetonitrile
A/B (min): 95/5 (0) → 95/5 (1) → 70/30 (6) → 70/30 (7) → 95/5 (7.01) → 95/5 (10)
Flow rate: 1 mL/min
Detection: UV visible detector, measured wavelength 254 nm
Injected volume: 2 μL
Compound concentration in liposome solution: The liposome solution was diluted with a trifluoroacetic acid/water/-methanol mixture (0.1/25/75) and centrifuged at 4°C for 10 minutes at 15,000× g to remove the insolubles. The compound concentration in the supernatant was measured by HPLC. HPLC measurement conditions are the following.

HPLC conditions

Column: Acquity UPLC BEH C18, 1.7 μm, 50 × 2.1 mm
Column temperature: 40°C
Mobile phase: A: 0.1% trifluoroacetic acid containing water B: acetonitrile
A/B (min): 95/5 (0) → 0/100 (3.5) → 0/100 (4) → 95/5 (4.01) → 95/5 (5)
Flow rate: 0.8 mL/min
Detection: UV visible detector, measured wavelength 254 nm
Injected volume: 5 μL

The encapsulation ratio was calculated by the following equation.

Encapsulation ratio (%) = (compound concentration in liposome solution - unencapsulated compound concentration) x100/compound concentration in liposome solution

A cell line from Luciferase expressing ovarian cancer ES-2 cells (ATCC) was intraperitoneally transplanted at 1 × $10^6$ cells/mouse in 5 week old BALB/c-nu/nu mice (CAnN.Cg-Foxn1nu/CrlCrlj, female, Charles River Laboratories Japan). After confirming engraftment of the ES-2 cells 5 to 14 days post-transplantation with an IVIS Imaging System (PerkinElmer), a solution formulation was intravenously administered once a week at a dose of 30 mg/kg or a liposome formulation was intravenously administered once a week at a dose of 4.5 mg/kg or 20 mg/kg. Luminescence due to luciferase was measured over time from the start of administration to evaluate the tumor regression action due to compound administration. The mice were observed based on the change in body weight, systemic symptoms, and the tumor size estimated from luminescence due to luciferase. The survival period was measured for mice exhibiting a healthy state without severe diagnosis.

The median survival period was calculated for a control administration group administered with only a solvent and a compound of the present disclosure administration group to evaluate the antitumor effect. For the control administration group, an empty liposome solution was used.

Table 21 shows the median survival period for peritoneal dissemination tumor-bearing mice transplanted with ES-2 cells at each dosage and dosing period of test compound.

[Table 21]

| Test | Dosage (mg/kg) | Dosing period (days) | Schedule (times/week) | Median survival period (days) |
|---|---|---|---|---|
| Empty liposome | - | 35 | 1 | 18.5 |
| Solution formulation of prexasertib | 30 (maximum tolerable dose) | 35 | 1 | 27 |
| Liposome formulation of prexasertib | 20 | 35 | 1 | 33.5 |
| Liposome formulation of Example 1 | 4.5 | 35 | 1 | 24 |
| Liposome formulation of Example 1 | 20 | 35 | 1 | 32 |

It was revealed by the drug efficacy evaluation test using peritoneal dissemination tumor-bearing mice that Example 1 prepared as a liposome formulation exhibits an excellent antitumor effect in the same manner as prexasertib prepared as a liposome formulation at a dosage of 20 mg/kg. These liposome formulations achieved prolongation of survival period, which cannot be achieved with prexasertib prepared as a solution formulation in an evaluation test using a dosage of 20 mg/kg. In view of the test results, Example 1 is a promising compound exhibiting an excellent antitumor effect, which is a significant effect.

Test Example 13. Drug efficacy evaluation test using orthotopic ovarian tumor-bearing mice transplanted with ES-2 cells

Antitumor effects were evaluated using prexasertib, a liposome formulation encapsulating prexasertib, and a liposome formulation encapsulating Example 1.

The test used a solution formulation of prexasertib, which was prepared by dissolving prexasertib in a 10 mM glycine/5% mannitol solution (pH2) containing 20% sulfobutylether-β-cyclodextrin and then filtering the mixture through a 0.22 μm membrane filter.

A liposome formulation encapsulating prexasertib was prepared as follows.

11.08 g of Presome ACD-1 (Nippon Fine Chemical Co., Ltd.) and 200 mL of 250 mM ammonium sulfate solution were mixed, and the resulting mixture was dispersed with a homogenizer (ULTRA-TURRAX, IKA) to obtain a crude liposome dispersion. The crude liposome dispersion was further dispersed with a high pressure homogenizer (Nano-Mizer NM2, Yoshida Kikai) at a pressure of 100 MPa to obtain a liposome with a mean particle size (Z-average) of 80 nm. A dialysis cassette (Slide-A-Lyzer G2 Dialysis Cassettes 20K MWCO, Thermo Scientific) was used to replace the outer aqueous phase of liposome with 10 mM L-histidine buffer/10% sucrose solution (pH 6.5). The mixture was filtered through a 0.22 μm membrane filter to obtain an empty liposome solution with a total lipid concentration of 75 mM. 60 mg of prexasertib, 10 mL of 10 mM L-histidine buffer/9.4% sucrose solution (pH6.5), and 20 mL of the empty liposome solution were added, and the pH was adjusted to 6.5. The solution was heated for 30 minutes in a 50°C water bath and then cooled with ice. The mixture was filtered through a 0.22 μm membrane filter. The prexasertib concentration in the liposome solution was 1.97 mg/mL, and the encapsulation ratio was 98%.

The compound concentration in liposome solution and encapsulation ratio were calculated as follows.

Unencapsulated compound concentration: 100 μL of liposome solution, 100 μL of aqueous 4% phosphoric acid solution,

and 300 μL of saline were mixed. The mixture was centrifuged for 60 minutes at 100,000× g to remove insolubles. The compound concentration in the supernatant was measured by HPLC. Compound concentration in liposome solution: The liposome solution was diluted with a trifluoroacetic acid/water/methanol mixture (0.1/25/75) and centrifuged at 4°C for 10 minutes at 15,000× g to remove the insolubles. The compound concentration in the supernatant was measured by HPLC.

HPLC measurement conditions are the following.

HPLC conditions

Column: Acquity UPLC BEH C18, 1.7 μm, 50 × 2.1 mm
Column temperature: 40°C
Mobile phase: A: 0.1% trifluoroacetic acid containing water B: acetonitrile
A/B (min): 90/10 (0) → 70/30 (3) → 0/100 (3.5) → 0/100 (4) → 90/10 (4.01) → 90/10 (5)
Flow rate: 0.8 mL/min
Detection: UV visible detector, measured wavelength 254 nm
Injected volume: 5 μL

The encapsulation ratio was calculated by the following equation.

Encapsulation ratio (%) = (compound concentration in liposome solution - unencapsulated compound concentration) × 100/compound concentration in liposome solution

A liposome formulation encapsulating Example 1 was prepared as follows.

11.08 g of Presome ACD-1 (Nippon Fine Chemical Co., Ltd.) and 200 mL of 250 mM ammonium sulfate solution were mixed, and the resulting mixture was dispersed with a homogenizer (ULTRA-TURRAX, IKA) to obtain a crude liposome dispersion. The crude liposome dispersion was further dispersed with a high pressure homogenizer (Nano-Mizer NM2, Yoshida Kikai) at a pressure of 100 MPa to obtain a liposome with a mean particle size (Z-average) of 81 nm. A dialysis cassette (Slide-A-Lyzer G2 Dialysis Cassettes 20K MWCO, Thermo Scientific) was used to replace the outer aqueous phase of liposome with 10 mM L-histidine buffer/9.4% sucrose solution (pH 6.5). The mixture was filtered through a 0.22 μm membrane filter to obtain an empty liposome solution with a total lipid concentration of 53.1 mg/mL. 80 mg of the compound of Example 1, 12.2 mL of 10 mM L-histidine buffer/9.4% sucrose solution (pH6.5), and 27.8 mL of the empty liposome solution were added, and the pH was adjusted to 6.5. The solution was heated for 30 minutes in a 50°C water bath and then cooled with ice. The mixture was filtered through a 0.22 μm membrane filter. The concentration of the compound of Example 1 in the liposome solution was 1.99 mg/mL, and the encapsulation ratio was 97%.

The liposome encapsulation ratio was calculated as follows.

Unencapsulated compound concentration: 100 μL of liposome solution was mixed with 100 μL of aqueous 4% phosphoric acid solution, and the compound concentration in the solution was measured by HPLC. HPLC measurement conditions are the following.

HPLC conditions

Column: MonoSelect nPEC (GL Sciences Inc.)
Column temperature: 30°C
Mobile phase: A: 0.1% trifluoroacetic acid containing water B: acetonitrile
A/B (min): 95/5 (0) → 95/5 (1) → 70/30 (6) → 70/30 (7) → 95/5 (7.01) → 95/5 (10)
Flow rate: 1 mL/min
Detection: UV visible detector, measured wavelength 254 nm
Injected volume: 2 μL
Compound concentration in liposome solution: The liposome solution was diluted with a trifluoroacetic acid/water/-methanol mixture (0.1/25/75) and centrifuged at 4°C for 10 minutes at 15,000× g to remove the insolubles. The compound concentration in the supernatant was measured by HPLC. HPLC measurement conditions are the following.

HPLC conditions

Column: Acquity UPLC BEH C18, 1.7 μm, 50 × 2.1 mm
Column temperature: 40°C
Mobile phase: A: 0.1% trifluoroacetic acid containing water B: acetonitrile
A/B (min): 95/5 (0) → 0/100 (3.5) → 0/100 (4) → 95/5 (4.01) → 95/5 (5)
Flow rate: 0.8 mL/min
Detection: UV visible detector, measured wavelength 254 nm
Injected volume: 5 μL

The encapsulation ratio was calculated by the following equation.

Encapsulation ratio (%) = (compound concentration in liposome solution - unencapsulated compound concentration) × 100/compound concentration in liposome solution

A cell line from Luciferase expressing ovarian cancer ES-2 cells (ATCC) was transplanted within the right ovary at $1 \times 10^6$ cells/mouse in 5 week old BALB/c-nu/nu mice (CAnN.Cg-Foxn1nu/CrlCrlj, female, Charles River Laboratories Japan). After confirming engraftment of the ES-2 cells 5 to 14 days post-transplantation with an IVIS Imaging System (PerkinElmer), a solution formulation was intravenously administered once a week at a dose of 30 mg/kg or a liposome formulation was intravenously administered once a week at a dose of 4.5 mg/kg or 20 mg/kg. Luminescence due to luciferase was measured over time from the start of administration to evaluate the tumor regression action due to compound administration. The mice were observed based on the tumor size estimated from luminescence due to the change in body weight, systemic symptoms, and luciferase. The survival period was measured for mice exhibiting a healthy state without severe diagnosis.

The median survival period was calculated for a control administration group administered with only a solvent and a compound of the present disclosure administration group to evaluate the antitumor effect. For the control administration group, an empty liposome solution was used.

Table 22 shows the median survival period for orthotopic ovarian tumor-bearing mice transplanted with ES-2 cells at each dosage and dosing period for test compound.

[Table 22]

| Test | Dosage (mg/kg) | Dosing period (days) | Schedule (times/week) | Median survival period (days) |
|---|---|---|---|---|
| Empty liposome | - | 56 | 1 | 31.5 |
| Solution formulation of prexasertib | 30 (maximum tolerable dose) | 56 | 1 | 36 |
| Liposome formulation of prexasertib | 20 | 56 | 1 | 48.5 |
| Liposome formulation of Example 1 | 4.5 | 56 | 1 | 39.5 |
| Liposome formulation of Example 1 | 20 | 56 | 1 | 46 |

It was revealed by the drug efficacy evaluation test using orthotopic ovarian tumor-bearing mice that Example 1 prepared as a liposome formulation exhibits an excellent antitumor effect in the same manner as prexasertib prepared as a liposome formulation at a dosage of 20 mg/kg. These liposome formulations achieved prolongation of survival period, which cannot be achieved with prexasertib prepared as a solution formulation, in an evaluation test using a dosage of 20 mg/kg. In view of the test results, Example 1 is a promising compound exhibiting an excellent antitumor effect, which is a significant effect.

Test Example 14. Drug efficacy evaluation test using tumor-bearing mice transplanted with various cancer cells

Antitumor effects were evaluated using a liposome formulation encapsulating Example 1.

A liposome formulation encapsulating Example 1 was one used in Test Example 12 or prepared by the following

method.

138.4g of Presome ACD-1 (Nippon Fine Chemical Co., Ltd.) was weighed out, 2437g of 250 mM ammonium sulfate solution was added, and the resulting mixture was dispersed with a homogenizer (CLEARMIX, M TECHNIQUE) and degassed by vacuum degassing to obtain a crude liposome dispersion. The crude liposome dispersion was further dispersed with a high pressure homogenizer (Microfluidizer M-110 EH-30, Microfluidics) at a pressure of 14500 Psi to obtain a liposome with a mean particle size (Z-average) of 73 nm. Tangential Flow Filtration system equipped with five Pellicon cassettes (Pellicon 2 Mini 300kD 0.1m2, Merck) was used to replace the outer aqueous phase of liposome with 10 mM L-histidine buffer/9.4% sucrose solution (pH 6.5). The mixture was filtered through a 0.2 μm membrane filter to obtain an empty liposome solution with a total lipid concentration of 58.2 mg/mL. 3.0 g of the compound of Example 1 was weighed out, 562 g of 10 mM L-histidine buffer/9.4% sucrose solution (pH6.5), and 980 g of the empty liposome solution were added, and the pH was adjusted to 6.5. The mixture was heated at 45°C for 60 minutes and then cooled. Tangential Flow Filtration system equipped with five Pellicon cassettes (Pellicon 2 Mini 300kD 0.1m2, Merck) was used to replace the outer aqueous phase of liposome with 10 mM L-histidine buffer/9.4% sucrose solution (pH 6.5). The mixture was filtered through a 0.2 μm membrane filter. The solution was dispensed in 50 mL portions into glass vials, and the vials were stoppered and wind-tightened. The concentration of the compound of Example 1 in the liposome solution was 1.98 mg/mL, and the encapsulation ratio was 97%.

The compound concentration in liposome solution and encapsulation ratio were calculated as follows.
Unencapsulated compound concentration: 100 μL of liposome solution, 100 μL of aqueous 4% phosphoric acid solution, and 300 μL of saline were mixed. The mixture was centrifuged for 60 minutes at 100,000× g to remove insolubles. The compound concentration in the supernatant was measured by HPLC. Compound concentration in liposome solution: The liposome solution was diluted with a trifluoroacetic acid/water/methanol mixture (0.1/25/75) and centrifuged at 4°C for 10 minutes at 15,000× g to remove the insolubles. The compound concentration in the supernatant was measured by HPLC.

HPLC measurement conditions are the following.

HPLC conditions

Column: Acquity UPLC BEH C18, 1.7 μm, 50 × 2.1 mm
Column temperature: 40°C
Mobile phase: A: 0.1% trifluoroacetic acid containing water B: acetonitrile
A/B (min): 95/5 (0) → 60/40 (3) → 0/100 (3.5) → 0/100 (4) → 95/5 (4.01) → 95/5 (5)
Flow rate: 0.8 mL/min
Detection: UV visible detector, measured wavelength 254 nm Injected volume: 5 μL

The encapsulation ratio was calculated by the following equation.

Encapsulation ratio (%) = (compound concentration in liposome solution - unencapsulated compound concentration) × 100/compound concentration in liposome solution

Alternatively, 11.08 g of Presome ACD-1 (Nippon Fine Chemical Co., Ltd.) and 200 mL of 250 mM ammonium sulfate solution were mixed, and the resulting mixture was dispersed with a homogenizer (ULTRA-TURRAX, IKA) to obtain a crude liposome dispersion. The crude liposome dispersion was further dispersed with a high pressure homogenizer (Nano-Mizer NM2, Yoshida Kikai) at a pressure of 100 MPa to obtain a liposome with a mean particle size (Z-average) of 81 nm. A dialysis cassette (Slide-A-Lyzer G2 Dialysis Cassettes 20K MWCO, Thermo Scientific) was used to replace the outer aqueous phase of liposome with 10 mM L-histidine buffer/9.4% sucrose solution (pH 6.5). The mixture was filtered through a 0.22 μm membrane filter to obtain an empty liposome solution with a total lipid concentration of 53.1 mg/mL. 60 mg of the compound of Example 1, 9.16 mL of 10 mM L-histidine buffer/9.4% sucrose solution (pH6.5), and 20.8 mL of the empty liposome solution were added, and the pH was adjusted to 6.5. The solution was heated for 30 minutes in a 50°C water bath and then cooled with ice. The mixture was filtered through a 0.22 μm membrane filter. The concentration of the compound of Example 1 in the liposome solution was 2.08 mg/mL, and the encapsulation ratio was 98%.

The compound concentration in liposome solution and encapsulation ratio were calculated as follows.
Unencapsulated compound concentration: 100 μL of liposome solution, 100 μL of aqueous 4% phosphoric acid solution, and 300 μL of saline were mixed. The mixture was centrifuged for 60 minutes at 100,000× g to remove insolubles. The compound concentration in the supernatant was measured by HPLC. Compound concentration in liposome solution: The liposome solution was diluted with a trifluoroacetic acid/water/methanol mixture (0.1/25/75) and centrifuged at 4°C for 10 minutes at 15,000× g to remove the insolubles. The compound concentration in the supernatant was measured by HPLC.

HPLC measurement conditions are the following.

HPLC conditions

Column: Acquity UPLC BEH C18, 1.7 μm, 50 × 2.1 mm
Column temperature: 40°C
Mobile phase: A: 0.1% trifluoroacetic acid containing water B: acetonitrile
A/B (min): 95/5 (0) → 0/100 (3.5) → 0/100 (4) → 95/5 (4.01) → 95/5 (5)
Flow rate: 0.8 mL/min
Detection: UV visible detector, measured wavelength 254 nm
Injected volume: 5 μL

The encapsulation ratio was calculated by the following equation.

Encapsulation ratio (%) = (compound concentration in liposome solution - unencapsulated compound concentration) × 100/compound concentration in liposome solution

Ovarian cancer SKOV-3 cells (ATCC) or sarcoma SJCRH30 cells (ATCC) or sarcoma HT-1080 cells (ATCC) or pancreatic cancer AsPC-1 cells (ATCC) or pancreatic cancer BxPC-3 cells (ATCC) or lung cancer Calu-6 cells (ATCC) or ovarian cancer OV5304 cells (Crown Bioscience) were intradermally transplanted at $5 \times 10^5$ cells/mouse or $1 \times 10^6$ cells/mouse or $3 \times 10^6$ cells/mouse or $5 \times 10^6$ cells/mouse or 8 $mm^3$ to 27 $mm^3$ tumor mass/mouse into the flank region in 4 to 7 week old BALB/c-nu/nu mice (CAnN.Cg-Foxn1nu/CrlCrlj, female, Charles River Laboratories Japan) or NOD SCID mice (NOD.CB17-Prkdc$^{scid}$/J, female, Charles River Laboratories Japan). After confirming engraftment of the various cancer cells 5 to 27 days post-transplantation, a liposome formulation was intravenously administered once a week at a dose of 4.5 mg/kg or 10 mg/kg or 20 mg/kg. The tumor volume was measured over time from the start of administration to evaluate the tumor volume reduction from administration of the compound. The tumor volume was calculated by the following equation by using the minor axis and major axis of tumor measured with an electronic caliper (Mitutoyo). It is known that BxPC-3 cells exhibit gemcitabine resistance, and OV5304 exhibits PARP inhibitor resistance.

$$\text{Tumor volume } [mm^3] = 0.5 \times (\text{minor axis } [mm])^2 \times \text{major axis } [mm]$$

A control administration group administered with only a solvent and a compound of the present disclosure administration group were compared. T/C was calculated from the following equation to evaluate the antitumor effect. For the control administration group, an empty liposome solution was used.

T/C(%) = (tumor volume as of the end of administration of the compound of the present disclosure administration group - tumor volume as of the start of administration of the compound of the present disclosure administration group)/(tumor volume as of the end of administration of the control administration group - tumor volume as of the start of administration of the control administration group) × 100

Table 23 shows T/C(%) for tumor-bearing mice transplanted with various cancer cells at each dosage and dosing period for the test compound.

[Table 23]

| Cancer cell line | Cancer type | Test | Dosage (mg/kg) | Dosing period (days) | Schedule (times/week) | T/C (%) |
|---|---|---|---|---|---|---|
| SKOV-3 | Ovarian cancer | Liposome formulation of Example 1 | 20 | 21 | 1 | 10 |
| SJCRH30 | Sarcoma | Liposome formulation of Example 1 | 10 | 14 | 1 | 3 |
| HT-1080 | Sarcoma | Liposome formulation of Example 1 | 20 | 14 | 1 | 5 |

(continued)

| Cancer cell line | Cancer type | Test | Dosage (mg/kg) | Dosing period (days) | Schedule (times/week) | T/C (%) |
|---|---|---|---|---|---|---|
| AsPC-1 | Pancreatic cancer | Liposome formulation of Example 1 | 4.5 | 21 | 1 | 73 |
| AsPC-1 | Pancreatic cancer | Liposome formulation of Example 1 | 20 | 21 | 1 | 27 |
| BxPC-3 | Pancreatic cancer | Liposome formulation of Example 1 | 4.5 | 21 | 1 | 45 |
| BxPC-3 | Pancreatic cancer | Liposome formulation of Example 1 | 20 | 21 | 1 | 21 |
| Calu-6 | Lung cancer | Liposome formulation of Example 1 | 10 | 14 | 1 | 2 |
| OV5304 | Ovarian cancer | Liposome formulation of Example 1 | 20 | 25 | 1 | 83 |

It was revealed by the concomitantly used drug efficacy evaluation test using tumor-bearing mice that Example 1 prepared as a liposome formulation exhibits an excellent antitumor effect on various cancer cell lines. In view of the test results, Example 1 is a promising compound exhibiting an excellent antitumor effect, which is a significant effect.

Test Example 14A. Drug efficacy evaluation test using tumor-bearing mice transplanted with various cancer cells

Antitumor effects were evaluated using a liposome formulation encapsulating Example 1.

A liposome formulation encapsulating Example 1 was prepared as follows.

498.2 g of Presome ACD-1 (Nippon Fine Chemical Co., Ltd.) was weighed out, 8772 g of 250 mM ammonium sulfate solution was added, and the resulting mixture was dispersed with a homogenizer (CLEARMIX, M TECHNIQUE) and degassed by vacuum degassing to obtain a crude liposome dispersion. The crude liposome dispersion was further dispersed with a high pressure homogenizer (Microfluidizer M-110 EH-30, Microfluidics) at a pressure of 14500 Psi to obtain a liposome with a mean particle size (Z-average) of 73 nm. Tangential Flow Filtration system equipped with five Pellicon cassettes (Pellicon 2 Mini 300kD 0.1m2, Merck) was used to replace the outer aqueous phase of liposome with water for injection, and L-histidine and sucrose were added to make a 10 mM L-histidine/9.4% sucrose solution, and the pH was adjusted to 6.5 with 1 mol/L hydrochloric acid. The mixture was then filtered through a 0.2 μm membrane filter to obtain an empty liposome solution with a total lipid concentration of 50.2 mg/mL.

10.0 g of the compound of Example 1 was weighed out, 1354 g of 10 mM L-histidine buffer/9.4% sucrose solution (pH6.5), 27 g of 1 mol/L hydrochloric acid, and 3786 g of the empty liposome solution were added, and the pH was adjusted to 6.5 with 1 mol/L hydrochloric acid. The mixture was heated at 45°C for 60 minutes and then cooled, and the pH was adjusted to 6.5. The mixture was then filtered through a 0.2 μm membrane filter. The solution was dispensed in 50 mL portions into glass vials, and the vials were stoppered and wind-tightened. The concentration of the compound of Example 1 in the liposome solution was 2.01 mg/mL, the encapsulation ratio was 97.5%, and the average particle size (Z-average) was 74 nm.

The compound concentration in liposome solution and encapsulation ratio were calculated as follows.
Unencapsulated compound concentration: 100 μL of liposome solution, 100 μL of aqueous 4% phosphoric acid solution, and 300 μL of saline were mixed. The mixture was centrifuged for 60 minutes at 100,000× g to remove insolubles. The compound concentration in the supernatant was measured by HPLC.

Compound concentration in liposome solution: The liposome solution was diluted with a trifluoroacetic acid/-water/methanol mixture (0.1/25/75) and centrifuged at 4°C for 10 minutes at 15,000× g to remove the insolubles. The compound concentration in the supernatant was measured by HPLC.

HPLC measurement conditions are the following.

HPLC conditions

Column: Acquity UPLC BEH C18, 1.7 μm, 50 × 2.1 mm
Column temperature: 40°C
Mobile phase: A: 0.1% trifluoroacetic acid containing water B: acetonitrile
A/B (min): 95/5 (0) → 60/40 (3) → 0/100 (3.5) → 0/100 (4) → 95/5 (4.01) → 95/5 (5)
Flow rate: 0.8 mL/min
Detection: UV visible detector, measured wavelength 254 nm
Injected volume: 5 μL

The encapsulation ratio was calculated by the following equation.

Encapsulation ratio (%) = (compound concentration in liposome solution - unencapsulated compound concentration) × --> 100/compound concentration in liposome solution

A2780 cells (ECACC) or HCC1806 cells (ATCC) or An3CA cells (ATCC) or Detroit562 cells (ATCC) or FaDu cells (ATCC) or NCI-H460 cells (ATCC) were intradermally transplanted at 1 x $10^6$ cells/mouse or 2 × $10^6$ cells/mouse or 1.2 × $10^6$ cells/mouse or 1 × $10^6$ cells/mouse or 3 × $10^6$ cells/mouse or 3 × $10^6$ cells/mouse into the flank region in 4 to 7 week old BALB/c-nu/nu mice (CAnN.Cg-Foxn1nu/CrlCrlj, female, Charles River Laboratories Japan). After confirming engraftment of the various cancer cells 5 to 27 days post-transplantation, a liposome formulation was intravenously administered once a week at a dose of 20 mg/kg. The tumor volume was measured over time from the start of administration to evaluate the tumor volume reduction from administration of the compound. The tumor volume was calculated by the following equation by using the minor axis and major axis of tumor measured with an electronic caliper (Mitutoyo). It is known that BxPC-3 cells exhibit gemcitabine resistance, and OV5304 exhibits PARP inhibitor resistance.

$$\text{Tumor volume } [mm^3] = 0.5 \times (\text{minor axis } [mm])^2 \times \text{major axis } [mm]$$

A control administration group administered with only a solvent and a compound of the present disclosure administration group were compared. T/C was calculated from the following equation to evaluate the antitumor effect. For the control administration group, an empty liposome solution was used.

T/C(%) = (tumor volume as of the end of administration of the compound of the present disclosure administration group - tumor volume as of the start of administration of the compound of the present disclosure administration group)/(tumor volume as of the end of administration of the control administration group - tumor volume as of the start of administration of the control --> administration group) × 100

Table 23A shows T/C(%) for tumor-bearing mice transplanted with various cancer cells at each dosage and dosing period for the test compound.

[Table 23A]

| Cancer cell line | Cancer type | Test | Dosage (mg/kg) | Dosing period (days) | Schedule (times/week) | T/C (%) |
|---|---|---|---|---|---|---|
| A2780 | Ovarian cancer | Liposome formulation of Example 1 | 20 | 14 | 1 | 55 |
| HCC1806 | Breast cancer | Liposome formulation of Example 1 | 20 | 21 | 1 | -8 |
| An3CA | endometrial cancer | Liposome formulation of Example 1 | 20 | 14 | 1 | 15 |
| Detroit562 | head and neck cancer | Liposome formulation of Example 1 | 20 | 14 | 1 | -1 |
| FaDu | head and neck cancer | Liposome formulation of Example 1 | 20 | 14 | 1 | 33 |
| NCI-H460 | head and neck cancer | Liposome formulation of Example 1 | 20 | 14 | 1 | 19 |

Test Example 15. Concomitantly used drug efficacy evaluation test using tumor-bearing mice transplanted with PA-1 cells

Antitumor effects were evaluated using a liposome formulation encapsulating Example 1 and cisplatin.
Cisplatin (Randa Injection 50 mg/100 mL, Nippon Kayaku) was used.
A liposome formulation encapsulating Example 1 was prepared as follows.
138.4g of Presome ACD-1 (Nippon Fine Chemical Co., Ltd.) was weighed out, 2437g of 250 mM ammonium sulfate solution was added, and the resulting mixture was dispersed with a homogenizer (CLEARMIX, M TECHNIQUE) and degassed by vacuum degassing to obtain a crude liposome dispersion. The crude liposome dispersion was further dispersed with a high pressure homogenizer (Microfluidizer M-110 EH-30, Microfluidics) at a pressure of 14500 Psi to obtain a liposome with a mean particle size (Z-average) of 73 nm. Tangential Flow Filtration system equipped with five Pellicon cassettes (Pellicon 2 Mini 300kD 0.1m2, Merck) was used to replace the outer aqueous phase of liposome with 10 mM L-histidine buffer/9.4% sucrose solution (pH 6.5). The mixture was filtered through a 0.2 μm membrane filter to obtain an empty liposome solution with a total lipid concentration of 58.2 mg/mL. 3.0 g of the compound of Example 1 was weighed out, 562 g of 10 mM L-histidine buffer/9.4% sucrose solution (pH6.5), and 980 g of the empty liposome solution were added, and the pH was adjusted to 6.5. The mixture was heated at 45°C for 60 minutes and then cooled. Tangential Flow Filtration system equipped with five Pellicon cassettes (Pellicon 2 Mini 300kD 0.1m2, Merck) was used to replace the outer aqueous phase of liposome with 10 mM L-histidine buffer/9.4% sucrose solution (pH 6.5). The mixture was filtered through a 0.2 μm membrane filter. The solution was dispensed in 50 mL portions into glass vials, and the vials were stoppered and wind-tightened. The concentration of the compound of Example 1 in the liposome solution was 1.98 mg/mL, and the encapsulation ratio was 97%.
The compound concentration in liposome solution and encapsulation ratio were calculated as follows.
Unencapsulated compound concentration: 100 μL of liposome solution, 100 μL of aqueous 4% phosphoric acid solution, and 300 μL of saline were mixed. The mixture was centrifuged for 60 minutes at 100,000× g to remove insolubles. The compound concentration in the supernatant was measured by HPLC. Compound concentration in liposome solution: The liposome solution was diluted with a trifluoroacetic acid/water/methanol mixture (0.1/25/75) and centrifuged at 4°C for 10 minutes at 15,000× g to remove the insolubles. The compound concentration in the supernatant was measured by HPLC.
HPLC measurement conditions are the following.

HPLC conditions

Column: Acquity UPLC BEH C18, 1.7 μm, 50 × 2.1 mm
Column temperature: 40°C
Mobile phase: A: 0.1% trifluoroacetic acid containing water B: acetonitrile
A/B (min): 95/5 (0) → 60/40 (3) → 0/100 (3.5) → 0/100 (4) → 95/5 (4.01) → 95/5 (5)
Flow rate: 0.8 mL/min
Detection: UV visible detector, measured wavelength 254 nm
Injected volume: 5 μL

The encapsulation ratio was calculated by the following equation.

Encapsulation ratio (%) = (compound concentration in liposome solution - unencapsulated compound concentration) × 100/compound concentration in liposome solution

Ovarian cancer PA-1 cells (ATCC) were intradermally transplanted at $5 \times 10^6$ cells/mouse into the flank region in 4 to 7 week old BALB/c-nu/nu mice (CAnN.Cg-Foxn1nu/CrlCrlj, female, Charles River Laboratories Japan). After confirming engraftment of the PA-1 cells 5 to 14 days post-transplantation, a liposome formulation was administered at 20 mg/kg, cisplatin was administered at 2.5 mg/kg, or they were administered at a combined dose, intravenously once a week, peritoneally twice a week, or concomitantly. The tumor volume was measured over time from the start of administration to evaluate the tumor volume reduction from administration of the compound. The tumor volume was calculated by the following equation by using the minor axis and major axis of tumor measured with an electronic caliper (Mitutoyo).

$$\text{Tumor volume [mm}^3\text{]} = 0.5 \times (\text{minor axis [mm]})^2 \times \text{major axis [mm]}$$

A control administration group administered with only a solvent and a compound of the present disclosure administration group were compared. T/C was calculated from the following equation to evaluate the antitumor effect. The complete tumor regression (CR) individual ratio as of the end of administration due to a liposome formulation, cisplatin, or concomitant administration was also recorded. For the control administration group, an empty liposome solution was used.

T/C(%) = (tumor volume as of the end of administration of the compound of the present disclosure administration group - tumor volume as of the start of administration of the compound of the present disclosure administration group)/(tumor volume as of the end of administration of the control administration group - tumor volume as of the start of administration of the control administration group) $\times$ 100

Table 24 shows the T/C(%) and CR individual ratio for tumor-bearing mice transplanted with PA-1 cells at each dosage and dosing period for the test compound.

[Table 24]

| Test | Route of administration | Dosage (mg/kg) | Dosing period (days) | Schedule (times/week) | T/C (%) | CR (%) |
|---|---|---|---|---|---|---|
| Liposome formulation of Example 1 | Intravenous | 20 | 21 | 1 | -5 | 0 |
| Cisplatin | Peritoneal | 2.5 | 21 | 2 | 52 | 0 |
| Liposome formulation of Example 1 and cisplatin | Intravenous and peritoneal | 20 and 2.5 | 21 | 1 and 2 | -11 | 50 |

It was revealed by the concomitantly used drug efficacy evaluation test using tumor-bearing mice that Example 1 prepared as a liposome formulation exhibits an excellent antitumor effect in combined use with cisplatin. In view of the test results, Example 1 is a promising compound exhibiting an excellent antitumor effect, which is a significant effect.

Test Example 16. Concomitantly used drug efficacy evaluation test using tumor-bearing mice transplanted with ES-2 cells

Antitumor effects were evaluated using a liposome formulation encapsulating Example 1 and gemcitabine.
Gemcitabine (Gemzar Injection 200 mg, Eli Lilly Japan) dissolved in saline (Otsuka Normal Saline, Otsuka Pharmaceutical Factory) was used.
A liposome formulation was prepared by the similar method to Test Example 6. The test compound was weighed out. An empty liposome solution with a total lipid concentration of 50 mM was added so that the compound concentration would be 2 mg/mL, and 1 mol/L hydrochloric acid was added to adjust the pH to 6 to 7. Alternatively, 10 mM L-histidine buffer/10% sucrose solution (pH 6.5) and hydrochloric acid were added to the test compound to dissolve or disperse the compound, and an empty liposome solution with a total lipid concentration of 75 mM was added so that the compound concentration would be 2 mg/mL, and the pH was adjusted to 6 to 7. The solution was heated for 10 to 30 minutes in a 65°C water bath, and then cooled with ice. The solution was left standing overnight or longer in a 5°C refrigerator and then filtered with a 0.22 μm membrane filter. The liposome encapsulation ratio calculated by the method described in Test Example 6 was 99% or higher.
Ovarian cancer ES-2 cells (ATCC) were intradermally transplanted at $1 \times 10^6$ cells/mouse into the flank region in 4 to 7 week old BALB/c-nu/nu mice (CAnN.Cg-Foxn1nu/CrlCrlj, female, Charles River Laboratories Japan). After confirming engraftment of the ES-2 cells 5 to 14 days post-transplantation, a liposome formulation was administered at 4.5 mg/kg, gemcitabine was administered at 30 mg/kg, or they were administered at a combined dose, intravenously once a week, peritoneally twice a week, or concomitantly. The tumor volume was measured over time from the start of administration to evaluate the tumor volume reduction due to compound administration. The tumor volume was calculated by the following equation by using the minor axis and major axis of tumor measured with an electronic caliper (Mitutoyo).

$$\text{Tumor volume } [mm^3] = 0.5 \times (\text{minor axis } [mm])^2 \times \text{major axis } [mm]$$

A control administration group administered with only a solvent and a compound of the present disclosure administration group were compared. T/C was calculated from the following equation to evaluate the antitumor effect. The

complete tumor regression (CR) individual ratio as of the end of administration due to a liposome formulation, gemcitabine, or concomitant administration was also recorded. For the control administration group, an empty liposome solution prepared by the similar method to Test Example 6 was used.

T/C(%) = (tumor volume as of the end of administration of the compound of the present disclosure administration group - tumor volume as of the start of administration of the compound of the present disclosure administration group)/(tumor volume as of the end of administration of the control administration group - tumor volume as of the start of administration of the control administration group) × 100

Table 25 shows the T/C(%) and CR individual ratio for tumor-bearing mice transplanted with ES-2 cells at each dosage and dosing period for the test compound.

[Table 25]

| Test | Route of administration | Dosage (mg/kg) | Dosing period (days) | Schedule (times/week) | T/C (%) | CR (%) |
|---|---|---|---|---|---|---|
| Liposome formulation of Example 1 | Intravenous | 4.5 | 14 | 1 | 58 | 0 |
| Gemcitabine | Peritoneal | 30 | 14 | 2 | 9 | 0 |
| Liposome formulation of Example 1 and gemcitabine | Intravenous and peritoneal | 4.5 and 30 | 14 | 1 and 2 | -15 | 100 |

It was revealed by the concomitantly used drug efficacy evaluation test using tumor-bearing mice that Example 1 prepared as a liposome formulation exhibits an excellent antitumor effect in combined use with gemcitabine. In view of the test results, Example 1 is a promising compound exhibiting an excellent antitumor effect, which is a significant effect.

Test Example 17. Measurement of neutrophil count using tumor-bearing mice transplanted with ES-2 cells

Neutrophil counts were measured using a liposome formulation encapsulating Example 1 and gemcitabine.

Gemcitabine (Gemzar Injection 200 mg, Eli Lilly Japan) dissolved in saline (Otsuka Normal Saline, Otsuka Pharmaceutical Factory) was used.

A liposome formulation was prepared by the similar method to Test Example 6. The test compound was weighed out. An empty liposome solution with a total lipid concentration of 50 mM was added so that the compound concentration would be 2 mg/mL, and 1 mol/L hydrochloric acid was added to adjust the pH to 6 to 7. Alternatively, 10 mM L-histidine buffer/10% sucrose solution (pH 6.5) and hydrochloric acid were added to the test compound to dissolve or disperse the compound, and an empty liposome solution with a total lipid concentration of 75 mM was added so that the compound concentration would be 2 mg/mL, and the pH was adjusted to 6 to 7. The solution was heated for 10 to 30 minutes in a 65°C water bath, and then cooled with ice. The solution was left standing overnight or longer in a 5°C refrigerator and then filtered with a 0.22 μm membrane filter. The liposome encapsulation ratio calculated by the method described in Test Example 6 was 99% or higher.

Ovarian cancer ES-2 cells (ATCC) were intradermally transplanted at 1 × $10^6$ cells/mouse into the flank region in 4 to 7 week old BALB/c-nu/nu mice (CAnN.Cg-Foxn1nu/CrlCrlj, female, Charles River Laboratories Japan). After confirming engraftment of the ES-2 cells 5 to 14 days post-transplantation, a liposome formulation was administered at 4.5 mg/kg, gemcitabine was administered at 30 mg/kg, or they were administered at a combined dose, intravenously once a week, peritoneally twice a week, or concomitantly. Blood was collected 96 hours after the final administration to measure the neutrophil count. For comparison in this test, an empty liposome solution prepared by the similar method to Test Example 6 was used.

A control administration group administered with an empty liposome solution was compared with each of administration groups administered with Example 1 prepared as a liposome formulation, gemcitabine, and a combination thereof. The ratio of residual neutrophils was calculated from the following equation to evaluate the safety of each formulation.

Ratio of residual neutrophils (%) = (neutrophil count of administration group 96 hours after administration)/(neutrophil count of control administration group 96 hours after administration) × 100

Table 26 shows the ratio of residual neutrophils for tumor-bearing mice transplanted with ES-2 cells.

[Table 26]

| Test | Dosage (mg/kg) | Dosing period (days) | Schedule (times/week) | Ratio of residual neutrophils (%) |
|---|---|---|---|---|
| Empty liposome | - | 14 | 1 | - |
| Liposome formulation of Example 1 | 4.5 | 14 | 1 | 87 |
| Gemcitabine | 30 | 14 | 2 | 39 |
| Liposome formulation of Example 1 and gemcitabine | 4.5 and 30 | 14 | 1 and 2 | 38 |

The ratio of residual neutrophils for Example 1 prepared as a liposome formulation is 87% at 4.5 mg/kg. As shown in Test Example 17, Example 1 prepared as a liposome formulation exhibits an antitumor effect while not exhibiting hemotoxic side effects. Furthermore, Example 1 prepared as a liposome formulation in concomitant administration with gemcitabine exhibits a high ratio of residual neutrophils for which tumor regression is observed as shown in Test Example 17, without exacerbating a hemotoxic side effect due to gemcitabine. In view of the above, Example 1 prepared as a liposome formulation is an excellent compound having a significant effect, with both high safety and efficacy.

Test Example 18. Drug efficacy evaluation test regarding the order of concomitant administration using tumor-bearing mice transplanted with ES-2 cells

Antitumor effects were evaluated using a liposome formulation encapsulating Example 1 and gemcitabine.
Gemcitabine (Gemzar Injection 200 mg, Eli Lilly Japan) dissolved in saline (Otsuka Normal Saline, Otsuka Pharmaceutical Factory) was used.
A liposome formulation encapsulating Example 1 was prepared as follows.
11.08 g of Presome ACD-1 (Nippon Fine Chemical Co., Ltd.) and 200 mL of 250 mM ammonium sulfate solution were mixed, and the resulting mixture was dispersed with a homogenizer (ULTRA-TURRAX, IKA) to obtain a crude liposome dispersion. The crude liposome dispersion was further dispersed with a high pressure homogenizer (Nano-Mizer NM2, Yoshida Kikai) at a pressure of 100 MPa to obtain a liposome with a mean particle size (Z-average) of 81 nm. A dialysis cassette (Slide-A-Lyzer G2 Dialysis Cassettes 20K MWCO, Thermo Scientific) was used to replace the outer aqueous phase of liposome with 10 mM L-histidine buffer/9.4% sucrose solution (pH 6.5). The mixture was filtered through a 0.22 μm membrane filter to obtain an empty liposome solution with a total lipid concentration of 53.1 mg/mL. 60 mg of the compound of Example 1, 9.16 mL of 10 mM L-histidine buffer/9.4% sucrose solution (pH6.5), and 20.8 mL of the empty liposome solution were added, and the pH was adjusted to 6.5. The solution was heated for 30 minutes in a 50°C water bath and then cooled with ice. The mixture was filtered through a 0.22 μm membrane filter. The concentration of the compound of Example 1 in the liposome solution was 2.08 mg/mL, and the encapsulation ratio was 98%.
The compound concentration in liposome solution and encapsulation ratio were calculated as follows.
Unencapsulated compound concentration: 100 μL of liposome solution, 100 μL of aqueous 4% phosphoric acid solution, and 300 μL of saline were mixed. The mixture was centrifuged for 60 minutes at 100,000× g to remove insolubles. The compound concentration in the supernatant was measured by HPLC. Compound concentration in liposome solution: The liposome solution was diluted with a trifluoroacetic acid/water/methanol mixture (0.1/25/75) and centrifuged at 4°C for 10 minutes at 15,000× g to remove the insolubles. The compound concentration in the supernatant was measured by HPLC.
HPLC measurement conditions are the following.

HPLC conditions

Column: Acquity UPLC BEH C18, 1.7 μm, 50 × 2.1 mm
Column temperature: 40°C
Mobile phase: A: 0.1% trifluoroacetic acid containing water B: acetonitrile
A/B (min): 95/5 (0) → 0/100 (3.5) → 0/100 (4) → 95/5 (4.01) → 95/5 (5)
Flow rate: 0.8 mL/min
Detection: UV visible detector, measured wavelength 254 nm

Injected volume: 5 μL

The encapsulation ratio was calculated by the following equation.

Encapsulation ratio (%) = (compound concentration in liposome solution - unencapsulated compound concentration) × 100/compound concentration in liposome solution

Ovarian cancer ES-2 cells (ATCC) were intradermally transplanted at $1 \times 10^6$ cells/mouse into the flank region in 4 to 7 week old BALB/c-nu/nu mice (CAnN.Cg-Foxn1nu/CrlCrlj, female, Charles River Laboratories Japan). After confirming engraftment of the ES-2 cells 5 to 14 days post-transplantation, a liposome formulation was administered at 4.5 mg/kg and gemcitabine was administered at 30 mg/kg, intravenously once or peritoneally once. The tumor volume was measured over time from the start of administration to evaluate the tumor volume reduction due to compound administration. The tumor volume was calculated by the following equation by using the minor axis and major axis of tumor measured with an electronic caliper (Mitutoyo).

$$\text{Tumor volume } [mm^3] = 0.5 \times (\text{minor axis } [mm])^2 \times \text{major axis } [mm]$$

A control administration group administered with only a solvent and a compound of the present disclosure administration group were compared. T/C was calculated from the following equation to evaluate the antitumor effect. The complete tumor regression (CR) individual ratio as of the end of administration due to liposome formulation or gemcitabine or concomitant administration was also recorded. For the control administration group, an empty liposome solution was used.

T/C(%) = (tumor volume as of the end of administration of the compound of the present disclosure administration group - tumor volume as of the start of administration of the compound of the present disclosure administration group)/(tumor volume as of the end of administration of the control administration group - tumor volume as of the start of administration of the control administration group) × 100

Table 27 shows the T/C(%) and CR individual ratio for tumor-bearing mice transplanted with ES-2 cells at each dosage and dosing period of the test compound.

[Table 27]

| Test | T/C (%) |
|---|---|
| Liposome formulation of Example 1 (Day1) and gemcitabine (Day1) | 22 |
| Liposome formulation of Example 1 (Day2) and gemcitabine (Day1) | 7 |
| Liposome formulation of Example 1 (Day5) and gemcitabine (Day1) | 66 |
| Liposome formulation of Example 1 (Day1) and gemcitabine (Day2) | 21 |
| Liposome formulation of Example 1 (Day1) and gemcitabine (Day5) | 34 |

It was revealed by the concomitantly used drug efficacy evaluation test using tumor-bearing mice that Example 1 prepared as a liposome formulation exhibits an excellent antitumor effect in combined use with gemcitabine. As shown in Test Example 18, an antitumor effect through combined use of Example 1 prepared as a liposome formulation and gemcitabine has the most potent effect from the order of administering a liposome formulation of Example 1 on the day after administration of gemcitabine, and next most potent effect was exhibited from simultaneous administration or administration of gemcitabine on the day after administration of a liposome formulation of Example 1. In view of the test results, Example 1 is a promising compound exhibiting an excellent antitumor effect, which is a significant effect.

Test Example 19. Concomitantly used drug efficacy evaluation test using tumor-bearing mice transplanted with MC38 cells

Antitumor effects were evaluated using a liposome formulation encapsulating Example 1 and an anti-PD-1 antibody.

An anti-PD-1 antibody (114116, Biolegend) diluted with PBS (Invitrogen) was used.

A liposome formulation was prepared by the similar method to Test Example 6. The test compound was weighed

out. An empty liposome solution with a total lipid concentration of 50 mM was added so that the compound concentration would be 2 mg/mL, and 1 mol/L hydrochloric acid was added to adjust the pH to 6 to 7. Alternatively, 10 mM L-histidine buffer/10% sucrose solution (pH 6.5) and hydrochloric acid were added to the test compound to dissolve or disperse the compound, and an empty liposome solution with a total lipid concentration of 75 mM was added so that the compound concentration would be 2 mg/mL, and the pH was adjusted to 6 to 7. The solution was heated for 10 to 30 minutes in a 65°C water bath, and then cooled with ice. The solution was left standing overnight or longer in a 5°C refrigerator and then filtered with a 0.22 μm membrane filter. The liposome encapsulation ratio calculated by the method described in Test Example 6 was 99% or higher.

Mouse colon cancer MC38 cells (Kerafast) were intradermally transplanted at $5 \times 10^5$ cells/mouse into the flank region in 4 to 7 week old c57BL/6J mice (C57BL/6J, female, Charles River Laboratories Japan). After confirming engraftment of the MC38 cells 5 to 14 days post-transplantation, a liposome formulation was administered at 10 mg/kg, anti-PD-1 antibody was administered at 200 μg/head they were administered at a combined dose, intravenously once a week, peritoneally twice a week, or concomitantly. The tumor volume was measured over time from the start of administration to evaluate the tumor volume reduction due to compound administration. The tumor volume was calculated by the following equation by using the minor axis and major axis of tumor measured with an electronic caliper (Mitutoyo).

Tumor volume [$mm^3$] = $0.5 \times$ (minor axis [mm])$^2$ $\times$ major axis [mm]

A control administration group administered with only a solvent and a compound of the present disclosure administration group were compared. T/C was calculated from the following equation to evaluate the antitumor effect. The complete tumor regression (CR) individual ratio as of the end of administration due to liposome formulation or anti-PD-1 antibody or concomitant administration was also recorded. For the control administration group, an empty liposome solution prepared by the similar method to Test Example 6 was used.

T/C(%) = (tumor volume as of the end of administration of the compound of the present disclosure administration group - tumor volume as of the start of administration of the compound of the present disclosure administration group)/(tumor volume as of the end of administration of the control administration group - tumor volume as of the start of administration of the control administration group) $\times$ 100

Table 28 shows the T/C(%) and CR individual ratio for tumor-bearing mice transplanted with MC38 cells at each dosage and dosing period for the test compound.

[Table 28]

| Test | Route of administration | Dosage | Dosing period (days) | Schedule (times/week) | T/C (%) | CR (%) |
|---|---|---|---|---|---|---|
| Liposome formulation of Example 1 | Intravenous | 10mg/kg | 14 | 1 | 43 | 0 |
| Anti-PD-1 antibody | Peritoneal | 200μg/head | 14 | 2 | 23 | 0 |
| Liposome formulation of Example 1 and anti-PD-1 antibody | Intravenous and peritoneal | 10mg/kg and 200μg/head | 14 | 1 and 2 | 5 | 60 |

It was revealed by the concomitantly used drug efficacy evaluation test using tumor-bearing mice that Example 1 prepared as a liposome formulation exhibits an excellent antitumor effect in combined use with an anti-PD-1 antibody. As shown in Test Example 19, tumor-bearing mice transplanted with MC38 cells are models in which an anti-PD-1 antibody exhibits an antitumor effect. In view of the test results, Example 1 is a promising compound exhibiting an excellent antitumor effect on a model in which an anti-PD-1 antibody exhibits an antitumor effect, which is a significant effect.

Test Example 20. Concomitantly used drug efficacy evaluation test using tumor-bearing mice transplanted with EMT6 cells

Antitumor effects were evaluated using a liposome formulation encapsulating Example 1 and an anti-PD-1 antibody.

An anti-PD-1 antibody (114116, Biolegend) diluted with PBS (Invitrogen) was used.

A liposome formulation encapsulating Example 1 was prepared as follows.

11.08 g of Presome ACD-1 (Nippon Fine Chemical Co., Ltd.) and 200 mL of 250 mM ammonium sulfate solution

were mixed, and the resulting mixture was dispersed with a homogenizer (ULTRA-TURRAX, IKA) to obtain a crude liposome dispersion. The crude liposome dispersion was further dispersed with a high pressure homogenizer (Nano-Mizer NM2, Yoshida Kikai) at a pressure of 100 MPa to obtain a liposome with a mean particle size (Z-average) of 81 nm. A dialysis cassette (Slide-A-Lyzer G2 Dialysis Cassettes 20K MWCO, Thermo Scientific) was used to replace the outer aqueous phase of liposome with 10 mM L-histidine buffer/9.4% sucrose solution (pH 6.5). The mixture was filtered through a 0.22 μm membrane filter to obtain an empty liposome solution with a total lipid concentration of 53.1 mg/mL. 60 mg of the compound of Example 1, 9.16 mL of 10 mM L-histidine buffer/9.4% sucrose solution (pH6.5), and 20.8 mL of the empty liposome solution were added, and the pH was adjusted to 6.5. The solution was heated for 30 minutes in a 50°C water bath and then cooled with ice. The mixture was filtered through a 0.22 μm membrane filter. The concentration of the compound of Example 1 in the liposome solution was 2.08 mg/mL, and the encapsulation ratio was 98%.

The compound concentration in liposome solution and encapsulation ratio were calculated as follows. Unencapsulated compound concentration: 100 μL of liposome solution, 100 μL of aqueous 4% phosphoric acid solution, and 300 μL of saline were mixed. The mixture was centrifuged for 60 minutes at 100,000× g to remove insolubles. The compound concentration in the supernatant was measured by HPLC. Compound concentration in liposome solution: The liposome solution was diluted with a trifluoroacetic acid/water/methanol mixture (0.1/25/75) and centrifuged at 4°C for 10 minutes at 15,000× g to remove the insolubles. The compound concentration in the supernatant was measured by HPLC.

HPLC measurement conditions are the following.

HPLC conditions

Column: Acquity UPLC BEH C18, 1.7 μm, 50 × 2.1 mm
Column temperature: 40°C
Mobile phase: A: 0.1% trifluoroacetic acid containing water B: acetonitrile
A/B (min): 95/5 (0) → 0/100 (3.5) → 0/100 (4) → 95/5 (4.01) → 95/5 (5)
Flow rate: 0.8 mL/min
Detection: UV visible detector, measured wavelength 254 nm
Injected volume: 5 μL

The encapsulation ratio was calculated by the following equation.

Encapsulation ratio (%) = (compound concentration in liposome solution - unencapsulated compound concentration) × 100/compound concentration in liposome solution

Mouse breast cancer EMT6 cells (ATCC) were intradermally transplanted at $5 \times 10^5$ cells/mouse into the flank region in 4 to 7 week old BALB/c mice (BALB/cAnNCrlCrlj, female, Charles River Laboratories Japan). After confirming engraftment of the EMT6 cells 5 to 14 days post-transplantation, a liposome formulation was administered at 10 mg/kg, anti-PD-1 antibody was administered at 200 μg/head they were administered at a combined dose, intravenously once a week, peritoneally twice a week, or concomitantly. The tumor volume was measured over time from the start of administration to evaluate the tumor volume reduction due to compound administration. The tumor volume was calculated by the following equation by using the minor axis and major axis of tumor measured with an electronic caliper (Mitutoyo).

$$\text{Tumor volume [mm}^3\text{]} = 0.5 \times (\text{minor axis [mm]})^2 \times \text{major axis [mm]}$$

A control administration group administered with only a solvent and a compound of the present disclosure administration group were compared. T/C was calculated from the following equation to evaluate the antitumor effect. The complete tumor regression (CR) individual ratio as of the end of administration due to liposome formulation or anti-PD-1 antibody or concomitant administration was also recorded. For the control administration group, an empty liposome solution was used.

T/C(%) = (tumor volume as of the end of administration of the compound of the present disclosure administration group - tumor volume as of the start of administration of the compound of the present disclosure administration group)/(tumor volume as of the end of administration of the control administration group - tumor volume as of the start of administration of the control administration group) × 100

Table 29 shows the T/C(%) and CR individual ratio for tumor-bearing mice transplanted with EMT6 cells at each

dosage and dosing period for the test compound.

[Table 29]

| Test | Route of administration | Dosage | Dosing period (days) | Schedule (times/week) | T/C (%) | CR (%) |
|---|---|---|---|---|---|---|
| Liposome formulation of Example 1 | Intravenous | 10mg/kg | 14 | 1 | 36 | 20 |
| Anti-PD-1 antibody | Peritoneal | 200μg/head | 14 | 2 | 102 | 20 |
| Liposome formulation of Example 1 and anti-PD-1 antibody | Intravenous and peritoneal | 10mg/kg and 200μg/head | 14 | 1 and 2 | -6 | 100 |

It was revealed by the concomitantly used drug efficacy evaluation test using tumor-bearing mice that Example 1 prepared as a liposome formulation exhibits an excellent antitumor effect in combined use with an anti-PD-1 antibody. As shown in Test Example 20, tumor-bearing mice transplanted with EMT6 cells are models in which an anti-PD-1 antibody exhibits an antitumor effect. In view of the test results, Example 1 is a promising compound exhibiting an excellent antitumor effect in combined use of Example 1 prepared as a liposome formulation and anti-PD-1 antibody on a model in which an anti-PD-1 antibody does not exhibit an antitumor effect, which is a significant effect.

Test Example 21. Drug efficacy evaluation test regarding the order of concomitant administration using tumor-bearing mice transplanted with EMT6 cells

Antitumor effects were evaluated using a liposome formulation encapsulating Example 1 and an anti-PD-1 antibody.

An anti-PD-1 antibody (114116, Biolegend) diluted with PBS (Invitrogen) was used.

A liposome formulation encapsulating Example 1 was prepared as follows.

498.2 g of Presome ACD-1 (Nippon Fine Chemical Co., Ltd.) was weighed out, 8772 g of 250 mM ammonium sulfate solution was added, and the resulting mixture was dispersed with a homogenizer (CLEARMIX, M TECHNIQUE) and degassed by vacuum degassing to obtain a crude liposome dispersion. The crude liposome dispersion was further dispersed with a high pressure homogenizer (Microfluidizer M-110 EH-30, Microfluidics) at a pressure of 14500 Psi to obtain a liposome with a mean particle size (Z-average) of 73 nm. Tangential Flow Filtration system equipped with five Pellicon cassettes (Pellicon 2 Mini 300kD 0.1m2, Merck) was used to replace the outer aqueous phase of liposome with water for injection, and L-histidine and sucrose were added to make a 10 mM L-histidine/9.4% sucrose solution, and the pH was adjusted to 6.5 with 1 mol/L hydrochloric acid. The mixture was then filtered through a 0.2 μm membrane filter to obtain an empty liposome solution with a total lipid concentration of 50.2 mg/mL.

10.0 g of the compound of Example 1 was weighed out, 1354 g of 10 mM L-histidine buffer/9.4% sucrose solution (pH6.5), 27 g of 1 mol/L hydrochloric acid, and 3786 g of the empty liposome solution were added, and the pH was adjusted to 6.5 with 1 mol/L hydrochloric acid. The mixture was heated at 45°C for 60 minutes and then cooled, and the pH was adjusted to 6.5. The mixture was then filtered through a 0.2 μm membrane filter. The solution was dispensed in 50 mL portions into glass vials, and the vials were stoppered and wind-tightened. The concentration of the compound of Example 1 in the liposome solution was 2.01 mg/mL, the encapsulation ratio was 97.5%, and the average particle size (Z-average) was 74 nm.

The compound concentration in liposome solution and encapsulation ratio were calculated as follows. Unencapsulated compound concentration: 100 μL of liposome solution, 100 μL of aqueous 4% phosphoric acid solution, and 300 μL of saline were mixed. The mixture was centrifuged for 60 minutes at 100,000× g to remove insolubles. The compound concentration in the supernatant was measured by HPLC. Compound concentration in liposome solution: The liposome solution was diluted with a trifluoroacetic acid/water/methanol mixture (0.1/25/75) and centrifuged at 4°C for 10 minutes at 15,000× g to remove the insolubles. The compound concentration in the supernatant was measured by HPLC.

HPLC measurement conditions are the following.

HPLC conditions

Column: Acquity UPLC BEH C18, 1.7 μm, 50 × 2.1 mm

Column temperature: 40°C
Mobile phase: A: 0.1% trifluoroacetic acid containing water B: acetonitrile
A/B (min): 95/5 (0) → 60/40 (3) → 0/100 (3.5) → 0/100 (4) → 95/5 (4.01) → 95/5 (5)
Flow rate: 0.8 mL/min
Detection: UV visible detector, measured wavelength 254 nm
Injected volume: 5 μL

The encapsulation ratio was calculated by the following equation.

Encapsulation ratio (%) = (compound concentration in liposome solution - unencapsulated compound concentration) × 100/compound concentration in liposome solution

Mouse breast cancer EMT6 cells (ATCC) were intradermally transplanted at $5 \times 10^5$ cells/mouse into the flank region in 5 week old BALB/c mice (BALB/cAnNCrlCrlj, female, Charles River Laboratories Japan). After confirming engraftment of the EMT6 cells 5 days post-transplantation, a liposome formulation was administered at 6 mg/kg and anti-PD-1 antibody was administered at 200 μg/head intravenously once a week and peritoneally once a week. The tumor volume was measured over time from the start of administration to evaluate the tumor volume reduction due to compound administration. The tumor volume was calculated by the following equation by using the minor axis and major axis of tumor measured with an electronic caliper (Mitutoyo).

$$\text{Tumor volume } [mm^3] = 0.5 \times (\text{minor axis } [mm])^2 \times \text{major axis } [mm]$$

A control administration group administered with only a solvent and a compound of the present disclosure administration group were compared. T/C was calculated from the following equation to evaluate the antitumor effect. The complete tumor regression (CR) individual ratio as of the end of administration due to a liposome formulation or anti-PD-1 antibody was also recorded. For the control administration group, an empty liposome solution was used.

T/C(%) = (tumor volume as of the end of administration of the --> compound of the present disclosure administration group - tumor volume as of the start of administration of the compound of the present disclosure administration group)/(tumor volume as of the end of administration of the control administration group - tumor volume as of the start of administration of the control administration group) × 100

Table 30 shows the T/C(%) and CR individual ratio for tumor-bearing mice transplanted with EMT6 cells at each dosage and dosing period of the test compound.

[Table 30]

| Test | T/C (%) | CR (%) |
|---|---|---|
| Liposome formulation of Example 1 (Days 1 and 8 and 15) and anti-PD-1 antibody (Days 1 and 8 and 15) | 19 | 40 |
| Liposome formulation of Example 1 (Days 1 and 8 and 15) and anti-PD-1 antibody (Days 4 and 11 and 18) | 33 | 0 |
| Liposome formulation of Example 1 (Days 4 and 11 and 18) and anti-PD-1 antibody (Days 1 and 8 and 15) | 46 | 0 |

It was revealed by the concomitantly used drug efficacy evaluation test using tumor-bearing mice that Example 1 prepared as a liposome formulation exhibits an excellent antitumor effect in combined use with an anti-PD-1 antibody. As shown in Test Example 21, an antitumor effect from combined use of Example 1 prepared as a liposome formulation and anti-PD-1 antibody exhibits the most potent effect with simultaneous administration and next most potent effect was exhibited from the order of concomitant administration of administering an anti-PD-1 antibody after administration of a liposome formulation of Example 1. In view of the test results, Example 1 is a promising compound exhibiting an excellent antitumor effect, which is a significant effect.

Test Example 22. Kinase selectivity test

The selectivity for 370 wild-type kinases of prexasertib, Example 1, and Example 3 was evaluated by HotSpot Full Panel Kinase profiling (Reaction Biology).

Buffer: 20 mM HEPES, pH 7.5, 10 mM $MgCl_2$, 2 mM $MnCl_2$ (as needed), 1 mM EGTA, 0.02% Brij35, 0.02 mg/ mL BSA, 0.1 mM $Na_3VO_4$, 2 mM DTT, 1% DMSO
ATP concentration: 10 μm
Reaction time: 2 hours
ATP conversion rate in 2 hours: 5 to 20%
Compound concentration: 100 nM
Compound concentration: 100 nM

The names of the kinase and residual activity (%) at 100 nM are shown in Table 31 to Table 33 for 370 wild-type kinases with residual activity at 100 nM of less than 25% for prexasertib, Example 1, and Example 3.

[Table 31]

| Kinase | Residual activity (%) after adding prexasertib (100 nM) |
|---|---|
| RSK2 | 0.98 |
| RSK3 | 1.69 |
| CHK1 | 1.97 |
| RSK1 | 2.23 |
| CAMK2A | 2.52 |
| SIK2 | 2.62 |
| RSK4 | 4.78 |
| CAMK2D | 6.07 |
| LOK/STK10 | 6.15 |
| CHK2 | 6.41 |
| RET | 7.12 |
| TNIK | 7.76 |
| MARK4 | 10.22 |
| MEK5 | 10.72 |
| MELK | 11.40 |
| SIK1 | 11.93 |
| MARK3 | 12.64 |
| TRKC | 12.67 |
| MARK1 | 14.10 |
| MARK2/PAR-1BA | 14.26 |
| BRSK2 | 14.44 |
| ARK5/NUAK1 | 17.86 |
| CDK8/CYCLIN C | 18.06 |
| CDK19/CYCLIN C | 19.87 |
| GLK/MAP4K3 | 20.81 |
| BRSK1 | 21.69 |
| LRRK2 | 23.54 |

[Table 32]

| Kinase | Residual activity (%) after adding Example 1 (100 nM) |
|---|---|
| CHK1 | 2.87 |
| RSK3 | 3.91 |
| RSK2 | 4.32 |
| RSK1 | 8.51 |
| RSK4 | 9.05 |
| CAMK2A | 13.90 |
| SIK2 | 14.12 |
| MELK | 17.18 |
| TRKC | 17.43 |
| TNIK | 17.81 |
| RET | 19.04 |
| MARK4 | 24.10 |

[Table 33]

| Kinase | Residual activity (%) after adding Example 3 (100 nM) |
|---|---|
| RSK2 | 2.22 |
| CHK1 | 2.24 |
| RSK3 | 3.13 |
| RSK1 | 4.74 |
| SIK2 | 4.85 |
| RSK4 | 6.11 |
| CAMK2A | 7.18 |
| MELK | 11.28 |
| MARK4 | 11.52 |
| MARK2/PAR-1BA | 13.37 |
| MARK1 | 13.99 |
| MARK3 | 14.18 |
| TNIK | 15.11 |
| CAMK2D | 15.60 |
| BRSK2 | 17.03 |
| TRKC | 19.27 |
| CHK2 | 19.41 |
| RET | 20.57 |
| LOK/STK10 | 22.15 |
| ARK5/NUAK1 | 22.68 |
| CDK8/CYCLIN C | 23.71 |
| BRSK1 | 24.80 |

As shown in the above table, the compounds of Examples 1 and 3 exhibited higher CHK1 selectivity than prexasertib. In particular, Example 1 has a significant effect of selectively inhibiting CHK1 activity, so that an excellent

antitumor effect due to CHK1 inhibition was exhibited while avoiding expression of toxicity due to non-selective kinase inhibition.

Test Example 23. CHK1/CHK2 selectivity test

A test compound was dissolved in dimethylsulfoxide (DMSO) and further diluted with DMSO to prepare a solution with a concentration that is 100-fold of the test concentration. The solution was further diluted 25-fold with an assay buffer as a test compound solution. A positive control was similarly processed to prepare a positive control solution. A 4-fold concentration test compound solution was prepared with an assay buffer (20 mM HEPES, 0.01% Triton X-100, 1 mM DTT, pH 7.5). A 4-fold concentration substrate/ATP/metal solution was prepared with a kit buffer (20 mM HEPES, 0.01% Triton X-100, 5 mM DTT, pH 7.5). A 2-fold concentration kinase solution was prepared with an assay buffer. 5 μL of the 4-fold concentration test compound solution, 5 μL of the 4-fold concentration substrate/ATP/metal solution, and 10 μL of the 2-fold concentration kinase solution were mixed in a well of a 384-well polypropylene plate and reacted for 1 hour at room temperature. 70 μL of Termination Buffer (QuickScout Screening Assist MSA; Carna Biosciences) was added to terminate the reaction. Substrate peptides and phosphorylated peptides in the reaction solution were separated and quantified using a LabChip™ system (Perkin Elmer). The kinase reaction was evaluated by the product ratio (P/(P+S)) calculated from the substrate peptide peak height (S) and phosphorylated peptide peak height (P). The inhibition ratio was calculated from the mean signal of each test compound test well, with the mean signal of a control well comprising all reaction components as 0% inhibition, and the mean signal of a background well (with no enzyme added) as 100% inhibition. The $IC_{50}$ value was found by approximating a plot of test substance concentration and inhibition ratio to a four parameter logistic curve by a nonlinear least square method.

Table 34 shows the $IC_{50}$ value with respect to CHK1 and CHK2 for prexasertib and Example 1.

[Table 34]

| Kinase | Prexasertib | Example 1 |
|---|---|---|
| CHK1 | $< 3.0 \times 10^{-10}$ M | $< 3.0 \times 10^{-10}$ M |
| CHK2 | $1.03 \times 10^{-8}$ M | $5.66 \times 10^{-8}$ M |

As shown in the above table, the compound of Example 1 exhibited higher CHK1 selectivity compared to prexasertib. Therefore, Example 1 exhibited an excellent antitumor effect due to CHK1 inhibition while avoiding expression of toxicity due to CHK2 inhibition.

Test Example 24. Intratumoral immune cell evaluation test using tumor-bearing mice transplanted with EMT6 cells

The fractionation of immune cells infiltrating into the tumor was evaluated using a liposome formulation encapsulating Example 1 and an anti-PD-1 antibody.

An anti-PD-1 antibody (114116, Biolegend) diluted with PBS (Invitrogen) was used.

A liposome formulation encapsulating Example 1 was prepared as follows.

138.4g of Presome ACD-1 (Nippon Fine Chemical Co., Ltd.) was weighed out, 2437g of 250 mM ammonium sulfate solution was added, and the resulting mixture was dispersed with a homogenizer (CLEARMIX, M TECHNIQUE) and degassed by vacuum degassing to obtain a crude liposome dispersion. The crude liposome dispersion was further dispersed with a high pressure homogenizer (Microfluidizer M-110 EH-30, Microfluidics) at a pressure of 14500 Psi to obtain a liposome with a mean particle size (Z-average) of 73 nm. Tangential Flow Filtration system equipped with five Pellicon cassettes (Pellicon 2 Mini 300kD 0.1m2, Merck) was used to replace the outer aqueous phase of liposome with 10 mM L-histidine buffer/9.4% sucrose solution (pH 6.5). The mixture was filtered through a 0.2 μm membrane filter to obtain an empty liposome solution with a total lipid concentration of 58.2 mg/mL. 3.0 g of the compound of Example 1 was weighed out, 562 g of 10 mM L-histidine buffer/9.4% sucrose solution (pH6.5), and 980 g of the empty liposome solution were added, and the pH was adjusted to 6.5. The mixture was heated at 45°C for 60 minutes and then cooled. Tangential Flow Filtration system equipped with five Pellicon cassettes (Pellicon 2 Mini 300kD 0.1m2, Merck) was used to replace the outer aqueous phase of liposome with 10 mM L-histidine buffer/9.4% sucrose solution (pH 6.5). The mixture was filtered through a 0.2 μm membrane filter. The solution was dispensed in 50 mL portions into glass vials, and the vials were stoppered and wind-tightened. The concentration of the compound of Example 1 in the liposome solution was 1.98 mg/mL, and the encapsulation ratio was 97%.

The compound concentration in liposome solution and encapsulation ratio were calculated as follows.

Unencapsulated compound concentration: 100 μL of liposome solution, 100 μL of aqueous 4% phosphoric acid

solution, and 300 μL of saline were mixed. The mixture was centrifuged for 60 minutes at 100,000× g to remove insolubles. The compound concentration in the supernatant was measured by HPLC.

Compound concentration in liposome solution: The liposome solution was diluted with a trifluoroacetic acid/water/-methanol mixture (0.1/25/75) and centrifuged at 4°C for 10 minutes at 15,000× g to remove the insolubles. The compound concentration in the supernatant was measured by HPLC.

HPLC measurement conditions are the following.

HPLC conditions

Column: Acquity UPLC BEH C18, 1.7 μm, 50 × 2.1 mm
Column temperature: 40°C
Mobile phase: A: 0.1% trifluoroacetic acid containing water B: acetonitrile
A/B (min): 95/5 (0) → 60/40 (3) → 0/100 (3.5) → 0/100 (4) → 95/5 (4.01) → 95/5 (5)
Flow rate: 0.8 mL/min
Detection: UV visible detector, measured wavelength 254 nm
Injected volume: 5 μL

The encapsulation ratio was calculated by the following equation.

Encapsulation ratio (%) = (compound concentration in liposome solution - unencapsulated compound concentration) × 100/compound concentration in liposome solution

Mouse breast cancer EMT6 cells (ATCC) were intradermally transplanted at $5 \times 10^5$ cells/mouse into the flank region in 4 to 7 week old BALB/c mice (BALB/cAnNCrlCrlj, female, Charles River Laboratories Japan). After confirming engraftment of the EMT6 cells 5 to 14 days post-transplantation, a liposome formulation was administered at 10 mg/kg, anti-PD-1 antibody was administered at 200 μg/head, or they were administered at a combined dose, intravenously once a week, peritoneally twice a week, or concomitantly. 10 days after the start of administration, the tumor was harvested from each of four individuals in each group, weighed out, and then pooled to one sample for each group (N = 4). The tumor mass of EMT6 cells was finely cut with scissors, and treated with gentle MACS Tissue Dissociators (Miltenyi Biotec) according to the protocol using Tumor Dissociation Kit, Mouse (Miltenyi Biotec), and the tumor mass was prepared into a cell suspension.

The prepared cells were passed through a 70 μm cell strainer and then washed with RPMI1640 containing 10% FBS. Thereafter, the cells were treated with a red blood cell lysate (Lonza) for 1 minute to remove red blood cells, further washed with RPMI1640 containing 10% FCS, and suspended in MACS Rinsing solution containing 5% BSA (Miltnyi Biotec, hereinafter, referred to as MACS buffer). The cell suspension was counted using LUNA FL (Logos Biosystems), and 1 x $10^6$ cells were stained using the following method and antibodies.

The cell suspension was blocked by adding Mouse BD Fc BlockTM (BD Biosciences), and then, staining was performed with BV510-labeled anti-mouse CD8a (Biolegend), BV711-labeled anti-mouse CD3 (Biolegend), FITC-labeled anti-mouse f4/80 (Biolegend), PE-labeled anti-mouse IAd (BD Biosciences), PerCP-labeled anti-mouse CD45 (Biolegend), PE-Cy7-labeled anti-mouse CD11b (Thermo Fisher Scientific Inc.), APC-labeled anti-mouse CD206 (Biolegend), and APC-Cy7-labeled anti-mouse CD4 (BD Biosciences) on ice for 20 minutes. After washing once with MACS buffer, Hoechst33342 (Thermo Fisher Scientific Inc.) diluted with PBS was added, and a reaction was allowed to proceed for 5 minutes on ice to stain dead cells. After staining twice with MACS buffer, the cells were fixed with 1% PFA/PBS and analyzed using a flow cytometer.

The percentage of CD45 + CD3 + Tcells (CD3 positive Tcells), CD45 + CD3 + CD8 + Tcells (CD8 positive Tcells), CD45 + CD11b + f4/80 + IAd low CD206 high cells (M2 macrophages), and CD45 + CD11b + f4/80 + IAd high CD206 low cells (M1 macrophages) in living cells was multiplied by the number of living cells counted by LUNA FL (Logos Biosystems) to calculate the number of cells, and then, the number of cells was divided by the tumor weight to calculate the number of cells per weight in each fraction.

Table 35 shows the number of CD3 positive Tcells and the number of CD8 positive Tcells, and Table 36 shows the number of 1 macrophages and the number of M2 macrophages, in the empty liposome, liposome formulation of Example 1, anti-PD-1 antibody and combined use groups.

[Table 35]

| Test | Route of administration | Dosage | Dosing period (days) | Schedule (times/week) | CD3 positive T cell number (cells/g) | CD8 positive T cell number (cells/g) |
|---|---|---|---|---|---|---|
| Empty liposome | Intravenous | -- | 10 | 1 | 93239 | 6480 |
| Liposome formulation of Example 1 | Intravenous | 10mg/kg | 10 | 1 | 361191 | 73321 |
| Anti-PD-1 antibody | Peritoneal | 200μg/head | 10 | 2 | 106370 | 5818 |
| Combined use of liposome formulation of Example 1 and anti-PD-1 antibody | Intravenous and peritoneal | 10mg/kg and 200μg/head | 10 | 1 and 2 | 244316 | 28829 |

[Table 36]

| Test | Route of administration | Dosage | Dosing period (days) | Schedule (times/week) | M2 macrophage (cells/g) | M1 macrophage number (cells/g) | M1-M2 ratio |
|---|---|---|---|---|---|---|---|
| Empty liposome | Intravenous | - | 10 | 1 | 825485 | 363318 | 0.440 |
| Liposome formulation of Example 1 | Intravenous | 10mg/kg | 10 | 1 | 390266 | 1315145 | 3.370 |
| Anti-PD-1 antibody | Peritoneal | 200μg/head | 10 | 2 | 187972 | 544623 | 2.897 |
| Combined use of liposome formulation of Example 1 and anti-PD-1 antibody | Intravenous and peritoneal | 10mg/kg and 200μg/head | 10 | 1 and 2 | 183634 | 1192282 | 6.493 |

By the administration of the liposome formulation of Example 1, CD3 positive T cells and CD8 positive T cells in the tumor increased by 3 times and 7 times or more, respectively, compared to the empty liposome administration group, indicating that the liposome formulation of Example 1 enhanced the infiltration of T cells into the tumor. By the administration of the liposome formulation of Example 1, M2 macrophages decreased and M1 macrophages increased compared to the empty liposome administration group, and the M1-M2 ratio increased by 7.7 times by the administration of the liposome formulation of Example 1 and increased by 14.8 times by the combined use with the anti-PD-1 antibody. From the above, it was shown that the liposome formulation of Example 1 changes the tumor microenvironment from an immunosuppressive environment to an inflammatory environment.

Test Example 25. Evaluation test of CD8 positive T cell fractionation in lymph nodes using tumor-bearing mice transplanted with EMT6 cells

The fractionation of CD8 positive T cells in lymph nodes was evaluated using a liposome formulation encapsulating Example 1 and an anti-PD-1 antibody.
An anti-PD-1 antibody (114116, Biolegend) diluted with PBS (Invitrogen) was used.
A liposome formulation encapsulating Example 1 was prepared as follows.

498.2 g of Presome ACD-1 (Nippon Fine Chemical Co., Ltd.) was weighed out, 8772 g of 250 mM ammonium sulfate solution was added, and the resulting mixture was dispersed with a homogenizer (CLEARMIX, M TECHNIQUE) and degassed by vacuum degassing to obtain a crude liposome dispersion. The crude liposome dispersion was further dispersed with a high pressure homogenizer (Microfluidizer M-110 EH-30, Microfluidics) at a pressure of 14500 Psi to obtain a liposome with a mean particle size (Z-average) of 73 nm. Tangential Flow Filtration system equipped with five Pellicon cassettes (Pellicon 2 Mini 300kD 0.1m2, Merck) was used to replace the outer aqueous phase of liposome with water for injection, and L-histidine and sucrose were added to make a 10 mM L-histidine/9.4% sucrose solution, and the pH was adjusted to 6.5 with 1 mol/L hydrochloric acid. The mixture was then filtered through a 0.2 μm membrane filter to obtain an empty liposome solution with a total lipid concentration of 50.2 mg/mL.

10.0 g of the compound of Example 1 was weighed out, 1354 g of 10 mM L-histidine buffer/9.4% sucrose solution (pH6.5), 27 g of 1 mol/L hydrochloric acid, and 3786 g of the empty liposome solution were added, and the pH was adjusted to 6.5 with 1 mol/L hydrochloric acid. The mixture was heated at 45°C for 60 minutes and then cooled, and the pH was adjusted to 6.5. The mixture was then filtered through a 0.2 μm membrane filter. The solution was dispensed in 50 mL portions into glass vials, and the vials were stoppered and wind-tightened. The concentration of the compound of Example 1 in the liposome solution was 2.01 mg/mL, the encapsulation ratio was 97.5%, and the average particle size (Z-average) was 74 nm.

The compound concentration in liposome solution and encapsulation ratio were calculated as follows.

Unencapsulated compound concentration: 100 μL of liposome solution, 100 μL of aqueous 4% phosphoric acid solution, and 300 μL of saline were mixed. The mixture was centrifuged for 60 minutes at 100,000× g to remove insolubles. The compound concentration in the supernatant was measured by HPLC.
Compound concentration in liposome solution: The liposome solution was diluted with a trifluoroacetic acid/water/-methanol mixture (0.1/25/75) and centrifuged at 4°C for 10 minutes at 15,000× g to remove the insolubles. The compound concentration in the supernatant was measured by HPLC.

HPLC measurement conditions are the following.

HPLC conditions

Column: Acquity UPLC BEH C18, 1.7 μm, 50 × 2.1 mm
Column temperature: 40°C
Mobile phase: A: 0.1% trifluoroacetic acid containing water B: acetonitrile
A/B (min): 95/5 (0) → 60/40 (3) → 0/100 (3.5) → 0/100 (4) → 95/5 (4.01) → 95/5 (5)
Flow rate: 0.8 mL/min
Detection: UV visible detector, measured wavelength 254 nm
Injected volume: 5 μL

The encapsulation ratio was calculated by the following equation.

Encapsulation ratio (%) = (compound concentration in liposome solution - unencapsulated compound concentration) × 100/compound concentration in liposome solution

Mouse breast cancer EMT6 cells (ATCC) were intradermally transplanted at 5 × $10^5$ cells/mouse into the flank region in 4 to 7 week old BALB/c mice (BALB/cAnNCrlCrlj, female, Charles River Laboratories Japan). After confirming engraftment of the EMT6 cells 5 to 14 days post-transplantation, a liposome formulation of Example 1 was administered at 10 mg/kg, anti-PD-1 antibody was administered at 200 μg/head, or they were administered at a combined dose, intravenously once a week, peritoneally twice a week, or concomitantly. The regional lymph nodes were harvested in HBSS buffer 10 days after the start of administration. The harvested lymph nodes were ground with a slide glass and suspended in RPMI1640 containing 10% FCS. The cell suspension was centrifugally precipitated, treated with a red blood cell lysate (Lonza) for 1 minute to remove red blood cells, further washed with RPMI1640 containing 10% FCS, and suspended in MACS Rinsing solution containing 5% BSA (Miltnyi Biotec, hereinafter, referred to as MACS buffer). The cell suspension was counted using LUNA FL (Logos Biosystems), and 5 × $10^5$ cells were stained using the following method and antibodies.

Mouse BD Fc BlockTM (BD Biosciences) and Live/Dead Fixable Dye, nearIR (Thermo Fisher Scientific Inc.) diluted with PBS were added to the cell suspension, the cell suspension was blocked on ice for 10 minutes and then

centrifuged and suspended in 100 μL of MACS buffer, and staining was performed with BV510-labeled anti-mouse CD8a (Biolegend), FITC-labeled anti-mouse CD44 (Biolegend), PE-labeled anti-mouse CD62L (BD Biosciences), and APC-labeled anti-mouse CD3 (Biolegend) antibodies on ice for 20 minutes. After washing twice with MACS buffer, the cells were fixed with 1% PFA/PBS and analyzed using a flow cytometer.

The percentage of CD45 + CD3 + CD8 + CD62L + CD44 + T cells (central memory CD8 positive T cells), CD45 + CD3 + CD8 + CD62L - CD44 + T cells (naive CD8 positive T cells), CD45 + CD3 + CD8 + CD62L + CD44 - T cells (effector memory CD8 positive T cells), and CD45 + CD3 + CD8 + CD62L - CD44 - T cells (effector CD8 positive T cells) in living cells was multiplied by the number of living cells counted using LUNA FL to calculate the number of cells, and the number of cells was shown in Table 37.

[Table 37]

| Test | Route of administration | Dosage | Dosing period (days) | Schedule (times/week) | CD62L negative CD44 positive cell number (cells/g) | CD62L negative CD44 negative cell number (cells/g) |
|---|---|---|---|---|---|---|
| Empty liposome | Intravenous | -- | 10 | 1 | 1889939 | 2643709 |
| Liposome formulation of Example 1 | Intravenous | 10mg/kg | 10 | 1 | 5115776 | 6535902 |
| Anti-PD-1 antibody | Peritoneal | 200μg/head | 10 | 2 | 5214578 | 8089262 |
| Combined use of liposome formulation of Example 1 and anti-PD-1 antibody | Intravenous and peritoneal | 10mg/kg and 200μg/head | 10 | 1 and 2 | 7328752 | 12595490 |

By the administration of the liposome formulation of Example 1, the number of CD62L negative CD44 positive cells and the number of CD62L negative CD44 negative cells in the lymph nodes increased by about 3 times compared to the empty liposome group. Furthermore, by the combined use of the liposome formulation of Example 1 and the anti-PD-1 antibody, these cells increased by 4 to 5 times compared to the empty liposome administration group. From the above, it was shown that an immune reaction centered on CD8 positive T cells was induced by administration of the liposome formulation of Example 1, and the reaction was further enhanced by combined use with the anti-PD-1 antibody.

Test Example 26. Study on improvement effect of immune checkpoint inhibitor-resistance-associated genes

The variation of immune checkpoint inhibitor-resistance-related gene was evaluated using a liposome formulation encapsulating Example 1 and an anti-PD-1 antibody.

An anti-PD-1 antibody (114116, Biolegend) diluted with PBS (Invitrogen) was used.

A liposome formulation encapsulating Example 1 was prepared as follows.

11.08 g of Presome ACD-1 (Nippon Fine Chemical Co., Ltd.) and 200 mL of 250 mM ammonium sulfate solution were mixed, and the resulting mixture was dispersed with a homogenizer (ULTRA-TURRAX, IKA) to obtain a crude liposome dispersion. The crude liposome dispersion was further dispersed with a high pressure homogenizer (Nano-Mizer NM2, Yoshida Kikai) at a pressure of 100 MPa to obtain a liposome with a mean particle size (Z-average) of 81 nm. A dialysis cassette (Slide-A-Lyzer G2 Dialysis Cassettes 20K MWCO, Thermo Scientific) was used to replace the outer aqueous phase of liposome with 10 mM L-histidine buffer/9.4% sucrose solution (pH 6.5). The mixture was filtered through a 0.22 μm membrane filter to obtain an empty liposome solution with a total lipid concentration of 53.1 mg/mL. 60 mg of the compound of Example 1, 9.16 mL of 10 mM L-histidine buffer/9.4% sucrose solution (pH6.5), and 20.8 mL of the empty liposome solution were added, and the pH was adjusted to 6.5. The solution was heated for 30 minutes in a 50°C water bath and then cooled with ice. The mixture was filtered through a 0.22 μm membrane filter. The concentration of the compound of Example 1 in the liposome solution was 2.08 mg/mL, and the encapsulation ratio was 98%.

The compound concentration in liposome solution and encapsulation ratio were calculated as follows.

Unencapsulated compound concentration: 100 μL of liposome solution, 100 μL of aqueous 4% phosphoric acid solution, and 300 μL of saline were mixed. The mixture was centrifuged for 60 minutes at 100,000× g to remove insolubles. The compound concentration in the supernatant was measured by HPLC.

Compound concentration in liposome solution: The liposome solution was diluted with a trifluoroacetic acid/water/-methanol mixture (0.1/25/75) and centrifuged at 4°C for 10 minutes at 15,000× g to remove the insolubles. The compound concentration in the supernatant was measured by HPLC.

HPLC measurement conditions are the following.

HPLC conditions

Column: Acquity UPLC BEH C18, 1.7 μm, 50 × 2.1 mm
Column temperature: 40°C
Mobile phase: A: 0.1% trifluoroacetic acid containing water B: acetonitrile
A/B (min): 95/5 (0) → 0/100 (3.5) → 0/100 (4) → 95/5 (4.01) → 95/5 (5)
Flow rate: 0.8 mL/min
Detection: UV visible detector, measured wavelength 254 nm
Injected volume: 5 μL

The encapsulation ratio was calculated by the following equation.

Encapsulation ratio (%) = (compound concentration in liposome solution - unencapsulated compound concentration) × 100/compound concentration in liposome solution

Mouse breast cancer EMT6 cells (ATCC) were intradermally transplanted at 5 × $10^5$ cells/mouse into the flank region in 4 to 7 week old BALB/c mice (BALB/cAnNCrlCrlj, female, Charles River Laboratories Japan). After confirming engraftment of the EMT6 cells 5 to 14 days post-transplantation, a liposome formulation was administered at 10 mg/kg, anti-PD-1 antibody was administered at 200 μg/head, or they were administered at a combined dose, intravenously once a week, peritoneally twice a week, or concomitantly. 10 days after the start of administration, the tumor was harvested and frozen with dry ice, and then RNA was extracted according to the protocol using Maxwell RSC simplyRNA Cells kit (Promega). The RNA concentration was measured with Qbit (Thermo Fisher Scientific Inc.), and RNA was sent in accordance with the specifications of the nCounter service by RIKEN GENESIS CO., LTD. For the analysis by RIKEN GENESIS CO., LTD., nCounter Mouse PanCancerIO 360 panel (NanoString Technologies) was used, and analysis was performed using nCounter FLEX Analysis System. Predetermined normalization results were used to calculate the difference of expression level of VEGF and PD-L1. The expression in anti-PD-1 antibody group set as 100% and the expression level in combined use group of anti-PD-1 antibody and the liposome formulation of Example 1 was calculated, and shown in Table 38.

[Table 38]

| Test | Route of administration | Dosage | Dosing period (days) | Schedule (times/week) | PDL-1 expression (%) | VEGFa expression (%) |
|---|---|---|---|---|---|---|
| Anti-PD-1 antibody | Peritoneal | 200μg/head | 10 | 2 | 100 | 100 |
| Combined use of liposome formulation of Example 1 and anti-PD-1 antibody | Intravenous and peritoneal | 10mg/kg and 200μg/head | 10 | 1 and 2 | 392 | 66 |

By the administration of the liposome formulation of Example 1, the expression of PD-L1 increased by 3.92 times and the expression of VEGFa decreased by 0.66 times compared to the empty liposome administration group. Enhanced expression of PD-L1 is also used clinically as a biomarker for predicting the therapeutic effect by anti-PD-1 antibody. It is considered that administration of the liposome formulation of Example 1 enhances expression of PD-L1, and thus, an environment in which anti-PD-1 is more likely to exert drug efficacy is created. It is known that enhanced expression of VEGFa inhibits infiltration of CD8 positive T cells into the tumor. It is suggested that a decrease in expression of VEGFa by administration of the liposome formulation of Example 1 helps to enhance infiltration of CD8 positive T cells into the tumor.

Test Example 27. Study on induction effect of long-term immune memory

Induction of long-term immune memory was evaluated using a liposome formulation encapsulating Example 1 and an anti-PD-1 antibody.

An anti-PD-1 antibody (114116, Biolegend) diluted with PBS (Invitrogen) was used.

A liposome formulation encapsulating Example 1 was prepared as follows.

11.08 g of Presome ACD-1 (Nippon Fine Chemical Co., Ltd.) and 200 mL of 250 mM ammonium sulfate solution were mixed, and the resulting mixture was dispersed with a homogenizer (ULTRA-TURRAX, IKA) to obtain a crude liposome dispersion. The crude liposome dispersion was further dispersed with a high pressure homogenizer (Nano-Mizer NM2, Yoshida Kikai) at a pressure of 100 MPa to obtain a liposome with a mean particle size (Z-average) of 81 nm. A dialysis cassette (Slide-A-Lyzer G2 Dialysis Cassettes 20K MWCO, Thermo Scientific) was used to replace the outer aqueous phase of liposome with 10 mM L-histidine buffer/9.4% sucrose solution (pH 6.5). The mixture was filtered through a 0.22 μm membrane filter to obtain an empty liposome solution with a total lipid concentration of 53.1 mg/mL. 60 mg of the compound of Example 1, 9.16 mL of 10 mM L-histidine buffer/9.4% sucrose solution (pH6.5), and 20.8 mL of the empty liposome solution were added, and the pH was adjusted to 6.5. The solution was heated for 30 minutes in a 50°C water bath and then cooled with ice. The mixture was filtered through a 0.22 μm membrane filter. The concentration of the compound of Example 1 in the liposome solution was 2.08 mg/mL, and the encapsulation ratio was 98%.

The compound concentration in liposome solution and encapsulation ratio were calculated as follows.

Unencapsulated compound concentration: 100 μL of liposome solution, 100 μL of aqueous 4% phosphoric acid solution, and 300 μL of saline were mixed. The mixture was centrifuged for 60 minutes at 100,000× g to remove insolubles. The compound concentration in the supernatant was measured by HPLC.
Compound concentration in liposome solution: The liposome solution was diluted with a trifluoroacetic acid/water/-methanol mixture (0.1/25/75) and centrifuged at 4°C for 10 minutes at 15,000× g to remove the insolubles. The compound concentration in the supernatant was measured by HPLC.

HPLC measurement conditions are the following.

HPLC conditions

Column: Acquity UPLC BEH C18, 1.7 μm, 50 × 2.1 mm
Column temperature: 40°C
Mobile phase: A: 0.1% trifluoroacetic acid containing water B: acetonitrile
A/B (min): 95/5 (0) → 0/100 (3.5) → 0/100 (4) → 95/5 (4.01) → 95/5 (5)
Flow rate: 0.8 mL/min
Detection: UV visible detector, measured wavelength 254 nm
Injected volume: 5 μL

The encapsulation ratio was calculated by the following equation.

Encapsulation ratio (%) = (compound concentration in liposome solution - unencapsulated compound concentration) × 100/compound concentration in liposome solution

Alternatively, 498.2 g of Presome ACD-1 (Nippon Fine Chemical Co., Ltd.) was weighed out, 8772 g of 250 mM ammonium sulfate solution was added, and the resulting mixture was dispersed with a homogenizer (CLEARMIX, M TECHNIQUE) and degassed by vacuum degassing to obtain a crude liposome dispersion. The crude liposome dispersion was further dispersed with a high pressure homogenizer (Microfluidizer M-110 EH-30, Microfluidics) at a pressure of 14500 Psi to obtain a liposome with a mean particle size (Z-average) of 73 nm. Tangential Flow Filtration system equipped with five Pellicon cassettes (Pellicon 2 Mini 300kD 0.1m2, Merck) was used to replace the outer aqueous phase of liposome with water for injection, and L-histidine and sucrose were added to make a 10 mM L-histidine/9.4% sucrose solution, and the pH was adjusted to 6.5 with 1 mol/L hydrochloric acid. The mixture was then filtered through a 0.2 μm membrane filter to obtain an empty liposome solution with a total lipid concentration of 50.2 mg/mL. 10.0 g of the compound of Example 1 was weighed out, 1354 g of 10 mM L-histidine buffer/9.4% sucrose solution (pH6.5), 27 g of 1 mol/L hydrochloric acid, and 3786 g of the empty liposome solution were added, and the pH was adjusted to 6.5 with 1 mol/L hydrochloric acid. The mixture was heated at 45°C for 60 minutes and then cooled, and the pH was adjusted to 6.5. The mixture was then filtered through a

0.2 μm membrane filter. The solution was dispensed in 50 mL portions into glass vials, and the vials were stoppered and wind-tightened. The concentration of the compound of Example 1 in the liposome solution was 2.01 mg/mL, the encapsulation ratio was 97.5%, and the average particle size (Z-average) was 74 nm.

The compound concentration in liposome solution and encapsulation ratio were calculated as follows.

Unencapsulated compound concentration: 100 μL of liposome solution, 100 μL of aqueous 4% phosphoric acid solution, and 300 μL of saline were mixed. The mixture was centrifuged for 60 minutes at 100,000× g to remove insolubles. The compound concentration in the supernatant was measured by HPLC.
Compound concentration in liposome solution: The liposome solution was diluted with a trifluoroacetic acid/water/-methanol mixture (0.1/25/75) and centrifuged at 4°C for 10 minutes at 15,000× g to remove the insolubles. The compound concentration in the supernatant was measured by HPLC.

HPLC measurement conditions are the following.

HPLC conditions

Column: Acquity UPLC BEH C18, 1.7 μm, 50 × 2.1 mm
Column temperature: 40°C
Mobile phase: A: 0.1% trifluoroacetic acid containing water B: acetonitrile
A/B (min): 95/5 (0) → 60/40 (3) → 0/100 (3.5) → 0/100 (4) → 95/5 (4.01) → 95/5 (5)
Flow rate: 0.8 mL/min
Detection: UV visible detector, measured wavelength 254 nm
Injected volume: 5 μL

The encapsulation ratio was calculated by the following equation.

Encapsulation ratio (%) = (compound concentration in liposome solution - unencapsulated compound concentration) × 100/compound concentration in liposome solution

Mouse breast cancer EMT6 cells (ATCC) were intradermally transplanted at 5 × $10^5$ cells/mouse into the flank region in 4 to 7 week old BALB/c mice (BALB/cAnNCrlCrlj, female, Charles River Laboratories Japan). After confirming engraftment of the EMT6 cells 5 to 14 days post-transplantation, a liposome formulation was administered at 10 mg/kg, anti-PD-1 antibody was administered at 200 μg/head, or they were administered at a combined dose, intravenously once a week, peritoneally twice a week, or concomitantly. When the liposome formulation of Example 1 was administered three times, complete remission was observed in the single administration group of the liposome formulation of Example 1 and in the combined use group of the liposome formulation of Example 1 and the anti-PD-1 antibody. The observation was further performed for 3 months, EMT6 cells (ATCC) were intradermally transplanted again at 5 × $10^5$ cells/mouse into the flank region to the individual who maintained complete remission during that time and to the non-transplanted mouse of the same age, and the tumor growth was observed (secondary transplantation). The tumor volume was measured over time from the secondary transplantation to evaluate the tumor volume reduction. The complete remission rate and the tumor volume 14 days after secondary transplantation in each group were shown in Table 39. The tumor volume was calculated by the following equation by using the minor axis and major axis of tumor measured with an electronic caliper (Mitutoyo).

$$\text{Tumor volume [mm}^3\text{]} = 0.5 \times (\text{minor axis [mm]})^2 \times \text{major axis [mm]}$$

[Table 39]

| Test | Route of administration | Dosage | Dosing period (days) | Schedule (times/week) | Complete remission rate (%) | Mean tumor volume 14 days after secondary transplantation ($mm^3$) |
|---|---|---|---|---|---|---|
| Empty liposome | Intravenous | -- | 10 | 1 | 0 | - |

(continued)

| Test | Route of administration | Dosage | Dosing period (days) | Schedule (times/week) | Complete remission rate (%) | Mean tumor volume 14 days after secondary transplantation ($mm^3$) |
|---|---|---|---|---|---|---|
| Liposome formulation of Example 1 | Intravenous | 10mg/kg | 10 | 1 | 5.5 | 0 |
| Anti-PD-1 antibody | Peritoneal | 200μg/head | 10 | 2 | 0 | - |
| Combined use of liposome formulation of Example 1 and anti-PD-1 antibody | Intravenous and peritoneal | 10mg/kg and 200μg/head | 10 | 1 and 2 | 50 | 36 |
| Not-transplanted | - | - | - | - | - | 336 |

The combined use group of the liposome formulation of Example 1 and the anti-PD-1 antibody achieved complete remission with higher probability than the single administration group of the liposome formulation of Example 1. Furthermore, for the individual who achieved complete remission, tumor engraftment was not observed or tumor growth was suppressed after the secondary transplantation of the cancer cells, as compared with the non-transplanted mouse, indicating that the complete remission mouse acquired long-term immune memory.

Test Example 28. Study on TCR repertoire of CD8 positive cells in tumor, CD8 score and M1-M2 macrophage ratio using tumor-bearing mice transplanted with EMT6 cells

The repertoire variation of CD8 positive T cells and the M1-M2 macrophage ratio in the tumor were examined using a liposome formulation encapsulating Example 1 and an anti-PD-1 antibody.

An anti-PD-1 antibody (114116, Biolegend) diluted with PBS (Invitrogen) was used.

A liposome formulation encapsulating Example 1 was prepared as follows.

498.2 g of Presome ACD-1 (Nippon Fine Chemical Co., Ltd.) was weighed out, 8772 g of 250 mM ammonium sulfate solution was added, and the resulting mixture was dispersed with a homogenizer (CLEARMIX, M TECHNIQUE) and degassed by vacuum degassing to obtain a crude liposome dispersion. The crude liposome dispersion was further dispersed with a high pressure homogenizer (Microfluidizer M-110 EH-30, Microfluidics) at a pressure of 14500 Psi to obtain a liposome with a mean particle size (Z-average) of 73 nm. Tangential Flow Filtration system equipped with five Pellicon cassettes (Pellicon 2 Mini 300kD 0.1m2, Merck) was used to replace the outer aqueous phase of liposome with water for injection, and L-histidine and sucrose were added to make a 10 mM L-histidine/9.4% sucrose solution, and the pH was adjusted to 6.5 with 1 mol/L hydrochloric acid. The mixture was then filtered through a 0.2 μm membrane filter to obtain an empty liposome solution with a total lipid concentration of 50.2 mg/mL.

10.0 g of the compound of Example 1 was weighed out, 1354 g of 10 mM L-histidine buffer/9.4% sucrose solution (pH6.5), 27 g of 1 mol/L hydrochloric acid, and 3786 g of the empty liposome solution were added, and the pH was adjusted to 6.5 with 1 mol/L hydrochloric acid. The mixture was heated at 45°C for 60 minutes and then cooled, and the pH was adjusted to 6.5. The mixture was then filtered through a 0.2 μm membrane filter. The solution was dispensed in 50 mL portions into glass vials, and the vials were stoppered and wind-tightened. The concentration of the compound of Example 1 in the liposome solution was 2.01 mg/mL, the encapsulation ratio was 97.5%, and the average particle size (Z-average) was 74 nm.

The compound concentration in liposome solution and encapsulation ratio were calculated as follows.

Unencapsulated compound concentration: 100 μL of liposome solution, 100 μL of aqueous 4% phosphoric acid solution, and 300 μL of saline were mixed. The mixture was centrifuged for 60 minutes at 100,000× g to remove insolubles. The compound concentration in the supernatant was measured by HPLC.

Compound concentration in liposome solution: The liposome solution was diluted with a trifluoroacetic acid/water/-methanol mixture (0.1/25/75) and centrifuged at 4°C for 10 minutes at 15,000× g to remove the insolubles. The compound concentration in the supernatant was measured by HPLC.

HPLC measurement conditions are the following.

HPLC conditions

Column: Acquity UPLC BEH C18, 1.7 μm, 50 × 2.1 mm
Column temperature: 40°C
Mobile phase: A: 0.1% trifluoroacetic acid containing water B: acetonitrile
A/B (min): 95/5 (0) → 60/40 (3) → 0/100 (3.5) → 0/100 (4) → 95/5 (4.01) → 95/5 (5)
Flow rate: 0.8 mL/min
Detection: UV visible detector, measured wavelength 254 nm
Injected volume: 5 μL

The encapsulation ratio was calculated by the following equation.

Encapsulation ratio (%) = (compound concentration in liposome solution - unencapsulated compound concentration) × --> 100/compound concentration in liposome solution

Mouse breast cancer EMT6 cells (ATCC) were intradermally transplanted at 5 × $10^5$ cells/mouse into the flank region in 4 to 7 week old BALB/c mice (BALB/cAnNCrlCrlj, female, Charles River Laboratories Japan). After confirming engraftment of the EMT6 cells 5 to 14 days post-transplantation, a liposome formulation was administered at 10 mg/kg, anti-PD-1 antibody was administered at 200 ug/head, or they were administered at a combined dose, intravenously once a week, peritoneally twice a week, or concomitantly. 10 days after the start of administration, the tumor was harvested, weighed out, and then pooled to one sample for each group (N = 4). The tumor mass of EMT6 cells was finely cut with scissors, and treated with gentle MACS Tissue Dissociators according to the protocol using Tumor Dissociation Kit, Mouse (Miltenyi Biotec), and the tumor mass was prepared into a cell suspension.

The prepared cells were passed through a 70 μm cell strainer and then washed with RPMI1640 containing 10% FBS. Thereafter, the cells were treated with a red blood cell lysate (Lonza) for 1 minute to remove red blood cells, further washed with RPMI1640 containing 10% FCS, and suspended in MACS Rinsing solution containing 5% BSA (Miltnyi Biotec, hereinafter, referred to as MACS buffer). The cell suspension was counted using LUNA FL (Logos Biosystems), CD45 MicroBeads, mouse (Miltenyi Biotec) was added to all the cells according to the protocol, and CD45 positive cells were sorted by autoMACS Pro Separator (Miltenyi Biotec). The sorted cells were further stained with PerCP-labeled anti-mouse CD45 antibody on ice for 20 minutes. After washing once with MACS buffer, Hoechst33342 (Thermo Fisher Scientific Inc.) diluted with PBS was added, and a reaction was allowed to proceed for 5 minutes on ice to stain dead cells. Staining was performed twice with MACS buffer. The stained cells were further sorted into CD45 positive living cells by a cell sorter. After the sorted cells were counted, a sequence library was created based on the protocol of Chromium Next GEN Single Cell 5' Kit v2 (10x GENOMICS), and the sequence was analyzed by Takara Bio Inc.

From the obtained sequence data, single-cell gene expression and TCR chronotypes were calculated using CellRanger software (10x GENOMICS), and further, the cells were clustered based on the obtained gene expression data. For each obtained cluster, the cell type was assigned based on the expression of the marker gene. From the lymphocyte cluster identified here, CD8 positive T cells were assigned based on the expression of Cd8a and Cd8b1 and the non-expression of Cd4 and Ncr1. Similarly, from the macrophage cluster, M1 macrophages were assigned based on the expression of Stat1 and Cd86, and M2 macrophages were assigned based on the expression of Arg1 and Mrc1. The number of CD8 positive T cells relative to the total number of obtained CD45 positive cells was defined as the proportion of CD8 positive T cells (Figure 12). Similarly, the proportion of M1 macrophages as well as M2 macrophages was determined and the ratio thereof was calculated as the M1-M2 macrophage ratio (Figure 13). T cell clonality was analyzed using TCR clonotype information output from CellRanger. As an index of clonality, a ratio of clones with a certain size or more (specifically, clones in which two or more cells were detected) to all clones, and a Gini coefficient determined from a cell ratio for each clone were used. In order to correct the difference in the number of detected CD8 positive T cells for each administration group, an equal number of cells (35 cells) were randomly sampled for each administration group, and then an index was calculated. This sampling was performed 100 times, and the average of the indices was calculated. The index was calculated per administration group and compared between the administration groups (Figure 11).

As shown in Figure 11, it was revealed that the T cell clonality was remarkably increased in the administration group of the liposome formulation of Example 1 as compared with the empty liposome administration group. The CD8 positive T cell proportion and the M1-M2 macrophage ratio were each 5 times or more in the administration group of the liposome formulation of Example 1 and in the combined use group of the liposome formulation of Example 1 and the anti-PD-1 antibody, as compared with the empty liposome administration group. When these values are compared between the anti-PD-1 antibody sensitive and insensitive patients in the clinic, the CD8 positive T cell proportion sensitive patients are 0.1 to 0.15 times the insensitive patients, and the M1-M2 macrophage ratio is about 2 to 3 times. Therefore, based on the literature (Pender et al. Clin Cancer Res 2021; 27:202-12), changes in the administration group of the liposome formulation of Example 1 and the combined use group are remarkable.

Test Example 29. Concomitantly used drug efficacy evaluation test using tumor-bearing mice transplanted with Renca cells

Antitumor effects were evaluated using a liposome formulation encapsulating Example 1 and an anti-PD-1 antibody.

An anti-PD-1 antibody (114116, Biolegend) diluted with PBS (Invitrogen) was used.

A liposome formulation encapsulating Example 1 was prepared as follows.

498.2 g of Presome ACD-1 (Nippon Fine Chemical Co., Ltd.) was weighed out, 8772 g of 250 mM ammonium sulfate solution was added, and the resulting mixture was dispersed with a homogenizer (CLEARMIX, M TECHNIQUE) and degassed by vacuum degassing to obtain a crude liposome dispersion. The crude liposome dispersion was further dispersed with a high pressure homogenizer (Microfluidizer M-110 EH-30, Microfluidics) at a pressure of 14500 Psi to obtain a liposome with a mean particle size (Z-average) of 73 nm. Tangential Flow Filtration system equipped with five Pellicon cassettes (Pellicon 2 Mini 300kD 0.1m2, Merck) was used to replace the outer aqueous phase of liposome with water for injection, and L-histidine and sucrose were added to make a 10 mM L-histidine/9.4% sucrose solution, and the pH was adjusted to 6.5 with 1 mol/L hydrochloric acid. The mixture was then filtered through a 0.2 μm membrane filter to obtain an empty liposome solution with a total lipid concentration of 50.2 mg/mL.

10.0 g of the compound of Example 1 was weighed out, 1354 g of 10 mM L-histidine buffer/9.4% sucrose solution (pH6.5), 27 g of 1 mol/L hydrochloric acid, and 3786 g of the empty liposome solution were added, and the pH was adjusted to 6.5 with 1 mol/L hydrochloric acid. The mixture was heated at 45°C for 60 minutes and then cooled, and the pH was adjusted to 6.5. The mixture was then filtered through a 0.2 μm membrane filter. The solution was dispensed in 50 mL portions into glass vials, and the vials were stoppered and wind-tightened. The concentration of the compound of Example 1 in the liposome solution was 2.01 mg/mL, the encapsulation ratio was 97.5%, and the average particle size (Z-average) was 74 nm.

The compound concentration in liposome solution and encapsulation ratio were calculated as follows.

Unencapsulated compound concentration: 100 μL of liposome solution, 100 μL of aqueous 4% phosphoric acid solution, and 300 μL of saline were mixed. The mixture was centrifuged for 60 minutes at 100,000× g to remove insolubles. The compound concentration in the supernatant was measured by HPLC.

Compound concentration in liposome solution: The liposome solution was diluted with a trifluoroacetic acid/water/-methanol mixture (0.1/25/75) and centrifuged at 4°C for 10 minutes at 15,000× g to remove the insolubles. The compound concentration in the supernatant was measured by HPLC.

HPLC measurement conditions are the following.

HPLC conditions

Column: Acquity UPLC BEH C18, 1.7 μm, 50 × 2.1 mm
Column temperature: 40°C
Mobile phase: A: 0.1% trifluoroacetic acid containing water B: acetonitrile
A/B (min): 95/5 (0) → 60/40 (3) → 0/100 (3.5) → 0/100 (4) - 95/5 (4.01) → 95/5 (5)
Flow rate: 0.8 mL/min
Detection: UV visible detector, measured wavelength 254 nm
Injected volume: 5 μL

The encapsulation ratio was calculated by the following equation.

Encapsulation ratio (%) = (compound concentration in liposome solution - unencapsulated compound concentration) × 100/compound concentration in liposome solution

Mouse renal cancer Renca cells were intradermally transplanted at 5 × $10^4$ cells/mouse into the flank region in 4 to 7 week old BALB/c mice (BALB/cAnNCrlCrlj, female, Jackson Laboratory Japan). After confirming engraftment of the Renca cells 23 days post-transplantation, a liposome formulation at 10 mg/kg, anti-PD-1 antibody at 200 μg/head, or concomitantly used thereof was administered intravenously once a week, peritoneally twice a week, or a combination thereof. The tumor volume was measured over time from the start of administration to evaluate the tumor volume reduction due to compound administration. The tumor volume was calculated by the following equation by using the minor axis and major axis of tumor measured with an electronic caliper (Mitutoyo).

$$\text{Tumor volume } [mm^3] = 0.5 \times (\text{minor axis } [mm])^2 \times \text{major axis } [mm]$$

A control administration group administered with only a solvent and a compound of the present disclosure administration group were compared. T/C was calculated from the following equation to evaluate the antitumor effect. For the control administration group, an empty liposome solution was used.

T/C(%) = (tumor volume as of the end of administration of the compound of the present disclosure administration group - tumor volume as of the start of administration of the compound of the present disclosure administration group)/(tumor volume as of the end of administration of the control administration group - tumor volume as of the start of administration of the control administration group) × 100

Table 40 shows the T/C(%) for tumor-bearing mice transplanted with Renca cells at each dosage and dosing period for the test compound.

[Table 40]

| Test | Route of administration | Dosage | Dosing period (days) | Schedule (times/week) | T/C (%)) |
|---|---|---|---|---|---|
| Liposome formulation of Example 1 | Intravenous | 10mg/kg | 15 | 1 | 80 |
| Anti-PD-1 antibody | Peritoneal | 200μg/head | 15 | 2 | 139 |
| Liposome formulation of Example 1 and anti-PD-1 antibody | Intravenous and peritoneal | 10mg/kg and 200μg/head | 15 | 1 and 2 | 59 |

In the concomitantly used drug efficacy evaluation test using tumor-bearing mice, "Example 1 prepared as a liposome formulation" exhibited an excellent antitumor effect in "single agent" or "combined use with an anti-PD-1 antibody". As shown in Test Example 29, tumor-bearing mice transplanted with Renca cells are models in which an anti-PD-1 antibody does not exhibits an antitumor effect. From the results of the present test, the liposome formulation of Example 1 exhibits an excellent antitumor effect on a model in which the anti-PD-1 antibody does not exhibit an antitumor effect by "single agent" or "combined use with an anti-PD-1 antibody", which is a significant effect.

Test Example 30. Concomitantly used drug efficacy evaluation test using tumor-bearing mice transplanted with LLC cells

Antitumor effects were evaluated using a liposome formulation encapsulating Example 1 and an anti-PD-1 antibody.

An anti-PD-1 antibody (114116, Biolegend) diluted with PBS (Invitrogen) was used.

A liposome formulation encapsulating Example 1 was prepared as follows.

498.2 g of Presome ACD-1 (Nippon Fine Chemical Co., Ltd.) was weighed out, 8772 g of 250 mM ammonium sulfate solution was added, and the resulting mixture was dispersed with a homogenizer (CLEARMIX, M TECHNIQUE) and degassed by vacuum degassing to obtain a crude liposome dispersion. The crude liposome dispersion was further dispersed with a high pressure homogenizer (Microfluidizer M-110 EH-30, Microfluidics) at a pressure of 14500 Psi to obtain a liposome with a mean particle size (Z-average) of 73 nm. Tangential Flow Filtration system equipped with five Pellicon cassettes (Pellicon 2 Mini 300kD 0.1m2, Merck) was used to replace the outer aqueous phase of liposome with water for injection, and L-histidine and sucrose were added to make a 10 mM L-histidine/9.4% sucrose solution, and the pH was adjusted to 6.5 with 1 mol/L hydrochloric acid. The mixture was then filtered through a 0.2 μm membrane filter to obtain an empty liposome solution with a total lipid concentration of 50.2 mg/mL.

10.0 g of the compound of Example 1 was weighed out, 1354 g of 10 mM L-histidine buffer/9.4% sucrose solution (pH6.5), 27 g of 1 mol/L hydrochloric acid, and 3786 g of the empty liposome solution were added, and the pH was adjusted to 6.5 with 1 mol/L hydrochloric acid. The mixture was heated at 45°C for 60 minutes and then cooled, and the pH was adjusted to 6.5. The mixture was then filtered through a 0.2 μm membrane filter. The solution was dispensed in 50 mL portions into glass vials, and the vials were stoppered and wind-tightened. The concentration of the compound of Example 1 in the liposome solution was 2.01 mg/mL, the encapsulation ratio was 97.5%, and the average particle size (Z-average) was 74 nm.

The compound concentration in liposome solution and encapsulation ratio were calculated as follows.

Unencapsulated compound concentration: 100 μL of liposome solution, 100 μL of aqueous 4% phosphoric acid solution, and 300 μL of saline were mixed. The mixture was centrifuged for 60 minutes at 100,000× g to remove insolubles. The compound concentration in the supernatant was measured by HPLC.

Compound concentration in liposome solution: The liposome solution was diluted with a trifluoroacetic acid/water/-methanol mixture (0.1/25/75) and centrifuged at 4°C for 10 minutes at 15,000× g to remove the insolubles. The compound concentration in the supernatant was measured by HPLC.

HPLC measurement conditions are the following.

HPLC conditions

Column: Acquity UPLC BEH C18, 1.7 μm, 50 × 2.1 mm
Column temperature: 40°C
Mobile phase: A: 0.1% trifluoroacetic acid containing water B: acetonitrile
A/B (min): 95/5 (0) → 60/40 (3) → 0/100 (3.5) → 0/100 (4) → 95/5 (4.01) → 95/5 (5)
Flow rate: 0.8 mL/min
Detection: UV visible detector, measured wavelength 254 nm
Injected volume: 5 μL

The encapsulation ratio was calculated by the following equation.

Encapsulation ratio (%) = (compound concentration in liposome solution - unencapsulated compound concentration) × 100/compound concentration in liposome solution

Mouse lung cancer LLC cells (RIKEN BRC) were intradermally transplanted at $1 \times 10^6$ cells/mouse into the flank region in 4 to 7 week old B6 mice (C57BL/6J, female, Jackson Laboratory Japan). After confirming engraftment of the LLC cells 7 days post-transplantation, a liposome formulation at 10 mg/kg, anti-PD-1 antibody at 200 μg/head, or concomitantly used thereof was administered intravenously once a week, peritoneally twice a week, or a combination thereof. The tumor volume was measured over time from the start of administration to evaluate the tumor volume reduction due to compound administration. The tumor volume was calculated by the following equation by using the minor axis and major axis of tumor measured with an electronic caliper (Mitutoyo).

$$\text{Tumor volume } [mm^3] = 0.5 \times (\text{minor axis } [mm])^2 \times \text{major axis } [mm]$$

A control administration group administered with only a solvent and a compound of the present disclosure administration group were compared. T/C was calculated from the following equation to evaluate the antitumor effect. For the control administration group, an empty liposome solution was used.

T/C(%) = (tumor volume as of the end of administration of the compound of the present disclosure administration group - tumor volume as of the start of administration of the compound of the present disclosure administration group)/(tumor volume as of --> the end of administration of the control administration group - tumor volume as of the start of administration of the control administration group) × 100

Table 41 shows the T/C(%) for tumor-bearing mice transplanted with LLC cells at each dosage and dosing period for the test compound.

[Table 41]

| Test | Route of administration | Dosage | Dosing period (days) | Schedule (times/week) | T/C (%) |
|---|---|---|---|---|---|
| Liposome formulation of Example 1 | Intravenous | 10mg/kg | 17 | 1 | 33 |
| Anti-PD-1 antibody | Peritoneal | 200μg/head | 17 | 2 | 43 |
| Liposome formulation of Example 1 and anti-PD-1 antibody | Intravenous and peritoneal | 10mg/kg and 200μg/head | 17 | 1 and 2 | 8 |

It was revealed that in the concomitantly used drug efficacy evaluation test using tumor-bearing mice, "Example 1 prepared as a liposome formulation" exhibits an excellent antitumor effect in "single agent" or "concomitant administration with an anti-PD-1 antibody". As shown in Test Example 30, tumor-bearing mice transplanted with LLC cells are models in which an anti-PD-1 antibody is unlikely to exhibit an antitumor effect. From the results of the present test, the liposome formulation of Example 1 exhibits an excellent antitumor effect on a model in which the anti-PD-1 antibody is unlikely to exhibit an antitumor effect by "single agent" or "combined use with an anti-PD-1 antibody", which is a significant effect.

Test Example 31. Intratumoral immune cell evaluation test using tumor-bearing mice transplanted with Renca cells

The fractionation of immune cells infiltrating into the tumor was evaluated using a liposome formulation encapsulating Example 1 and an anti-PD-1 antibody.
An anti-PD-1 antibody (114116, Biolegend) diluted with PBS (Invitrogen) was used.
A liposome formulation encapsulating Example 1 was prepared as follows.
498.2 g of Presome ACD-1 (Nippon Fine Chemical Co., Ltd.) was weighed out, 8772 g of 250 mM ammonium sulfate solution was added, and the resulting mixture was dispersed with a homogenizer (CLEARMIX, M TECHNIQUE) and degassed by vacuum degassing to obtain a crude liposome dispersion. The crude liposome dispersion was further dispersed with a high pressure homogenizer (Microfluidizer M-110 EH-30, Microfluidics) at a pressure of 14500 Psi to obtain a liposome with a mean particle size (Z-average) of 73 nm. Tangential Flow Filtration system equipped with five Pellicon cassettes (Pellicon 2 Mini 300kD 0.1m2, Merck) was used to replace the outer aqueous phase of liposome with water for injection, and L-histidine and sucrose were added to make a 10 mM L-histidine/9.4% sucrose solution, and the pH was adjusted to 6.5 with 1 mol/L hydrochloric acid. The mixture was then filtered through a 0.2 μm membrane filter to obtain an empty liposome solution with a total lipid concentration of 50.2 mg/mL.
10.0 g of the compound of Example 1 was weighed out, 1354 g of 10 mM L-histidine buffer/9.4% sucrose solution (pH6.5), 27 g of 1 mol/L hydrochloric acid, and 3786 g of the empty liposome solution were added, and the pH was adjusted to 6.5 with 1 mol/L hydrochloric acid. The mixture was heated at 45°C for 60 minutes and then cooled, and the pH was adjusted to 6.5. The mixture was then filtered through a 0.2 μm membrane filter. The solution was dispensed in 50 mL portions into glass vials, and the vials were stoppered and wind-tightened. The concentration of the compound of Example 1 in the liposome solution was 2.01 mg/mL, the encapsulation ratio was 97.5%, and the average particle size (Z-average) was 74 nm.
The compound concentration in liposome solution and encapsulation ratio were calculated as follows.

Unencapsulated compound concentration: 100 μL of liposome solution, 100 μL of aqueous 4% phosphoric acid solution, and 300 μL of saline were mixed. The mixture was centrifuged for 60 minutes at 100,000× g to remove insolubles. The compound concentration in the supernatant was measured by HPLC.
Compound concentration in liposome solution: The liposome solution was diluted with a trifluoroacetic acid/water/-methanol mixture (0.1/25/75) and centrifuged at 4°C for 10 minutes at 15,000× g to remove the insolubles. The compound concentration in the supernatant was measured by HPLC.

HPLC measurement conditions are the following.

HPLC conditions

Column: Acquity UPLC BEH C18, 1.7 μm, 50 × 2.1 mm
Column temperature: 40°C

Mobile phase: A: 0.1% trifluoroacetic acid containing water B: acetonitrile
A/B (min): 95/5 (0) → 60/40 (3) → 0/100 (3.5) → 0/100 (4) - 95/5 (4.01) → 95/5 (5)
Flow rate: 0.8 mL/min
Detection: UV visible detector, measured wavelength 254 nm
Injected volume: 5 μL

The encapsulation ratio was calculated by the following equation.

Encapsulation ratio (%) = (compound concentration in liposome solution - unencapsulated compound concentration) × 100/compound concentration in liposome solution

Mouse renal cancer Renca cells were intradermally transplanted at $5 \times 10^4$ cells/mouse into the flank region in 4 to 7 week old BALB/c mice (BALB/cAnNCrlCrlj, female, Charles River Laboratories Japan). After confirming engraftment of the Renca cells 23 days post-transplantation, a liposome formulation was administered at 10 mg/kg, anti-PD-1 antibody was administered at 200 ug/head, or they were administered at a combined dose, intravenously once a week, peritoneally twice a week, or concomitantly. 15 days after the start of administration, the tumor was harvested from four individuals in each group. The tumor mass of Renca cells was finely cut with scissors, and treated with gentle MACS Tissue Dissociators (Miltenyi Biotec) according to the protocol using Tumor Dissociation Kit, Mouse (Miltenyi Biotec), and the tumor mass was prepared into a cell suspension.

The prepared cells were passed through a 70 μm cell strainer and then washed with RPMI1640 containing 10% FBS. Thereafter, the cells were treated with a red blood cell lysate (Lonza) for 1 minute to remove red blood cells, further washed with RPMI1640 containing 10% FCS, and suspended in MACS Rinsing solution containing 5% BSA (Miltnyi Biotec, hereinafter, referred to as MACS buffer).

Live/Dead Fixable Dye, nearIR (Thermo Fisher Scientific Inc.) diluted with PBS was added to the cell suspension, and a reaction was allowed to proceed for 5 minutes on ice to stain dead cells. After washing once with MACS buffer, the cell suspension was blocked by adding Mouse BD Fc BlockTM (BD Biosciences), and then, staining was performed with V450-labeled mouse CD45 (BD Biosciences), BV510-labeled anti-mouse CD8a (Biolegend), PE-Cy7-labeled anti-mouse CD3 (Thermo Fisher Scientific Inc.), and APC-labeled anti-mouse CD4 (BD Biosciences) on ice for 20 minutes. After staining twice with MACS buffer, the cells were fixed with 1% PFA/PBS and analyzed using a flow cytometer.

The average of data of six individuals in each group was calculated for the percentage of CD45 + lymphocyte fractionated CD8 + T cells (CD8 positive T cells) in living cells.

Table 42 shows the CD8 positive T cell rate in the empty liposome, liposome formulation of Example 1, anti-PD-1 antibody, and combined use groups.

[Table 42]

| Test | Route of administration | Dosage | Dosing period (days) | Schedule (times/week) | CD8 positive T cell rate (%) |
|---|---|---|---|---|---|
| Empty liposome | Intravenous | -- | 15 | 1 | 0.025 |
| Liposome formulation of Example 1 | Intravenous | 10mg/kg | 15 | 1 | 0.094 |
| Anti-PD-1 antibody | Peritoneal | 200μg/head | 15 | 2 | 0.034 |
| Combined use of liposome formulation of Example 1 and anti-PD-1 antibody | Intravenous and peritoneal | 10mg/kg and 200μg/head | 15 | 1 and 2 | 0.427 |

By the administration of the liposome formulation of Example 1, the number of CD8 positive T cells in the tumor increased by 3.5 times or more compared to the empty liposome administration group, indicating that the liposome formulation of Example 1 enhanced the infiltration of T cells into the tumor. From the above, it was shown that, also in the mouse renal cancer Renca model, the liposome formulation of Example 1 changes the tumor microenvironment from an immunosuppressive environment to an inflammatory environment.

Test Example 32. Intratumoral immune cell evaluation test using tumor-bearing mice transplanted with LLC cells

The fractionation of immune cells infiltrating into the tumor was evaluated using a liposome formulation

encapsulating Example 1 and an anti-PD-1 antibody.

An anti-PD-1 antibody (114116, Biolegend) diluted with PBS (Invitrogen) was used.

A liposome formulation encapsulating Example 1 was prepared as follows.

498.2 g of Presome ACD-1 (Nippon Fine Chemical Co., Ltd.) was weighed out, 8772 g of 250 mM ammonium sulfate solution was added, and the resulting mixture was dispersed with a homogenizer (CLEARMIX, M TECHNIQUE) and degassed by vacuum degassing to obtain a crude liposome dispersion. The crude liposome dispersion was further dispersed with a high pressure homogenizer (Microfluidizer M-110 EH-30, Microfluidics) at a pressure of 14500 Psi to obtain a liposome with a mean particle size (Z-average) of 73 nm. Tangential Flow Filtration system equipped with five Pellicon cassettes (Pellicon 2 Mini 300kD 0.1m2, Merck) was used to replace the outer aqueous phase of liposome with water for injection, and L-histidine and sucrose were added to make a 10 mM L-histidine/9.4% sucrose solution, and the pH was adjusted to 6.5 with 1 mol/L hydrochloric acid. The mixture was then filtered through a 0.2 μm membrane filter to obtain an empty liposome solution with a total lipid concentration of 50.2 mg/mL.

10.0 g of the compound of Example 1 was weighed out, 1354 g of 10 mM L-histidine buffer/9.4% sucrose solution (pH6.5), 27 g of 1 mol/L hydrochloric acid, and 3786 g of the empty liposome solution were added, and the pH was adjusted to 6.5 with 1 mol/L hydrochloric acid. The mixture was heated at 45°C for 60 minutes and then cooled, and the pH was adjusted to 6.5. The mixture was then filtered through a 0.2 μm membrane filter. The solution was dispensed in 50 mL portions into glass vials, and the vials were stoppered and wind-tightened. The concentration of the compound of Example 1 in the liposome solution was 2.01 mg/mL, the encapsulation ratio was 97.5%, and the average particle size (Z-average) was 74 nm.

The compound concentration in liposome solution and encapsulation ratio were calculated as follows.

Unencapsulated compound concentration: 100 μL of liposome solution, 100 μL of aqueous 4% phosphoric acid solution, and 300 μL of saline were mixed. The mixture was centrifuged for 60 minutes at 100,000× g to remove insolubles. The compound concentration in the supernatant was measured by HPLC.
Compound concentration in liposome solution: The liposome solution was diluted with a trifluoroacetic acid/water/-methanol mixture (0.1/25/75) and centrifuged at 4°C for 10 minutes at 15,000× g to remove the insolubles. The compound concentration in the supernatant was measured by HPLC.

HPLC measurement conditions are the following.

HPLC conditions

Column: Acquity UPLC BEH C18, 1.7 μm, 50 × 2.1 mm
Column temperature: 40°C
Mobile phase: A: 0.1% trifluoroacetic acid containing water B: acetonitrile
A/B (min): 95/5 (0) → 60/40 (3) → 0/100 (3.5) → 0/100 (4) → 95/5 (4.01) → 95/5 (5)
Flow rate: 0.8 mL/min
Detection: UV visible detector, measured wavelength 254 nm
Injected volume: 5 μL

The encapsulation ratio was calculated by the following equation.

Encapsulation ratio (%) = (compound concentration in liposome --> solution - unencapsulated compound concentration) × 100/compound concentration in liposome solution

Mouse lung cancer LLC cells (RIKEN BRC) were intradermally transplanted at $1 \times 10^6$ cells/mouse into the flank region in 4 to 7 week old B6 mice (C57BL/6J, female, THE JACKSON LABORATORY JAPAN, INC.). After confirming engraftment of the LLC cells 7 days post-transplantation, a liposome formulation was administered at 10 mg/kg, anti-PD-1 antibody was administered at 200 ug/head, or they were administered at a combined dose, intravenously once a week, peritoneally twice a week, or concomitantly. 17 days after the start of administration, the tumor was harvested from four individuals in each group. The tumor mass of LLC cells was finely cut with scissors, and treated with gentle MACS Tissue Dissociators (Miltenyi Biotec) according to the protocol using Tumor Dissociation Kit, Mouse (Miltenyi Biotec), and the tumor mass was prepared into a cell suspension.

The prepared cells were passed through a 70 μm cell strainer and then washed with RPMI1640 containing 10% FBS. Thereafter, the cells were treated with a red blood cell lysate (Lonza) for 1 minute to remove red blood cells, further

washed with RPMI1640 containing 10% FCS, and suspended in MACS Rinsing solution containing 5% BSA (Miltnyi Biotec, hereinafter, referred to as MACS buffer).

Live/Dead Fixable Dye, nearIR (Thermo Fisher Scientific Inc.) diluted with PBS was added to the cell suspension, and a reaction was allowed to proceed for 5 minutes on ice to stain dead cells. After washing once with MACS buffer, the cell suspension was blocked by adding Mouse BD Fc BlockTM (BD Biosciences), and then, staining was performed with V450-labeled mouse CD45 (BD Biosciences), BV510-labeled anti-mouse CD8a (Biolegend), PE-Cy7-labeled anti-mouse CD3 (Thermo Fisher Scientific Inc.), and PerCP-labeled anti-mouse CD4 (BD Biosciences) on ice for 20 minutes. After staining twice with MACS buffer, the cells were fixed with 1% PFA/PBS and analyzed using a flow cytometer.

The average of data of six individuals in each group was calculated for the percentage of CD45 + lymphocyte fractionated CD8 + T cells (CD8 positive T cells) in living cells.

Table 43 shows the CD8 positive T cell rate in the empty liposome, liposome formulation of Example 1, anti-PD-1 antibody, and combined use groups.

[Table 43]

| Test | Route of administration | Dosage | Dosing period (days) | Schedule (times/week) | CD8 positive T cell rate (%) |
|---|---|---|---|---|---|
| Empty liposome | Intravenous | -- | 17 | 1 | 0.92 |
| Liposome formulation of Example 1 | Intravenous | 10mg/kg | 17 | 1 | 1.20 |
| Anti-PD-1 antibody | Peritoneal | 200μg/head | 17 | 2 | 1.36 |
| Combined use of liposome formulation of Example 1 and anti-PD-1 antibody | Intravenous and peritoneal | 10mg/kg and 200μg/head | 17 | 1 and 2 | 2.14 |

By the administration of the "liposome formulation of Example 1", the number of CD8 positive T cells in the tumor increased by 1.3 times or more compared to the empty liposome administration group, indicating that the liposome formulation of Example 1 enhanced the infiltration of T cells into the tumor. At the same time, in the present model, a similar number of CD8 positive T cells in the tumor are observed also in the "anti-PD-1 antibody administration group", and in the "combined use group of the liposome formulation and the anti-PD-1 antibody", an increase in both combined is observed. This is consistent with the drug efficacy data shown in Test Example 30. From the above, it was shown that, also in the mouse renal cancer LLC model, the liposome formulation of Example 1 changes the tumor microenvironment from an immunosuppressive environment to an inflammatory environment.

Test Example 33. Intratumoral immune environment evaluation test using tumor-bearing mice transplanted with Renca cells

The gene expression related to immune cells infiltrating into the tumor was evaluated using a liposome formulation encapsulating Example 1 and an anti-PD-1 antibody.

An anti-PD-1 antibody (114116, Biolegend) diluted with PBS (Invitrogen) was used.

A liposome formulation encapsulating Example 1 was prepared as follows.

498.2 g of Presome ACD-1 (Nippon Fine Chemical Co., Ltd.) was weighed out, 8772 g of 250 mM ammonium sulfate solution was added, and the resulting mixture was dispersed with a homogenizer (CLEARMIX, M TECHNIQUE) and degassed by vacuum degassing to obtain a crude liposome dispersion. The crude liposome dispersion was further dispersed with a high pressure homogenizer (Microfluidizer M-110 EH-30, Microfluidics) at a pressure of 14500 Psi to obtain a liposome with a mean particle size (Z-average) of 73 nm. Tangential Flow Filtration system equipped with five Pellicon cassettes (Pellicon 2 Mini 300kD 0.1m2, Merck) was used to replace the outer aqueous phase of liposome with water for injection, and L-histidine and sucrose were added to make a 10 mM L-histidine/9.4% sucrose solution, and the pH was adjusted to 6.5 with 1 mol/L hydrochloric acid. The mixture was then filtered through a 0.2 μm membrane filter to obtain an empty liposome solution with a total lipid concentration of 50.2 mg/mL.

10.0 g of the compound of Example 1 was weighed out, 1354 g of 10 mM L-histidine buffer/9.4% sucrose solution (pH6.5), 27 g of 1 mol/L hydrochloric acid, and 3786 g of the empty liposome solution were added, and the pH was adjusted to 6.5 with 1 mol/L hydrochloric acid. The mixture was heated at 45°C for 60 minutes and then cooled, and the pH was adjusted to 6.5. The mixture was then filtered through a 0.2 μm membrane filter. The solution was dispensed in 50 mL portions into glass vials, and the vials were stoppered and wind-tightened. The concentration of the compound of Example

1 in the liposome solution was 2.01 mg/mL, the encapsulation ratio was 97.5%, and the average particle size (Z-average) was 74 nm.

The compound concentration in liposome solution and encapsulation ratio were calculated as follows.

Unencapsulated compound concentration: 100 μL of liposome solution, 100 μL of aqueous 4% phosphoric acid solution, and 300 μL of saline were mixed. The mixture was centrifuged for 60 minutes at 100,000× g to remove insolubles. The compound concentration in the supernatant was measured by HPLC.

Compound concentration in liposome solution: The liposome solution was diluted with a trifluoroacetic acid/water/-methanol mixture (0.1/25/75) and centrifuged at 4°C for 10 minutes at 15,000× g to remove the insolubles. The compound concentration in the supernatant was measured by HPLC.

HPLC measurement conditions are the following.

HPLC conditions

Column: Acquity UPLC BEH C18, 1.7 μm, 50 × 2.1 mm
Column temperature: 40°C
Mobile phase: A: 0.1% trifluoroacetic acid containing water B: acetonitrile
A/B (min): 95/5 (0) → 60/40 (3) → 0/100 (3.5) → 0/100 (4) → 95/5 (4.01) → 95/5 (5)
Flow rate: 0.8 mL/min
Detection: UV visible detector, measured wavelength 254 nm
Injected volume: 5 μL

The encapsulation ratio was calculated by the following equation.

Encapsulation ratio (%) = (compound concentration in liposome solution - unencapsulated compound concentration) × 100/compound concentration in liposome solution

Mouse renal cancer Renca cells were intradermally transplanted at $5 \times 10^4$ cells/mouse into the flank region in 4 to 7 week old BALB/c mice (BALB/cAnNCrlCrlj, female, Charles River Laboratories Japan). After confirming engraftment of the Renca cells 23 days post-transplantation, a liposome formulation was administered at 10 mg/kg, anti-PD-1 antibody was administered at 200 ug/head, or they were administered at a combined dose, intravenously once a week, peritoneally twice a week, or concomitantly. 15 days after the start of administration, the tumor was harvested from four individuals in each group. The tumor mass of Renca cells was finely cut with scissors, and treated with gentle MACS Tissue Dissociators (Miltenyi Biotec) according to the protocol using Tumor Dissociation Kit, Mouse (Miltenyi Biotec), and the tumor mass was prepared into a cell suspension.

The prepared cells were passed through a 70 μm cell strainer and then washed with RPMI1640 containing 10% FBS. Thereafter, the cells were treated with a red blood cell lysate (Lonza) for 1 minute to remove red blood cells, further washed with RPMI1640 containing 10% FCS, and suspended in MACS Rinsing solution containing 5% BSA (Miltnyi Biotec, hereinafter, referred to as MACS buffer).

The cell suspension was centrifuged, the tumor was frozen with dry ice, and then RNA was extracted according to the protocol using Maxwell RSC simplyRNA Cells kit (Promega). The extracted RNA was reverse-transcribed at xxx, the expression levels of CD8 positive T cell-associated markers (Granzyme B, Interferon γ), M1 macrophage-associated genes (H2ab1, CD80), M2 macrophage-associated genes (Mrc1, Arginase1), and housekeeping gene GAPDH were quantified using Taqman probe, and the expression level of each gene was standardized with the value of GAPDH.

Tables 44 to 46 show the variations of CD8 positive T cell-associated markers (Granzyme B, Interferon γ), M1 macrophage-associated genes (H2ab1, CD80), and M2 macrophage-associated genes (Mrc1, Arg1), which are calculated by averaging the data from five or six individuals in each group and presented as ratio data when the vehicle value is set to 1.

[Table 44]

| Test | Route of administration | Dosage | Dosing period (days) | Schedule (times/week) | Granzyme B | Interferon $\gamma$ |
|---|---|---|---|---|---|---|
| Empty liposome | Intravenous | -- | 15 | 1 | 1 | 1 |
| Liposome formulation of Example 1 | Intravenous | 10mg/kg | 15 | 1 | 2.6 | 12.9 |
| Anti-PD-1 antibody | Peritoneal | 200μg/head | 15 | 2 | 0.7 | 1.2 |
| Combined use of liposome formulation of Example 1 and anti-PD-1 antibody | Intravenous and peritoneal | 10mg/kg and 200μg/head | 15 | 1 and 2 | 3.4 | 17.8 |

[Table 45]

| Test | Route of administration | Dosage | Dosing period (days) | Schedule (times/week) | CD80 | H2ab1 |
|---|---|---|---|---|---|---|
| Empty liposome | Intravenous | -- | 15 | 1 | 1 | 1 |
| Liposome formulation of Example 1 | Intravenous | 10mg/kg | 15 | 1 | 1.9 | 1.6 |
| Anti-PD-1 antibody | Peritoneal | 200μg/head | 15 | 2 | 0.8 | 1.1 |
| Combined use of liposome formulation of Example 1 and anti-PD-1 antibody | Intravenous and peritoneal | 10mg/kg and 200μg/head | 15 | 1 and 2 | 1.7 | 1.6 |

[Table 46]

| Test | Route of administration | Dosage | Dosing period (days) | Schedule (times/week) | Mrc1 | Arg1 |
|---|---|---|---|---|---|---|
| Empty liposome | Intravenous | -- | 15 | 1 | 1 | 1 |
| Liposome formulation of Example 1 | Intravenous | 10mg/kg | 15 | 1 | 0.6 | 0.7 |
| Anti-PD-1 antibody | Peritoneal | 200μg/head | 15 | 2 | 0.8 | 0.5 |
| Combined use of liposome formulation of Example 1 and anti-PD-1 antibody | Intravenous and peritoneal | 10mg/kg and 200μg/head | 15 | 1 and 2 | 0.5 | 0.7 |

By the administration of the liposome formulation of Example 1, it was shown that in the "liposome formulation group of Example 1" or the "combined use group of the liposome formulation and the anti-PD-1 antibody", the CD8 positive T cell-associated genes (Granzyme B, Interferon $\gamma$) in the tumor increased by 2 times and 10 times or more, respectively, M1 macrophage-associated genes (H2ab1, CD80) increased by 1.5 times and 1.5 times or more, respectively, and M2 macrophage-associated genes (Mrc1, Arginase1) decreased by 0.6 times and 0.7 times or less, respectively, compared to the empty liposome administration group. From the above, it was shown that, also in the mouse renal cancer Renca model, the tumor microenvironment was changed from an immunosuppressive environment dominated by M2 macrophages to an inflammatory environment dominated by M1 macrophages by the liposome formulation of Example 1. This change suggested that, in the Renca model, the single agent of the liposome formulation of Example 1 and the combined use of the liposome formulation and the anti-PD-1 antibody exhibited remarkable drug efficacy.

Test Example 34. Intracytoplasmic ssDNA evaluation test in cells

Replication stress in the tumor was evaluated using Example 1.

After 24 hours from seeding of mouse breast cancer cells EMT6 and mouse renal cancer cells Renca, the cells were treated with Example 1 and DMSO and cultured for 24 hours. Thereafter, the cells were collected, and cytoplasmic components were extracted using NE-PER Nuclear and Cytoplasmic Extraction Reagents (Thermo Fisher Scientific Inc.). After protein quantification and concentration adjustment, ssDNA was quantified using Qbit ssDNA Assay Kit (Thermo Fisher Scientific Inc.).

The quantitative results in each cell are shown in Figure 14.

As shown in Figure 14, by the treatment of Example 1, the ssDNA in cytoplasm increased 1.3 times in EMT6 and 1.5 times in Renca. Since the increase in ssDNA in cytoplasm is one of markers of DNA replication stress, Example 1 was shown to induce replication stress in these cells.

As shown in Test Example 34, the treatment of Example 1 resulted in accumulation of ssDNA in cytoplasm. The accumulation of ssDNA is caused by DNA damage due to DNA replication stress and repair abnormalities. The accumulated cytoplasmic ssDNA activates the STING pathway, releases Type I interferons and cytokines, and activates immune cells in tumor including dendritic cells as shown in Test Example 32. The above action is considered to be one of the mechanisms by which Example 1 exhibits a remarkable drug efficacy when used concomitantly with an anti-PD-1 antibody in an anti-PD-1 antibody-resistant tumor.

In view of the results of Test Examples 1 to 13, the compounds of the invention exhibited high CHK1 inhibitory activity (Test Example 1) and a high cancer cell growth suppressing effect (Test Example 2). Furthermore, the compounds of the invention are characterized by low risk of cardiotoxicity (Test Example 3) and efficient encapsulation into a liposome (Test Examples 6, 6A, and 6B).

The inventors newly found a problem to be solved of having a risk of hepatotoxicity from prexasertib, and the compound represented by formula (3) (pyridine derivative with a nitrogen atom at a specific position) in particular, overcomes the problem with high safety (Test Example 4). In addition, the compound represented by formula (3) in particular, has excellent pharmacokinetics (Test Example 7).

The compound of Example 1, which is a representative compound encompassed by formula (3), has a 5-fold or greater CHK1 inhibitory activity relative to prexasertib (Test Example 1), and has a significant effect of exhibiting a potent cell growth suppression effect (Test Example 2). In addition, it was found that the compound of Example 1 has a much lower cardiotoxicity than prexasertib (Test Example 3) with no risk of hepatotoxicity, which is the new problem of prexasertib (Test Example 4), and has a different effect of significantly reduced risk of hemotoxicity relative to prexasertib (Test Example 5 and Test Example 10). Furthermore, the compound of Example 1 exhibits excellent pharmacokinetics (Test Example 7), is efficiently encapsulated into a liposome, and exhibits excellent liposome encapsulation operability (Test Examples 6, 6A, and 6B).

Furthermore, the compound of Example 1 prepared as a liposome formulation has a significant effect of exhibiting an excellent antitumor effect *in vitro* and *in vivo,* while having a different effect of high safety (Test Example 8, Test Example 9, and Test Example 11 to Test Example 14). Combined use of an existing anticancer agent with a compound of Example 1 has a feature of both excellent antitumor effect and high safety (Test Example 15 to Test Example 21). Since the compound of Example 1 has a more significant effect of selectively inhibiting CHK1 compared to prexasertib, the compound exhibits an excellent antitumor effect from CHK1 inhibition while avoiding expression of toxicity due to non-selective kinase inhibition (Test Example 22 to Test Example 23).

In view of the above, the compound of Example 1 has an unexpected effect from having a significant effect of higher efficacy as well as a different effect of exhibiting higher safety compared to prexasertib. The liposome formulation of Example 1 is an excellent agent having an unexpected effect with excellent blood retention, antitumor effect, and high safety.

The present disclosure relates to a CHK1 inhibitor, which is an antitumor agent with a high therapeutic effect while reducing side effects. As a result of diligent study, the inventors found that the compound represented by formula (1) has a potent inhibitory action against CHK1 and an excellent antitumor action. In particular, a series of compounds having a pyridine ring with a nitrogen atom at a specific position, represented by formula (3), was confirmed to exhibit a significant and different effect to complete the present invention.

Specifically, the inventors created the series of compounds of Examples 1 to 38, which exhibits a very high CHK1 inhibitory activity (Test Example 1), high cancer cell toxicity (Test Example 2), as well as a different effect of high safety in view of weak hERG inhibition (Test Example 3). Furthermore, the inventors have found the problem to be solved of having a risk of hepatotoxicity from prexasertib, and found that the compound of the invention has a different effect of overcoming the problem (Test Example 4). In particular, "compound represented by formula (3) having a pyridine ring with a nitrogen atom at a specific substitution position" represented by Example 1 and the like has a very significant and different effect.

The compounds of Example 1 and the like represented by formula (3) exhibit high safety with respect to hemotoxicity without suppressing human bone marrow cell colony formation, unlike prexasertib (Test Example 5), with a very high liposome encapsulation efficiency and excellent liposome encapsulation operability (Test Examples 6, 6A, and 6B), and high blood retention due to preparation into a liposome formulation (Test Example 7). Such a liposome formulation, despite having a tumor regression effect that cannot be achieved by prexasertib in a tumor-bearing model

(Test Example 8), has very high safety (Test Examples 9 and 10). Example 1 itself has excellent pharmacokinetic and pharmacodynamic profiles (Test Example 11), and the liposome formulation exhibited an effect of prolonging survival period that cannot be achieved by prexasertib (Test Examples 12 and 13) and broad efficacy against various cancers (Test Example 14). Combined use of Example 1 prepared as a liposome formulation and various anticancer agents (cisplatin, gemcitabine, or PD-1 antibody) exhibited a significant effect (Test Examples 15 to 21). The compound of Example 1, since it has a more significant effect of selectively suppressing CHK1 activity compared to prexasertib, exhibits an excellent antitumor effect due to CHK1 inhibition while avoiding expression of toxicity due to non-selective kinase inhibition (Test Examples 22 to 23). The compound of Example 1 was shown to change the tumor microenvironment from an immunosuppressive environment to an inflammatory environment (Test Example 24).

It was shown that a CD8 positive T cell centered immune reaction was induced by administration of the liposome formulation of Example 1, and the reaction was further enhanced by combined use with an anti-PD-1 antibody (Test Example 25). Administration of Example 1 was shown to reduce expression of VEGF$\alpha$ (Test Example 26). It was shown that the combined use group of the liposome formulation of Example 1 and an anti-PD-1 antibody achieved complete remission with higher probability than the single administration group of the liposome formulation of Example 1, and the complete remission mouse acquired long-term immune memory (Test Example 27). It was revealed that the T cell clonality was remarkably increased in the administration group of Test Example 1 as compared with the empty liposome administration group, and the CD8 positive T cell proportion and the M1-M2 macrophage ratio were each 5 times or more in the administration group of Test Example 1 and in the combined use group of Test Example 1 and the anti-PD-1 antibody, as compared with the empty liposome administration group (Test Example 28).

Example 1 prepared as a liposome formulation, when administered as a single agent or combined use with an anti-PD-1 antibody, exhibited an exceptionally remarkably antitumor effect against "tumor-bearing mice transplanted with Renca or LLC cells", which are models in which an anti-PD-1 antibody does not exhibits an antitumor effect or is unlikely to exhibit an antitumor effect (Test Examples 29 and 30). Furthermore, Example 1 prepared as a liposome formulation, when administered as a single agent or combined use with an anti-PD-1 antibody, increased the CD8 positive T cell rate in the tumor of tumor-bearing mice transplanted with Renca or LLC cells, and changes the tumor microenvironment from an immunosuppressive environment to an inflammatory environment (Test Examples 31 and 32). In addition, Example 1 prepared as a liposome formulation, when administered as a single agent or combined use with an anti-PD-1 antibody, increased the CD8 positive T cell-associated genes (Granzyme B, Interferon $\gamma$) and M1 macrophage-associated genes (H2ab1, CD80) and decreased M2 macrophage-associated genes (Mrc1, Arginase1) of tumor-bearing mice transplanted with Renca cells, indicating that it has an exceptionally remarkably effect on changing the tumor microenvironment from an immunosuppressive environment dominated by M2 macrophages to an inflammatory environment dominated by M1 macrophages (Test Example 33). Moreover, when mouse breast cancer cells EMT6 and mouse renal cancer cells Renca were treated with Example 1, the ssDNA in cytoplasm increased, indicating that Example 1 has an exceptionally remarkably effect on inducing DNA replication stress in the cells (Test Example 34).

As described above, the present disclosure is exemplified by the use of its preferred embodiments. It is understood that the scope of the present disclosure should be interpreted solely based on the Claims. The present application claims priority to Japanese Patent Application No. 2022-87166 (filed on May 27, 2022). It is understood that the entire content thereof is incorporated herein by reference. It is also understood that any patent, any patent application, and any references cited herein should be incorporated herein by reference in the same manner as the contents are specifically described herein.

[Industrial Applicability]

The compounds of the present disclosure potently inhibit Checkpoint Kinase 1 (CHK1). These liposome formulations exhibit a potent antitumor effect based on CHK1 inhibitory action by achieving sustained drug exposure due to the controlled release effect thereof. Therefore, the compound of the present disclosure and pharmaceutically acceptable salt thereof, a pharmaceutical composition comprising the same, and liposome formulation comprising the same are useful as a therapeutic agent or prophylactic agent for a pathological condition associated with CHK1.

## Claims

1. A therapeutic agent and/or prophylactic agent for a cancer patient exhibiting therapeutic resistance to an immune checkpoint inhibitor, comprising, as an active ingredient, a CHK1 inhibitor, or a liposome encapsulating a CHK1 inhibitor.

2. The therapeutic agent and/or prophylactic agent of claim 1, wherein the CHK1 inhibitor is used concomitantly with an immune checkpoint inhibitor.

**3.** The therapeutic agent and/or prophylactic agent of claim 2, wherein the CHK1 inhibitor and the immune checkpoint inhibitor are administered simultaneously.

**4.** The therapeutic agent and/or prophylactic agent of any one of claim 2 or 3, wherein the immune checkpoint inhibitor is administered after the CHK1 inhibitor is administered.

**5.** The therapeutic agent and/or prophylactic agent of any one of claims 2 to 4, wherein the anti-PD-1 antibody is further administered after the CHK1 inhibitor and the immune checkpoint inhibitor are administered simultaneously.

**6.** The therapeutic agent and/or prophylactic agent of any one of claims 2 to 4, wherein the anti-PD-1 antibody is further administered three days or later after the CHK1 inhibitor and the immune checkpoint inhibitor are administered simultaneously.

**7.** The therapeutic agent and/or prophylactic agent of any one of claims 2 to 6, wherein the immune checkpoint inhibitor is an anti-PD-1 antibody or an anti-PD-L1 antibody.

**8.** The therapeutic agent and/or prophylactic agent of any one of claims 2 to 6, wherein the immune checkpoint inhibitor is an anti-PD-1 antibody.

**9.** The therapeutic agent and/or prophylactic agent of any one of claims 2 to 6, wherein the anti-PD-1 antibody is pembrolizumab, nivolumab, spartalizumab, or cemiplimab.

**10.** The therapeutic agent and/or prophylactic agent of any one of claims 1 to 9, wherein the number of CD3 positive cells in the tumor tissue of the cancer patient is lower than that in tumor tissue of an anti-PD-1 antibody-responsive patient.

**11.** The therapeutic agent and/or prophylactic agent of any one of claims 1 to 10, wherein the number of CD8 positive cells in the tumor tissue of the cancer patient is lower than that in tumor tissue of an anti-PD-1 antibody-responsive patient.

**12.** The therapeutic agent and/or prophylactic agent of any one of claims 1 to 11, wherein the number of CD8 positive cells in the tumor tissue of the cancer patient is 0.135 or less.

**13.** The therapeutic agent and/or prophylactic agent of any one of claims 1 to 12, wherein an M1-M2 ratio of macrophages in the tumor tissue of the cancer patient is 21 or less.

**14.** The therapeutic agent and/or prophylactic agent of any one of claims 1 to 13, wherein the number of CD206 positive cells in the tumor tissue of the cancer patient is higher than that in tumor tissue of an anti-PD-1 antibody-responsive patient.

**15.** The therapeutic agent and/or prophylactic agent of any one of claims 1 to 14, wherein the number of IAd positive cells in the tumor tissue of the cancer patient is lower than that in tumor tissue of an anti-PD-1 antibody-responsive patient.

**16.** The therapeutic agent and/or prophylactic agent of any one of claims 1 to 15, wherein the number of CD62L negative CD44 positive cells in the lymph nodes of the cancer patient is decreased compared to lymph nodes in an anti-PD-1 antibody-responsive patient.

**17.** The therapeutic agent and/or prophylactic agent of any one of claims 1 to 16, wherein PD-L1 expression in the tumor tissue of the cancer patient is lower than that in tumor tissue of an anti-PD-1 antibody-responsive patient.

**18.** The therapeutic agent and/or prophylactic agent of any one of claims 1 to 17, wherein VEGFa expression in the tumor tissue of the cancer patient is higher than that in tumor tissue of an anti-PD-1 antibody-responsive patient.

**19.** The therapeutic agent and/or prophylactic agent of any one of claims 1 to 17, wherein Granzyme B expression in the tumor tissue of the cancer patient is lower than that in tumor tissue of an anti-PD-1 antibody-responsive patient.

**20.** The therapeutic agent and/or prophylactic agent of any one of claims 1 to 17, wherein Interferon $\gamma$ expression in the tumor tissue of the cancer patient is lower than that in tumor tissue of an anti-PD-1 antibody-responsive patient.

**21.** The therapeutic agent and/or prophylactic agent of any one of claims 1 to 17, wherein H2ab1 expression in the tumor

tissue of the cancer patient is lower than that in tumor tissue of an anti-PD-1 antibody-responsive patient.

**22.** The therapeutic agent and/or prophylactic agent of any one of claims 1 to 17, wherein CD80 expression in the tumor tissue of the cancer patient is lower than that in tumor tissue of an anti-PD-1 antibody-responsive patient.

**23.** The therapeutic agent and/or prophylactic agent of any one of claims 1 to 17, wherein Mrc1 expression in the tumor tissue of the cancer patient is higher than that in tumor tissue of an anti-PD-1 antibody-responsive patient.

**24.** The therapeutic agent and/or prophylactic agent of any one of claims 1 to 17, wherein Arginase1 expression in the tumor tissue of the cancer patient is higher than that in tumor tissue of an anti-PD-1 antibody-responsive patient.

**25.** The therapeutic agent and/or prophylactic agent of any one of claims 1 to 17, wherein DNA replication stress in the tumor tissue of the cancer patient is lower than that in tumor tissue of an anti-PD-1 antibody-responsive patient.

**26.** A therapeutic agent and/or prophylactic agent for the prophylaxis of recurrence of cancer after remission, comprising, as an active ingredient, a CHK1 inhibitor, or a liposome encapsulating a CHK1 inhibitor.

**27.** The therapeutic agent and/or prophylactic agent of claim 26, wherein the CHK1 inhibitor is used concomitantly with an immune checkpoint inhibitor.

**28.** The therapeutic agent and/or prophylactic agent of any one of claim 26 or 27, wherein the immune checkpoint inhibitor is an anti-PD-1 antibody or an anti-PD-L1 antibody.

**29.** The therapeutic agent and/or prophylactic agent of any one of claim 26 or 27, wherein the immune checkpoint inhibitor is an anti-PD-1 antibody.

**30.** The therapeutic agent and/or prophylactic agent of any one of claim 26 or 27, wherein the anti-PD-1 antibody is pembrolizumab, nivolumab, spartalizumab, or cemiplimab.

**31.** The therapeutic agent and/or prophylactic agent of any one of claims 26 to 30, wherein the cancer is at least one type of cancer selected from the group consisting of squamous cell carcinoma, melanoma, acute leukemia, chronic lymphocytic leukemia, chronic myelogenous leukemia, myelodysplastic syndrome, polycythemia vera, malignant lymphoma, plasma cell tumor, multiple myeloma, brain tumor, head and neck cancer, head and neck squamous cell carcinoma, esophageal cancer, thyroid cancer, lung cancer, small cell lung cancer, non small cell lung cancer, thymoma/thymic carcinoma, breast cancer, triple negative breast cancer, gastric cancer, gallbladder/bile duct cancer, liver cancer, hepatocellular carcinoma, pancreatic cancer, colon cancer, rectal cancer, MSI-high colon cancer, anal cancer, gastrointestinal stromal tumor, choriocarcinoma, endometrial cancer, cervical cancer, ovarian cancer, platinum-resistant ovarian cancer, PARP inhibitor-resistant ovarian cancer, bladder cancer, urothelial cancer, renal cancer, renal cell cancer, prostate cancer, testicular tumor, testicular germ cell tumor, ovarian germ cell tumor, Wilms tumor, malignant melanoma, neuroblastoma, osteosarcoma, Ewing sarcoma, chondrosarcoma, soft tissue sarcoma, or skin cancer.

**32.** The therapeutic agent and/or prophylactic agent of any one of claims 26 to 30, wherein the cancer is melanoma, squamous cell carcinoma, non small cell lung cancer, small cell lung cancer, head and neck squamous cell carcinoma, urothelial cancer, non-muscle invasive bladder cancer, kidney cancer, hepatocellular carcinoma, MSI-high colon cancer, esophageal cancer, colon cancer, rectal cancer, breast cancer, endometrial cancer, or ovarian cancer.

**33.** The therapeutic agent and/or prophylactic agent of any one of claims 26 to 30, wherein the cancer is at least one type of cancer selected from the group consisting of head and neck cancer, squamous cell carcinoma, non small cell lung cancer, anal cancer, breast cancer, triple negative breast cancer, ovarian cancer, platinum-resistant ovarian cancer, or endometrial cancer.

**34.** The therapeutic agent and/or prophylactic agent of any one of claims 26 to 30, wherein the cancer is a Tumor Mutation Burden-High (TMB-High) solid tumor.

**35.** The therapeutic agent and/or prophylactic agent of any one of claims 1 to 34, wherein the liposome further comprises a phospholipid.

**36.** The therapeutic agent and/or prophylactic agent of claim 35, wherein the phospholipid is one selected from the group consisting of phosphatidylcholine, phosphatidylglycerol, phosphatidic acid, phosphatidylethanolamine, phosphatidylserine, phosphatidylinositol, sphingomyelin, soybean lecithin, egg yolk lecithin, hydrogenated egg yolk lecithin, and hydrogenated soybean lecithin or a combination of two or more thereof.

**37.** The therapeutic agent and/or prophylactic agent of any one of claims 1 to 36, wherein the liposome further comprises sterol.

**38.** The therapeutic agent and/or prophylactic agent of any one of claims 1 to 37, wherein the sterol is cholesterol.

**39.** The therapeutic agent and/or prophylactic agent of any one of claims 1 to 38, wherein the liposome further comprises a polymer-modified lipid.

**40.** The therapeutic agent and/or prophylactic agent of claim 39, wherein a polymer moiety of the polymer-modified lipid is polyethylene glycol, polypropylene glycol, polyvinyl alcohol, polyvinylpyrrolidone, methoxypolyethylene glycol, methoxypolypropylene glycol, methoxypolyvinyl alcohol, methoxypolyvinylpyrrolidone, ethoxypolyethylene glycol, ethoxypolypropylene glycol, ethoxypolyvinyl alcohol, ethoxypolyvinylpyrrolidone, propoxypolyethylene glycol, propoxypolypropylene glycol, propoxypolyvinyl alcohol, or propoxypolyvinylpyrrolidone.

**41.** The therapeutic agent and/or prophylactic agent of claim 39 or 40, wherein a lipid moiety of the polymer-modified lipid is phosphatidylethanolamine or diacylglycerol.

**42.** The therapeutic agent and/or prophylactic agent of any one of claims 1 to 41, wherein the liposome encapsulating a CHK1 inhibitor comprises

(1) 40 to 70 mol% of phospholipid,
(2) 30 to 50 mol% of cholesterol, and
(3) 1 to 10 mol% of polymer-modified lipid.

**43.** The therapeutic agent and/or prophylactic agent of any one of claims 1 to 42, wherein the liposome further comprises an additive selected from the group consisting of inorganic acids, inorganic acid salts, organic acids, organic acid salts, saccharides, buffer, antioxidants, and polymers.

**44.** The therapeutic agent and/or prophylactic agent of any one of claims 1 to 43, wherein the CHK1 inhibitor is a compound represented by formula (1):

[Chemical Formula 1]

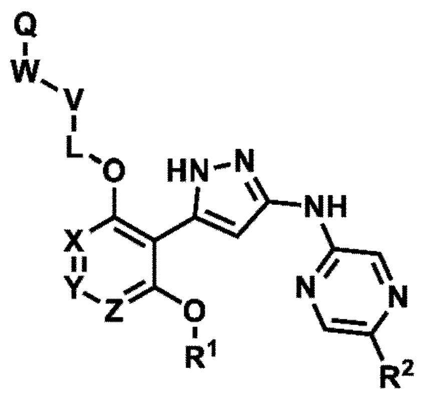

(1)

or a pharmaceutically acceptable salt thereof, wherein

$R^1$ represents a hydrogen atom, optionally substituted $C_{1-6}$ alkyl, optionally substituted $C_{3-10}$ cycloalkyl, an optionally substituted 3- to 10-membered saturated heterocyclic group, optionally substituted $C_{6-10}$ aryl, or optionally substituted 5-to 12-membered heteroaryl,

$R^2$ represents a hydrogen atom, a halogen atom, cyano, nitro, carboxyl, sulfonic acid, phosphoric acid, $-OR^3$, $-SR^3$, $-COR^4$, $-CO_2R^4$, $-CONR^5R^6$, $-SO_2R^4$, $-SO_2NR^5R^6$, $-OCOR^4$, $-OCO_2R^4$, $-OCONR^5R^6$, $-NR^5R^6$, $-NR^7COR^4$, $-NR^7CO_2R^4$, $-NR^7CONR^5R^6$, $-NR^7SO_2R^4$, $-NR^7SO_2NR^5R^6$, optionally substituted $C_{1-6}$ alkyl, optionally substituted $C_{2-6}$ alkenyl, optionally substituted $C_{2-6}$ alkynyl, optionally substituted $C_{3-10}$ cycloalkyl, an optionally substituted 3- to 10-membered saturated heterocyclic group, optionally substituted $C_{6-10}$ aryl, or optionally substituted 5-to 12-membered heteroaryl,

$R^3$ represents a hydrogen atom or $C_{1-6}$ alkyl,

$R^4$ represents $C_{1-6}$ alkyl,

$R^5$, $R^6$, and $R^7$ each independently represent a hydrogen atom or $C_{1-6}$ alkyl, wherein $R^5$ and $R^6$ that attach to the same nitrogen atom, when both are $C_{1-6}$ alkyl, together with the nitrogen atom to which each is attached, may form a 3- to 8-membered nitrogen-containing saturated heterocycle,

X, Y, and Z each independently represent $CR^8$ or a nitrogen atom, wherein X, Y, and Z are not simultaneously $CR^8$,

$R^8$, if there are multiple instances, each independently represent a hydrogen atom, a halogen atom, cyano, nitro, carboxyl, sulfonic acid, phosphoric acid, $-OR^9$, $-SR^9$, $-COR^{10}$, $-CO_2R^{10}$, $-CONR^{11}R^{12}$, $-SO_2R^{10}$, $-SO_2NR^{11}R^{12}$, $-OCOR^{10}$, $-OCO_2R^{10}$, $-OCONR^{11}R^{12}$, $-NR^{11}R^{12}$, $-NR^{13}COR^{10}$, $-NR^{13}CO_2R^{10}$, $-NR^{13}CONR^{11}R^{12}$, $-NR^{13}SO_2R^{10}$, $-NR^{13}SO_2NR^{11}R^{12}$, optionally substituted $C_{1-6}$ alkyl, optionally substituted $C_{2-6}$ alkenyl, optionally substituted $C_{2-6}$ alkynyl, optionally substituted $C_{3-10}$ cycloalkyl, an optionally substituted 3- to 10-membered saturated heterocyclic group, optionally substituted $C_{6-10}$ aryl, or optionally substituted 5-to 12-membered heteroaryl,

$R^9$ represents a hydrogen atom or $C_{1-6}$ alkyl,

$R^{10}$ represents $C_{1-6}$ alkyl,

$R^{11}$, $R^{12}$, and $R^{13}$ each independently represent a hydrogen atom or $C_{1-6}$ alkyl, wherein $R^{11}$ and $R^{12}$ that attach to the same nitrogen atom, when both are $C_{1-6}$ alkyl, together with the nitrogen atom to which each is attached, may form a 3- to 8-membered nitrogen-containing saturated heterocycle,

L represents a single bond or optionally substituted $C_{1-6}$ alkylene,

V represents a single bond, optionally substituted $C_{3-10}$ cycloalkylene, or an optionally substituted 3- to 10-membered divalent saturated heterocyclic group,

W represents a single bond or optionally substituted $C_{1-6}$ alkylene,

Q represents a hydrogen atom or $NHR^{14}$, and

$R^{14}$ represents a hydrogen atom, optionally substituted $C_{1-6}$ alkyl, optionally substituted $C_{3-10}$ cycloalkyl, or an optionally substituted 3- to 10-membered saturated heterocyclic group.

**45.** The therapeutic agent and/or prophylactic agent of claim 44, wherein formula (1) is represented by formula (2):

[Chemical Formula 2]

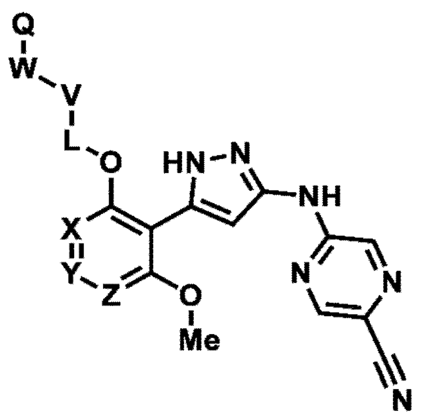

**(2)**

wherein

X, Y, and Z each independently represent $CR^8$ or a nitrogen atom, wherein X, Y, and Z are not simultaneously $CR^8$,

$R^8$, if there are multiple instances, each independently represent

a hydrogen atom,

a fluorine atom,

a chlorine atom,
a bromine atom,
$C_{1-6}$ alkyl (wherein the alkyl is optionally substituted with 1 to 2 of the same or different substituents selected from the group consisting of a fluorine atom, a hydroxyl group, $C_{1-3}$ alkoxy, $-NR^{16}R^{17}$, and cyano), or
5- to 6-membered heteroaryl (wherein the heteroaryl is optionally substituted with 1 to 2 of the same or different substituents selected from the group consisting of a fluorine atom, a hydroxyl group, $C_{1-3}$ alkyl, $C_{1-3}$ alkoxy, $-NR^{16}R^{17}$, and cyano),

L represents

a single bond, or
$C_{1-6}$ alkylene (wherein the alkylene is optionally substituted with 1 to 3 of the same or different substituents selected from the group consisting of a fluorine atom, a hydroxyl group, $C_{1-3}$ alkoxy, $-NR^{16}R^{17}$, and cyano),

V represents

a single bond,
$C_{3-10}$ cycloalkylene (wherein the cycloalkylene is optionally substituted with 1 to 3 of the same or different substituents selected from the group consisting of a fluorine atom, a hydroxyl group, $C_{1-3}$ alkyl, $C_{1-3}$ alkoxy, $-NR^{16}R^{17}$, and cyano), or
a 3- to 10-membered divalent saturated heterocyclic group (wherein the saturated heterocyclic group is optionally substituted with 1 to 3 of the same or different substituents selected from the group consisting of a fluorine atom, a hydroxyl group, $C_{1-3}$ alkyl optionally substituted with 1 to 2 hydroxyl groups or fluorine atoms, $C_{1-3}$ alkoxy, $-NR^{16}R^{17}$, and cyano),

W represents

a single bond, or
$C_{1-6}$ alkylene (wherein the alkylene is optionally substituted with 1 to 3 of the same or different substituents selected from the group consisting of a fluorine atom, a hydroxyl group, $C_{1-3}$ alkoxy, $-NR^{16}R^{17}$, and cyano),

Q represents a hydrogen atom or $NHR^{14}$,
$R^{14}$ represents

a hydrogen atom,
$C_{1-6}$ alkyl (wherein the alkyl is optionally substituted with 1 to 3 of the same or different substituents selected from the group consisting of a fluorine atom, a hydroxyl group, $C_{1-3}$ alkoxy, $-NR^{16}R^{17}$, and cyano),
$C_{3-10}$ cycloalkyl (wherein the cycloalkyl is optionally substituted with 1 to 3 of the same or different substituents selected from the group consisting of a fluorine atom, a hydroxyl group, $C_{1-3}$ alkyl, $C_{1-3}$ alkoxy, $-NR^{16}R^{17}$, and cyano), or
a 3- to 10-membered saturated heterocyclic group (wherein the saturated heterocyclic group is optionally substituted with 1 to 3 of the same or different substituents selected from the group consisting of a fluorine atom, a hydroxyl group, $C_{1-3}$ alkyl, $C_{1-3}$ alkoxy, $-NR^{16}R^{17}$, and cyano), and

$R^{16}$ and $R^{17}$ each independently represent a hydrogen atom or $C_{1-6}$ alkyl, and if there are multiple instances of $R^{16}$ or $R^{17}$, each of $R^{16}$ or $R^{17}$ may be the same or different, wherein $R^{16}$ and $R^{17}$ that attach to the same nitrogen atom, when both are $C_{1-6}$ alkyl, together with the nitrogen atom to which each is attached, may form a 3- to 8-membered nitrogen-containing saturated heterocycle.

**46.** The therapeutic agent and/or prophylactic agent of claim 44, wherein formula (1) is represented by formula (3):

[Chemical Formula 3]

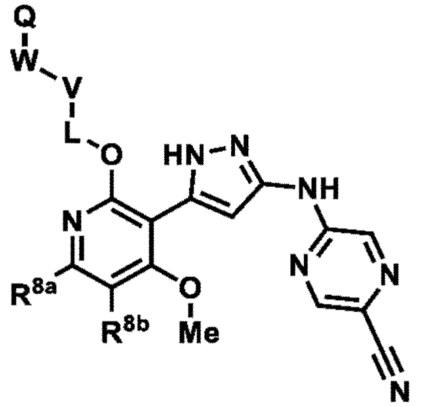

(3)

wherein,

$R^{8a}$ and $R^{8b}$ each independently represent a hydrogen atom, a fluorine atom, a chlorine atom, a bromine atom, or $C_{1-3}$ alkyl,
L represents a single bond or $C_{1-6}$ alkylene optionally substituted with 1 hydroxyl group,
V represents

a single bond,
$C_{3-7}$ cycloalkylene (wherein the cycloalkylene is optionally substituted with 1 substituent selected from the group consisting of a hydroxyl group and $C_{1-3}$ alkyl), or
a 3- to 7-membered divalent saturated heterocyclic group (wherein the saturated heterocyclic group is optionally substituted with 1 substituent selected from the group consisting of a fluorine atom, cyano, a hydroxyl group, and $C_{1-3}$ alkyl),

W represents

a single bond, or
$C_{1-3}$ alkylene optionally substituted with 1 hydroxyl group, and

Q is a hydrogen atom or $NH_2$.

**47.** The therapeutic agent and/or prophylactic agent of claim 46, wherein $R^{8a}$ and $R^{8b}$ are each independently a hydrogen atom, a fluorine atom or a chlorine atom.

**48.** The therapeutic agent and/or prophylactic agent of claim 46, wherein $R^{8a}$ and $R^{8b}$ are each independently a hydrogen atom, or a chlorine atom.

**49.** The therapeutic agent and/or prophylactic agent of any one of claims 46 to 48, wherein L is $C_{1-3}$ alkylene.

**50.** The therapeutic agent and/or prophylactic agent of any one of claims 46 to 49, wherein V is a single bond.

**51.** The therapeutic agent and/or prophylactic agent of any one of claims 46 to 50, wherein V is $C_{3-7}$ cycloalkylene.

**52.** The therapeutic agent and/or prophylactic agent of any one of claims 46 to 51, wherein W is a single bond.

**53.** The therapeutic agent and/or prophylactic agent of any one of claims 46 to 51, wherein W is $C_{1-3}$ alkylene.

**54.** The therapeutic agent and/or prophylactic agent of any one of claims 46 to 53, wherein Q is $NH_2$.

**55.** The therapeutic agent and/or prophylactic agent of claim 44, wherein formula (1) is represented by formula (4):

[Chemical Formula 4]

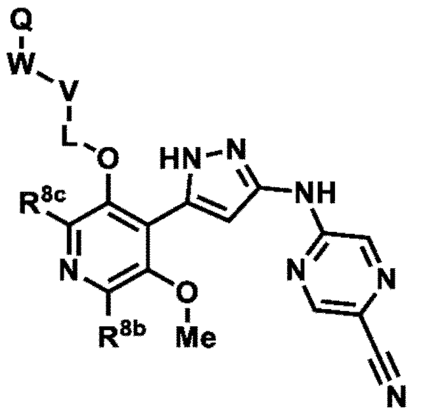

(4)

wherein

$R^{8b}$ and $R^{8c}$ each independently represent a hydrogen atom, a fluorine atom, a chlorine atom, a bromine atom, or $C_{1-3}$ alkyl,
L represents

a single bond, or
$C_{1-6}$ alkylene (wherein the alkylene is optionally substituted with 1 substituent selected from the group consisting of a fluorine atom, a hydroxyl group, and cyano),

V represents

a single bond,
$C_{3-7}$ cycloalkylene (wherein the cycloalkylene is optionally substituted with 1 substituent selected from the group consisting of a hydroxyl group and $C_{1-3}$ alkyl), or
a 3- to 7-membered divalent saturated heterocyclic group (wherein the saturated heterocyclic group is optionally substituted with 1 substituent selected from the group consisting of a fluorine atom, cyano, a hydroxyl group, and $C_{1-3}$ alkyl optionally substituted with 1 to 3 hydroxyl groups and fluorine atoms),

W represents

a single bond, or
$C_{1-3}$ alkylene optionally substituted with 1 hydroxyl group, and

Q is a hydrogen atom, $NH_2$, or NHMe.

**56.** The therapeutic agent and/or prophylactic agent of claim 55, wherein $R^{8b}$ and $R^{8c}$ are hydrogen atoms.

**57.** The therapeutic agent and/or prophylactic agent of any one of claim 55 or 56, wherein L is

a single bond, or
$C_{1-6}$ alkylene optionally substituted with 1 hydroxyl group or 1 fluorine atom.

**58.** The therapeutic agent and/or prophylactic agent of any one of claims 55 to 57, wherein V is

a single bond,
$C_{3-7}$ cycloalkylene, or
a 3- to 7-membered divalent saturated heterocyclic group (wherein the saturated heterocyclic group is optionally substituted with 1 substituent selected from the group consisting of a fluorine atom, a hydroxyl group, and $C_{1-3}$ alkyl).

**59.** The therapeutic agent and/or prophylactic agent of any one of claims 55 to 58, wherein W is

a single bond, or
$C_{1-3}$ alkylene.

**60.** The therapeutic agent and/or prophylactic agent of any one of claims 55 to 59, wherein Q is a hydrogen atom.

**61.** The therapeutic agent and/or prophylactic agent of any one of claims 55 to 59, wherein Q is $NH_2$.

**62.** The therapeutic agent and/or prophylactic agent of any one of claims 55 to 59, wherein Q is NHMe.

**63.** The therapeutic agent and/or prophylactic agent of claim 44, wherein formula (1) is represented by formula (5):

[Chemical Formula 5]

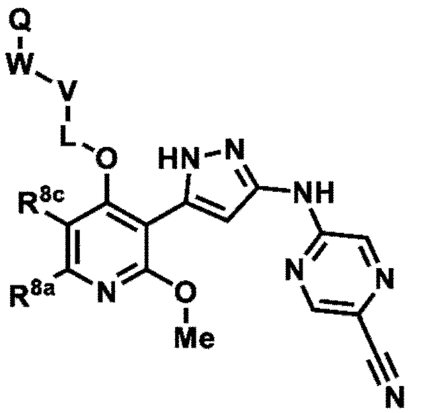

(5)

wherein

$R^{8a}$ and $R^{8c}$ each independently represent a hydrogen atom, a fluorine atom, a chlorine atom, a bromine atom, or $C_{1-3}$ alkyl,
L represents a single bond, or $C_{1-6}$ alkylene optionally substituted with 1 hydroxyl group,
V represents

a single bond,
$C_{3-7}$ cycloalkylene (wherein the cycloalkylene is optionally substituted with 1 substituent selected from the group consisting of a hydroxyl group and $C_{1-3}$ alkyl), or
a 3- to 7-membered divalent saturated heterocyclic group (wherein the saturated heterocyclic group is optionally substituted with 1 substituent selected from the group consisting of a fluorine atom, cyano, a hydroxyl group, and $C_{1-3}$ alkyl optionally substituted with 1 hydroxyl group),

W represents

a single bond, or
$C_{1-3}$ alkylene optionally substituted with 1 hydroxyl group, and

Q is a hydrogen atom or $NH_2$.

**64.** The therapeutic agent and/or prophylactic agent of claim 63, wherein $R^{8a}$ and $R^{8c}$ are hydrogen atoms.

**65.** The therapeutic agent and/or prophylactic agent of any one of claim 63 or 64, wherein L is $C_{1-6}$ alkylene optionally substituted with 1 hydroxyl group.

**66.** The therapeutic agent and/or prophylactic agent of any one of claims 63 to 65, wherein V is

a single bond,
$C_{3-7}$ cycloalkylene, or
a 3- to 7-membered divalent saturated heterocyclic group (wherein the saturated heterocyclic group is optionally substituted with 1 substituent selected from the group consisting of a hydroxyl group and $C_{1-3}$ alkyl optionally substituted with 1 hydroxyl group).

**67.** The therapeutic agent and/or prophylactic agent of any one of claims 63 to 66, wherein W is

a single bond, or
$C_{1-3}$ alkylene.

**68.** The therapeutic agent and/or prophylactic agent of any one of claims 63 to 67, wherein Q is a hydrogen atom.

**69.** The therapeutic agent and/or prophylactic agent of any one of claims 63 to 67, wherein Q is $NH_2$.

**70.** The therapeutic agent and/or prophylactic agent of claim 44, comprising a compound selected from the following compounds, or a pharmaceutically acceptable salt thereof:

5-({5-[2-(3-aminopropoxy)-4-methoxypyridin-3-yl]-1H-pyrazol-3-yl}amino)pyrazine-2-carbonitrile,
5-({5-[2-(3-aminopropoxy)-6-chloro-4-methoxypyridin-3-yl]-1H-pyrazol-3-yl}amino)pyrazine-2-carbonitrile,
5-({5-[3-(3-aminopropoxy)-5-methoxypyridin-4-yl]-1H-pyrazol-3-yl}amino)pyrazine-2-carbonitrile,
5-({5-[4-(3-aminopropoxy)-2-methoxypyridin-3-yl]-1H-pyrazol-3-yl}amino)pyrazine-2-carbonitrile,
5-[(5-{3-[(3-fluoroazetidin-3-yl)methoxy]-5-methoxypyridin-4-yl}-1H-pyrazol-3-yl)amino]pyrazine-2-carbonitrile,
5-[(5-{2-methoxy-4-[(3-methylazetidin-3-yl)methoxy]pyridin-3-yl}-1H-pyrazol-3-yl)amino]pyrazine-2-carbonitrile,
5-[(5-{4-[(3-hydroxyazetidin-3-yl)methoxy]-2-methoxypyridin-3-yl}-1H-pyrazol-3-yl)amino]pyrazine-2-carbonitrile,
5-{[5-(4-{[3-(hydroxymethyl)azetidin-3-yl]methoxy}-2-methoxypyridin-3-yl)-1H-pyrazol-3-yl]amino}pyrazine-2-carbonitrile,
5-[(5-{3-[(3R)-3-aminobutoxy]-5-methoxypyridin-4-yl}-1H-pyrazol-3-yl)amino]pyrazine-2-carbonitrile,
5-[(5-{3-[(3S)-3-aminobutoxy]-5-methoxypyridin-4-yl}-1H-pyrazol-3-yl)amino]pyrazine-2-carbonitrile,
5-{[5-(3-{[1-(aminomethyl)cyclopropyl]methoxy}-5-methoxypyridin-4-yl)-1H-pyrazol-3-yl]amino}pyrazine-2-carbonitrile,
5-[(5-{3-methoxy-5-[(morpholin-2-yl)methoxy]pyridin-4-yl}-1H-pyrazol-3-yl)amino]pyrazine-2-carbonitrile,
5-[(5-{3-methoxy-5-[(morpholin-2-yl)methoxy]pyridin-4-yl}-1H-pyrazol-3-yl)amino]pyrazine-2-carbonitrile,
5-[(5-{3-[(2S)-3-amino-2-hydroxypropoxy]-5-methoxypyridin-4-yl}-1H-pyrazol-3-yl)amino]pyrazine-2-carbonitrile,
N-{5-[2-(3-aminopropoxy)-4-methoxypyridin-3-yl]-1H-pyrazol-3-yl}-5-chloropyrazin-2-amine,
N-{5-[2-(3-aminopropoxy)-4-methoxypyridin-3-yl]-1H-pyrazol-3-yl}-5-(trifluoromethyl)pyrazin-2-amine,
5-({5-[4-(3-aminopropoxy)-6-methoxypyrimidin-5-yl]-1H-pyrazol-3-yl}amino)pyrazine-2-carbonitrile,
5-({5-[3-(azetidin-3-yl)methoxy-5-methoxypyridin-4-yl]-1H-pyrazol-3-yl}amino)pyrazine-2-carbonitrile,
5-{[5-(3-{[(1R,3S)-3-aminocyclohexyl]oxy}-5-methoxypyridin-4-yl)-1H-pyrazol-3-yl]amino}pyrazine-2-carbonitrile,
(S)-5-[(5-{3-[(3-fluoropyrrolidin-3-yl)methoxy]-5-methoxypyridin-4-yl}-1H-pyrazol-3-yl)amino]pyrazine-2-carbonitrile,
(S)-5-[(5-{3-methoxy-[5-(pyrrolidin-3-yl)methoxy]pyridin-4-yl}-1H-pyrazol-3-yl)amino]pyrazine-2-carbonitrile,
(R)-5-[(5-{3-[(3-fluoropyrrolidin-3-yl)methoxy]-5-methoxypyridin-4-yl}-1H-pyrazol-3-yl)amino]pyrazine-2-carbonitrile,
5-{[5-(4-{[1-(aminomethyl)cyclopropyl]methoxy}-6-methoxypyrimidin-5-yl)-1H-pyrazol-3-yl]amino}pyrazine-2-carbonitrile,
5-({5-[3-(3-aminopropoxy)-5-(fluoromethoxy)pyridin-4-yl]-1H-pyrazol-3-yl}amino)pyrazine-2-carbonitrile,
5-[(5-{3-methoxy-5-[(3-methylazetidin-3-yl)methoxy]pyridin-4-yl}-1H-pyrazol-3-yl)amino]pyrazine-2-carbonitrile,
5-{[5-(3-{[3-(difluoromethyl)azetidin-3-yl]methoxy}-5-methoxypyridin-4-yl)-1H-pyrazol-3-yl]amino}pyrazine-2-carbonitrile,
5-[(5-{3-[(2S)-3-amino-2-methoxypropoxy]-5-methoxypyridin-4-yl}-1H-pyrazol-3-yl)amino]pyrazine-2-carbonitrile,

5-[(5-{3-[(2R)-3-amino-2-methoxypropoxy]-5-methoxypyridin-4-yl}-1H-pyrazol-3-yl)amino]pyrazine-2-carbonitrile,
5-[(5-{3-[(2S)-3-amino-2-fluoropropoxy]-5-methoxypyridin-4-yl}-1H-pyrazol-3-yl)amino]pyrazine-2-carbonitrile,
5-[(5-{3-[(2R)-3-amino-2-fluoropropoxy]-5-methoxypyridin-4-yl}-1H-pyrazol-3-yl)amino]pyrazine-2-carbonitrile,
5-[(5-{3-methoxy-5-[3-(methylamino)propoxy]pyridin-4-yl}-1H-pyrazol-3-yl)amino]pyrazine-2-carbonitrile,
5-{[5-(3-{[(1R,3R)-3-aminocyclopentyl]oxy}-5-methoxypyridin-4-yl)-1H-pyrazol-3-yl]amino}pyrazine-2-carbonitrile,
5-[(5-{3-[(1R)-1-(azetidin-3-yl)ethoxy]-5-methoxypyridin-4-yl}-1H-pyrazol-3-yl)amino]pyrazine-2-carbonitrile,
5-[(5-{3-[(1R)-1-(3-hydroxyazetidin-3-yl)ethoxy]-5-methoxypyridin-4-yl}-1H-pyrazol-3-yl)amino]pyrazine-2-carbonitrile,
5-{[5-(3-methoxy-5-{[(1R,3R)-3-(methylamino)cyclopentyl]oxy}pyridin-4-yl)-1H-pyrazol-3-yl]amino}pyrazine-2-carbonitrile,
5-{[5-(3-{[(1R,2S,4S,5S)-4-aminobicyclo[3.1.0]hexan-2-yl]oxy}-5-methoxypyridin-4-yl)-1H-pyrazol-3-yl]amino}pyrazine-2-carbonitrile,
5-{[5-(2-{[1-(aminomethyl)cyclopropyl]methoxy}-4-methoxypyridin-3-yl)-1H-pyrazol-3-yl]amino}pyrazine-2-carbonitrile, and
5-{[5-(4-{[1-(aminomethyl)cyclopropyl]methoxy}-2-methoxypyridin-3-yl)-1H-pyrazol-3-yl]amino}pyrazine-2-carbonitrile.

**71.** The therapeutic agent and/or prophylactic agent of claim 44, comprising a compound selected from the following compounds, or a pharmaceutically acceptable salt thereof:

5-({5-[2-(3-aminopropoxy)-4-methoxypyridin-3-yl]-1H-pyrazol-3-yl}amino)pyrazine-2-carbonitrile,
5-({5-[3-(3-aminopropoxy)-5-methoxypyridin-4-yl]-1H-pyrazol-3-yl}amino)pyrazine-2-carbonitrile, and
5-({5-[4-(3-aminopropoxy)-2-methoxypyridin-3-yl]-1H-pyrazol-3-yl}amino)pyrazine-2-carbonitrile.

**72.** The therapeutic agent and/or prophylactic agent of claim 44, comprising a compound selected from the following compound, or a pharmaceutically acceptable salt thereof:
5-({5-[4-(3-aminopropoxy)-2-methoxypyridin-3-yl]-1H-pyrazol-3-yl}amino)pyrazine-2-carbonitrile.

**73.** The therapeutic agent and/or prophylactic agent of claim 44, comprising the following compound or a pharmaceutically acceptable salt thereof:
5-({5-[2-(3-aminopropoxy)-4-methoxypyridin-3-yl]-1H-pyrazol-3-yl}amino)pyrazine-2-carbonitrile.

**74.** The therapeutic agent and/or prophylactic agent of claim 44, comprising the following compound or a pharmaceutically acceptable salt thereof:
5-({5-[3-(3-aminopropoxy)-5-methoxypyridin-4-yl]-1H-pyrazol-3-yl}amino)pyrazine-2-carbonitrile.

Fig.1

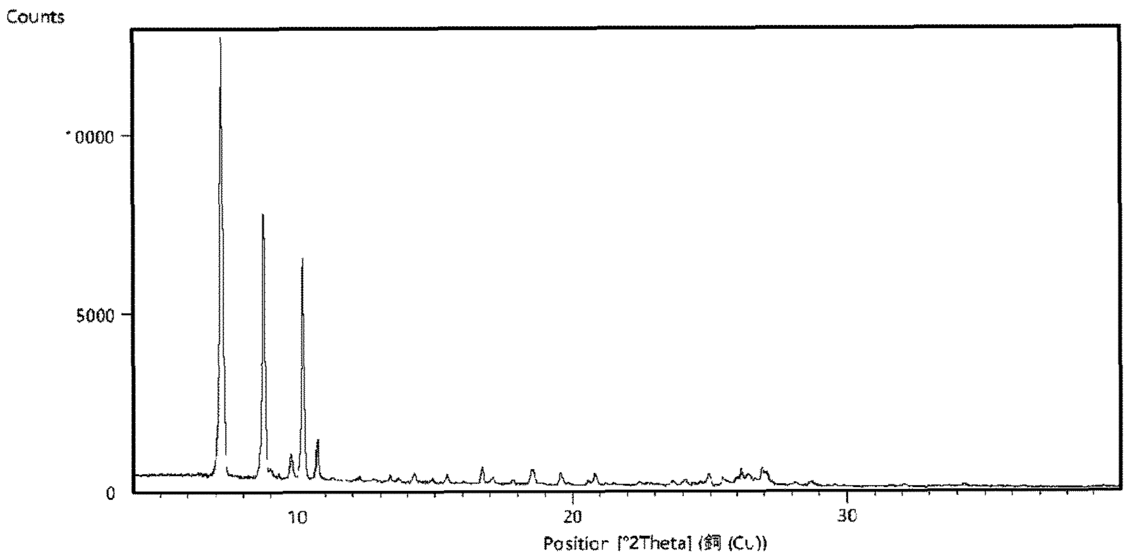
Counts
10000
5000
0
10
20
30
Position [°2Theta] (銅 (Cu))

Fig.2

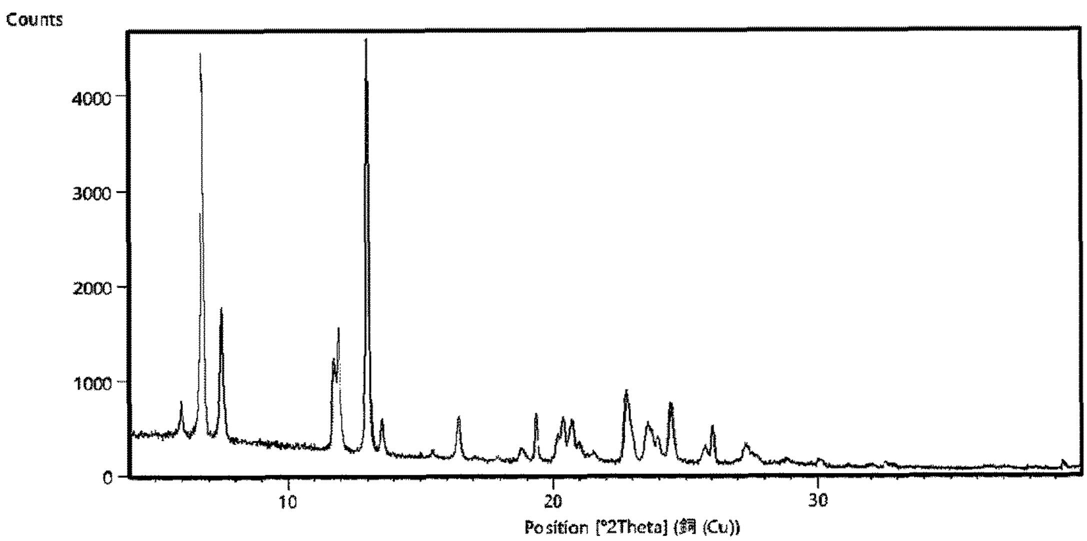
Counts
4000
3000
2000
1000
0
10
20
30
Position [°2Theta] (銅 (Cu))

Fig.3

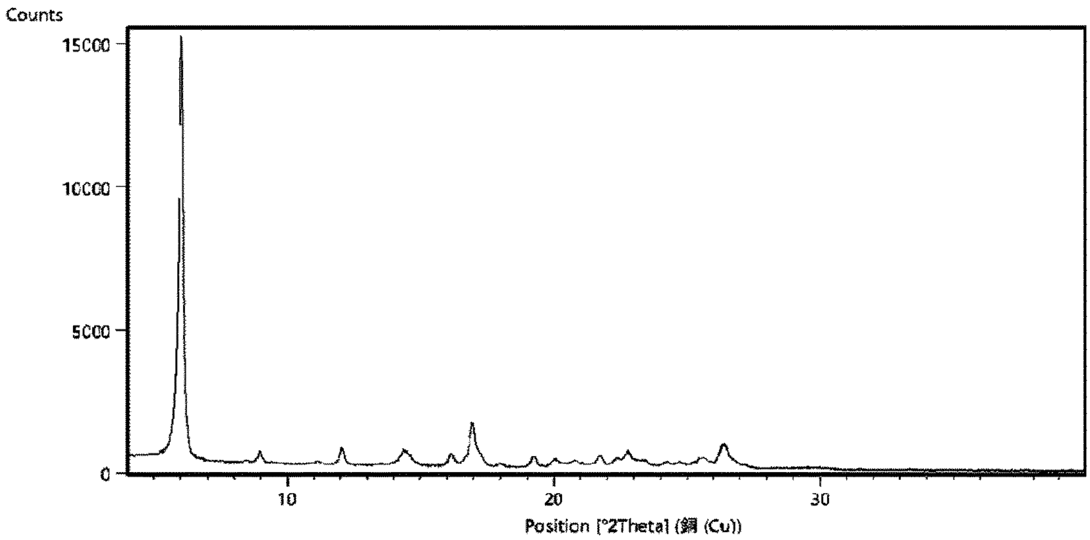
Counts
15000
10000
5000
0
10
20
30
Position [°2Theta] (銅 (Cu))

Fig.4

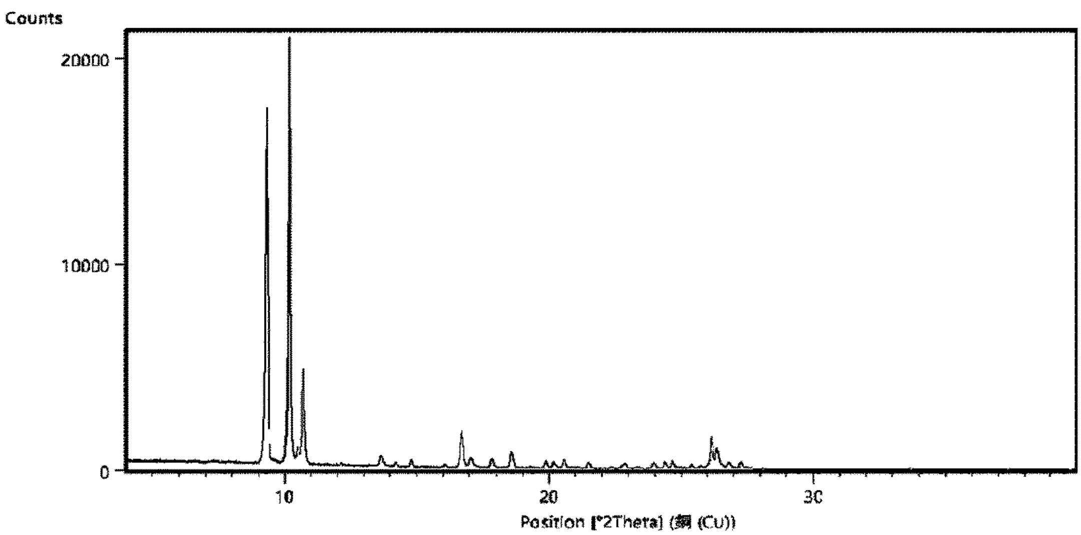
Counts
20000
10000
0
10
20
30
Position [°2Theta] (銅 (Cu))

Fig.5

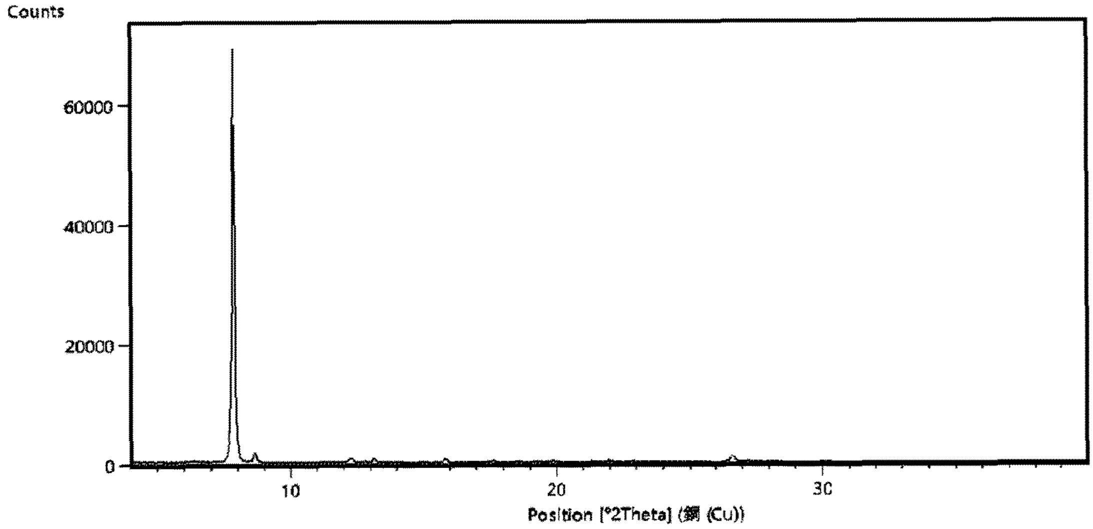
Counts
60000
40000
20000
0
10
20
30
Position [°2Theta] (銅 (Cu))

Fig.6

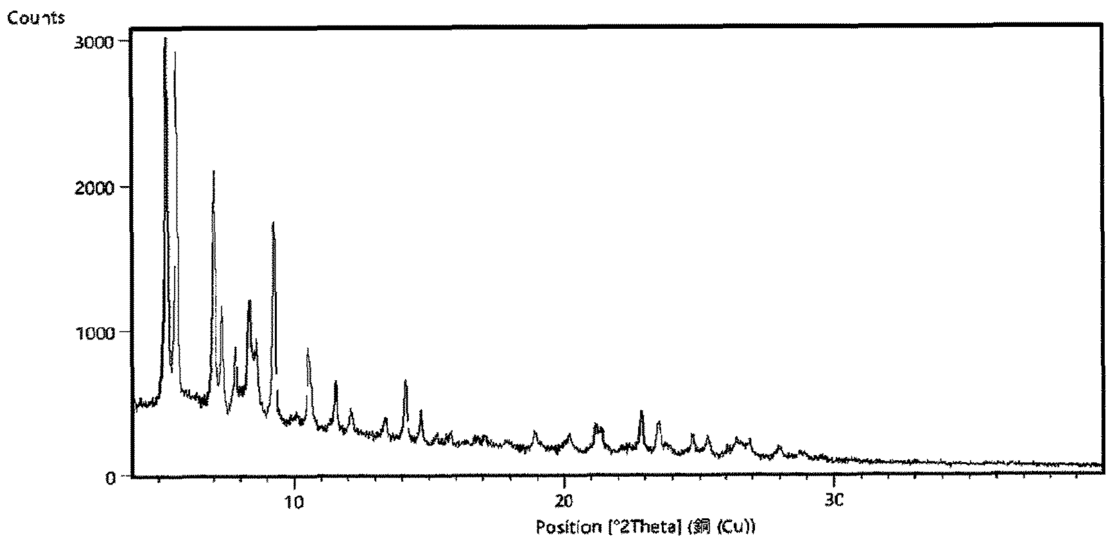
Counts
3000
2000
1000
0
10
20
30
Position [°2Theta] (銅 (Cu))

Fig.7

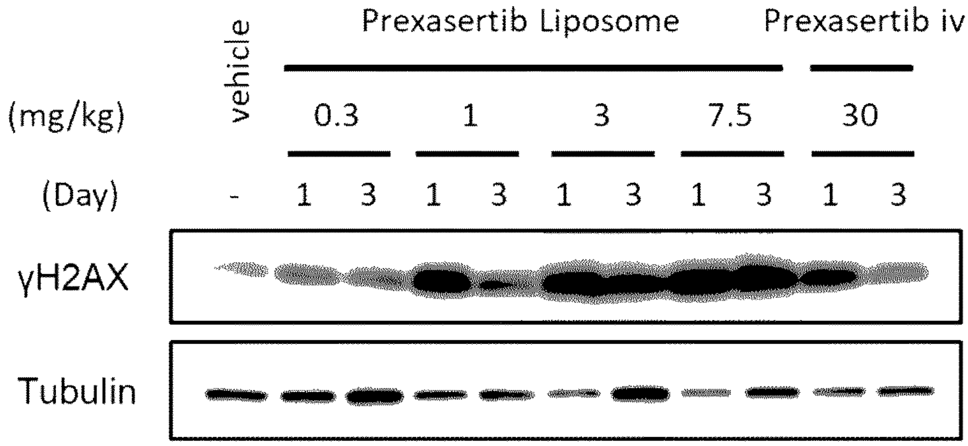
vehicle
Prexasertib Liposome
Prexasertib iv
(mg/kg)
0.3
1
3
7.5
30
(Day)
-
1 3 1 3 1 3 1 3 1 3
γH2AX
Tubulin

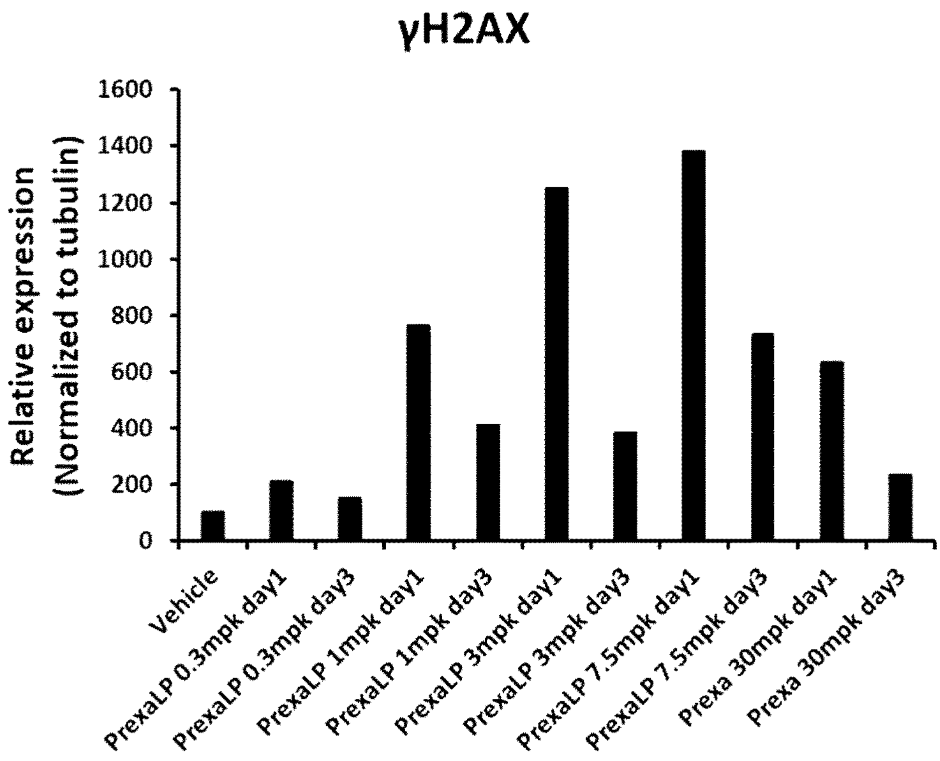
γH2AX
Relative expression
(Normalized to tubulin)
0
200
400
600
800
1000
1200
1400
1600
Vehicle
PrexaLP 0.3mpk day1
PrexaLP 0.3mpk day3
PrexaLP 1mpk day1
PrexaLP 1mpk day3
PrexaLP 3mpk day1
PrexaLP 3mpk day3
PrexaLP 7.5mpk day1
PrexaLP 7.5mpk day3
Prexa 30mpk day1
Prexa 30mpk day3

Fig.8

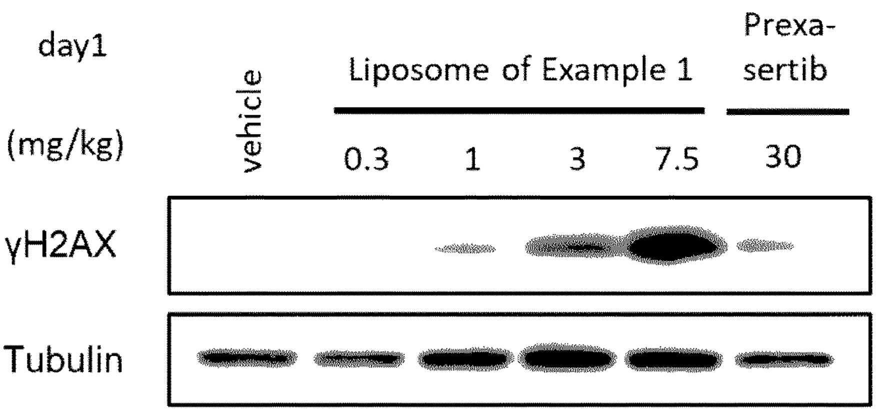
day1
(mg/kg)
vehicle
Liposome of Example 1
Prexa-
sertib
0.3
1
3
7.5
30
γH2AX
Tubulin

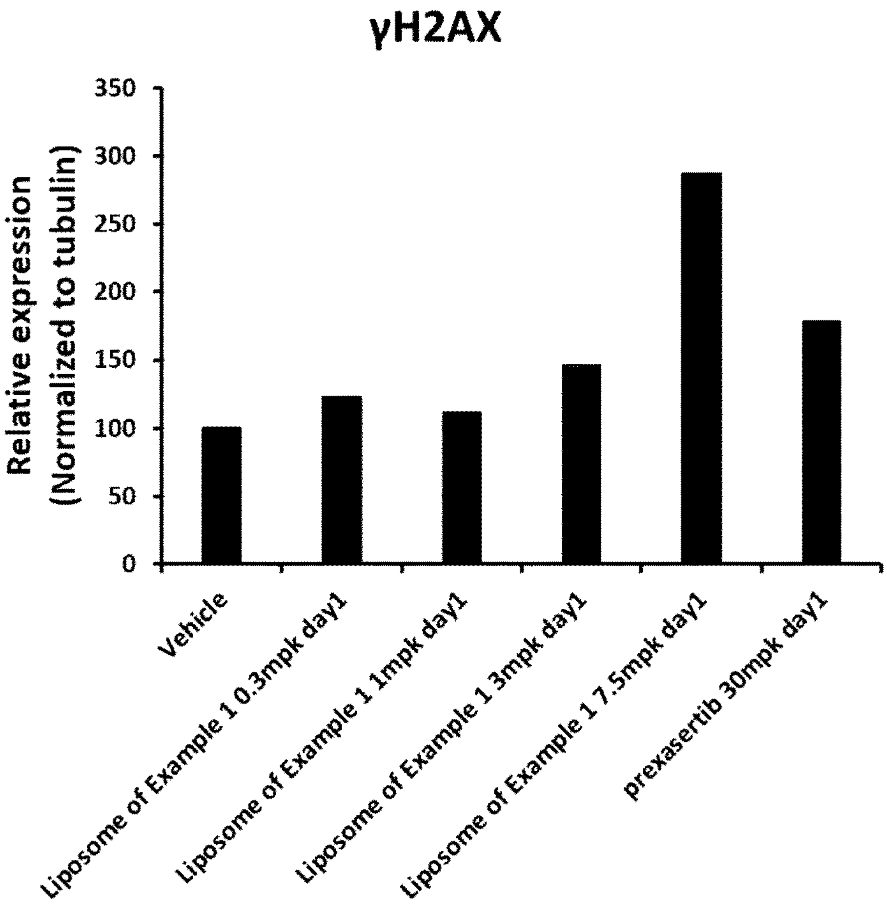
γH2AX
Relative expression
(Normalized to tubulin)
350
300
250
200
150
100
50
0
Vehicle
Liposome of Example 1 0.3mpk day1
Liposome of Example 1 1mpk day1
Liposome of Example 1 3mpk day1
Liposome of Example 1 7.5mpk day1
prexasertib 30mpk day1

Fig.9

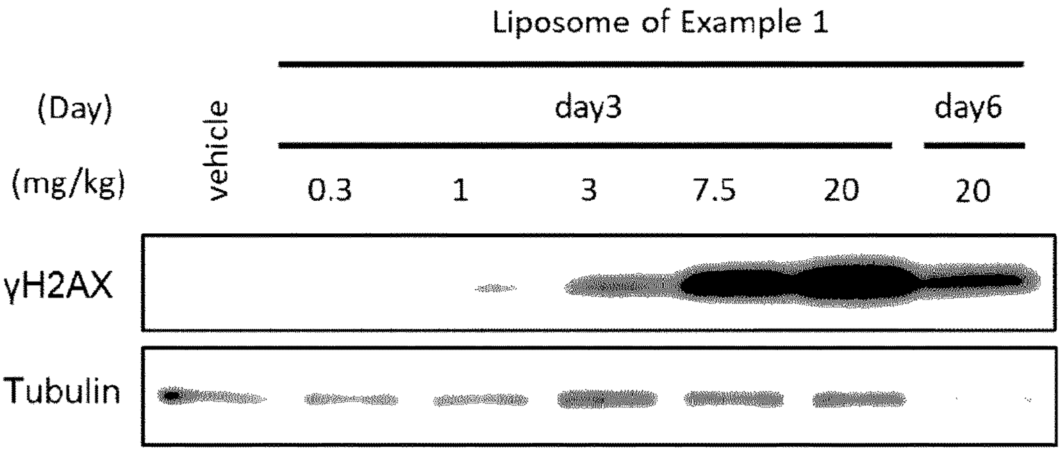
Liposome of Example 1
(Day)
vehicle
day3
day6
(mg/kg)
0.3
1
3
7.5
20
20
γH2AX
Tubulin

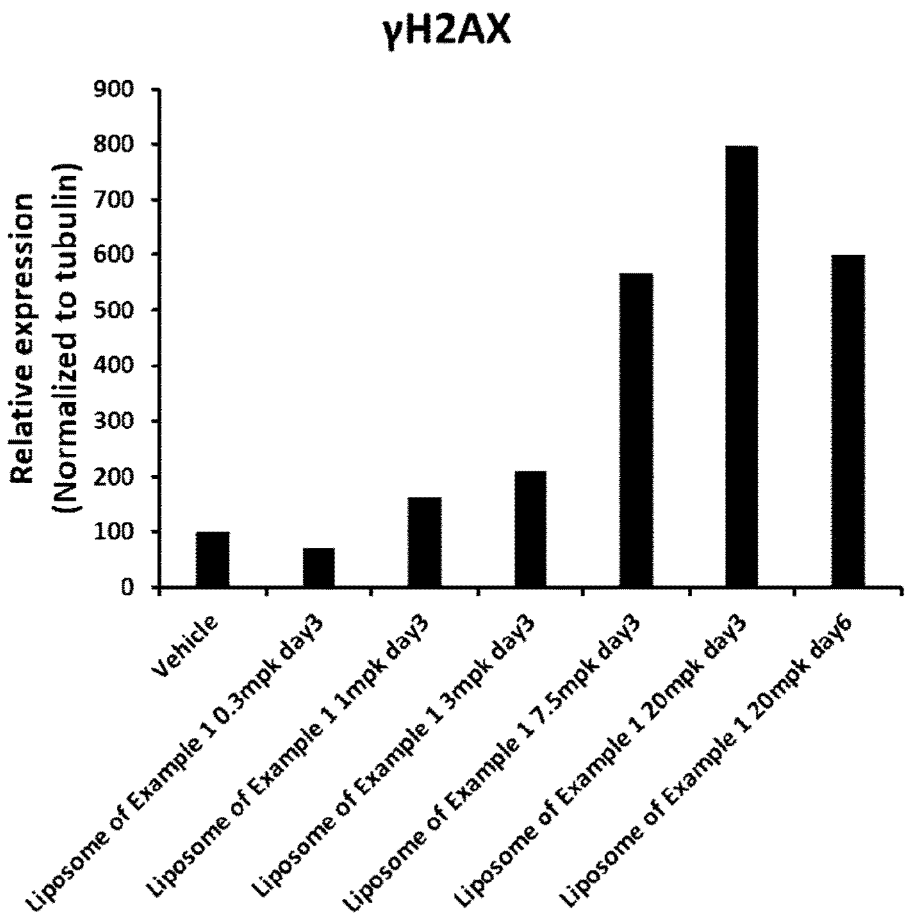
γH2AX
Relative expression
(Normalized to tubulin)
900
800
700
600
500
400
300
200
100
0
Vehicle
Liposome of Example 1 0.3mpk day3
Liposome of Example 1 1mpk day3
Liposome of Example 1 3mpk day3
Liposome of Example 1 7.5mpk day3
Liposome of Example 1 20mpk day3
Liposome of Example 1 20mpk day6

Fig.10

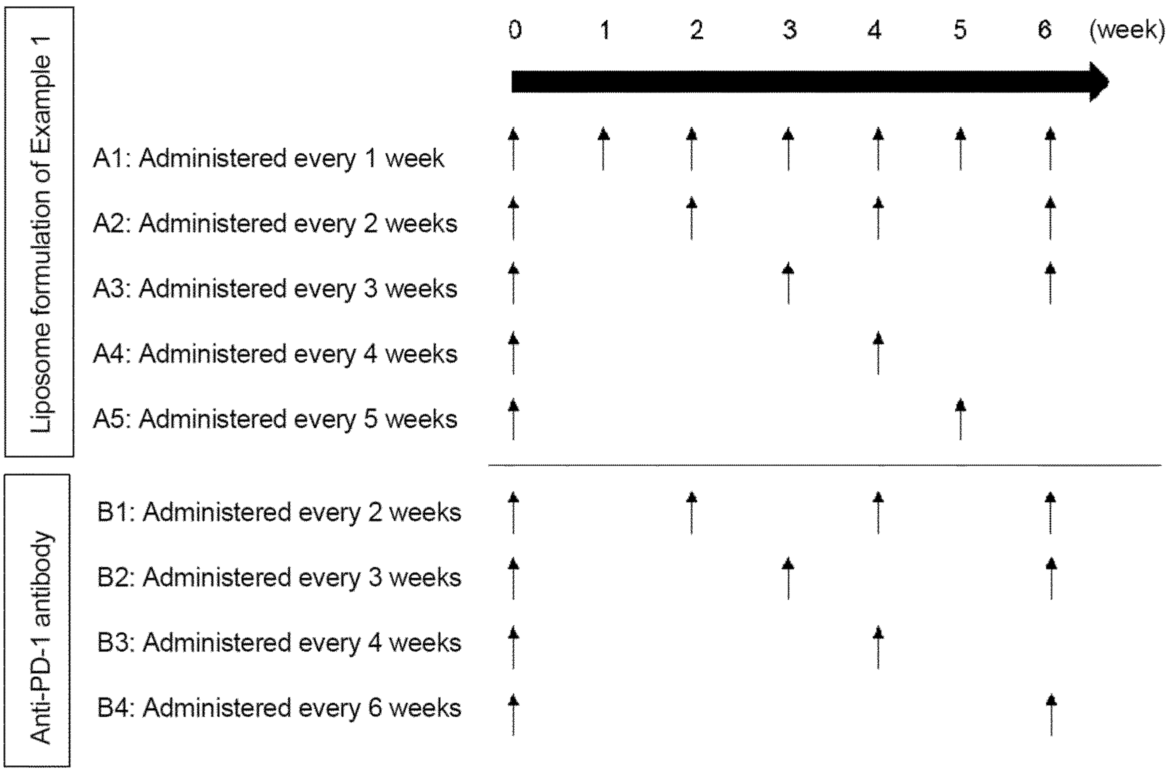
Liposome formulation of Example 1
Anti-PD-1 antibody
0 1 2 3 4 5 6 (week)
A1: Administered every 1 week
A2: Administered every 2 weeks
A3: Administered every 3 weeks
A4: Administered every 4 weeks
A5: Administered every 5 weeks
B1: Administered every 2 weeks
B2: Administered every 3 weeks
B3: Administered every 4 weeks
B4: Administered every 6 weeks

Fig.11

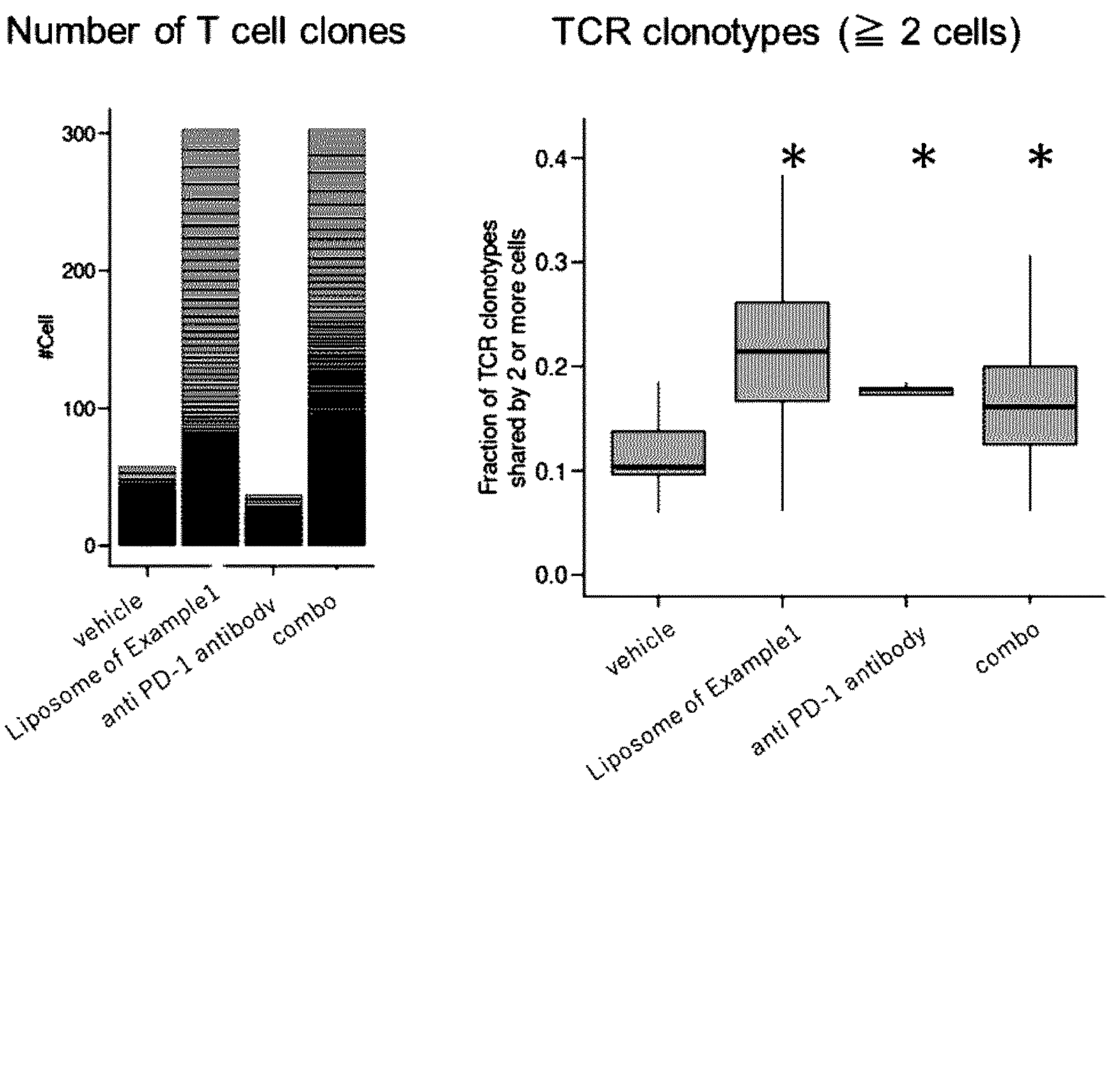
Number of T cell clones
#Cell
300
200
100
0
vehicle
Liposome of Example1
anti PD-1 antibody
combo
TCR clonotypes (≧ 2 cells)
Fraction of TCR clonotypes shared by 2 or more cells
0.4
0.3
0.2
0.1
0.0
*
*
*
vehicle
Liposome of Example1
anti PD-1 antibody
combo

Fig.12

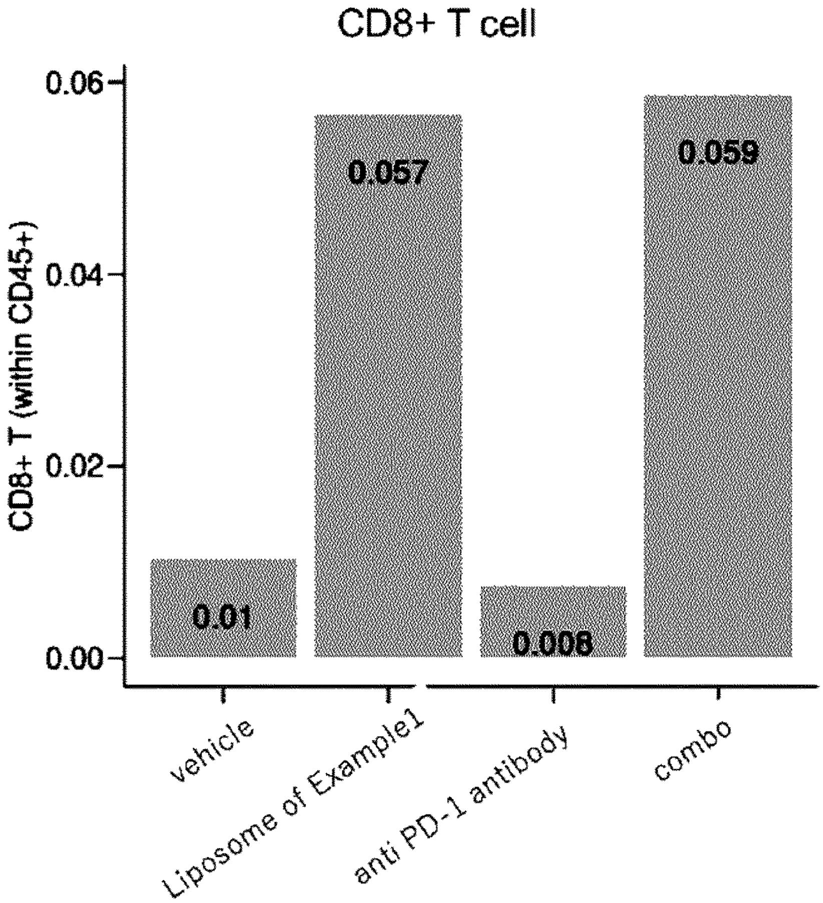
CD8+ T cell
CD8+ T (within CD45+)
0.06
0.04
0.02
0.00
0.057
0.059
0.01
0.008
vehicle
Liposome of Example1
anti PD-1 antibody
combo

Fig.13

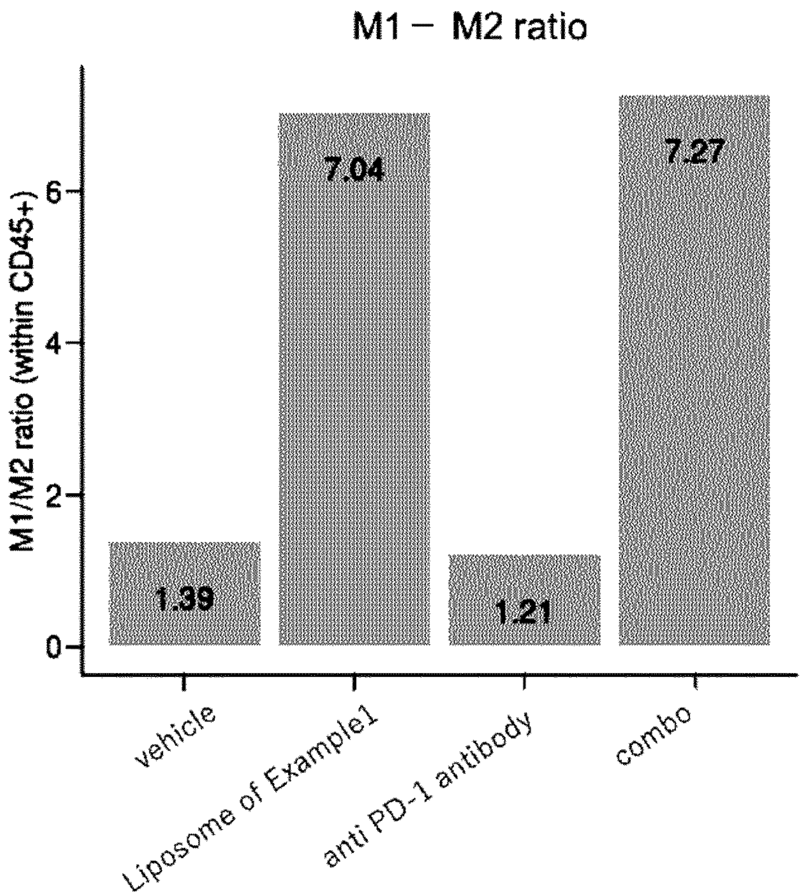
M1 – M2 ratio
M1/M2 ratio (within CD45+)
0
2
4
6
1.39
7.04
1.21
7.27
vehicle
Liposome of Example1
anti PD-1 antibody
combo

Fig.14

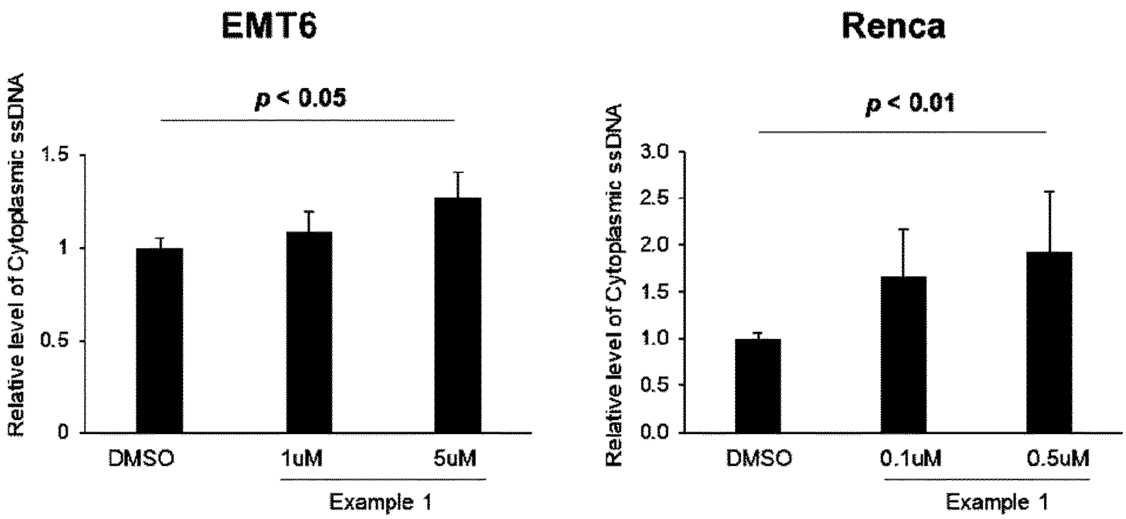
EMT6
p < 0.05
Relative level of Cytoplasmic ssDNA
1.5
1
0.5
0
DMSO
1uM
5uM
Example 1
Renca
p < 0.01
Relative level of Cytoplasmic ssDNA
3.0
2.5
2.0
1.5
1.0
0.5
0.0
DMSO
0.1uM
0.5uM
Example 1

## INTERNATIONAL SEARCH REPORT

International application No.
**PCT/JP2023/019755**

**A. CLASSIFICATION OF SUBJECT MATTER**

***A61K 45/00***(2006.01)i; ***A61K 9/127***(2006.01)i; ***A61K 31/497***(2006.01)i; ***A61K 31/506***(2006.01)i; ***A61K 31/5377***(2006.01)i; ***A61K 39/395***(2006.01)i; ***A61K 47/02***(2006.01)i; ***A61K 47/24***(2006.01)i; ***A61K 47/26***(2006.01)i; ***A61K 47/28***(2006.01)i; ***A61K 47/32***(2006.01)i; ***A61K 47/34***(2017.01)i; ***A61P 35/00***(2006.01)i; ***A61P 43/00***(2006.01)i
FI: A61K45/00; A61K9/127; A61P35/00; A61P43/00 121; A61K39/395 T; A61K47/24; A61K47/28; A61K47/34; A61K47/32; A61K47/02; A61K47/26; A61K31/497; A61K31/5377; A61K31/506; A61P43/00 111

According to International Patent Classification (IPC) or to both national classification and IPC

**B. FIELDS SEARCHED**

Minimum documentation searched (classification system followed by classification symbols)

A61K45/00; A61K9/127; A61K31/497; A61K31/506; A61K31/5377; A61K39/395; A61K47/02; A61K47/24; A61K47/26; A61K47/28; A61K47/32; A61K47/34; A61P35/00; A61P43/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2020/198510 A1 (SIERRA ONCOLOGY, INC.) 01 October 2020 (2020-10-01) claim 1, examples 9-12, fig. 9, 10, 15, 21, 22, 26, 27 | 1-25 |
| Y | | 26-70 |
| A | | 71-74 |
| X | US 2021/0332136 A1 (RESEARCH DEVELOPMENT FOUNDATION) 28 October 2021 (2021-10-28) claims 1, 2, 4, 8, paragraph [0010], examples 7, 12, fig. 22, 27, 30 | 1-25 |
| Y | | 35-70 |
| A | | 71-74 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **03 August 2023** | **15 August 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No. **PCT/JP2023/019755**

**C. DOCUMENTS CONSIDERED TO BE RELEVANT**

| Category* | Citation of document, with indication, where appropriate, of the relevant passages | Relevant to claim No. |
|---|---|---|
| X | SEN, T. et al. Targeting DNA Damage Response Promotes Antitumor Immunity through STING-Mediated T-cell Activation in Small Cell Lung Cancer. Cancer Discovery. 2019, vol. 9, no. 5, pp. 646-661, DOI: 10.1158/2159-8290.CD-18-1020<br>abstract, "CHK1i Augments Anti–PD-L1 Antibody–Induced Antitumor Immunity", fig. 1, 2 | 1-25 |
| Y | | 26-70 |
| A | | 71-74 |
| Y | LAMPERT, E. J. et al. Cell cycle checkpoint kinase (CHK)1 inhibition induces immune modulation in recurrent BRCA wild-type high-grade serous ovarian cancer (HGSOC) patients. Cancer Research. 2019, vol. 79, no. 13, supp., abstract number: 5000, DOI: 10.1158/1538-7445.SABCS18-5000<br>particularly, "results", "conclusions" | 26-70 |
| A | | 71-74 |
| Y | YOSHIMOTO, Y. et al. Anti-tumor immune responses induced by radiotherapy: A review. Fukushima J. Med. Sci. 2015, 61(1), pp. 13-22, DOI: 10.5387/fms.2015-6<br>p. 13, right column, second paragraph to p. 14, left column, first paragraph | 26-70 |
| A | | 71-74 |
| Y | WO 2012/064548 A1 (ELI LILLY AND COMPANY) 18 May 2012 (2012-05-18)<br>examples 1-6 | 44-70 |
| A | | 71-74 |
| A | WO 2010/077758 A1 (ELI LILLY AND COMPANY) 08 July 2010 (2010-07-08)<br>examples 1-5 | 71-74 |
| A | WO 2017/132928 A1 (PHARMAENGINE, INC.) 10 August 2017 (2017-08-10)<br>examples 1-17 | 71-74 |
| A | JP 2017-508787 A (CASCADIAN THERAPEUTICS, INC.) 30 March 2017 (2017-03-30)<br>examples 1-163 | 71-74 |
| P, A | WO 2022/114189 A1 (SUMITOMO PHARMA CO., LTD.) 02 June 2022 (2022-06-02)<br>claims, examples | 1-74 |

Form PCT/ISA/210 (second sheet) (January 2015)

**INTERNATIONAL SEARCH REPORT**

International application No.
**PCT/JP2023/019755**

**Box No. III Observations where unity of invention is lacking (Continuation of item 3 of first sheet)**

This International Searching Authority found multiple inventions in this international application, as follows:

Document 1: WO 2020/198510 A1 (SIERRA ONCOLOGY, INC.) 01 October 2020 (2020-10-01), claim 1, examples 9-11, fig. 9, 10, 15, 22 & US 2022/0184091 A1, claim 1, examples 9-11, fig. 9, 10, 15, 22
Document 2: US 2021/0332136 A1 (RESEARCH DEVELOPMENT FOUNDATION) 28 October 2021 (2021-10-28), claims 1, 2, 4, 8, paragraph [0010], examples 7, 12, fig. 22, 27, 30 & WO 2021/183973 A1, claims 1, 2, 4, 8, paragraph [0010], examples 7, 12, fig. 22, 27, 30

Document 1 (claim 1) discloses a method for treating a cancer, comprising administering to a subject with the cancer effective amounts of a chemotherapeutic agent, a Chk1 inhibitor and an immune checkpoint inhibitor.

Specifically, document 1 indicates that the Chk1 inhibitor, SRA737, significantly increases both the total PD-L1 levels and cell surface PD-L1 expression in the SCLC cell lines, and has anti-proliferative and potential immunomodulatory effects in multiple cancer models (example 9 and fig. 9 and 10), while an anti-PD-L1 agent alone showed no anti-tumor response in cancer model mice having SCLC cells, animals treated with a single agent of SRA737 showed significant reduction in tumor growth as compared to either the vehicle or the anti-PD-L1 treatment, and further complete inhibition of tumor growth was observed in mice treated with the combination therapy comprising SRA737 and the anti-PD-L1 agent of B7-H11 (example 10 and fig. 15), and similarly, while monotherapy treatment with anti-PD1 showed very limited activity in model mice of colon (MC38), bladder (MBT-2) and pancreatic (Pan02) cancer, SRA737 monotherapy induced moderate tumor growth inhibition and combination treatment of SRA737 and anti-PD1 resulted in improved efficacy (example 11 and fig. 22).

The SCLC, MC38, MBT-2 and Pan02 cells are resistant to anti-PD-1 or anti-PD-L1 antibody and thus are "cancer resistant to immune checkpoint inhibitors" and thus document 1 is considered to disclose treatment of the cells with a Chk1 inhibitor SRA737 alone or a combination thereof with anti-PD-1 or anti-PD-L1 antibody.

Document 2 (claim 1) discloses a method for treating a cancer, comprising, if the expression of cytoplasmic or intracellular PD-L1 is decreased relative to a normal control, administering an anti-cancer therapy to a subject. Document 2 indicates that the anti-cancer therapy is a DDR (DNA damage response) inhibitor such as a Chk1 inhibitor (claims 2, 4 and 8), and tumor PD-L1 can be a negative biomarker for DDR inhibitor efficacy (paragraph [0010]).

Specifically, document 2 indicates that in 4T1 cells or ID8agg cells that are resistant to αPD-L1 immune checkpoint blockade immunotherapy despite expressing PD-L1, PD-L1 knockdown by shRNA (PD-L1lo) or PD-L1 deletion by CRISPR/Cas9 (PD-L1KO) enhanced sensitivity to Chk1 inhibitor rabusertib in vitro (example 7 and fig. 22 and 30), and combination of αPD-L1 antibody with rabusertib significantly decreased murine 4T1 tumors in highly immunodeficient NSG mice (example 12 and fig. 27).

The 4T1 cells or ID8agg cells are resistant to αPD-L1 immune checkpoint blockade immunotherapy and thus are "cancer resistant to immune checkpoint inhibitors" and thus document 2 is considered to disclose treatment of the cells with a Chk1 inhibitor rabusertib and αPD-L1 antibody.

(Invention 1) Parts of claims 1-25 and claims 35-74

Claim 1-3 lack novelty in light of documents 1 and 2, and thus do not have a special technical feature.

However, claim 4 dependent on claim 1 has the special technical feature. Thus, claims 1-4 are classified as invention 1.

Furthermore, parts of claims 5-25 and claims 35-74 directly or indirectly referring to claim 1 are dependent on claim 1 are inventively linked with claim 1, and thus are classified as invention 1.

(Invention 2) Parts of claims 26-34 and claims 35-74

Parts of claims 26-34 and claims 35-74 which do not directly or indirectly refer to claim 1 cannot be said to share a same or corresponding special technical feature with claims 1-25 classified as invention 1.

Further, the parts of claims 26-34 and claims 35-74 which do not directly or indirectly refer to claim 1 are not dependent on claim 1 classified as invention 1, and are not substantially identical to or similarly closely related to any of the claims classified as invention 1.

Therefore, the parts of claims 26-34 and claims 35-74 which do not directly or indirectly refer to claim 1 cannot be classified as invention 1.

The parts of claims 26-34 and claims 35-74 which do not directly or indirectly refer to claim 1 have a special technical feature, and thus are classified as invention 2.

Form PCT/ISA/210 (continuation of first sheet) (January 2015)

**INTERNATIONAL SEARCH REPORT**

International application No.
**PCT/JP2023/019755**

**Box No. III Observations where unity of invention is lacking (Continuation of item 3 of first sheet)**

1. ☑ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☑ No protest accompanied the payment of additional search fees.

Form PCT/ISA/210 (continuation of first sheet) (January 2015)

**INTERNATIONAL SEARCH REPORT**
**Information on patent family members**

International application No.
**PCT/JP2023/019755**

| Patent document cited in search report | Publication date (day/month/year) | Patent family member(s) | Publication date (day/month/year) |
|---|---|---|---|
| WO 2020/198510 A1 | 01 October 2020 | US 2022/0184091 A1<br>claim 1, examples 9-12, fig. 9, 10, 15, 21, 22, 26, 27 | |
| US 2021/0332136 A1 | 28 October 2021 | WO 2021/183973 A1<br>claims 1, 2, 4, 8, paragraph [0010], examples 7, 12, fig. 22, 27, 30 | |
| WO 2012/064548 A1 | 18 May 2012 | JP 2013-541586 A<br>examples 1-6 | |
| WO 2010/077758 A1 | 08 July 2010 | JP 2012-512249 A<br>examples 1-5 | |
| WO 2017/132928 A1 | 10 August 2017 | JP 2019-509990 A<br>examples 1-17 | |
| JP 2017-508787 A | 30 March 2017 | WO 2015/120390 A1<br>examples 1-163 | |
| WO 2022/114189 A1 | 02 June 2022 | US 2022/0273649 A1<br>claims, examples | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010077758 A
- WO 2012064548 A
- WO 2017132928 A
- JP 2022087166 A


### Non-patent literature cited in the description


- **DAI Y** ; **GRANT S**. *Clin Cancer Res*, 2010, vol. 16 (2), 376-383
- **BENADA J** ; **MACUREK L**. *Biomolecules*, 2015, vol. 5, 1912-1937
- **KING C**. *Mol Cancer Ther*, 2015, vol. 14 (9), 2004-2013
- **DENT P**. *Expert Opinion on Investigational drugs*, 2019, vol. 28 (12), 1095-1100
- **EVANGELISTI G et al.** *Expert Opinion on Investigational Drugs*, 2020, vol. 29 (8), 779-792
- **DAVID H et al.** *J Clin Oncol*, 2016, vol. 34, 1764-1771
- **RAFFAELE A**. *Expert Opinion on Biological Therapy*, 2019, vol. 19:3, 169-171
- **THEODORA W. GREENE** ; **PETER G. M. WUTS**. Protective Groups in Organic Synthesis. John Wiley & Sons, 1999
- **R. C. LAROCK**. Comprehensive Organic Transformations. John Wiley & Sons, 1999
- *Curr Opin Drug Discov Devel*, January 2010, vol. 13 (1), 66-77
- *Therapeutics and Clinical Risk Management*, 2005, vol. 1 (1), 3-13
- *Xenobiotica*, 2009, vol. 39 (2), 99-112
- *Drug Metabolism and Disposition*, 2011, vol. 39 (7), 1247-1254